# EUROPÄISCHE PATENTSCHRIFT

(11) **EP 1 858 890 B1**
(45) Veröffentlichungstag und Bekanntmachung des Hinweises auf die Patenterteilung: **14.04.2010**
(21) Anmeldenummer: 06724863.3
(22) Anmeldetag: 22.02.2006
(51) Int. Cl.: C07D 409/12, C07D 409/14, C07D 405/12, C07D 207/26, A61K 31/4015, A61K 31/4025, A61P 7/02

(54) **NEUE SUBSTITUIERTE PYRROLIDINONE MIT ANTITHROMBOTISCHER WIRKUNG, DEREN HERSTELLUNG UND DEREN VERWENDUNG ALS ARZNEIMITTEL**
NOVEL ANTITHROMBOTIC SUBSTITUTED PYRROLIDINONES, THE PRODUCTION AND USE THEREOF IN THE FORM OF MEDICAMENTS
NOUVELLES PYRROLIDINONES SUBSTITUEES A EFFET ANTITHROMBOTIQUE, LEUR PRODUCTION ET LEUR UTILISATION COMME MEDICAMENTS

(30) Priorität: 25.02.2005 DE 102005008649
(43) Veröffentlichungstag der Anmeldung: 28.11.2007
(73) Patentinhaber: Boehringer Ingelheim Pharma GmbH & Co. KG, 55216 Ingelheim am Rhein (DE); Boehringer Ingelheim International GmbH, 55216 Ingelheim am Rhein (DE)
(72) Erfinder: GERLACH, Kai, 88441 Mittelbiberach (DE); PRIEPKE, Henning, 88447 Warthausen (DE); PFAU, Roland, 88400 Biberach (DE); DAHMANN, Georg, 88448 Attenweiler (DE); WIENEN, Wolfgang, 88400 Biberach (DE); SCHULER-METZ, Annette, 89081 Ulm (DE); NAR, Herbert, 88416 Ochsenhausen (DE)
(74) Vertreter: Hammann, Heinz
(86) Internationale Anmeldenummer: PCT/EP2006/060182
(87) Internationale Veröffentlichungsnummer: WO 2006/089909

(56) Entgegenhaltungen:
- WO-A-03/053925
- WO-A-2004/110434
- MEDERSKI W W K R ET AL: "Chlorothiophenecarboxamides as P1 surrogates of inhibitors of blood coagulation factor Xa" BIOORGANIC & MEDICINAL CHEMISTRY LETTERS, OXFORD, GB, Bd. 14, Nr. 23, 6. Dezember 2004 (2004-12-06), Seiten 5817-5822, XP004611126 ISSN: 0960-894X
- EWING W R ET AL: "DESIGN AND STRUCTURE-ACTIVITY RELATIONSHIPS OF POTENT AND SELECTIVEINHIBITORS OF BLOOD COAGULATON FACTOR XA" JOURNAL OF MEDICINAL CHEMISTRY, AMERICAN CHEMICAL SOCIETY, US, Bd. 42, Nr. 18, 21. August 1999 (1999-08-21), Seiten 3557-3571, XP001025122 ISSN: 0022-2623

## Beschreibung

Gegenstand der vorliegenden Erfindung sind neue substituierte Pyrrolidinone der allgemeinen Formel 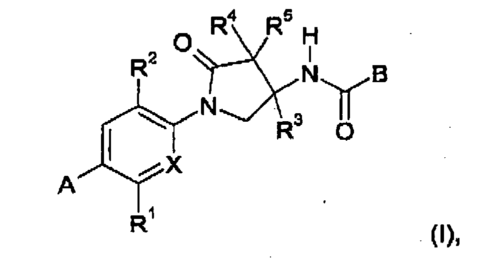 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel I sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

WO 03/053925 und WO 2004/110434 beschreiben 3-Sulfonylamino-2-pyrrolidinonederivete und deren Verwendung als antithrombotische Faktor Xa-Inhibitoren. Mederski W W K R et al., Bioorganic & Medicinal Chemistry Letters, Bd. 14, Nr. 23, Seiten 5817-5822, beschreibt offenkettige Chlorothiophenecarboxamide und deren Verwendung als antithrombotische Faktor Xa-Inhibitoren. Ewing W R et al., Journal of Medicinal Chemistry, Vol. 42, 18, Seiten 3557-3571 beschreibt 3-Sulfonylamino-2-pyrrolidinonederivate und deren Verwendung als antithrombotische Faktor Xa-Inhibitoren.

Gegenstand der vorliegenden Anmeldung sind neue Verbindungen der obigen allgemeinen Formel I, deren Herstellung, die die pharmakologisch wirksamen Verbindungen enthaltenden Arzneimittel, deren Herstellung und Verwendung.

Eine erste Ausführungsform der vorliegenden Erfindung umfaßt diejenigen Verbindungen der allgemeinen Formel I, in denen
- A: eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluoratome, eine oder zwei C₁₋₃-Alkyl-, C₂₋₃-Alkenyl-, C₂₋₃-Alkinyl-, Hydroxy-C₁₋₃-alkyl-, C₁₋₃-Alkyloxy-C₁₋₃-alkyl-, Phenyl-C₁₋₃-alkyl-, 1,1- Diphenyl-C₁₋₃-alkyl-, Heteroaryl-C₁₋₃-alkyl-, Amino-C₁₋₃-alkyl-, C₃₋₆-Cycloalkylamino-C₁₋₃-alkyl-, Phenylamino-C₁₋₃-alkyl-, C₁₋₅-Alkylamino-C₁₋₃-alkyl-, Di-(C₁₋₅-alkyl)-amino-C₁₋₅-alkyl-, N- (C₃₋₆-Cycloalkyl)-C₁₋₃-alkylamino-C₁₋₃-alkyl-, eine 4- bis 7-gliedrige Cycloalkylenimino-C₁₋₃-alkyl-, N-(C₁₋₃-Alkylcarbonyl)-C₁₋₃-alkylamino- C₁₋₃-alkyl-, Carboxy-C₁₋₃-alkyl-, C₁₋₃-Alkyloxycarbonyl-C₁₋₃-alkyl-, Aminocarbonyl-C₁₋₃-alkyl-, C₁₋₃-Alkylaminocarbonyl-C₁₋₃-alkyl-, Di- (C₁₋₃-alkyl)-aminocarbonyl-C₁₋₃-alkyl-, eine 4- bis 7-gliedrige Cycloalkyleniminocarlbonyl-C₁₋₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino-C₁₋₃-alkyl-, Aminocarbonylamino-C₁₋₃-alkyl-, C₁₋₅-Alkylaminocarbonylamino-C₁₋₃-alkyl-, Di-(C₁₋₃-alkyl)- aminocarbonylamino-C₁₋₃-alkyl-, C₁₋₅-Alkylsulfonylamino, C₁₋₅- alkylaminocarbonylamino, Carboxy-, C₁₋₃-Alkyloxycarbonyl-, Benzyloxycarbonyl-, C₁₋₃-Alkylcarbonyl-, Aminocarbonyl-, C₁₋₃-Alkylaminocarbonyl-, Di-(C₁₋₃-alkyl)-aminocarbonyl-, N-(C₃₋₇-Cycloalkyl)-C₁₋₅-alkylaminocarbonyl-, N-(Phenyl-C₁₋₃- alkyl)C₁₋₅-alkylaminocarbonyl-, eine 4- bis 7-gliedrige Cycloalkyleniminocarbonyl-, Aminocarbonyl-C₁₋₃-alkylaminocarbonyl- , Hydroxy-, C₁₋₃-Alkyloxy-, Allyloxy-, Propargyloxy-, Benzyloxy-, Amino-, C₁₋₃-Alkylamino-, Di-(C₁₋₃-alkyl)-amino-, eine 4- bis 7- gliedrige Cycloalkylenimino-, Trifluormethylcarbonylamino-, N-(C₁₋₃-Alkyl)-piperazin-4-yl-C₁₋₃-alkyl-, eine Phenyl- oder eine 5- bis
6-gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom, eine Sulfinyl- oder Sulfonylgruppe ersetzt sein kann oder
eine Methylengruppe in 4-Position einer 6- oder 7-gliedrigen Cycloalkyleniminogruppe durch ein Sauerstoff- oder Schwefelatom, eine Carbonyl-, Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7- gliedrigen Cycloalkyleniminoreste, bei denen eine Methylengruppe durch ein Sauerstoff oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Hydroxy-C₁₋₃-alkyl, C_{1- 5}-alkoxy-C₁₋₃-alkyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₃₋₆-Cycloalkylamino-C₁₋₃-alkyl-, Phenyl-, Phenyl-C₁₋₃-alkyl-, Heteroaryl-, Heteroaryl-C₁₋₃-alkyl-, Aminocarbonyl-, C₁₋₃-Alkylaminocarbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl-, C₂₋₃-Alkenyl-, C₂₋₃-Alkinyl- oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Benzyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₁₋₅-Alkyloxycarbonylamino-C₁₋₃-alkyl-, C₃₋₆-Cycloalkylamino-C₁₋₃-alkyl-, Aminocarbonyl-, C₁₋₃-Alkylamino-carbonyl-, N-(C₃₋₇-Cycloalkyl)-C₁₋₃-alkylaminocarbonyl-, N-(Phenyl-C₁₋₃-alkyl)-C₁₋₅-alkylaminocarbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 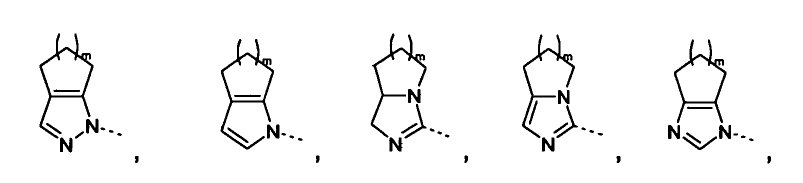 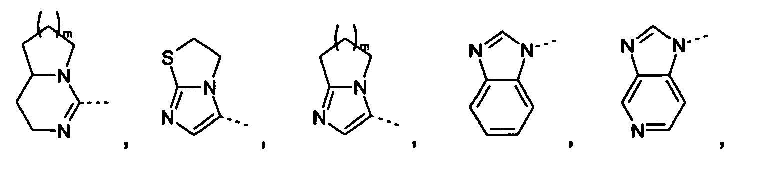 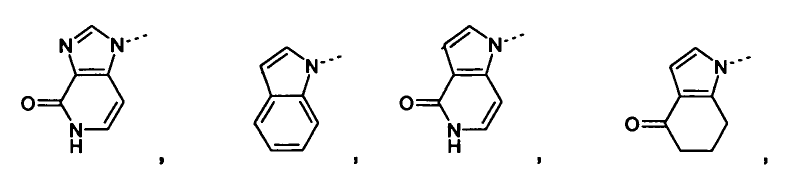 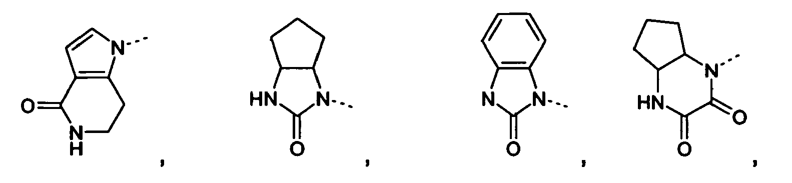 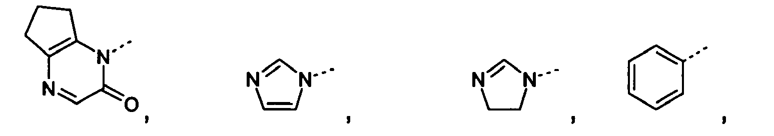 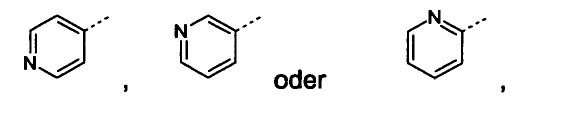 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, C₁₋₃-alkylsulfonyl-C₁₋₃-alkyl, C₁₋₃-alkylsulfonylamino-C₁₋₃-alkyl, C₁₋₃-alkylsulfonylamino, C₁₋₃-alkylcarbonylamino-C₁₋₃-alkyl-, C₁₋₃-alkylcarbonylamino-, Amino-C₁₋₃-alkyl-, C₁₋₃-Alkylamino-C₁₋₃-alkyl-, Di-(C₁₋₃-alkyl)-amino-C₁₋₃-alkyl-, Hydroxy-C₁₋₃-alkyl-, C₁₋₅-alkoxy-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, N-(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
- R¹: ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
- R²: ein Wasserstoff- oder Halogenatom oder eine C₁₋₃-Alkylgruppe bedeutet,
- X: ein Stickstoffatom oder eine CH-Gruppe bedeutet,
- R³: ein Wasserstoffatom oder eine C₁₋₃-Alkylgruppe bedeutet,
- R⁴ und R⁵: jeweils unabhängig voneinander
ein Wasserstoffatom, eine Hydroxygruppe, eine OR⁹-Gruppe, eine C₂₋₆-Alkenyl- oder C₂₋₆-Alkinylgruppe,
eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, Aminosulfonyl-, C₁₋₆-Al- kylaminosulfonyl-, Di-(C₁₋₅-alkyl)-aminosulfonyl-, C₄₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, N-(C₁₋₅-Alkylsulfonyl)-C₁₋₅-alkylamino- oder C₃₋₆-Cycloalkylcarbonylaminogruppe substituiert sein kann, wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
eine Phenyl-, oder Heteroarylgruppe die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, oder eine C₁₋₅-Alkyloxycarbonyl- C₁₋₅-alkyloxygruppe substituiert sein kann,
eine 3- bis 7-gliedrige Cycloalkyl-, Cycloalkylenimino-, Cycloalkyl- C₁₋₅-alkyl- oder Cycloalkylenimino-C₁₋₃-alkylgruppe, in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkylenimino-, Cycloalkyl-C₁₋₅-alkyl- oder Cycloalkylenimino-C₁₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkylenimino-, Cycloalkyl-C₁₋₅-alkyl- oder Cycloalkylenimino-C₁₋₃-alkylgruppe an einer oder zwei -CH₂- Gruppen durch jeweils eine oder zwei C₁₋₃-Alkylgruppen substituiert sein kann,
bedeutet,
mit der Maßgabe, dass R⁴ und R⁵ nicht gleichzeitig als Hydroxy- oder OR⁹-Gruppen definiert sein können, oder
- R⁴ und R⁵: zusammen mit dem Kohlenstoffatom, an dem sie gebunden sind, eine C₃₋₈-Cycloalkyl- oder C₃₋₈-Cycloalkenylgruppe bilden,
wobei eine der Methylengruppen einer C₄₋₈-Cycloalkylgruppe durch ein Sauerstoff- oder Schwefelatom oder eine -N(R⁷)-, oder eine Carbonyl-, Sulfinyl- oder Sulfonylgruppe ersetzt sein kann, und/oder
zwei direkt benachbarte Methylengruppen einer C₄₋₈-Cycloalkylgruppe zusammen durch eine -C(O)N(R⁸)- oder - S(O)₂N(R⁸)-Gruppe ersetzt sein können, und/oder
drei direkt benachbarte Methylengruppen einer C₆₋₈- Cycloalkylgruppe zusammen durch eine -OC(O)N(R⁸)-, - N(R⁸)C(O)N(R⁸)- oder -N(R⁸)S(O)₂N(R⁸)-Gruppe ersetzt sein können,
wobei 1 bis 3 Kohlenstoffatome einer C₃₋₈-Cycloalkylgruppe gegebenenfalls unabhängig voneinander durch jeweils ein oder zwei gleiche oder unterschiedliche Halogenatome oder C₁₋₅-Alkyl-, Nitril-, Hydroxy-, C₁₋₅-Alkyloxy-, C₁₋₅-Alkylcarbonyloxy-, Carboxy- C₁₋₅-alkyl, C₁₋₅-Alkyloxycarbonyl-C₁₋₅-alkyl, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, N-(C₁₋₅-Alkylsulfonyl)-C₁₋₅-alkylamino- oder C₃₋₆-Cycloalkylcarbonylaminogruppen substituiert sein können,
wobei 1 bis 2 Kohlenstoffatome einer C₃₋₈-Cycloalkenylgruppe gegebenenfalls unabhängig voneinander durch jeweils eine C₁₋₅- Alkyl-, Nitril-, Carboxy-C₁₋₅-alkyl, C₁₋₅-Alkyloxycarbonyl-C₁₋₅-alkyl, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonylgruppen substituiert sein können,
und 1 bis 2 Kohlenstoffatome einer C₄₋₈-Cycloalkenylgruppe, die nicht durch eine Doppelbindung an ein anderes Kohlenstoffatom gebunden sind, gegebenenfalls unabhängig voneinander durch ein Fluoratom oder eine Hydroxy-, C₁₋₅-Alkyloxy-, C₁₋₅-Alkylcarbonyloxy-, C₁₋₅-Alkylsulfanyl-, C₁₋₅-Alkylsulfonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, N-(C₁₋₅-Alkylsulfonyl)-C₁₋₅-alkylamino- oder C₃₋₆-Cycloalkylcarbonylaminogruppen substituiert sein können,
mit der Maßgabe, dass eine solche, zusammen aus R⁴ und R⁵ gebildete C₃₋₈-Cycloalkyl- oder C₃₋₈-Cycloalkenylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder
in der ein an die cyclische Gruppe gebundener Substituent, der sich dadurch auszeichnet, daß ein Heteroatom aus der Gruppe Sauerstoff, Stickstoff, Schwefel und Halogenatom direkt an die cyclische Gruppe gebunden ist, von einem anderen Heteroatom aus der Gruppe Sauerstoff, Stickstoff und Schwefel, mit Ausnahme der Sulfongruppe, durch genau eine, gegebenenfalls substituierte, Methylengruppe getrennt ist, und/oder
in der zwei Sauerstoffatome direkt miteinander verbunden sind,
ausgeschlossen ist,
- R⁷: jeweils unabhängig voneinander ein Wasserstoffatom, eine Hydroxy-, eine Formyl-, eine C₁₋₅-Alkyl-, C₁₋₅-Alkylcarlbonyl-, C₁₋₅-Alkyloxycarbonyl- oder C₁₋₅-Alkylsulfonylgruppe bedeutet,
- R⁸: jeweils unabhängig voneinander ein Wasserstoffatom oder eine C₁₋₅-Alkylgruppe bedeutet,
- R⁹: eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarlbonyl-C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, C₁₋₅-alkyloxycarbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, N-(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
mit der Maßgabe, dass der Ersatz von Wasserstoffatomen des ersten Kohlenstoffatoms der geradkettigen oder verzweigten C₁₋₆-Alkylgruppe durch Substituenten aus der Gruppe Sauerstoff, Schwefel oder Stickstoff ausgeschlossen ist,
eine Phenyl-, Heteroaryl-, Phenyl-C₁₋₅-alkyl- oder Heteroaryl- C₁₋₆-alkylgruppe,
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können,
eine 3- bis 7-gliedrige Cycloalkyl-, Cycloalkyl-C₁₋₅-alkyl- oder Cycloalkylenimino-C₂₋₃-alkylgruppe,
in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkyl-C₁₋₅-alkyl- oder Cycloalkylenimino-C₂₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkyl-C₁₋₅-alkyl- oder Cycloalkylenimino-C₂₋₃-alkylgruppe an einer oder zwei -CH₂-Gruppen durch jeweils eine oder zwei C₁₋₃- Alkylgruppen substituiert sein kann,
bedeutet,
- B: einen Rest der allgemeinen Formel 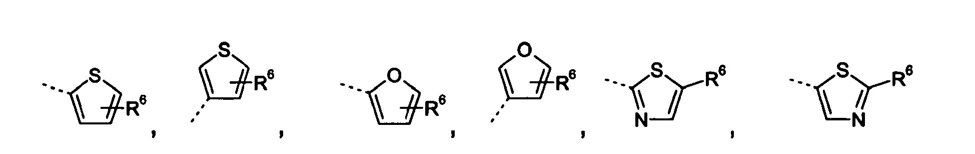 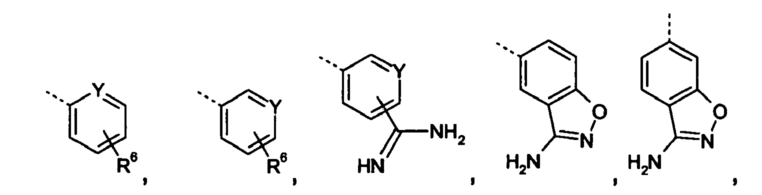 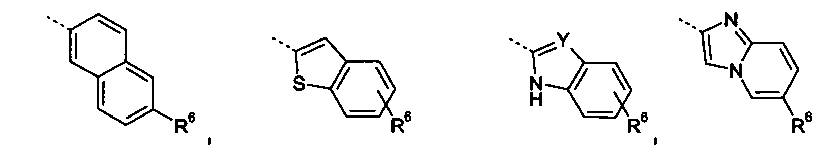 oder 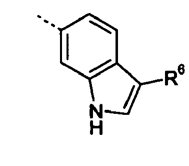 bedeutet,
- Y: ein Stickstoffatom oder eine CH-Gruppe bedeutet,
- R⁶: ein Wasserstoff- , ein Halogenatom, eine Nitrilgruppe, eine C₂₋₃-Alkenyl-, C₂₋₃-Alkinyl-, eine C₁₋₃-Alkylgruppe, oder eine C₁₋₃-Alkoxygruppe bedeutet, wobei die Wasserstoffatome der C₁₋₃-Alkyl- oder C₁₋₃-Alkoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine C₃₋₆-Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Eine zweite Ausführungsform der vorliegenden Erfindung umfasst diejenigen Verbindungen der allgemeinen Formel I, in denen
- A: eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, C₂₋₃-Alkenyl-, C₂₋₃-Alkinyl-, Hydroxy- C₁₋₃-alkyl-, C₁₋₃-Alkyloxy-C₁₋₃-alkyl-, Phenyl-C₁₋₃-alkyl-, 1,1-Diphenyl- C₁₋₃-alkyl-, Heteroaryl-C₁₋₃-alkyl-, Amino-C₁₋₃-alkyl-, C₃₋₆-Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, N-(C₃₋₆-Cycloalkyl)-C₁₋₃-alkylamino-C₁₋₃-alkyl-, eine 4- bis 7-gliedrige Cycloalkylenimino-C₁₋₃-alkyl-, N-(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine 4- bis 7-gliedrige Cycloalkyleniminocarbonyl-C₁₋₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino- C₁₋₃-alkyl-, Aminocarbonylamino-C₁₋₃-alkyl-, C₁₋₅-Alkylaminocarbonylamino- C₁₋₃-alkyl-, Di-(C₁₋₃-alkyl)-aminocarbonylamino-C₁₋₃-alkyl-, C₁₋₅- Alkylsulfonylamino, C₁₋₅-alkylaminocarbonylamino, Carboxy-, C₁₋₃-Alkyloxycarbonyl-, Benzyloxycarbonyl-, C₁₋₃-Alkylcarbonyl-, Aminocarbonyl-, C₁₋₃-Alkylaminocarbonyl-, Di-(C₁₋₃-alkyl)-aminocarbonyl-, N-(C₃₋₇-Cycloalkyl)-C₁₋₅-alkylaminocarbonyl-, N-(Phenyl-C₁₋₃-alkyl)-C₁₋₅- alkylaminocarbonyl-, eine 4- bis 7-gliedrige Cycloalkyleniminocarbonyl-, Aminocarbonyl-C₁₋₃-alkylaminocarbonyl-, Hydroxy-, C₁₋₃-Alkyloxy-, Allyloxy-, Propargyloxy-, Benzyloxy-, Amino-, C₁₋₃-Alkylamino-, Di-(C₁₋₃-alkyl)-amino-, eine 4- bis 7-gliedrige Cycloalkylenimino-, Trifluormethylcarbonylamino-, N-(C₁₋₃-Alkyl)-piperazin-4-yl-C₁₋₃-alkyl-, eine Phenyl- oder eine 5- bis 6- gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom, eine Sulfinyl- oder Sulfonylgruppe ersetzt sein kann oder
eine Methylengruppe in 4-Position einer 6- oder 7-gliedrigen Cycloalkyleniminogruppe durch ein Sauerstoff- oder Schwefelatom, eine Carbonyl-, Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Hydroxy-C₁₋₃-alkyl, C₁₋₅-alkoxy-C₁₋₃-alkyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₃₋₆-Cycloalkylamino-C₁₋₃-alkyl-, Phenyl-, Phenyl-C₁₋₃-alkyl-, Heteroaryl-, Heteroaryl-C₁₋₃-alkyl-, Aminocarbonyl-, C₁₋₃-Alkylaminocarbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl-, C₂₋₃-Alkenyl-, C₂₋₃-Alkinyl-, oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Benzyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₁₋₅-Alkyloxycarbonylamino-C₁₋₃-alkyl-, C₃₋₆-Cycloalkylamino-C₁₋₃-alkyl-, Aminocarbonyl-, C₁₋₃-Alkylamino-carlbonyl-, N-(C₃₋₇-Cycloalkyl)-C₁₋₃-alkylaminocarbonyl-, N-(Phenyl-C₁₋₃-alkyl)-C₁₋₅-alkylaminocarbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 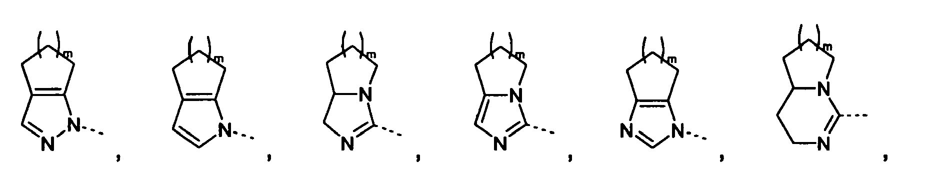 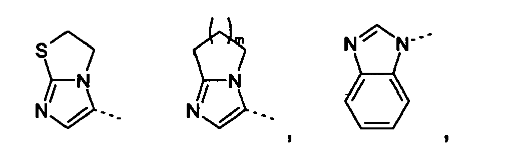 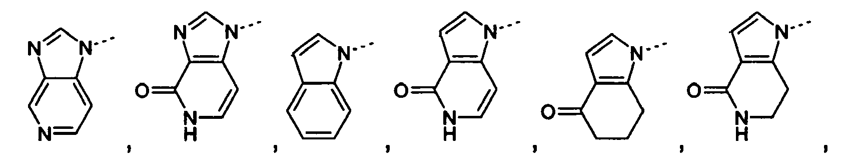 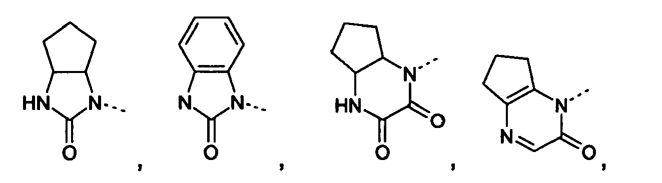 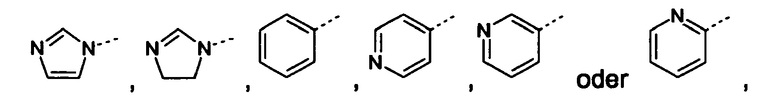 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, C₁₋₃-alkylsulfonyl-C₁₋₃-alkyl, C₁₋₃-alkylsulfonylamino-C₁₋₃-alkyl, C₁₋₃-alkylsulfonylamino, C₁₋₃-alkylcarbonylamino-C₁₋₃-alkyl-, C₁₋₃-alkylcarbonylamino-, Amino-C₁₋₃-alkyl-, C₁₋₃-Alkylamino-C₁₋₃-alkyl-, Di-(C₁₋₃-alkyl)-amino-C₁₋₃-alkyl-, Hydroxy-C₁₋₃-alkyl-, C₁₋₅-alkoxy-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, N-(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
- R¹: ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
- R²: ein Wasserstoff- oder Halogenatom oder eine C₁₋₃-Alkylgruppe bedeutet,
- X: ein Stickstoffatom oder eine CH-Gruppe bedeutet,
- R³: ein Wasserstoffatom oder eine C₁₋₃-Alkylgruppe bedeutet,
- R⁴ und R⁵: jeweils unabhängig voneinander
ein Wasserstoffatom, eine Hydroxygruppe, eine OR⁹-Gruppe, eine C₂₋₆-Alkenyl- oder C₂₋₆-Alkinylgruppe,
eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, Aminosulfonyl-, C₁₋₅-Al- kylaminosulfonyl-, Di-(C₁₋₅-alkyl)-aminosulfonyl-, C₄₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, N-(C₁₋₅-Alkylsulfonyl)-C₁₋₅-alkylamino- oder C₃₋₆-Cycloalkylcarbonylaminogruppe substituiert sein kann, wobei die 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
eine Phenyl-, oder Heteroarylgruppe die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, oder eine C₁₋₅-Alkyloxycarbonyl- C₁₋₅-alkyloxygruppe substituiert sein kann,
eine 3- bis 7-gliedrige Cycloalkyl-, Cycloalkylenimino-, Cycloalkyl- C₁₋₅-alkyl- oder Cycloalkylenimino-C₁₋₃-alkylgruppe, in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkylenimino-, Cycloalkyl-C₁₋₅-alkyl- oder Cycloalkylenimino-C₁₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkylenimino-, Cycloalkyl-C₁₋₅-alkyl- oder Cycloalkylenimino-C₁₋₃-alkylgruppe an einer oder zwei -CH₂- Gruppen durch jeweils eine oder zwei C₁₋₃-Alkylgruppen substituiert sein kann,
bedeutet,
mit der Maßgabe, dass R⁴ und R⁵ nicht gleichzeitig als Hydroxy- oder OR⁹-Gruppen definiert sein können, oder
- R⁴ und R⁵: zusammen mit dem Kohlenstoffatom, an dem sie gebunden sind, eine C₃₋₈-Cycloalkyl- oder C₃₋₈-Cycloalkenylgruppe bilden,
wobei eine der Methylengruppen einer C₄₋₈-Cycloalkylgruppe durch ein Sauerstoff- oder Schwefelatom oder eine -N(R⁷)-, oder eine Carbonyl-, Sulfinyl- oder Sulfonylgruppe ersetzt sein kann, und/oder
zwei direkt benachbarte Methylengruppen einer C₄₋₈-Cycloalkylgruppe zusammen durch eine -C(O)N(R⁸)- oder - S(O)₂N(R⁸)-Gruppe ersetzt sein können, und/oder
drei direkt benachbarte Methylengruppen einer C₆₋₈- Cycloalkylgruppe zusammen durch eine -OC(O)N(R⁸)-, - N(R⁸)C(O)N(R⁸)- oder -N(R⁸)S(O)₂N(R⁸)-Gruppe ersetzt sein können,
wobei 1 bis 3 Kohlenstoffatome einer C₃₋₈-Cycloalkylgruppe gegebenenfalls unabhängig voneinander durch jeweils ein oder zwei gleiche oder unterschiedliche Halogenatome oder C₁₋₅-Alkyl-, Nitril-, Hydroxy-, C₁₋₅-Alkyloxy-, C₁₋₆-Alkylcarbonyloxy-, Carboxy- C₁₋₅-alkyl, C₁₋₅-Alkyloxycarbonyl-C₁₋₅-alkyl, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, N-(C₁₋₅-Alkylsulfonyl)-C₁₋₅-alkylamino- oder C₃₋₆-Cycloalkylcarbonylaminogruppen substituiert sein können,
wobei 1 bis 2 Kohlenstoffatome einer C₃₋₈-Cycloalkenylgruppe gegebenenfalls unabhängig voneinander durch jeweils eine C₁₋₅- Alkyl-, Nitril-, Carboxy-C₁₋₅-alkyl, C₁₋₅-Alkyloxycarbonyl-C₁₋₅-alkyl, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonylgruppen substituiert sein können,
und 1 bis 2 Kohlenstoffatome einer C₄₋₈-Cycloalkenylgruppe, die nicht durch eine Doppelbindung an ein anderes Kohlenstoffatom gebunden sind, gegebenenfalls unabhängig voneinander durch ein Fluoratom oder eine Hydroxy-, C₁₋₅-Alkyloxy-, C₁₋₅-Alkylcarbonyloxy-, C₁₋₅-Alkylsulfanyl-, C₁₋₅-Alkylsulfonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, N-(C₁₋₅-Alkylsulfonyl)-C₁₋₅-alkylamino- oder C₃₋₆-Cycloalkylcarbonylaminogruppen substituiert sein können,
mit der Maßgabe, dass eine solche, zusammen aus R⁴ und R⁵ gebildete C₃₋₈-Cycloalkyl- oder C₃₋₈-Cycloalkenylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder
in der ein an die cyclische Gruppe gebundener Substituent, der sich dadurch auszeichnet, daß ein Heteroatom aus der Gruppe Sauerstoff, Stickstoff, Schwefel und Halogenatom direkt an die cyclische Gruppe gebunden ist, von einem anderen Heteroatom aus der Gruppe Sauerstoff, Stickstoff und Schwefel, mit Ausnahme der Sulfongruppe, durch genau eine, gegebenenfalls substituierte, Methylengruppe getrennt ist, und/oder
in der zwei Sauerstoffatome direkt miteinander verbunden sind,
ausgeschlossen ist,
- R⁷: jeweils unabhängig voneinander ein Wasserstoffatom, eine Hydroxy-, eine Formyl-, eine C₁₋₅-Alkyl-, C₁₋₅-Alkylcarbonyl-, C₁₋₅-Alkyloxycarbonyl- oder C₁₋₅-Alkylsulfonylgruppe bedeutet,
- R⁸: jeweils unabhängig voneinander ein Wasserstoffatom oder eine C₁₋₅-Alkylgruppe bedeutet,
- R⁹: eine geradkettige oder verzweigte C₁₋₆-Alkylgruppe,
wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆ Cycloalkyleniminocarbonyl-, C₁₋₅-alkyloxycarbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, N-(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷ -Gruppe durch eine Carbonylgruppe ersetzt sein kann,
mit der Maßgabe, dass der Ersatz von Wasserstoffatomen des ersten Kohlenstoffatoms der geradkettigen oder verzweigten C₁₋₆-Alkylgruppe durch Substituenten aus der Gruppe Sauerstoff, Schwefel oder Stickstoff ausgeschlossen ist,
eine Phenyl-, Heteroaryl-, Phenyl-C₁₋₅-alkyl- oder Heteroaryl- C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können,
eine 3- bis 7-gliedrige Cycloalkyl-, Cycloalkyl-C₁₋₅-alkyl- oder Cycloalkylenimino-C₂₋₃-alkylgruppe,
in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkyl-C₁₋₅-alkyl- oder Cycloalkylenimino-C₂₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkyl-C₁₋₅-alkyl- oder Cycloalkylenimino-C₂₋₃-alkylgruppe an einer oder zwei -CH₂-Gruppen durch jeweils eine oder zwei C₁₋₃- Alkylgruppen substituiert sein kann,
bedeutet,
- B: einen Rest der allgemeinen Formel 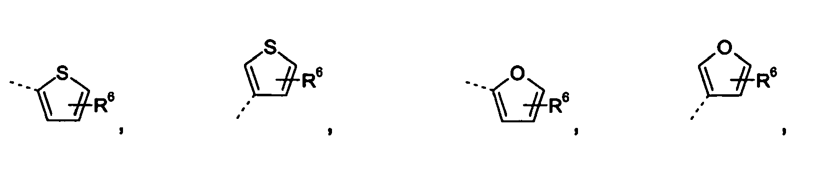 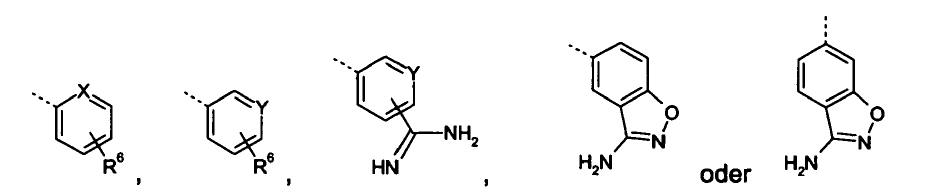
bedeutet,
- Y: ein Stickstoffatom oder eine CH-Gruppe bedeutet,
- R⁶: ein Wasserstoff- , ein Halogenatom, eine Nitrilgruppe, eine C₂₋₃-Alkenyl-, C₂₋₃-Alkinyl-, eine C₁₋₃-Alkylgruppe, oder eine C₁₋₃-Alkoxygruppe bedeutet, wobei die Wasserstoffatome der C₁₋₃-Alkyl- oder C₁₋₃-Alkoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarytgruppe" eine monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine C₃₋₆-Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält, und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Eine dritte Ausführungsform der vorliegenden Erfindung umfasst diejenigen Verbindungen der allgemeinen Formel I, in denen
- A: eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, C₂₋₃-Alkenyl-, C₂₋₃-Alkinyl-, Hydroxy- C₁₋₃-alkyl-, C₁₋₃-Alkyloxy-C₁₋₃-alkyl-, Phenyl-C₁₋₃-alkyl-, 1,1-Diphenyl- C₁₋₃-alkyl-, Heteroaryl-C₁₋₃-alkyl-, Amino-C₁₋₃-alkyl-, C₃₋₆-Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, N-(C₃₋₆-Cycloalkyl)-C₁₋₃-alkylamino-C₁₋₃-alkyl-, eine 4- bis 7-gliedrige Cycloalkylenimino-C₁₋₃-alkyl-, N-(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine 4- bis 7-gliedrige Cycloalkyleniminocarbonyl-C₁₋₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino- C₁₋₃-alkyl-, Aminocarbonylamino-C₁₋₃-alkyl-, C₁₋₅-Alkylaminocarbonylamino- C₁₋₃-alkyl-, Di-(C₁₋₃-alkyl)-aminocarbonylamino-C₁₋₃-alkyl-, C₁₋₅- Alkylsulfonylamino, C₁₋₅-alkylaminocarbonylamino, Carboxy-, C₁₋₃-Alkyloxycarbonyl-, Benzyloxycarbonyl-, C₁₋₃-Alkylcarlbonyl-, Aminocarbonyl-, C₁₋₃-Alkylaminocarbonyl-, Di-(C₁₋₃-alkyl)-aminocarbonyl-, N-(C₃₋₇-Cycloalkyl)-C₁₋₅-alkylaminocarbonyl-, N-(Phenyl-C₁₋₃-alkyl)-C₁₋₅- alkylaminocarbonyl-, eine 4- bis 7-gliedrige Cycloalkyleniminocarbonyl-, Aminocarbonyl-C₁₋₃-alkylaminocarbonyl-, Hydroxy-, C₁₋₃-Alkyloxy-, Allyloxy-, Propargyloxy-, Benzyloxy-, Amino-, C₁₋₃-Alkylamino-, Di-(C₁₋₃-alkyl)-amino-, eine 4- bis 7-gliedrige Cycloalkylenimino-, Trifluormethylcarbonylamino-, N-(C₁₋₃-Alkyl)-piperazin-4-yl-C₁₋₃-alkyl-, eine Phenyl- oder eine 5- bis 6- gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder eine Methylengruppe in 4-Position einer 6- oder 7-gliedrigen Cycloalkyleniminogruppe durch ein Sauerstoff- oder Schwefelatom, eine Carbonyl-, Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Hydroxy-C₁₋₃-alkyl, C₁₋₅-alkoxy-C₁₋₃-alkyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₃₋₆-Cycloalkylamino-C₁₋₃-alkyl-, Phenyl-, Phenyl-C₁₋₃-alkyl-, Heteroaryl-, Heteroaryl-C₁₋₃-alkyl-, Aminocarbonyl-, C₁₋₃-Alkylaminocarbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl-, C₂₋₃-Alkenyl-, C₂₋₃-Alkinyl-, oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Benzyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₁₋₅-Alkyloxycarbonylamino-C₁₋₃-alkyl-, C₃₋₆-Cycloalkylamino-C₁₋₃-alkyl-, Aminocarbonyl-, C₁₋₃-Alkylamino-carbonyl-, *N-*(C₃₋₇-Cycloalkyl)-C₁₋₃-alkylaminocarbonyl-, *N*-(Phenyl-C₁₋₃-alkyl)-C₁₋₅-alkylaminocarbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 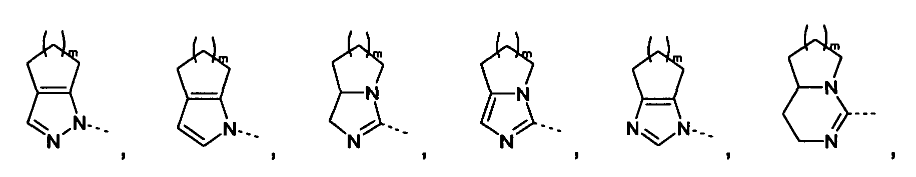 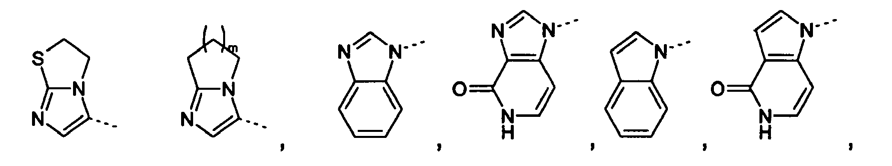 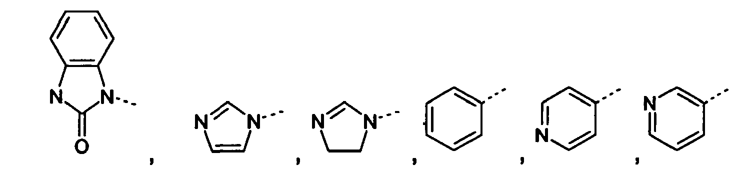 oder 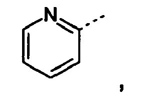 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, C₁₋₃-alkylsulfonyl-C₁₋₃-alkyl, C₁₋₃-alkylsulfonylamino-C₁₋₃-alkyl, C₁₋₃-alkylsulfonylamino, C₁₋₃-alkylcarbonylamino-C₁₋₃-alkyl-, C₁₋₃-alkylcarbonylamino-, Amino-C₁₋₃-alkyl-, C₁₋₃-AlkylaminO-C₁₋₃-alkyl-, Di-(C₁₋₃-alkyl)-amino-C₁₋₃-alkyl-, Hydroxy-C₁₋₃-alkyl-, C₁₋₅-alkoxy-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
- R¹: ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
- R²: ein Wasserstoff- oder Halogenatom oder eine Methylgruppe bedeutet,
- X: ein Stickstoffatom oder eine CH-Gruppe bedeutet,
- R³: ein Wasserstoffatom oder eine C₁₋₃-Alkylgruppe bedeutet,
- R⁴ und R⁵: jeweils unabhängig voneinander
ein Wasserstoffatom, eine Hydroxygruppe, eine OR⁹-Gruppe, eine C₂₋₆-Alkenyl- oder C₂₋₆-Alkinylgruppe,
eine geradkettige oder verzweigte C₁₋₆-Alkylgruppe, wobei die Wasserstoffatome der geradkettigen oder verzweigten C₁₋₆Alkylgruppe gegebenenfalls ganz oder teilweise durch Fluoratome ersetzt sein können, und die gegebenenfalls durch eine C₃₋₅-Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarlbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, Aminosulfonyl-, C₁₋₅-Al- kylaminosulfonyl-, Di-(C₁₋₅-alkyl)-aminosulfonyl-, C₄₋₆-Cycloalkyleniminosulfonyl-, Amino-, C₁₋₅-Alkylamino-, Di- (C₁₋₅-alkyl)-amino-, C₁₋₅-Alkylcarlbonylamino-, C₁₋₅-Alkyl- sulfonylamino-, *N-*(C₁₋₅-Alkylsulfonyl)-C₁₋₅-alkylamino- oder C₃₋₆-Cycloalkycarbonylaminogruppe substituiert sein kann, wobei in den 6- bis 7-gliedrigen Cyclen der C₄₋₆ Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR**⁷**-Gruppe ersetzt sein kann und zusätzlich eine Methylengruppe benachbart zu einer vorstehend erwähnten - NR⁷-Gruppe durch eine Carbonylgruppe ersetzt sein kann,
eine Phenyl-, Heteroaryl-, Phenyl-C₁₋₅-alkyl- oder Heteroaryl- C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können,
eine 3- bis 7-gliedrige Cycloalkyl-, Cycloalkylenimino-, Cycloalkyl- C₁₋₅-alkyl- oder Cycloalkylenimino-C₁₋₃-alkylgruppe, in der bei 4- bis 7-gliedrigen Cyclen im cyclischen Teil eine Methylengruppe gegebenenfalls durch eine -N(R⁷)-Gruppe, ein Sauerstoff oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder-N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkylenimino-, Cycloalkyl-C₁₋₅-alkyl- oder Cycloalkylenimino-C₁₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkylenimino-, Cycloalkyl-C₁₋₅-alkyl- oder Cycloalkylenimino-C₁₋₃-alkylgruppe an einer oder zwei -CH₂- Gruppen durch jeweils eine oder zwei C₁₋₃-Alkylgruppen substituiert sein kann,
bedeutet,
mit der Maßgabe, dass R⁴ und R⁵ nicht gleichzeitig als Hydroxy- oder OR⁹-Gruppen definiert sein können,
- R⁷: jeweils unabhängig voneinander ein Wasserstoffatom, eine Hydroxy-, eine Formyl-, eine C₁₋₃-Alkyl-, C₁₋₃-Alkylcarbonyl-, C₁₋₃-Alkyloxycarbonyl- oder C₁₋₃-Alkylsulfonylgruppe bedeutet,
- R⁸: jeweils unabhängig voneinander ein Wasserstoffatom oder eine C₁₋₃-Alkylgruppe bedeutet,
- R⁹: eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅- Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl- C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarlbonyl-, C₄₋₆- Cycloalkyleniminocarbonyl-, C₁₋₅-alkyloxycarbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, *N-*(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann, mit der Maßgabe, dass der Ersatz von Wasserstoffatomen des ersten Kohlenstoffatoms der geradkettigen oder verzweigten C₁₋₆-Alkylgruppe durch Substituenten aus der Gruppe Sauerstoff, Schwefel oder Stickstoff ausgeschlossen ist,
eine Phenyl-, oder Heteroarylgruppe
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, d₁₋₅-Alkyloxycarbonyloxy-, Carboxy-C₁₋₅-alkyloxy-, oder eine C₁₋₅-Alkyloxycarlbonyl- C₁₋₅-alkyloxygruppe substituiert sein kann;
eine 3- bis 7-gliedrige Cycloalkyl-, Cycloalkyl-C₁₋₅-alkyl- oder Cycloalkylenimino-C₂₋₃-alkylgruppe,
in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkyl-C₁₋₅-alkyl- oder Cycloalkylenimino-C₂₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkyl-C₁₋₅-alkyl- oder Cycloalkylenimino-C₂₋₃-alkylgruppe an einer oder zwei -CH₂-Gruppen durch jeweils eine oder zwei C₁₋₃- Alkylgruppen substituiert sein kann,
bedeutet,
- B: einen Rest der allgemeinen Formel 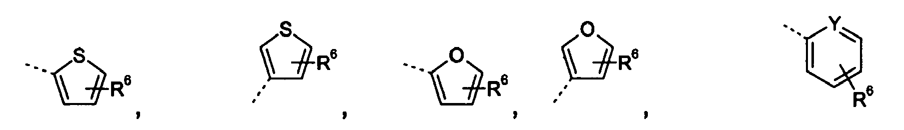 oder 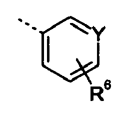 bedeutet,
- Y: ein Stickstoffatom oder eine CH-Gruppe bedeutet,
- R⁶: ein Wasserstoff- , ein Halogenatom, eine Ethinyl-, eine Methylgruppe, eine Methoxygruppe bedeutet, wobei die Wasserstoffatome der Methoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-AlkylaminO-C₂₋₃-alkyl-, Di-(C₁₋₃-alkyl)amino-C₂₋₃-alkyl-, eine C₃₋₆-Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Eine vierte Ausführungsform der vorliegenden Erfindung umfasst diejenigen Verbindungen der allgemeinen Formel I, in denen
- A: eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, Hydroxy-C₁₋₃-alkyl-, C₁₋₃-Alkyloxy- C₁₋₃-alkyl-, Heteroaryl-C₁₋₃-alkyl-, Amino-C₁₋₃-alkyl-, C₃₋₆-Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, *N-*(C₃₋₆-Cycloalkyl)-C₁₋₃-alkylamino-C₁₋₃-alkyl-, eine 4- bis 7-gliedrige Cycloalkylenimino-C₁₋₃-alkyl-, *N-*(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine 4- bis 7-gliedrige Cycloalkyleniminocarbonyl-C₁₋₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino- C₁₋₃-alkyl-, Aminocarbonylamino-C₁₋₃-alkyl-, C₁₋₅-Alkylaminocarbonylamino- C₁₋₃-alkyl-, Di-(C₁₋₃-alkyl)-aminocarbonylamino-C₁₋₃-alkyl-, C₁₋₅- Alkylsulfonylamino, C₁₋₅-alkylaminocarbonylamino, Carboxy-, C₁₋₃-Alkyloxycarbonyl-, Benzyloxycarbonyl-, C₁₋₃-Alkylcarbonyl-, Aminocarbonyl-, C₁₋₃-Alkylaminocarbonyl-, Di-(C₁₋₃-alkyl)-aminocarbonyl-, *N-*(C₃₋₇-Cycloalkyl)-C₁₋₅-alkylaminocarbonyl-, *N-*(Phenyl-C₁₋₃-alkyl)-C₁₋₅- alkylaminocarbonyl-, eine 4- bis 7-gliedrige Cycloalkyleniminocarbonyl-, Aminocarbonyl-C₁-₃-alkylaminocarbonyl-, Hydroxy-, C₁₋₃-Alkyloxy-, Allyloxy-, Propargyloxy-, Benzyloxy-, Amino-, C₁₋₃-Alkylamino-, Di-(C₁₋₃-alkyl)-amino-, eine 4- bis 7-gliedrige Cycloalkylenimino-, *N-*(C₁₋₃-Alkyl)-piperazin-4-yl- C₁₋₃-alkyl-, eine Phenyl- oder eine 5- bis 6-gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder
eine Methylengruppe in 4-Position einer 6- oder 7-gliedrigen Cycloalkyleniminogruppe durch ein Sauerstoff- oder Schwefelatom, eine Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte - NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₃₋₆-Cycloalkylamino-C₁₋₃-alkyl-, Heteroaryl-, Heteroaryl-C₁₋₃-alkyl-, Aminocarbonyl-, C₁₋₃-Alkylaminocarbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl-, oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Amino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, Aminocarbonyl-, C₁₋₃-Alkylamino-carbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 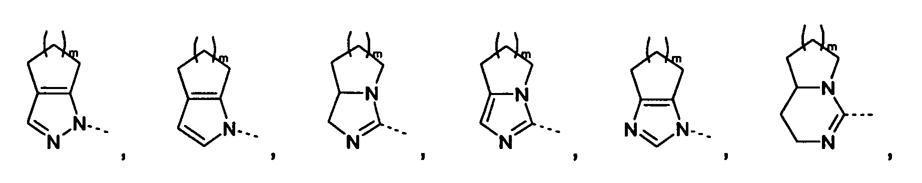 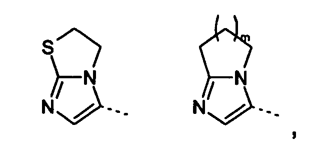 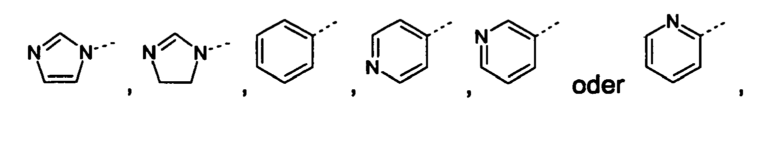 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁-₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
- R¹: ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
- R²: ein Wasserstoff- oder Fluoratom oder eine Methylgruppe bedeutet,
- X: ein Stickstoffatom oder eine CH-Gruppe bedeutet,
- R³: ein Wasserstoffatom oder eine C₁₋₃-Alkylgruppe bedeutet,
- R⁴: ein Wasserstoffatom,
eine geradkettige oder verzweigte C₁₋₄-Alkylgruppe, wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine C₁₋₃-Alkyloxygruppe, wobei die Wasserstoffatome der C₁₋₃-Alkyloxygruppe gegebenenfalls ganz oder teilweise durch Fluoratome ersetzt sein können, substituiert sein kann, bedeutet
- R⁵: ein Wasserstoffatom, eine Hydroxygruppe, eine OR⁹-Gruppe, eine C₂₋₄-Alkenyl- oder C₂₋₄-Alkinylgruppe,
eine geradkettige oder verzweigte C₁₋₄-Alkylgruppe, wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine C₃₋₅- Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarlbonyloxy-, Carboxy-C₁₋₅-alkyloxy-, C₁₋₅-Alkyloxycarbonyl- C₁₋₅-alkyloxy-, Mercapto-, C₁₋₅-Alkylsulfanyl-, C₁₋₅-Alkylsulfonyl-, Carboxy- , C₁₋₅-Alkyloxycarbonyl-, Aminocarbonyl-, C₁₋₅-Alkylaminocarbonyl-, Di- (C₁₋₅-alkyl)-aminocarbonyl-, C₄₋₆-Cycloalkyleniminocarbonyl-, Aminosulfonyl-, C₁₋₅-Alkylaminosulfonyl-, Di-(C₁₋₅-alkyl)-aminosulfonyl-, C₄₋₆-Cycloalkyleniminosulfonyl-, Amino-, C₁₋₅-Alkylamino-, Di-(C₁₋₅-alkyl)- amino-, C₁₋₅-Alkylcarlbonylamino-, C₁₋₅-Alkylsulfonylamino-, *N-*(C₁₋₅-Alkylsulfonyl)-C₁₋₅-alkylamino- oder C₃₋₆-Cycloalkylcarbonyl- aminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten -NR⁷-Gruppe durch eine Carbonylgruppe ersetzt sein kann,
eine Phenyl-, Heteroaryl-, Phenyl-C₁₋₅-alkyl- oder Heteroaryl- C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können,
bedeutet,
- R⁷: jeweils unabhängig voneinander ein Wasserstoffatom, eine C₁₋₃-Alkyl-, C₁₋₃-Alkylcarbonyl-, C₁₋₃-Alkyloxycarbonyl- oder C₁₋₃-Alkylsulfonylgruppe bedeutet,
- R⁹: eine geradkettige oder verzweigte C₁₋₄-Alkylgruppe,
wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine C₃₋₅-Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, C₁₋₅-alkyloxycarbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, *N-*(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann, mit der Maßgabe, dass der Ersatz von Wasserstoffatomen des ersten Kohlenstoffatoms der geradkettigen oder verzweigten C₁₋₄-Alkylgruppe durch Substituenten aus der Gruppe Sauerstoff, Schwefel oder Stickstoff ausgeschlossen ist,
eine Phenyl-, oder Heteroarylgruppe die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, oder eine C₁₋₅-Alkyloxycarbonyl- C₁₋₅-alkyloxygruppe substituiert sein kann;
bedeutet,
- B: einen Rest der allgemeinen Formel 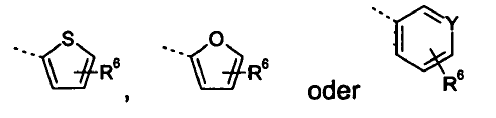 bedeutet,
Y ein Stickstoffatom oder eine CH-Gruppe bedeutet,
R⁶ ein Wasserstoff- , ein Halogenatom, eine Ethinyl-, eine Methylgruppe, eine Methoxygruppe bedeutet, wobei die Wasserstoffatome der Methoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-AlkylaminO-C₂₋₃-alkyl-, Di-(C₁₋₃-alkyl)amino-C₂₋₃-alkyl-, eine C₃₋₆-Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Eine fünfte Ausführungsform der vorliegenden Erfindung umfasst diejenigen Verbindungen der allgemeinen Formel I, in denen
- A: eine 5- bis 6-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, Hydroxy-C₁₋₃-alkyl-, C₁₋₃-Alkyloxy- C₁₋₃-alkyl-, Heteroaryl-C₁₋₃-alkyl-, Amino-C₁₋₃-alkyl-, C₃₋₆₋Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, *N-*(C₃₋₆-Cycloalkylyl)-C₁₋₃-alkylamino-C₁₋₃-alkyl-, eine 4- bis 7-gliedrige Cycloalkylenimino-C₁₋₃-alkyl-, *N-*(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine C₁₋₃-Alkylcarbonylamino- C₁₋₃-alkyl-, C₁₋₅-Alkylaminocarbonylamino-C₁₋₃-alkyl-, Di-(C₁₋₃-alkyl)- aminocarbonylamino-C₁₋₃-alkyl-, C₁₋₅-Alkylsulfonylamino, C₁₋₅- alkylaminocarbonylamino, Carboxy-, C₁₋₃-Alkyloxycarbonyl-, Aminocarbonyl-, C₁₋₃-Alkylaminocarbonyl-, Di-(C₁₋₃-alkyl)-aminocarbonyl-, *N-*(C₃₋₇-Cycloalkyl)-C₁₋₅-alkylaminocarbonyl-, *N-*(Phenyl-C₁₋₃-alkyl)-C₁₋₅- alkylaminocarbonyl-, Hydroxy-, C₁₋₃-Alkyloxy, Amino-, C₁₋₃-Alkylamino-, Di- (C₁₋₃-alkyl)-amino-, *N-*(C₁₋₃-Alkyl)-piperazin-4-yl-C₁₋₃-alkyl-, eine Phenyl- oder eine 6-gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder
eine Methylengruppe in 4-Position einer 6- bis 7-gliedrigen Cycloalkyleniminogruppe durch ein Sauerstoff oder Schwefelatom oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom ersetzt ist, zwei Heteroatome durch mindestens zwei Kohlenstoffatome voneinander getrennt sind,
oder A eine Gruppe der Formel 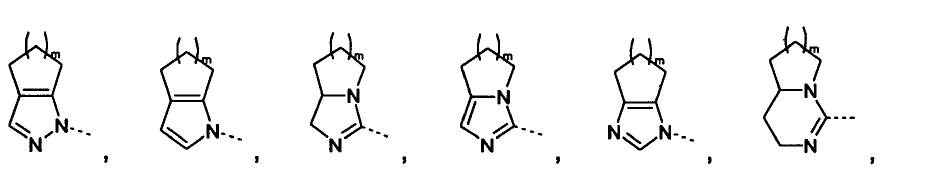 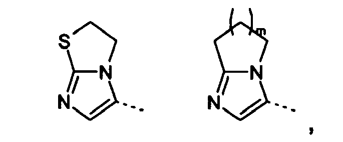 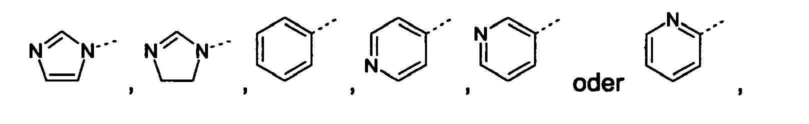 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
- R¹: ein Wasserstoff- , Fluor-, Chlor- oder Bromatom, eine Methyl- oder Methoxygruppe, wobei die Wasserstoffatome der Methyl- oder Methoxygruppe gegebenenfalls ganz oder teilweise durch Fluoratome ersetzt sein können, bedeutet,
- R²: ein Wasserstoff- oder Fluoratom bedeutet,
- X: ein Stickstoffatom oder eine CH-Gruppe bedeutet,
- R³: ein Wasserstoffatom oder eine C₁₋₃-Alkylgruppe bedeutet,
- R⁴: ein Wasserstoffatom bedeutet,
- R⁵: ein Wasserstoffatom, eine Hydroxygruppe, eine OR⁹-Gruppe, eine C₂₋₄-Alkenyl- oder C₂₋₄-Alkinylgruppe,
eine geradkettige oder verzweigte C₁₋₄-Alkylgruppe, wobei die Wasserstoffatome gegebenenfalls ganz oder teilweise durch Fluoratome ersetzt sein können, und die gegebenenfalls durch eine Hydroxy-, eine C₁₋₃-Alkyloxygruppe, wobei die Wasserstoffatome der C₁₋₃-Alkyloxygruppe ganz oder teilweise durch Fluoratome ersetzt sein können, eine Benzyloxy-, C₁₋₃-Alkylcarlbonyloxy-, C₁₋₃-Alkyloxycarbonyl-, Aminocarbonyl-, C₁₋₃-Alkylaminocarbonyl-, Di-(C₁₋₃-alkyl)-aminocarbonyl- , C₄-₆-Cycloalkyleniminocarbonyl-, Amino-, C₁₋₃-Alkylamino-, Di- (C₁₋₃-alkyl)-amino-, C₁₋₃-Alkylcarlbonylamino-, oder C₁₋₃-Alkyl- sulfonylaminogruppe substituiert sein kann,
eine Phenyl-, oder C-verknüpfte Heteroarylgruppe wobei die Heteroarylgruppe ausgewählt ist aus der Gruppe bestehend aus Pyrrolyl, Oxazolyl, Imidazolyl, Furanyl, Thiophenyl, Thiazolyl, 1,2,3- Triazolyl, 1,2,4-Triazolyl, Tetrazolyl, Pyridinyl, Pyrimidinyl und Pyrazinyl, und die im Heteroarylteil gegebenenfalls ein- bis zweifach durch gleiche oder unterschiedliche Substituenten ausgewählt aus der Gruppe bestehend aus Halogenatomen, C₁₋₃-Alkyl-, Hydroxy-, C₁₋₃-Alkyloxy-, Mono-, Di- und Trifluormethoxygruppen substituiert sein kann,
eine Phenyl-C₁₋₃-alkyl-, Heteroaryl-C₁₋₃-alkyl-gruppe, wobei die Heteroarylgruppe ausgewählt ist aus der Gruppe bestehend aus Pyrrolyl, Oxazolyl, Imidazolyl, Furanyl, Thiophenyl, Thiazolyl, 1,2,3- Triazolyl, 1,2,4-Triazolyl, Tetrazolyl, Pyridinyl, Pyrimidinyl und Pyrazinyl, und die im Heteroarylteil gegebenenfalls ein- bis zweifach durch gleiche oder unterschiedliche Substituenten ausgewählt aus der Gruppe bestehend aus Halogenatomen, C₁₋₃-Alkyl-, Hydroxy-, C₁₋₃-Alkyloxy-, Mono-, Di- und Trifluormethoxygruppen substituiert sein kann, und die gegebenenfalls im C₁₋₃-alkyl-Teil durch eine Hydroxy-, eine C₁₋₃-Alkyloxygruppe, wobei die Wasserstoffatome der C₁₋₃-Alkyloxygruppe gegebenenfalls ganz oder teilweise durch Fluoratome ersetzt sein können, eine C₁₋₅-Alkylcarbonyloxy-, oder eine C₁₋₅-Alkyloxycarbonyloxygruppe substituiert sein kann;
bedeutet,
- R⁹: eine geradkettige oder verzweigte C₁₋₄-Alkylgruppe, wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine Hydroxy-, eine C₁₋₃-Alkyloxygruppe, wobei die Wasserstoffatome der C₁₋₃-Alkyloxygruppe gegebenenfalls ganz oder teilweise durch Fluoratome ersetzt sein können, eine Benzyloxy-, C₁₋₃-Alkyloxycarbonyl-, Aminocarbonyl-, C₁₋₃-Alkylaminocarbonyl-, Di-(C₁₋₃-alkyl)-aminocarbonyl-, C₄₋₆-Cycloalkyleniminocarbonyl-, Amino-, C₁₋₃-Alkylamino-, Di-(C₁₋₃-alkyl)- amino-, C₁₋₃-Alkylcarbonylamino-, C₁₋₃-Alkylsulfonylaminogruppe substituiert sein kann,
mit der Maßgabe, dass der Ersatz von Wasserstoffatomen des ersten Kohlenstoffatoms der geradkettigen oder verzweigten C₁₋₄-Alkylgruppe durch Substituenten aus der Gruppe Sauerstoff, Schwefel oder Stickstoff ausgeschlossen ist,
eine Phenyl-, Phenyl-C₁₋₂-alkyl-, Heteroaryl-C₁₋₂-alkyl- oder C-verknüpfte Heteroarylgruppe
wobei die Heteroarylgruppe ausgewählt ist aus der Gruppe bestehend aus Pyrrolyl, Oxazolyl, Imidazolyl, Furanyl, Thiophenyl, Thiazolyl, 1,2,3-Triazolyl, 1,2,4-Triazolyl, Tetrazolyl, Pyridinyl, Pyrimidinyl und Pyrazinyl, und die im Heteroarylteil gegebenenfalls ein- bis zweifach durch gleiche oder unterschiedliche Substituenten ausgewählt aus der Gruppe bestehend aus Halogenatomen, C₁₋₃-Alkyl-, Hydroxy-, C₁₋₃-Alkyloxy-, Mono-, Di- und Trifluormethoxygruppen substituiert sein kann,
bedeutet,
- B: einen Rest der allgemeinen Formel 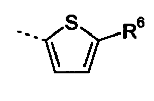 bedeutet,
- R⁶: ein Wasserstoff-, ein Chlor- oder Bromatom, eine Ethinyl-, eine Methyl- oder eine Methoxygruppe bedeutet,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Eine sechste Ausführungsform der vorliegenden Erfindung umfasst diejenigen Verbindungen der allgemeinen Formel I, in denen
- A: eine 5- bis 6-gliedrige Cycloalkyleniminocarbonyl- oder Cycloalkyleniminosulfonylgruppe, wobei
der Cycloalkyleniminoteil im Kohlenstoffgerüst durch eine oder zwei C₁₋₃-Alkyl-, Hydroxy-C₁₋₃-alkyl-, C₁₋₃-Alkyloxy-C₁₋₃-alkyl-, Pyridinyl-C₁₋₃-alkyl-, Amino-C₁₋₃-alkyl-, C₃₋₆-Cycloalkylamino-C₁₋₃-alkyl-, Phenylamino-C₁₋₃-alkyl-, C₁₋₅-Alkylamino-C₁₋₃-alkyl-, Di-(C₁₋₅-alkyl)-amino-C₁₋₅-alkyl-, *N-*Pyrrolidinyl- C₁₋₃-alkyl-, C₁₋₃-Alkyloxycarlbonyl-C₁₋₃-alkyl-, C₁₋₃-Alkyloxycarbonyl-, Aminocarbonyl-, C₁₋₃-Alkylaminocarbonyl-, Di-(C₁₋₃-alkyl)-aminocarbonyl-, Hydroxy-, C₁₋₃-Alkyloxy, C₁₋₃-Alkylamino-, Di-(C₁₋₃-alkyl)-amino-, *N-*(C₁₋₃-Alkyl)piperazin-4-yl-C₁₋₃-alkyl-, eine Phenyl- oder eine Pyridinylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder
eine Methylengruppe in 4-Position einer 6- bis 7-gliedrigen Cycloalkyleniminogruppe durch ein Sauerstoff- oder Schwefelatom, durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom ersetzt ist, zwei Heteroatome durch mindestens zwei Kohlenstoffatome voneinander getrennt sind,
oder A eine Gruppe der Formel 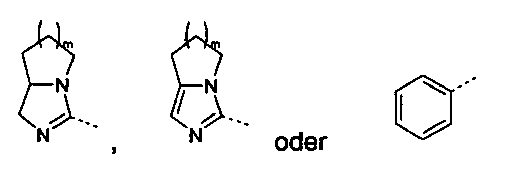 die jeweils an einem Kohlenstoffatom durch eine C₁₋₃-Alkyl-, Methylsulfonylmethyl-, Aminosulfonyl, Amino-C₁₋₃-alkyl-, C₁₋₃-Alkylamino-C₁₋₃-alkyl- oder eine Di-(C₁₋₃-alkyl)-amino-C₁₋₃-alkyl-gruppe substituiert sein kann und wobei
m die Zahl 1 oder 2 bedeutet,
- R¹: ein Wasserstoff- , Fluor-, Chlor- oder Bromatom, eine Methyl-, Trifluormethyl- oder Methoxygruppe bedeutet,
- R²: ein Wasserstoffatom bedeutet,
- X: ein Stickstoffatom oder eine CH-Gruppe bedeutet,
- R³: ein Wasserstoffatom oder eine Methylgruppe bedeutet,
- R⁴: ein Wasserstoffatom bedeutet,
- R⁵: ein Wasserstoffatom, eine Hydroxygruppe, eine OR⁹-Gruppe, eine Allyl- oder Methallylgruppe,
eine Methylgruppe, die durch eine C₁₋₃-alkyl-, Hydroxy-, OR⁹-Gruppe-, Aminocarbonyl-, Pyridin-4-yl, Pyridin-3-yl, Pyridin-2-yl, Pyrazin-2-yl, Pyrazin-3-yl- oder Phenylgruppe substituiert sein kann,
eine Phenylgruppe
bedeutet,
- R⁹: eine geradkettige oder verzweigte C₁₋₄-Alkylgruppe, die gegebenenfalls durch eine Hydroxy-, eine C₁₋₃-Alkoxygruppe, eine Benzyloxy- oder eine Di-(C₁₋₃-alkyl)-aminogruppe substituiert sein kann, mit der Maßgabe, dass der Ersatz von Wasserstoffatomen des ersten Kohlenstoffatoms der geradkettigen oder verzweigten C₁₋₄-Alkylgruppe durch Substituenten aus der Gruppe Sauerstoff oder Stickstoff ausgeschlossen ist,
bedeutet,
- B: einen Rest der allgemeinen Formel 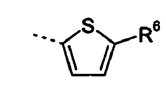 bedeutet,
- R⁶: ein Chlor- oder Bromatom oder eine Ethinylgruppe bedeutet,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Eine siebte Ausführungsform der vorliegenden Erfindung umfasst diejenigen Verbindungen der allgemeinen Formel I, in denen der Rest B die Gruppe 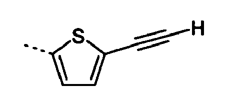 bedeutet,
deren Tautomere, deren Enantiomere, deren Diastereomere, deren Gemische und deren Salze.

Eine achte Ausführungsform der vorliegenden Erfindung umfasst diejenigen Verbindungen der allgemeinen Formel I, in denen der Rest B die Gruppe 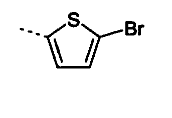 bedeutet,
deren Tautomere, deren Enantiomere, deren Diastereomere, deren Gemische und deren Salze.

Eine neunte Ausführungsform der vorliegenden Erfindung umfasst diejenigen Verbindungen der allgemeinen Formel I, in denen der Rest A die Gruppe 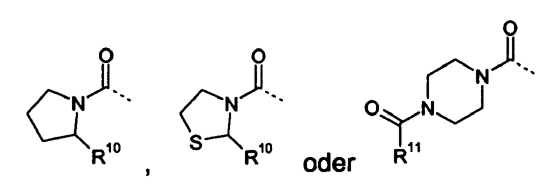 bedeutet, wobei
R¹⁰ das Wasserstoffatom, eine Methyl-, Aminomethyl-, C₁₋₃-Alkylamino-C₁₋₂-alkyl-, Di-(C₁₋₃-alkyl)-amino-C₁₋₂-alkyl-, Pyrrolidin-1-yl-methyl- oder 2-(Pyrrolidin-1-yl)-ethylgruppe,
R¹¹ das Wasserstoffatom oder eine Methylgruppe bedeutet,
deren Tautomere, deren Enantiomere, deren Diastereomere, deren Gemische und deren Salze.

Im Rahmen der vorliegenden Anmeldung sind, falls nicht anders definiert, folgende in den Definitionen erwähnten allgemeine Ausdrücke wie hier unten näher definiert oder durch Beispiele dargestellt.

Beispiele für die voranstehend in den Definitionen erwähnten monocyclische Heteroarylgruppen sind die Pyridyl-, *N-*Oxy-pyridyl-, Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thiophenyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für die voranstehend in den Definitionen erwähnten bicyclische Heteroarylgruppen sind die Benzimidazolyl, Benzofuranyl-, Benzo[c]furanyl-, Benzothiophenyl-, Benzo[c]thiophenyl-, Benzothiazolyl-, Benzo[c]isothiazolyl-, Benzo[*d*]isothiazolyl-, Benzooxazolyl-, Benzo[c]isoxazolyl-, Benzo[*d*]isoxazolyl-, Benzo[1,2,5]oxadiazolyl-, Benzo[1,2,5]thiadiazolyl-, Benzo[1,2,3]thiadiazolyl-, Benzo[*d*][1,2,3]thazinyl-, Benzo[1,2,4]triazinyl-, Benzotriazolyl-, Cinnolinyl-, Chinolinyl-, *N-*Oxy-chinolinyl-, Isochinolinyl-, Chinazolinyl-, *N-*Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₆-Alkylkgruppen sind die Methyl-, Ethyl-, 1-Propyl-, 2-Propyl-, n-Butyl-, sec-Butyl-, *tert*-Butyl-, 1-Pentyl-, 2-Pentyl-, 3-Pentyl-, *neo*-Pentyl-, 3-Methyl-2-butyl-, 1-Hexyl-, 2-Hexyl-, 3-Hexyl, 3-Methyl-2-pentyl-, 4-Methyl-2-pentyl-, 3-Methyl-3-pentyl-, 2-Methyl-3-pentyl-, 2,2-Dimethyl-3-butyl- oder 2,3-Dimethyl-2-butyl-Gruppe.

Beispiele für die voranstehend in den Definitionen erwähnten C₁₋₅-Alkyloxygruppen sind die Methyloxy-, Ethyloxy-, 1-Propyloxy-, 2-Propyloxy-, n-Butyloxy-, sec-Butyloxy-, *tert*-Butyloxy-, 1-Pentyloxy-, 2-Pentyloxy-, 3-Pentyloxy- oder *neo*-Pentyloxy-Gruppe.

Beispiele für die voranstehend in den Definitionen erwähnten C₂₋₆-Alkenylgruppen sind die Ethenyl-, 1-Propen-1-yl-, 2-Propen-1-yl-, 1-Buten-1-yl-, 2-Buten-1-yl-, 3-Buten-1-yl-, 1-Penten-1-yl-, 2-Penten-1-yl-, 3-Penten-1-yl-, 4-Penten-1-yl-, 1-Hexen-1-yl-, 2-Hexen-1-yl-, 3-Hexen-1-yl-, 4-Hexen-1-yl-, 5-Hexen-1-yl-, But-1-en-2-yl-, But-2-en-2-yl-, But-1-en-3-yl-, 2-Methyl-prop-2-en-1-yl-, Pent-1-en-2-yl-, Pent-2-en-2-yl-, Pent-3-en-2-yl-, Pent-4-en-2-yl-, Pent-1-en-3-yl-, Pent-2-en-3-yl-, 2-Methyl-but-1-en-1-yl-, 2-Methyl-but-2-en-1-yl-, 2-Methyl-but-3-en-1-yl-, 2-Ethyl-prop-2-en-1-yl-, Hex-1-en-2-yl-, Hex-2-en-2-yl-, Hex-3-en-2-yl-, Hex-4-en-2-yl-, Hex-5-en-2-yl-, Hex-1-en-3-yl-, Hex-2-en-3-yl-, Hex-3-en-3-yl-, Hex-4-en-3-yl-, Hex-5-en-3-yl-, Hex-1-en-4-yl-, Hex-2-en-4-yl-, Hex-3-en-4-yl-, Hex-4-en-4-yl-, Hex-5-en-4-yl-, 4-Methyl-pent-1-en-3-yl-, 3-Methyl-pent-1-en-3-yl-, 2-Methyl-pent-1-en-3-yl-, 2,3-Dimethyl-but-1-en-3-yl-, 3,3-Dimethyl-but-1-en-2-yl- oder 2-Ethyl-but-1-en-3-yl-Gruppe,

Beispiele für die voranstehend in den Definitionen erwähnten C₂₋₆-Alkinylgruppen sind die Ethinyl-, 1-Propinyl-, 2-Propinyl-, 1-Butin-1-yl-, 1-Butin-3-yl-, 2-Butin-1-yl-, 3-Butin-1-yl-, 1-Pentin-1-yl-, 1-Pentin-3-yl-, 1-Pentin-4-yl-, 2-Pentin-1-yl-, 2-Pentin-3-yl-, 3-Pentin-1-yl-, 4-Pentin-1-yl-, 2-Methyl-1-butin-4-yl-, 3-Methyl-1-butin-1-yl-, 3-Methyl-1-butin-3-yl-, 1-Hexin-1-yl-, 2-Hexin-1-yl-, 3-Hexin-1-yl-, 4-Hexin-1-yl-, 5-Hexin-1-yl-, 1-Hexin-3-yl-, 1-Hexin-4-yl-, 1-Hexin-5-yl-, 2-Hexin-4-yl-, 2-Hexin-5-yl-, 3-Hexin-5-yl-, 3-Methyl-1-pentin-3-yl-, 4-Methyl-1-pentin-3-yl-, 3-Methyl-1-pentin-4-yl-, 4-Methyl-1-pentin-4-yl-, 4-Methyl-2-pentin-4-yl-, 4-Methyl-2-pentin-1-yl-, 2,2-Dimethyl-3-butin-1-yl- oder 2-Ethyl-3-butin-1-yl-Gruppe.

Unter einer *in vivo* in eine Carboxygruppe überführbare Gruppe ist beispielsweise eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, ein C₅₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, dass keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol oder ein Alkohol der Formel

R¹²-CO-O-(R¹³CR¹⁴)-OH,

in der
R¹² eine C₁₋₈-Alkyl-, C₅₋₇-Cycloalkyl-, Phenyl- oder Phenyl-C₁₋₃-alkylgruppe,
R¹³ ein Wasserstoffatom, eine C₁₋₃-Alkyl-, C₅₋₇-Cycloalkyl- oder Phenylgruppe und
R¹⁴ ein Wasserstoffatom oder eine C₁₋₃-Alkylgruppe darstellen,
zu verstehen.

Als bevorzugte von einer Carboxygruppe *in vivo* abspaltbare Reste kommen eine C₁₋₆-Alkoxygruppe wie die Methoxy-, Ethoxy-, n-Propyloxy-, Isopropyloxy-, n-Butyloxy-, n-Pentyloxy-, n-Hexyloxy- oder Cyclohexyloxygruppe oder eine Phenyl-C₁₋₃-alkoxygruppe wie die Benzyloxygruppe in Betracht.

Unter einer *in vivo* in eine Hydroxylgruppe überführbare Gruppe ist beispielsweise eine mit einer Carbonsäure veresterte Hydroxylgruppe, in der der Carbonsäureteil vorzugsweise eine C₁₋₇-Alkansäure, eine Phenyl-C₁₋₃-alkansäure, ein C₃₋₉-Cycloalkylcarbonsäure, eine C₅₋₇-Cycloalkencarbonsäure, eine C₃₋₇-Alkensäure, eine Phenyl-C₃₋₅-alkensäure, eine C₃₋₇-Alkinsäure oder Phenyl-C₃₋₅-alkinsäure, wobei einzelne Methylengruppen des Carbonsäurerestes durch Sauerstoffatome ersetzt sein können mit der Maßgabe, dass keine Bindung an das Sauerstoffatom von einem Kohlenstoffatom ausgeht, welches eine Doppel- oder Dreifachbindung trägt, zu verstehen.

Als bevorzugte von einer Hydroxylgruppe *in vivo* abspaltbare Reste kommen eine C₁₋₇-Acylgruppe wie die Formyl-, Acetyl-, n-Propionyl-, Isopropionyl-, n-Propanoyl-, n-Butanoyl-, n-Pentanoyl-, n-Hexanoyl- oder Cyclohexylcarbonylgruppe oder eine Benzoylgruppe sowie auch eine Methoxyacetyl-, 1-Methoxypropionyl-, 2-Methoxypropionyl- oder 2-Methoxyethoxyacetylgruppe in Betracht.

Diejenigen Verbindungen der allgemeinen Formel I, in denen A, R⁴ und/oder R⁵ eine *in vivo* in eine Carboxy- oder Hydroxylgruppe überführbare Gruppe enthält, stellen Prodrugs für diejenigen Verbindungen der allgemeinen Formel I dar, in denen A, R⁴ und/oder R⁵ eine Carboxy- oder Hydroxylgruppe enthält.

Beispielsweise seien folgende bevorzugte Verbindungen der allgemeinen Formel I erwähnt:
(1) 5-Brom-thiophen-2-carbonsäure-{1-[3-methyl-4-(3-xo-piperazin-1-carbonyl)-phenyl]-5-oxo-pyrrolidin-3-yl}-amid
(2) 5-Brom-thiophen-2-carbonsäure-{1-[4-(4-hydroxy-piperazin-1-carbonyl)-3-methyl-phenyl]-5-oxo-pyrrolidin-3-yl}-amid
(3) (2*S*)-1-(4-{4-[(5-Brom-thiophen-2-carbonyl)-amino]-2-oxo-pyrrolidin-1-yl}-2-methyl-benzoyl)-pyrrolidin-2-carbonsäuredimethylamid
(4) 1-(4-{4-[(5-Brom-thiophen-2-carbonyl)-amino]-2-oxo-pyrrolidin-1-yl}-2-methyl-benzoyl)-pyrrolidin-2-carbonsäuremethylester
(5) (25)-1-(4-{4-[(5-Brom-thiophen-2-carbonyl)-amino]-2-oxo-pyrrolidin-1-yl}-2-methyl-benzoyl)-pyrrolidin-2-carbonsäuremethylamid
(6) 5-Brom-thiophen-2-carbonsäure-{1-[3-methyl-4-(3,4,5,6-tetrahydro-2H-[2,3']bipyridinyl-1-carbonyl)-phenyl]-5-oxo-pyrrolidin-3-yl}amid
(7) 5-Brom-thiophen-2-carbonsäure-{1-[3-methyl-4-((2*S*)-2-pyrrolidin-1-ylmethyl-pyrrolidin-1-carbonyl)-phenyl]-5-oxo-pyrrolidin-3-yl}-amid
(8) 1-(4-{4-[(5-Brom-thiophen-2-carbonyl)-amino]-2-oxo-pyrrolidin-1-yl}-2-methyl-benzoyl)-piperidin-2-carbonsäuremethylester
(9) (2R)-1-(4-{4-[(5-Brom-thiophen-2-carbonyl)-amino]-2-oxo-pyrrolidin-1-yl}-2-methyl-benzoyl)-pyrrolidin-2-carbonsäureamid
(10) 5-Brom-thiophen-2-carbonsäure-(1-{4-[3-(butan-1-sulfonylamino)-piperidin-1-carbonyl]-3-methyl-phenyl}-5-oxo-pyrrolidin-3-yl)-amid
(11) 1-(4-{4-[(5-Brom-thiophen-2-carbonyl)-amino]-2-oxo-pyrrolidin-1-yl}-2-methyl-benzoyl)-piperidin-4-carbonsäureamid
(12) 5-Brom-thiophen-2-carbonsäure-{1-[3-methyl-4-(3-methyl-piperidin-1-carbonyl)-phenyl]-5-oxo-pyrrolidin-3-yl}-amid
(13) 5-Brom-thiophen-2-carbonsäure-{1-[3-methyl-4-(4-methyl-piperidin-1-carbonyl)-phenyl]-5-oxo-pyrrolidin-3-yl}amid
(14) 5-Brom-thiophen-2-carbonsäure-(1-{4-[3-(3-butyl-ureido)-piperidin-1-carbonyl]-3-methyl-phenyl}-5-oxo-pyrrolidin-3-yl)-amid
(15) 5-Brom-thiophen-2-carbonsäure-{1-[4-(dimethylcarbamoylmethyl-methylcarbamoyl)-3-methyl-phenyl]-5-oxo-pyrrolidin-3-yl}-amid
(16) 5-Brom-thiophen-2-carbonsäure-(1-{4-[2-(4-dimethylamino-butyl)-piperidin-1-carbonyl]-3-methyl-phenyl}-5-oxo-pyrrolidin-3-yl)-amid
(17) (2*S*,4R)-1-(4-{4-[(5-Brom-thiophen-2-carbonyl)-amino]-2-oxo-pyrrolidin-1-yl}-2-methyl-benzoyl)-4-hydroxy-pyrrolidin-2-carbonsäuremethylester
(18) 5-Brom-thiophen-2-carbonsäure-{1-[4-(R-2-hydroxymethyl-pyrrolidin-1-carbonyl)-3-methyl-phenyl]-5-oxo-pyrrolidin-3-yl}amid
(19) 5-Brom-thiophen-2-carbonsäure-{1-[4-(3,5-dimethyl-piperidin-1-carbonyl)-3-methyl-phenyl]-5-oxo-pyrrolidin-3-yl}-amid
(20) 5-Brom-thiophen-2-carbonsäure-{1-[3-methyl-4-(thiomorpholin-4-carbonyl)-phenyl]-5-oxo-pyrrolidin-3-yl}-amid
(21) 5-Brom-thiophen-2-carbonsäure-{1-[3-methyl-4-(2-methyl-morpholin-4-carbonyl)-phenyl]-5-oxo-pyrrolidin-3-yl}-amid
(22) 5-Brom-thiophen-2-carbonsäure-{1-[3-methyl-4-(1-oxo-1λ⁴-thiomorpholin-4-carbonyl)-phenyl]-5-oxo-pyrrolidin-3-yl}-amid
(23) 5-Brom-thiophen-2-carbonsäure-(1-{3-methyl-4-[2-(4-methyl-piperazin-1-ylmethyl)-piperidin-1-carbonyl]-phenyl}-5-oxo-pyrrolidin-3-yl)-amid
(24) 1-(4-{4-[(5-Brom-thiophen-2-carbonyl)-amino]-2-oxo-pyrrolidin-1-yl}-2-methyl-benzoyl)-piperidin-3-yl]essigsäuremethylester
(25) 5-Brom-thiophen-2-carbonsäure-{1-[4-((2R)-2-methoxymethyl-pyrrolidin-1-carbonyl)-3-methyl-phenyl]-5-oxo-pyrrolidin-3-yl}-amid
(26) 5-Brom-thiophen-2-carbonsäure-{1-[4-(3-hydroxy-piperidin-1-carbonyl)-3-methyl-phenyl]-5-oxo-pyrrolidin-3-yl}-amid
(27) 5-Brom-thiophen-2-carbonsäure-{1-[4-((2*S*)-2-hydroxymethyl-pyrrolidin-1-carbonyl)-3-methyl-phenyl]-5-oxo-pyrrolidin-3-yl}-amid
(28) 5-Brom-thiophen-2-carbonsäure-{1-[4-((2*S*)-2-methoxymethyl-pyrrolidin-1-carbonyl)-3-methyl-phenyl]-5-oxo-pyrrolidin-3-yl}-amid
(29) 5-Brom-thiophen-2-carbonsäure-{1-[4-(4-methoxy-piperidin-1-carbonyl)-3-methyl-phenyl]-5-oxo-pyrrolidin-3-yl}-amid
(30) 5-Brom-thiophen-2-carbonsäure-(1-{4-[2-(2-diethylamino-ethyl)-piperidin-1-carbonyl]-3-methyl-phenyl}-5-oxo-pyrrolidin-3-yl)-amid
(31) 5-Brom-thiophen-2-carbonsäure-{1-[3-methyl-4-(2-methyl-pyrrolidin-1-carbonyl)-phenyl]-5-oxo-pyrrolidin-3-yl}-amid
(32) 5-Brom-thiophen-2-carbonsäure-{1-[3-methyl-4-(4-methyl-[1,4]diazepan-1-carbonyl)-phenyl]-5-oxo-pyrrolidin-3-yl}-amid
(33) 5-Brom-thiophen-2-carbonsäure-(1-{4-[2-(3-hydroxy-propyl)-piperidin-1-carbonyl]-3-methyl-phenyl}-5-oxo-pyrrolidin-3-yl)-amid
(34) 5-Brom-thiophen-2-carbonsäure-{1-[3-methyl-4-(piperidin-1-carbonyl)-phenyl]-5-oxo-pyrrolidin-3-yl}-amid
(35) 5-Brom-thiophen-2-carbonsäure-{1-[3-methyl-4-(2-methyl-piperidin-1-carbonyl)-phenyl]-5-oxo-pyrrolidin-3-yl}-amid
(36) 1-(4-{4-[(5-Brom-thiophen-2-carbonyl)-amino]-2-oxo-pyrrolidin-1-yl}-2-methyl-benzoyl)-piperidin-3-carbonsäureamid
(37) 5-Brom-thiophen-2-carbonsäure-{1-[4-(4-hydroxy-piperidin-1-carbonyl)-3-methyl-phenyl]-5-oxo-pyrrolidin-3-yl}-amid
(38) 5-Brom-thiophen-2-carbonsäure-{1-[4-(4-acetyl-piperazin-1-carbonyl)-3-methyl-phenyl]-5-oxo-pyrrolidin-3-yl}-amid
(39) 5-Brom-thiophen-2-carbonsäure-{1-[3-methyl-4-((2R)-2-phenylaminomethyl-pyrrolidin-1-carbonyl)-phenyl]-5-oxo-pyrrolidin-3-yl}-amid
(40) 5-Brom-thiophen-2-carbonsäure-{1-[3-methyl-4-(morpholin-4-carbonyl)-phenyl]-5-oxo-pyrrolidin-3-yl}-amid
(41) 5-Brom-thiophen-2-carbonsäure-{1-[3-methyl-4-(3-oxo-[1,4]diazepan-1-carbonyl)-phenyl]-5-oxo-pyrrolidin-3-yl}-amid
(42) 5-Brom-thiophen-2-carbonsäure-{1-[3-methyl-4-(2-methyl-5-phenyl-pyrrolidin-1-carbonyl)-phenyl]-5-oxo-pyrrolidin-3-yl}amid
(43) 5-Brom-thiophen-2-carbonsäure-{1-[3-methyl-4-(4-methyl-piperazin-1-carbonyl)-phenyl]-5-oxo-pyrrolidin-3-yl}-amid
(44) 5-Brom-thiophen-2-carbonsäure-{1-[4-(3,3-dimethyl-piperidin-1-carbonyl)-3-methyl-phenyl]-5-oxo-pyrrolidin-3-yl}-amid
(45) 5-Brom-thiophen-2-carbonsäure-{1-[4-(2-ethyl-piperidin-1-carbonyl)-3-methyl-phenyl]-5-oxo-pyrrolidin-3-yl}-amid
(46) 1-(4-{4-[(5-Brom-thiophen-2-carbonyl)-amino]-2-oxo-pyrrolidin-1-yl}-2-methyl-benzoyl)-piperidin-3-carbonsäureethylester
(47) 5-Brom-thiophen-2-carbonsäure-{1-[3-methyl-4-(thiazolidin-3-carbonyl)-phenyl]-5-oxo-pymolidin-3-yl}-amid
(48) 5-Brom-thiophen-2-carbonsäure-[1-(4-{2-[(ethyl-methyl-amino)-methyl]-piperidin-1-carbonyl}-3-methyl-phenyl)-5-oxo-pyrrolidin-3-yl]-amid
(49) 5-Brom-thiophen-2-carbonsäure-{1-[4-(3,6-dihydro-2H-pyridin-1-carbonyl)-3-methyl-phenyl]-5-oxo-pyrrolidin-3-yl}-amid
(50) 5-Brom-thiophen-2-carbonsäure-(1-{4-[2-(3-dimethylamino-propyl)-piperidin-1-carbonyl]-3-methyl-phenyl}-5-oxo-pyrrolidin-3-yl)-amid
(51) 5-Brom-thiophen-2-carbonsäure-{1-[4-(4-formyl-piperazin-1-carbonyl)-3-methyl-phenyl]-5-oxo-pyrrolidin-3-yl}amid
(52) 5-Brom-thiophen-2-carbonsäure-{1-[3-methyl-4-(2-piperidin-1-ylmethyl-piperidin-1-carbonyl)-phenyl]-5-oxo-pyrrolidin-3-yl}-amid
(53) 5-Brom-thiophen-2-carbonsäure-{1-[4-(azepan-1-carbonyl)-3-methylphenyl]-5-oxo-pyrrolidin-3-yl}-amid
(54) 5-Brom-thiophen-2-carbonsäure-{1-[3-methyl-4-(5-oxo-[1,4]diazepan-1-carbonyl)phenyl]-5-oxo-pyrrolidin-3-yl}-amid
(55) 5-Brom-thiophen-2-carbonsäure-{1-[4-(4-dimethylamino-piperidin-1-carbonyl)-3-methyl-phenyl]-5-oxo-pyrrolidin-3-yl}-amid
(56) 5-Brom-thiophen-2-carbonsäure-{1-[3-methyl-4-(2-pyridin-2-yl-pyrrolidin-1-carbonyl)-phenyl]-5-oxo-pyrrolidin-3-yl}-amid
(57) 5-Brom-thiophen-2-carbonsäure-{1-[3-methyl-4-(2-pyridin-4-yl-pyrrolidin-1-carbonyl)-phenyl]-5-oxo-pyrrolidin-3-yl}-amid
(58) 5-Brom-thiophen-2-carbonsäure-{1-[3-methyl-4-((2*S*)-2-phenylaminomethyl-pyrrolidin-1-carbonyl)-phenyl]-5-oxo-pyrrolidin-3-yl}-amid
(59) 5-Brom-thiophen-2-carbonsäure-{1-[4-(3-dimethylamino-pyrrolidin-1-carbonyl)-3-methyl-phenyl]-5-oxo-pyrrolidin-3-yl}-amid
(60) 5-Brom-thiophen-2-carbonsäure-(1-{4-[3-(4-diethylamino-butyl)-piperidin-1-carbonyl]-3-methyl-phenyl}-5-oxo-pyrrolidin-3-yl)-amid
(61) 5-Brom-thiophen-2-carbonsäure-{1-[4-(2-aminomethyl-piperidin-1-carbonyl)-3-methyl-phenyl]-5-oxo-pyrrolidin-3-yl}-amid
(62) 5-Brom-thiophen-2-carbonsäure-{1-[4-((2R)-2-aminomethyl-pyrrolidin-1-carbonyl)-3-methyl-phenyl]-5-oxo-pyrrolidin-3-yl}-amid
(63) 5-Brom-thiophen-2-carbonsäure-{1-[4-(3-aminomethyl-piperidin-1-carbonyl)-3-methyl-phenyl]-5-oxo-pyrrolidin-3-yl}-amid
(64) 5-Brom-thiophen-2-carbonsäure-(1-{4-[(2*S*)-2-(2-amino-ethyl)-pyrrolidin-1-carbonyl]-3-methyl-phenyl}-5-oxo-pyrrolidin-3-yl)-amid
(65) 5-Brom-thiophen-2-carbonsäure-{1-[3-methyl-4-(4-methylamino-piperidin-1-carbonyl)-phenyl]-5-oxo-pyrrolidin-3-yl}-amid
(66) 5-Brom-thiophen-2-carbonsäure-{1-[4-(3-amino-pyrrolidin-1-carbonyl)-3-methyl-phenyl]-5-oxo-pyrrolidin-3-yl}-amid
(67) 5-Brom-thiophen-2-carbonsäure-(1-{4-[3-(2-amino-ethyl)-piperidin-1-carbonyl]-3-methyl-phenyl}-5-oxo-pyrrolidin-3-yl)-amid
(68) 5-Brom-thiophen-2-carbonsäure-{1-[4-([1,4]diazepan-1-carbonyl)-3-methyl-phenyl]-5-oxo-pyrrolidin-3-yl}-amid
(69) 5-Brom-thiophen-2-carbonsäure-{1-[3-methyl-4-(4-methylaminomethyl-piperidin-1-carbonyl)-phenyl]-5-oxo-pyrrolidin-3-yl}-amid
(70) 5-Brom-thiophen-2-carbonsäure-{1-[4-(4-amino-4-methyl-piperidin-1-carbonyl)-3-methyl-phenyl]-5-oxo-pyrrolidin-3-yl}-amid
(71) 5-Brom-thiophen-2-carbonsäure-(1-{4-[2-(2-amino-ethyl)-piperidin-1-carbonyl]-3-methyl-phenyl}-5-oxo-pyrrolidin-3-yl)-amid
(72) 5-Brom-thiophen-2-carbonsäure-{1-[4-(4-aminomethyl-piperidin-1-carbonyl)-3-methyl-phenyl]-5-oxo-pyrrolidin-3-yl}amid
(73) 5-Brom-thiophen-2-carbonsäure-(1-{4-[4-(3-ethylamino-propyl)-piperidin-1-carbonyl]-3-methyl-phenyl}-5-oxo-pyrrolidin-3-yl)-amid
(74) 5-Brom-thiophen-2-carbonsäure-{1-[4-(dimethylcarbamoylmethyl-carbamoyl)-3-methyl-phenyl]-5-oxo-pyrrolidin-3-yl}-amid
(75) 5-Brom-thiophen-2-carbonsäure-{1-[4-(1-dimethylcarbamoyl-2-methyl-propylcarbamoyl)-3-methyl-phenyl]-5-oxo-pyrrolidin-3-yl}-amid
(76) 5-Brom-thiophen-2-carbonsäure-{1-[4-(carbamoylmethyl-carbamoyl)-3-methyl-phenyl]-5-oxo-pyrrolidin-3-yl}-amid
(77) 5-Brom-thiophen-2-carbonsäure-{1-[4-(carbamoylmethyl-methylcarbamoyl)-3-methyl-phenyl]-5-oxo-pyrrolidin-3-yl}-amid
(78) 5-Brom-thiophen-2-carbonsäure-{1-[3-methyl-4-(methyl-methylcarbamoylmethyl-carbamoyl)-phenyl]-5-oxo-pyrrolidin-3-yl}-amid
(79) 5-Brom-thiophen-2-carbonsäure-{1-[4-(cyclopropyl-methyl-carbamoyl)-3-methyl-phenyl]-5-oxo-pyrrolidin-3-yl}-amid
(80) 5-Brom-thiophen-2-carbonsäure-(1-{4-[(2-amino-ethyl)-ethyl-carbamoyl]-3-methyl-phenyl)-5-oxo-pyrrolidin-3-yl)-amid
(81) 5-Brom-thiophen-2-carbonsäure-(1-{3-methyl-4-[methyl-(2-methylaminoethyl)-carbamoyl]-phenyl}-5-oxo-pyrrolidin-3-yl)-amid
(82) 5-Brom-thiophen-2-carbonsäure-(1-{4-[(3-amino-propyl)-ethyl-carbamoyl]-3-methyl-phenyl}-5-oxo-pyrrolidin-3-yl)-amid
(83) 5-Brom-thiophen-2-carbonsäure-{1-[3-methyl-4-(pyrrolidin-1-carbonyl)-phenyl]-5-oxo-pyrrolidin-3-yl}-amid
(84) 5-Brom-thiophen-2-carbonsäure[1-(2'-dimethylaminomethyl-biphenyl-4-yl)-5-oxo-pyn-olidin-3-yl]amid
(85) 5-Brom-thiophen-2-carbonsäure[1-(2'-dimethylaminomethyl-biphenyl-4-yl)-5-oxo-pyrrolidin-3-yl]amid
(86) 5-Chlor-thiophen-2-carbonsäure-[1-(2'-methansulfonyl-biphenyl-4-yl)-5-oxo-pyrrolidin-3-yl]-amid
(87) 5-Chlor-thiophen-2-carbonsäure-[1-(2'-methansulfonyl-biphenyl-4-yl)-5-oxo-pyrrolidin-3-yl]-amid
(88) 5-Brom-furan-2-carbonsäure-[1-(2'-methansulfonyl-biphenyl-4-yl)-5-oxo-pyrrolidin-3-yl]-amid
(89) 4-Brom-*N*-[1-(2'-methansulfonyl-biphenyl-4-yl)-5-oxo-pyrrolidin-3-yl]-benzamid
(90) 4-Chlor-*N*-[1-(2'-methansulfonyl-biphenyl-4-yl)-5-oxo-pyrrolidin-3-yl]-benzamid
(91) 4-Brom-*N*-[1-(2'-dimethylaminomethyl-biphenyl-4-yl)-5-oxo-pyrrolidin-3-yl]-benzamid
(92) 4-Chlor-*N*-[1-(2' -dimethylaminomethyl-biphenyl-4-yl)-5-oxo-pyrrolidin-3-yl]-benzamid
(93) (*R*)-5-Brom-thiophen-2-carbonsäure-{1-[3-methyl-4-(pyrrolidin-1-sulfonyl)-phenyl]-5-oxo-pyrrolidin-3-yl}-amid
(94) (*R*)-5-Ethinyl-thiophen-2-carbonsäure-{1-[3-methyl-4-(pyrrolidin-1-sulfonyl)-phenyl]-5-oxo-pyrrolidin-3-yl}-amid
(95) 5-Brom-thiophen-2-carbonsäure-{4-hydroxy-1-[3-methyl-4-(pyrrolidin-1-carbonyl)-phenyl]-5-oxo-pyrrolidin-3-yl}-amid
(96) 5-Chlor-thiophen-2-carbonsäure-{4-allyl-1-[3-methyl-4-(pyrrolidin-1-carbonyl)-phenyl]-5-oxo-pyrrolidin-3-yl}-amid
(97) 5-Ethinyl-thiophen-2-carbonsäure-{4-hydroxy-1-[3-methyl-4-(pyrrolidin-1-carbonyl)-phenyl]-5-oxo-pyrrolidin-3-yl}-amid
(98) 5-Brom-thiophen-2-carbonsäure-{4-methoxy-1-[3-methyl-4-(pyrrolidin-1-carbonyl)-phenyl]-5-oxo-pyrrolidin-3-yl}-amid
(99) 5-Brom-thiophen-2-carbonsäure-{4-(2-hydroxy-ethyl)-1-[3-methyl-4-(pyrrolidin-1-carbonyl)-phenyl]-5-oxo-pyrrolidin-3-yl}-amid
(100) 5-Brom-thiophen-2-carbonsäure-{4-(1,2-dihydroxy-ethyl)-1-[3-methyl-4-(pyrrolidin-1-carbonyl)-phenyl]-5-oxo-pyrrolidin-3-yl}-amid
(101) 5-Brom-thiophen-2-carbonsäure-1-[3-methyl-4-(5,6,7,7a-tetrahydro-1H-pyrrolo[1,2c]imidazol-3-yl)-phenyl]-5-oxo-pyrrolidin-3-yl}-amid
(102) 5-Brom-thiophen-2-carbonsäure-{1-[4-(6,7-dihydro-5H-pyrrolo[1,2c]imidazol-3-yl)-3-methyl-phenyl]-5-oxo-pyrrolidin-3-yl}-amid
(103) 5-Brom-thiophen-2-carbonsäure-{1-[4-(2,5-dihydropyrrol-1-carbonyl)-3-methyl-phenyl]-5-oxo-pyrrolidin-3-yl}-amid
(104) 5-Brom-thiophen-2-carbonsäure-{4-methoxymethyl-1-[3-methyl-4-(pyrrolidin-1-carbonyl)-phenyl]-5-oxo-pyrrolidin-3-yl}-amid
(105) 5-Chlor-thiophen-2-carbonsäure-{4-propyl-1-[3-methyl-4-(pyrrolidin-1-carbonyl)-phenyl]-5-oxo-pyrrolidin-3-yl}-amid
deren Tautomere, deren Enantiomere, deren Diastereomere, deren Gemische und deren Salze.

Besonders bevorzugt von den oben genannten Verbindungen sind die Verbindungen:
(1), (2), (3), (4), (5), (6), (7), (8), (9), (10), (16), (17), (18), (20), (21), (23), (25), (27), (28), (29), (30), (31), (32), (33), (34), (37), (38), (39), (40), (41), (43), (46), (47), (48), (50), (51), (52), (53), (54), (56), (57), (58), (61), (62), (64), (65), (66), (67), (68), (69), (71), (2), (79), (80), (81), (82), (83), (84), (85), (86), (87), (93), (94), (95), (96), (97), (98), (99), (103), (104), (105),
deren Tautomere, deren Enantiomere, deren Diastereomere, deren Gemische und deren Salze.

Ganz besonders bevorzugt von den oben genannten Verbindungen sind die Verbindungen:
(7), (16), (18), (25), (27), (28), (30), (31), (33), (38), (39), (40), (41), (47), (51), (54), (58), (61), (62), (64), (83), (84), (85), (93), (94), (95), (96), (97), (98), (99), (104), (105),
deren Tautomere, deren Enantiomere, deren Diastereomere, deren Gemische und deren Salze.

Im Rahmen der vorliegenden Anmeldung ist, falls vorhanden, unter der Begriff "Isomere", "Stereoisomere", "Diastereomere", "Enantiomere", "chiral", "Racemat" oder "racemische Mischung" folgendes zu verstehen. Verbindungen der gleichen Summenformel, die sich in der Art oder Anordnung der Bindung ihrer Atome bzw. deren Konnektivität oder der räumlichen Anordnung der Atome im Molekül unterscheiden, werden "Isomere" genannt. Isomere, die sich bei gleicher Art und Weise der Konnektivität Ihrer Atome durch die räumliche Anordnung der Atome im Molekül unterscheiden und nicht deckungsgleich sind, werden "Stereoisomere" genannt. Stereoisomere, die sich nicht wie Bild und Spiegelbild zueinander verhalten, werden "Diastereomere" genannt, und Stereoisomere, die sich wie Bild und Spiegelbild zueinander verhalten, werden "Enantiomere" genannt. Bei Anwesenheit eines asymmetrischen Zentrums oder Atoms (auch Stereozentrum oder Chiralitätszentrum genannt), beispielsweise bei einem durch vier unterschiedliche Substituenten substituierten Kohlenstoffatom, hat das Molekül die Eigenschaft "chiral", und ein Paar von Enantiomeren ist möglich. Ein Enantiomer kann durch die absolute Konfiguration seines Stereozentrums charakterisiert werden. Die Beschreibung der absoluten Konfiguration geschieht mittels der Descriptoren (*R*) und (*S*), die durch die Anwendung der Sequenzregeln nach Cahn, Ingold und Prelog ermittelt werden, oder durch Beschreibung der Drehung der Ebene von polarisiertem Licht bei Wechselwirkung mit dem Molekül, die als rechtsdrehend oder linksdrehend (also entsprechend mit (+) oder (-) als Descriptor) bezeichnet wird. Eine chirale Verbindung kann sowohl als individuelles Enantiomer oder als Gemisch der entsprechenden Enantiomere vorliegen. Ein Gemisch, das gleiche Anteile beider Enantiomeren einer Verbindung enthält, wird "Racemat" oder "racemische Mischung" genannt.

Erfindungsgemäß erhält man die Verbindungen der allgemeinen Formel (I) nach an sich bekannten Verfahren, beispielsweise nach folgenden Verfahren:
(a) Zur Herstellung einer Verbindung der allgemeinen Formel 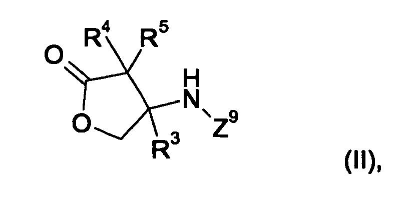 wobei Z⁹ eine Schutzgruppe der Aminofunktion darstellt, die nachfolgend nach literaturbekannten Verfahren abgespalten werden kann, und R³ bis R⁵ wie vorhin definiert sind:
   1) Reduktion und nachfolgende Lactonisierung einer Verbindung der
      allgemeinen Formel 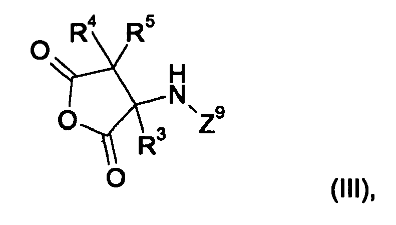 wobei Z⁹ eine Schutzgruppe der Aminofunktion darstellt, die nachfolgend nach literaturbekannten Verfahren abgespalten werden kann, und R³ bis R⁵ wie vorhin definiert sind:
      Die Reduktion zur intermediären Hydroxy-Säure wird beispielsweise zweckmäßigerweise in einem Lösungsmittel oder Lösungsmittelgemisch wie Tetrahydrofuran, Dioxan, Glykoldimethylether, Diethylenglykoldimethylether, Pentan, Hexan, Cyclohexan, Heptan, Benzol, Toluol oder Xylol mit komplexen Hydriden wie Natriumborhydrid, Lithiumborhydrid, Natriumcyanoborhydrid beispielsweise bei Temperaturen zwischen -80 und 250 °C, vorzugsweise jedoch zwischen -30 und 150 °C durchgeführt.
      Die anschließende Lactonisierung des Intermediats wird beispielsweise zweckmäßigerweise in einem Lösungsmittel oder Lösungsmittelgemisch wie Benzol, Chlorbenzol, Toluol, Xylol, Dichloromethan, Chloroform, Tetrachlormethan, 1,2-Dichlorethan in Gegenwart eines Katalysators wie *para-*Toluolsulfonsäure, Camphersulfonsäure oder sauren lonenaustauscher gegebenenfalls in Gegenwart eines Trockenmittels wie Natriumsulfat, Magnesiumsulfat oder Molsieben, beispielsweise bei Temperaturen zwischen-30 und 250 °C, vorzugsweise jedoch zwischen Temperaturen von 0 und 200 °C, durchgeführt. Beispielsweise kann diese Umsetzung wie beschrieben bei G. J. McGarvey, J. M. Williams, R. N. Hiner, Y. Matsubara, T. Oh J. Am. Chem. Soc. 1986, 108, 4943-4952*,* durchgeführt werden.
   2) (Sequentielle) Alkyierung einer Verbindung der allgemeinen Formel 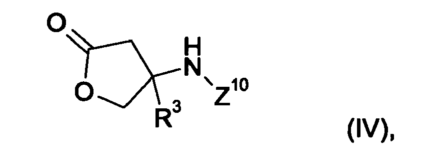 wobei R³ wie vorhin definiert ist und Z¹⁰ eine Schutzgruppe der Aminofunktion darstellt, die nachfolgend nach literaturbekannten Verfahren abgespalten werden kann, aber auch eine Acyl-Gruppe der Formel 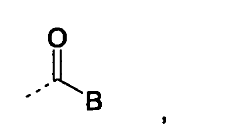 in der B wie vorhin definiert ist, bedeuten kann,
      mit einer Verbindung der allgemeinen Formel

      T-Z¹¹ (V),

      in der die Gruppe T die vorhin definierten Gruppen R⁴ oder R⁵ bedeutet, mit der Maßgabe, dass T nicht die Gruppe OR⁹ bedeuten kann, und Z¹¹ eine nucleofuge Gruppe, beispielsweise ein lod-, Brom- oder Chloratom oder eine Tosylat-, Triflat- oder Mesylatgruppe, darstellt:
      Die Alkyierung kann mit einem gleichen oder anderen Alkylierungsmittel der Formel (V) wiederholt werden, so dass α,α-disubstituierte Lactone der Verbindung (II) entstehen.
      Die Alkylierungen können analog den unter (a) 1) i) b) beschriebenen Bedingungen oder wie beschrieben bei A. EI Hadri, A. Ahbouabdellah, U. Thomet, R. Baur, R. Furtmüller, E. Sigel, W. Sieghart, R. H. Dodd, J. Med. Chem. 2002, 45, 2824-2831, durchgeführt werden.
   3) Nucleophile Substitution einer Verbindung der allgemeinen Formel 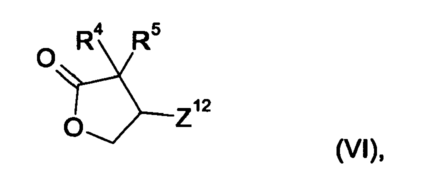 wobei Z¹² eine nucleofuge Gruppe, beispielsweise ein lod-, Brom- oder Chloratom oder eine Tosylat-, Triflat- oder Mesylatgruppe, darstellt, und R⁴ und R⁵ wie vorhin definiert sind,
      mit einer Verbindung beispielsweise aus der Gruppe Lithium-,Natrium-, Kaliumazid, Natrium-, Kaliumphthalimid, 4-Methoxybenzylamin, Benzylamin, 2,4-Dimethoxybenzylamin, Dibenzylamin, Kalium- oder Natriumcyanid,
      und anschließende Reduktion der so eingeführten Gruppe und Schutzgruppenmanipulation
      Die nucleophile Substitution wird zweckmäßigerweise in einem Lösungsmittel oder Lösungsmittelgemisch wie Ethanol, Isopropanol, Benzol, Chlorbenzol, Toluol, Xylol, Glykol, Glykoldimethylether, Diethylenglykoldimethylether, Dimethylformamid, *N*-Methylpyrrolidinon, Tetralin, Dimethylsulfoxid, Sulfolan, Methylenchlorid, Chloroform, Tetrachlormethan oder aber *N*-Ethyl-diisopropylamin, *N*-C₁₋₅-Alkylmorpholin, *N*-C₁₋₅-Alkylpiperidin, *N*-C₁₋₅-Alkylpyrrolidin, Triethylamin, Pyridin, beispielsweise bei Temperaturen zwischen -30 und 250°C, vorzugsweise jedoch zwischen 0 und 150°C, gegebenenfalls zweckmäßigerweise in Gegenwart von Basen wie Lithium-, Natrium-, Kalium-, Cäsiumcarbonat, Kalium-*tert*.-butylat, Natrium-ethanolat, Kaliumhexamethyldisilazan, Natriumhydrid oder Lithiumdiisopropylamid durchgeführt. Beispielsweise kann diese Umsetzung wie beschrieben bei R. N. Salvatore, A. S. Nagle, K. W. Jung, J. Org. Chem. 2002, 67, 674-683, durchgeführt werden.
      Die nachfolgende Reduktion des so eingeführten Stickstoff-Nucleophils wird beispielsweise analog wie unter (a) 1) beschrieben, durchgeführt.
      Das so erhaltene Aminolacton wird beispielsweise durch literaturbekannte Verfahren mit einer Schutzgruppe versehen.
      Die Herstellung von Verbindungen der Formel (VI) kann beispielsweise aus Malonsäuren wie beschrieben bei J.-L. Canet, A. Fadel, J. Salaun, J. Org. Chem. 1992, 57, 3463-3473, durchgeführt werden.
(c) Zur Herstellung einer Verbindung der allgemeinen Formel 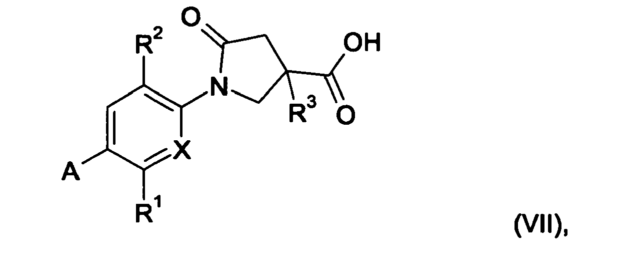 in der A, X und R¹ bis R³ wie vorhin definiert sind:
   Tandem-Michael-Addition-Lactamisierung einer Verbindung der allgemeinen Formel 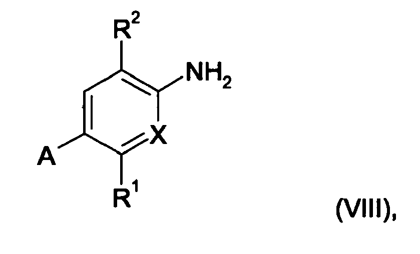 in der A, X, R¹ und R² wie vorhin definiert sind, mit Itaconsäure und
   eine sich gegebenenfalls anschließende Sequenz aus Veresterung, α-Alkylierung mit einer Verbindung der allgemeinen Formel

   T¹-Z¹¹ (IX),

   in der T¹ eine C₁₋₃-Alkylgruppe bedeutet und Z¹¹ eine nucleofuge Gruppe, beispielsweise ein lod-, Brom- oder Chloratom oder eine Tosylat-, Triflat- oder Mesylatgruppe, darstellt, und
   Deblockierung der Carbonsäure:
   Die Tandem-Michael-Addition-Lactamisierung wird zweckmäßigerweise bei einer Temperatur von 50 - 250 °C, vorzugsweise jedoch bei 80 - 200 °C, in Abwesenheit oder Gegenwart eines Lösungsmittels oder Lösungsmittelgemisches wie Wasser, Ethanol, Propanol, Butanol, Toluol, Xylol, Chlorbenzol, Tetralin, Diphenylether, mit Itaconsäure durchgeführt. Diese Reaktion erlaubt die Synthese von Verbindungen der allgemeinen Formel (VII) mit der Maßgabe, dass R³ ein Wasserstoffatom bedeutet.
   Eine sich gegebenenfalls anschließende Substitution wird vorbereitet durch eine Blockierung der Carbonsäurefunktion durch Veresterung nach literaturbekannten Verfahren.
   Die Alkylierung kann analog den unter (a) 1) i) b) beschriebenen Bedingungen oder wie beschrieben bei X.-H. Jiang, Y.-L. Song, Y.-Q. Long, Bioorg. Med. Chem. Lett 2004, 14, 3675-3678, durchgeführt werden.
   Die Deblockierung der veresterten Carbonsäure nach literatur-bekannten Verfahren ermöglicht die Herstellung von α-subsitutierten Carbonsäuren der allgemeinen Formel(VII), in der R³ dann auch eine C₁₋₃-Alkylgruppe bedeutet.
(d) Zur Herstellung einer Verbindung der allgemeinen Formel 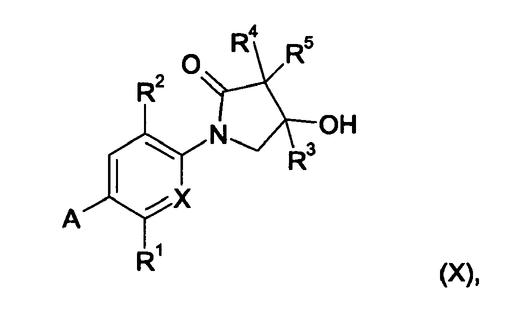 in der A, X und R¹ bis R⁵ wie vorhin definiert sind:
   1) Übergangsmetallkatalysierte Kupplungsreaktion einer Verbindung der allgemeinen Formel 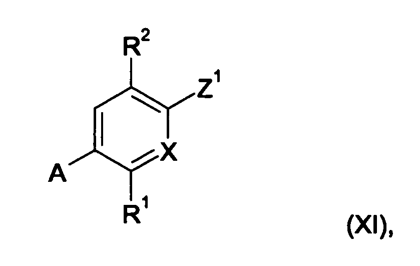 in der A, X, R¹ und R² wie vorhin definiert sind und Z¹ ein Chlor-, Brom- oder lodatom oder eine Triflatgruppe darstellen, mit
      einer Verbindung der allgemeinen Formel 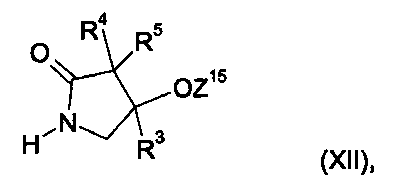 in der R³ bis R⁵ wie vorhin definiert sind und Z¹⁵ eine Schutzgruppe für die Hydroxyfunktion darstellt, und
      nachfolgende Deblockierung der Hydroxyfunktion:

   Die Kupplungsreaktion kann beispielsweise analog den unter (a) 1) a) ii) beschriebenen Bedingungen durchgeführt werden.
   Die Deblockierung der Hydroxyfunktion kann nach literatur-bekannten Verfahren durchgeführt werden.
   Die Verbindungen der allgemeinen Formel (XI) können nach literaturbekannten Verfahren wie bespielsweise der Sandmeyer-Reaktion aus den korrespondierenden Aminen der allgemeinen Formel (VIII) hergestellt werden.
(e) Zur Herstellung einer Verbindung der allgemeinen Formel 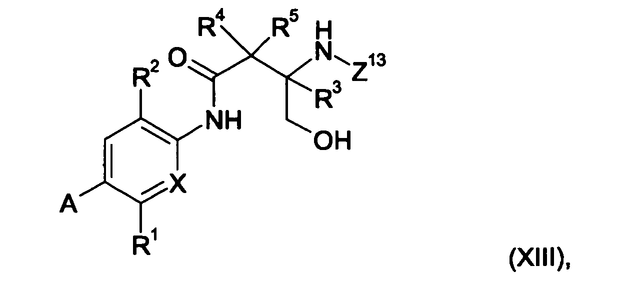 in der A, X und R¹ bis R⁵ wie vorhin definiert sind und Z¹³ eine Schutzgruppe der Aminofunktion darstellt, die nachfolgend nach literaturbekannten Verfahren abgespalten werden kann, aber auch eine Acyl-Gruppe der Formel 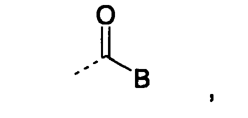 in der B wie vorhin definiert ist, bedeuten kann:
   Lewissäure-assistierte Lacton-Öffnung einer Verbindung der allgemeinen Formel 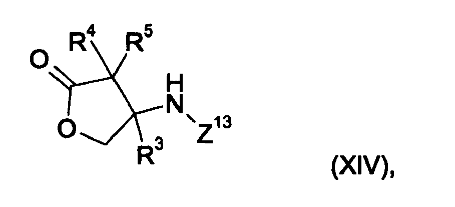 in der R³ bis R⁵ wie vorhin definiert sind und Z¹³ eine Schutzgruppe der Aminofunktion darstellt, die nachfolgend nach literaturbekannten Verfahren abgespalten werden kann, aber auch eine Acyl-Gruppe der Formel 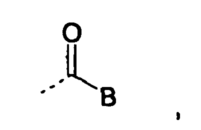 in der B wie vorhin definiert ist, bedeuten kann,
   mit einer Verbindung der allgemeinen Formel 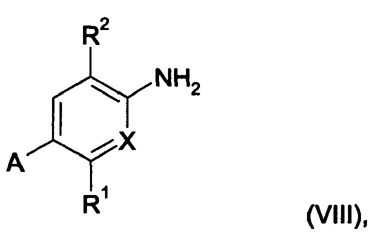 in der A, X, R¹ und R² wie vorhin definiert sind:
   Die Verbindung der allgemeinen Formel (VIII) wird mit einer Organoaluminium-Verbindung wie beispielsweise Trimethylaluminium, Triethylaluminium, Tripropylaluminium, Trisobutylaluminium, Tributylaluminium, Triphenylaluminium in einem Lösungsmittel oder Lösungsmittelgemisch wie Dichlormethan, Toluol, Xylol, Benzol, Hexan, Cyclohexan, Heptan, Tetrahydrofuran bei einer Temperatur von -100 bis 100 °C, vorzugsweise jedoch zwischen -80 und 80 °C, aktiviert und mit dem Lacton der allgemeinen Formel (XIV) umgesetzt.
(f) Zur Herstellung einer Verbindung der allgemeinen Formel 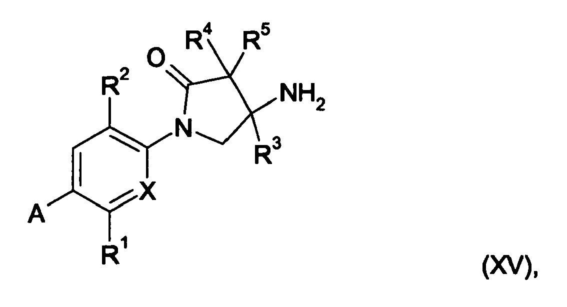 in der A, X und R¹ bis R⁵ wie vorhin definiert sind:
   1) Nucleophile Ringöffnung einer Verbindung der allgemeinen Formel 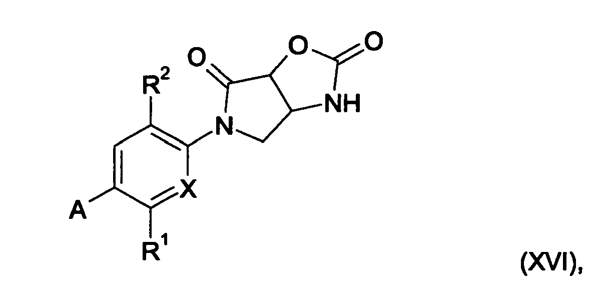 in der A, X, R¹ und R² wie vorhin definiert sind,
      mit einem Alkalisalz der Verbindung R⁹OH,
      in dem R⁹ wie vorhin definiert ist:
      Die nucleophile Ringöffnung des Carbamats zum freien Amin wird zweckmäßigerweise in einem Lösungsmittel oder Lösungsmittelgemisch wie Wasser, Methanol, Ethanol, Isopropanol, Pentan, Hexan, Cyclohexan, Heptan, Benzol, Toluol, Xylol, Glykol, Glykoldimethylether, Diethylenglykoldimethylether, Dioxan, Tetrahydrofuran, *N*-Methylpyrrolidinon, Dimethylformamid mit dem Lithium-, Natrium- oder Kalium-Salz der Verbindung R⁹OH beispielsweise bei Temperaturen zwischen -30 und 250 °C, vorzugsweise jedoch zwischen 0 und 150 °C, durchgeführt.
      Die Herstellung von Verbindungen der Formel (XVI) kann beispielsweise wie beschrieben bei T. Kametani, Y. Kigawa, M. Ihara, Tetrahedron. 1979, 35, 313-316, durchgeführt werden.
   2) Säureabbau-Reaktion einer Verbindung der allgemeinen Formel 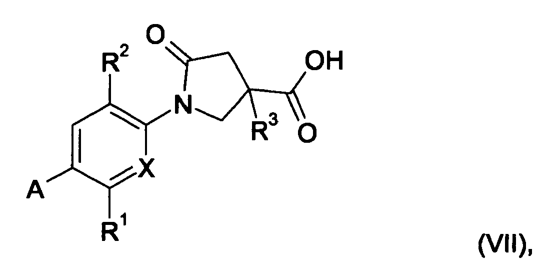 in der A, X, R¹ bis R³ wie vorhin definiert sind:
      Die Carbonsäuren werden durch literatur-bekannte Verfahren in beispielsweise aktivierte Carbonylamide oder Carbonylazide überführt. Durch eine Umlagerungsreaktion (beispielsweise eine Hofmann- , Lossen- oder Curtius-Umlagerung) werden diese Intermediate in Isocyanate umgewandelt.
      Die so entstandenen Isocyanate werden durch Reaktion mit einem Alkohol in die üblichen als Schutzgruppe für die Aminfunktion dienenden Carbamate überführt.
      Diese Carbamat-Schutzgruppen werden nachfolgend nach literatur-bekannten Verfahren abgespalten und setzen das Amin der Formel (XV) frei.
      Die Isocycanate können gebenenfalls auch unter Einwirkung von wässriger Säure direkt in das Amin der Formel (XV) überführt werden.
      Die Bildung der aktivierten Carbonsäurederivate kann beispielsweise durch Aktivierung der vorgenannten Carbonsäuren der Formel (VII) als Carbonylhalogenide oder als unsymmetrische Anhydride und nachfolgender Reaktion mit Lithium-, Natrium-, Kaliumazid oder Hydrazin oder Hydroxyl-Amin in einem Lösungsmittel oder Lösungsmittelgemisch wie Aceton, Butanon, Wasser, Dimethylformamid, Benzol, Toluol, Xylol, Chlorbenzol, Acetonitril, Nitromethan, Tetrahydrofuran, Dioxan, Glykoldimethylether, Diethylenglykoldimethylether, Dimethylformamid, *N*-Methylpyrrolidinon, Dimethylsulfoxid, Sulfolan, Methylenchlorid, Chloroform, Tetrachlormethan, gegebenenfalls in Gegenwart einer Base wie beispielsweise *N*-Ethyl-diisopropylamin, *N*-C₁₋₅-Alkylmorpholin, *N*-C₁₋₅-Alkylpiperidin, *N*-C₁₋₅-Alkylpyrrolidin, Triethylamin, Pyridin bei Temperaturen zwischen -80 und 250 °C, vorzugsweise jedoch zwischen -30 und 150 °C, durchgeführt werden.
      Die Säureabbau-Reaktion (i.e. Umlagerung zum Isocyanat und Carbamat-Bildung) ausgehend von der Carbonsäure der Formel (VII) wird zweckmäßigerweise mit Diphenylphosphorylazid und einer Base wie beispielsweise *N*-Ethyl-diisopropylamin, *N*-C₁₋₅-Alkylmorpholin, *N*-C₁₋₅-Alkylpiperidin, *N*-C₁₋₅-Alkylpyrrolidin, Triethylamin, Pyridin, in einem Lösungsmittel oder Lösungsmittelgemisch wie Benzol, Toluol, Chlorbenzol, Xylol, Tetrahydrofuran, Dioxan, Glykoldimethylether, Diethylenglykoldimethylether, Dimethylformamid, *N*-Methylpyrrolidinon, Dimethylsulfoxid, Sulfolan, Methylenchlorid, Chloroform, Tetrachlormethan bei Temperaturen zwischen -30 und 250 °C, vorzugsweise jedoch zwischen 0 und 200 °C, in Gegenwart eines Alkohols wie beispielsweise tert.-Butanol, Benzylalkohol, para-Methoxybenzylalkohol, Fluorenylmethanol durchgeführt. Diese Carbamat-Schutzgruppen werden nachfolgend nach literatur-bekannten Verfahren abgespalten und setzen das Amin der Formel (XV) frei.
   3) Mitsunobu-Cyclodehydratisierung und nachfolgende SchutzgruppenAbspaltung einer Verbindung der allgemeinen Formel 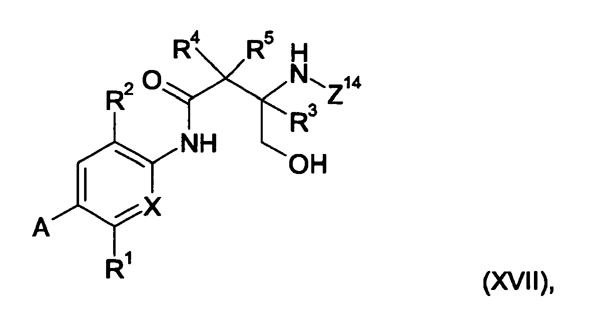 in der A, X und R¹ bis R⁵ wie vorhin definiert sind und Z¹⁴ eine Schutzgruppe für die Aminofunktion bedeutet:
      Die Lactamisierung unter Mitsunubo-Bedingungen wird zweckmäßigerweise in einem inerten Lösungsmittel oder Lösungsmittelgemisch wie zum Beispiel Tetrahydrofuran, Dioxan, Benzol, Toluol, Xylol, Acetonitril in der Gegenwart von Phosphinen wie zum Beispiel Triphenylphosphin, Tributylphosphin mit Dialkylazodicarboxylaten wie beispielsweise Diethylazodicarboxylat, Diisopropylazodicarboxylat, Di(tert.-butyl)azodicarboxylat, beispielsweise bei einer Temperatur von -50 bis 200 °C, vorzugsweise jedoch zwischen -20 und 150 °C, durchgeführt.
      Die anschließende Deblockierung der Amino-Funktion kann nach literaturbeschriebenen Verfahren durchgeführt werden.
   4) Aktivierung, nucleophile Substitution und Reduktion der so eingeführten Gruppe einer Verbindung der allgemeinen Formel 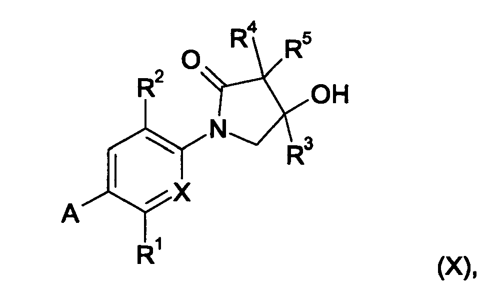 in der A, X und R¹ bis R⁵ wie vorhin definiert sind,
      und nucleophile Substitution
      mit einer Verbindung beispielsweise aus der Gruppe Lithium-,Natrium-, Kaliumazid, Natrium-, Kaliumphthalimid, 4-Methoxybenzylamin, Benzylamin, 2,4-Dimethoxybenzylamin, Dibenzylamin, Kalium- oder Natriumcyanid,
      und anschließende Reduktion der so eingeführten Stickstoff-haltigen Gruppe:
      Die Aktivierung der Alkohol-Funktion einer Verbindung der Formel (X) wird nach literatur-bekannten Verfahren wie zum Beispiel Umwandlung in eine Chlor-, Brom-, lodgruppe oder Überführung in eine nucleofuge Gruppe wie zum Beispiel Mesylat, Triflat- oder Tosylat durchgeführt.
      Die nucleophile Substitution mit einem Stickstoff-Nucleophil und die nachfolgende Reduktion des so eingeführten Stickstoff-Nucleophils wird beispielsweise analog wie unter (f) 3) beschrieben, durchgeführt.
   5) Reduktion der aliphatischen Nitrogruppe einer Verbindung der allgemeinen Formel 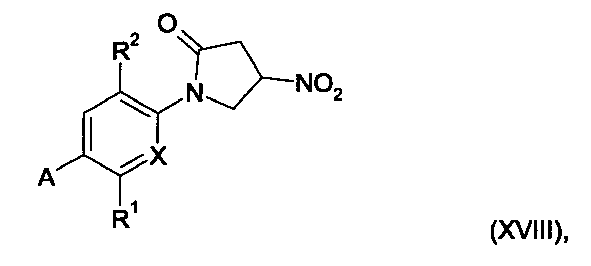 in der A, X, R¹ und R² wie vorhin definiert sind:
      Die Reduktion der Nitrogruppe wird zweckmäßigerweise in einem Lösungsmittel oder Lösungsmittelgemisch wie zum Beispiel Methanol, Ethanol, Isopropanol, Propanol, Butanol, Wasser in Gegenwart von Übergangsmetallsalzen wie zum Beispiel Nickel(II)chlorid oder Cobalt(II)chlorid mit einem Reduktionsmittel wie zum Beispiel Lithiumborhydrid, Natriumborhydrid, beispielsweise bei einer Temperatur von -80°C bis 150 °C, vorzugsweise jedoch zwischen -20 und 100 °C, durchgeführt.
      Verbindungen der allgemeinen Formel (XVIII) können analog den unter (c) beschriebenen Bedingungen durch eine Tandem-Michael-Addition-Lactamisierung aus Verbindungen der allgemeinen Formel (VIII) durch Reaktion mit beispielsweise 3-Nitro-but-3-ensäuremethylester hergestellt werden.
(g) Zur Herstellung einer Verbindung der allgemeinen Formel 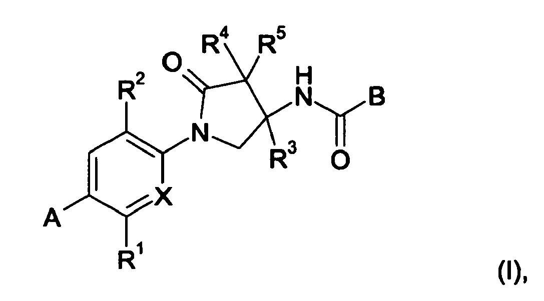 in der A, X, B und R¹ bis R⁵ wie vorhin definiert sind:
   1) Mitsunobu-Cyclodehydratisierung einer Verbindung der allgemeinen Formel 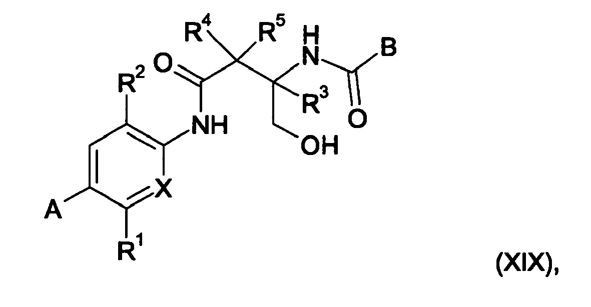 in der A, X, B und R¹ bis R⁵ wie vorhin definiert sind:
      Die Lactamisierung unter Mitsunobu-Bedingungen wird beispielsweise analog wie unter (e) 3) beschrieben, durchgeführt.
   2) Reduktion der aromatischen Nitrogruppe, nachfolgende Umwandlung der so freigelegten Aminogruppe in den wie vorhin definierten Rest A und Schutzgruppenabspaltung einer Verbindung der allgemeinen Formel 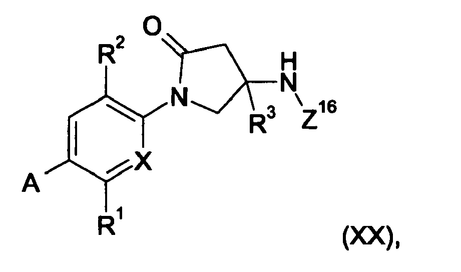 in der A, X und R¹ bis R³ wie vorhin definiert sind und Z¹⁶ eine Schutzgruppe für die Aminofunktion bedeutet,
      durch (sequentielle) Alkylierung analog der (a) 1) i) b) beschriebenen Bedingungen, durch Mono-Hydroxylierung mit Oxaziridinen des Davis-Typs und gegebenenfalls nachfolgender Veretherung oder durch Aldol-Reaktion mit Aldehyden der allgemeinen Formel 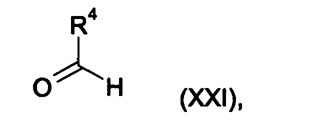 in der R⁴ wie vorhin definiert ist, wobei die Hydroxy-Gruppe als R⁴-Rest ausgeschlossen ist.
      hergestellt werden.
      Die Alkyierung kann mit einem gleichen oder anderen Alkylierungsmittel wiederholt werden, so dass α,α-disubstituierte Lactame der Verbindung (XVIII) entstehen.
      Bei der Alkylierung, Mono-Hydroxylierung und der Aldol-Reaktion kann das Lactam der allgemeinen Formel (XX) jeweils analog der unter (a) 1) i) b) beschriebenen Bedingungen deprotoniert und mit einem Elektrophil wie z.B. einem Oxaziridin (beispielsweisel Phenylsulfonyloxaziridin oder Camphersulfonyloxaziridin) oder einen Aldehyd der allgemeinen Formel (XXI) umgesetzt werden.
   3) Acylierung einer Verbindung der allgemeinen Formel 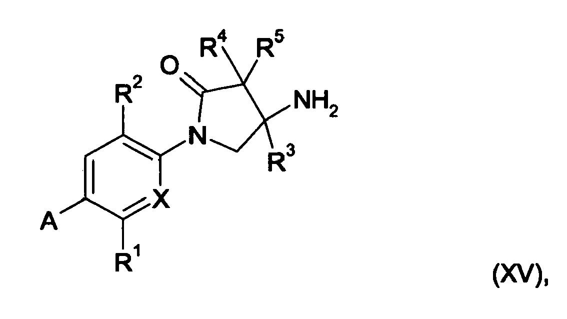 in der A, X, R¹ bis R⁵ wie vorhin definiert sind, mit einer Carbonsäure oder einem reaktiven Carbonsäurederivat der allgemeinen Formel 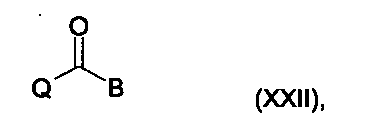 in der B wie vorhin definiert ist und Q eine Hydroxy- oder C₁₋₄-Alkoxygruppe, ein Halogenatom oder eine Acyloxygruppe darstellt.

Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 160°C, durchgeführt.

Die Acylierung kann jedoch auch mit der freien Säure gegebenenfalls in Gegenwart eines die Säure aktivierenden Mittels oder eines wasserentziehenden Mittels, beispielsweise in Gegenwart von Chlorameisensäureisobutylester, Thionylchlorid, Trimethylchlorsilan, Chlorwasserstoff, Schwefelsäure, Methansulfonsäure, p-Toluolsulfonsäure, Phosphortrichlorid, Phosphorpentoxid, *N*,*N*'-Dicyclohexylcarbodiimid, *N*,*N*'-Dicyclohexylcarbodiimid/*N*-Hydroxysuccinimid oder 1-Hydroxy-benztriazol, *N*,*N*'-Carbonyldiimidazol, *N*,*N*'-Carbonylditriazol, *O*-(Benzotriazol-1-yl)-*N*,*N*,*N*',*N*'-tetramethyl-uroniumtetrafluorborat/*N*-Methylmorpholin, *O*-(Benzotriazol-1-yl)-*N*,*N*,*N*',*N*'-tetramethyl-uroniumtetrafluorborat/N-Ethyldüsopropylamin, *O*-Pentafluorophenyl-*N*,*N*,*N*',*N*-tetramethyluronium-hexafluorophosphat/Triethylamin, *N*,*N*'-Thionyldiimidazol oder Triphenylphosphin/Tetrachlorkohlenstoff, bei Temperaturen zwischen -20 und 200°C, vorzugsweise jedoch bei Temperaturen zwischen -10 und 160°C, durchgeführt werden.

Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995 beschrieben.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden. Beispielsweise kommt als Schutzrest für eine Hydroxygruppe die Methoxy-, Benzyloxy-, Trimethylsilyl-, Acetyl-, Benzoyl-, *tert*.-Butyl-, Trityl-, Benzyl- oder Tetrahydropyranylgruppe,
als Schutzreste für eine Carboxylgruppe die Trimethylsilyl-, Methyl-, Ethyl-, *tert.-*Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, *tert*.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Weitere Schutzgruppen und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von Iodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines *tert*.-Butyl- oder *tert*.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Ferner können die erhaltenen Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel I sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und auf einer Hemmwirkung auf verwandte Serinproteasen wie z. B. Urokinase, Faktor VIIa, Faktor IX, Faktor XI und Faktor XII.

Die im Experimentellen Teil angeführten Verbindungen wurden auf ihre Wirkung auf die Hemmung des Faktors Xa wie folgt untersucht:

### Methodik:

Enzymkinetische Messung mit chromogenem Substrat. Die durch humanen Faktor Xa aus dem farblosen chromogenen Substrat freigesetzte Menge an p-Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material:

Tris(hydroxymethyl)-aminomethan-Puffer (100 mMol) und Natriumchlorid (150 mMol), pH 8.0 plus 1 mg/ml Human Albumin Fraction V, Proteasefrei
Faktor Xa (Calbiochem), Spez. Aktivität: 217 IU/mg, Endkonzentration: 7 IU/ml pro Reaktionsansatz
Substrat S 2765 (Chromogenix), Endkonzentration: 0.3 mMol/l (1 KM) pro Reaktionsansatz
Testsubstanz: Endkonzentration 100, 30, 10, 3, 1, 0.3, 0.1, 0.03, 0.01, 0.003, 0.001 µMol/l
Durchführung: 10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Auswertung:

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigten IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulcerativen Colitis, der Prophylaxe und Behandlung der Koronarthrombose, der Prophylaxe des Schlaganfalls und der Verhinderung der Occlusion von Shunts. Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von Fibrin-abhängigen Gewebsadhäsionen und/oder Narbengewebebildung sowie zur Förderung von Wundheilungsprozessen geeignet. Die neuen Verbindungen und deren physiologisch verträgliche Salze können therapeutisch in Kombination mit Acetylsalicylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern, ohne diese jedoch in ihrem Umfang zu beschränken:

Für die hergestellten Verbindungen liegen in der Regel Schmelzpunkte, IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX^{™}, 35-70 µm) verwendet. Falls nähere Angaben zur Konfiguration fehlen, bleibt offen, ob es sich um reine Stereoisomere oder um Enantiomeren-/Diastereomerengemische handelt.

In den Versuchsbeschreibungen werden die folgenden Abkürzungen verwendet:
- Boc: *tert*.-Butoxycarbonyl
- DCC: *N*,*N*'-Dicyclohexylcarbodiimid
- DIPEA: *N*-Ethyl-diisopropylamin
- DMSO: Dimethylsulfoxid
- DMF: *N*,*N*-Dimethylformamid
- DPPA: Diphenylphosphorylazid
- ges.: gesättigt
- i. Vak.: im Vakuum
- konz.: Konzentriert
- NMM: *N*-Methyl-morpholin
- NMP: *N*-Methyl-pyrrolidin-2-on
- *o*: ortho
- PfTU: *O*-Pentafluorophenyl-*N*,*N*,*N*',*N*'-tetramethyluroniumhexafluorophosphat
- PPA: Propanphosphonsäurecycloanhydrid
- quant.: quantitativ
- R_{f}: Retentionsfaktor
- Rₜ: Retentionszeit
- *rac.*: Racemisch
- TBTU: *O*-(Benzotriazol-1-yl)-*N*,*N*,*N*',*N*'-tetramethyluroniumtetrafluorborat
- TEA: Triethylamin
- TFA: Trifluoressigsäure
- THF: Tetrahydrofuran
- *tert.*: tertiär
- Σ: Ausbeute über alle analog durchgeführten beschriebenen Stufen

Die HPLC/MS-Daten für die Beispiele 2 bis 82 wurden unter den folgenden Bedingungen erzeugt:
Waters ZQ2000 Massenspektrometer (Massenbereich: 120-1000 m/z), HP1100 HPLC + DAD, Gilson 215 Autosampler
Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.0 | 95 | 5 | 1.00 |
| 0.4 | 95 | 5 | 1.00 |
| 4.0 | 2 | 98 | 1.00 |
| 4.35 | 2 | 98 | 1.00 |
| 4.5 | 95 | 5 | 1.00 |

Als stationäre Phase diente eine Säule XTerra®, MS C₁₈ 3.5 µm, 4.6 mm x 50 mm (Säulentemperatur: konstant bei 40°C).

Die Diodenarraydetektion erfolgte im Wellenlängenbereich 210-500 nm.

Die HPLC-Daten für alle anderen Beispiele wurden unter den folgenden Bedingungen erzeugt:
Waters ZMD, Alliance 2695 HPLC, Waters 2700 Autosampler, Waters 2996 Diodenarraydetektor
Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.0 | 95 | 5 | 1.00 |
| 0.1 | 95 | 5 | 1.00 |
| 3.1 | 2 | 98 | 1.00 |
| 4.5 | 2 | 98 | 1.00 |
| 5.0 | 95 | 5 | 1.00 |

Als stationäre Phase diente eine Säule XTerra®, MS C₁₈ 2.5 µm, 4.6 mm x 30 mm (Säulentemperatur: konstant bei 25°C).

Die Diodenarraydetektion erfolgte im Wellenlängenbereich 210-300 nm.

### Beispiel 1

### 5-Brom-thiophen-2-carbonsäure-{1-[3-methyl-4-(3-oxo-piperazin-1-carbonyl)-phenyl]-5-oxo-pyrrolidin-3-yl}-amid

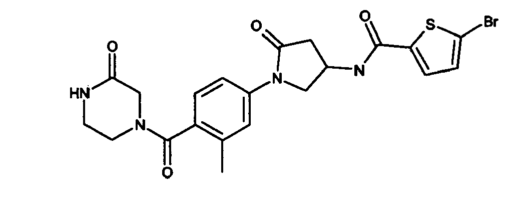

### (a) 4-Amino-2-methyl-benzoesäuremethylester

15 g (78 mmol) 4-Acetamido-2-methyl-benzoesäure werden in 150 ml Methanol suspendiert und mit 11.2 ml (210 mmol) konz. Schwefelsäure versetzt. Man erhitzt für drei Stunden unter Rückfluß. Anschließend enfernt man im Vakuum überschüssiges Methanol. Der Rückstand wird auf Eiswasser gegossen, mit 5 N Natronlauge alkalisch gestellt und zweimal mit Ethylacetat extrahiert. Die vereinten organischen Phasen werden über Natriumsulfat getrocknet und zur Trockene eingeengt.
Ausbeute: quantitativ
Rₜ-Wert: 2.0 min
C₉H₁₁NO₂ (165.19)
Massenspektrum: (M+H)⁺ = 166

### (b) 1-(4-Methoxycarbonyl-3-methyl-phenyl)-5-oxo-pyrrolidin-3-carbonsäure

10 g (60.5 mmol) 4-Amino-2-methyl-benzoesäuremethylester und 7.9 g (60.5 mmol) Itaconsäure werden in 50 ml Xylol suspendiert und sieben Stunden unter Rückfluß erhitzt. Man kühlt die Reaktionsmischung auf Raumtemperatur ab und engt zur Trockene ein. Der Rückstand wird in Methanol suspendiert. Man filtriert den nichtgelösten Feststoff ab und trocknet zur Gewichtskonstanz.
Ausbeute: 6.9 g (42 %)
Rₜ-Wert: 2.53 min
C₁₄H₁₅NO₅ (277.27)
Massenspektrum: (M+H)⁺ = 278

### (c) 4-(4-tert.-butoxycarbonylamino-2-oxo-pyrrolidin-1-yl)-2-methyl-benzoesäuremethylester

3 g (70%ig, 7.6 mmol) 1-(4-Methoxycarbonyl-3-methyl-phenyl)-5-oxo-pyrrolidin-3-carbonsäure werden in 105 ml tert.-Butanol suspendiert, mit 1.05 ml (7.6 mmol) Triethylamin und anschließend mit 1.7 ml (7.6 mmol) DPPA versetzt. Die Mischung wird fünf Stunden unter Rückfluß, dann 2 Tage bei Raumtemperatur, gerührt. Man engt zur Trockene ein und reinigt den Rückstand chromatographisch an Kieselgel (Eluens: Dichlormethan/Isopropanol 95:5) Ausbeute: 2.38 g (75%ig, 68 % korrigierte Ausbeute)
Rₜ-Wert: 3.02 min
C₁₈H₂₄N₂O₅ (348.39)
Massenspektrum: (M+H)⁺ = 349

### (d) 4-(4-Amino-2-oxo-pyrrolidin-1-yl)-2-methyl-benzoesäuremethylester Trifluoracetat

1.9 g (75%ig, 5.5 mmol) 4-(4-tert.-butoxycarbonylamino-2-oxo-pyrrolidin-1-yl)-2-methyl-benzoesäuremethylester werden in 25 ml Dichlormethan gelöst und mit 4.4 ml TFA versetzt. Man rührt 18 Stunden bei Raumtemperatur, engt dann zur Trockene ein und reinigt den Rückstand chromatographisch an Kieselgel (Eluens: Gradient Dichlormethan/Isopropanol/Ammoniak 90:10:0.2 - Dichlormethan/Methanol/Ammoniak 50:50:0.4).
Ausbeute: quantitativ
Rₜ-Wert: 2.0 min
C₁₃H₁₆N₂O₃ (248.28)
Massenspektrum: (M+H)⁺ = 249

### (e) 4-{4-[(5-Brom-thiophen-2-carbonyl)-aminol]-2-oxo-pyrrolidin-1-yl}-2-methyl-benzoesäuremethylester

1.1 g (5.3 mmol) 5-Brom-thiophen-2-carbonsäure werden in 10 ml DMF mit 4.2 ml (38.2 mmol) NMM und 1.7 g (5.2 mmol) TBTU versetzt und anschließend unter Stickstoff-Atmosphäre bei Raumtemperatur für 10 min gerührt. Dann werden 2.5 g (5.2 mmol) 4-(4-Amino-2-oxo-pyrrolidin-1-yl)-2-methyl-benzoesäuremethylester-Trifuoracetat in 10 ml DMF gelöst zugefügt und 16 Stunden bei Raumtemperatur gerührt. Danach wird die Reaktionsmischung mit ges. Natriumhydrogencarbonat-Lösung und Wasser versetzt und mit Ethylacetat extrahiert. Die wässrige Phase wird zweimal mit Ethylacetat ausgeschüttelt. Die vereinigten organischen Phasen werden über Natriumsulfat getrocknet und i. Vak. vollständig eingeengt. Der Rückstand wird an Kieselgel chromatographiert (Eluens: Petrolether/Ethylacetat 3:2).
Ausbeute: 1.6 g (70 %)
Rₜ-Wert: 3.17 min
C₁₈H₁₇BrN₂O₄S(437.31)
Massenspektrum: (M+H)⁺ = 437/439 (Bromisotope)

### (f) 4-{4-[(5-Brom-thiophen-2-carbonyl)-amino]-2-oxo-pyrrolidin-1-yl}-2-methyl-benzoesäure

1.98 g (4.54 mmol) 4-{4-[(5-Brom-thiophen-2-carbonyl)-amino]-2-oxo-pyrrolidin-1-yl}-2-methyl-benzoesäuremethylester werden in 10 ml Ethanol suspendiert, mit 8.1 ml (27 mmol) wässriger 8%iger Lithiumhydroxid-Lösung versetzt und drei Tage bei Raumtemperatur gerührt. Man engt die Mischung auf ein Drittel des Volumens ein und versetzt mit Wasser, Ethylacetat und gibt 2 N Salzsäure zu, bis ein pH-Wert von 5 erreicht wird. Man extrahiert die wässrige Phase zweimal mit Ethylacetat. Die vereinigten organischen Phasen werden über Natriumsulfat getrocknet und zur Trockene eingeengt. Der Rückstand wird chromatographisch an Kieselgel gereinigt (Eluens: Gradient Dichlormethan/Isopropanol 9:1 -0:100).
Ausbeute: 160 mg (8 %)
Rₜ-Wert: 2.81 min
C₁₇H₁₅BrN₂O₄S (423.28)
Massenspektrum: (M+H)⁺ = 423/425 (Bromisotope)

### (g) 5-Brom-thiophen-2-carbonsäure-{1-[3-methyl-4-(3-oxo-piperazin-1-carbonyl)-phenyl]-5-oxo-pyrrolidin-3-yl}-amid

Hergestellt analog Beispiel 1e aus 4-{4-[(5-Brom-thiophen-2-carbonyl)-amino]-2-oxo-pyrrolidin-1-yl}-2-methyl-benzoesäure und Piperazin-2-on mit TBTU und NMM in DMF und anschließender Reinigung durch Chromatographie (Kieselgel, Eluens: Dichlormethan/Isopropanol 95:5).
Ausbeute: 84 %
R_{f}-Wert: 0.28 (Kieselgel; Dichlormethan/Isopropanol 9:1)
C₂₁H₂₁BrN₄O₄S (505.39)
Massenspektrum: (M+H)⁺ = 505/507 (Bromisotope)

Analog wurden folgenden Verbindungen hergestellt:

| **Nr.** | **Strukturformel** | **Massenpeak(s)** | **R_{f-}Wert bzw. Rₜ** |
|---|---|---|---|
| | Name | | |
| **2** | 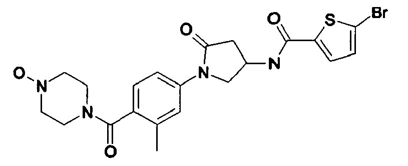 | (M⁺H)⁺ = 508.42 | 3.20 min |
| | 5-Brom-thiophen-2-carbonsäure-{1-[4-(4-hydroxy-piperazin-1-carbonyl)-3-methyl-phenyl]-5-oxo-pyrrolidin-3-yl}-amid | | |
| **3** | 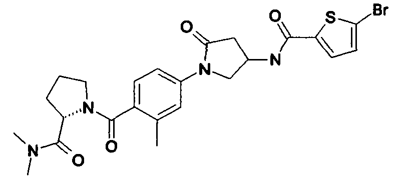 | (M⁺H)⁺ = 548.48 | 3.55 min |
| | (2S)-1-(4-{4-[(5-Brom-thiophen-2-carbonyl)-amino]-2-oxo-pyrrolidin-1-yl}-2-methyl-benzoyl)-pyrrolidin-2-carbonsäuredimethylamid | | |
| **4** | 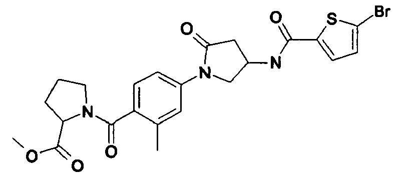 | (M+H)⁺ = 535.44 | 3.81 min |
| | 1-(4-{4-[(5-Brom-thiophen-2-carbonyl)-amino]-2-oxo-pyrrolidin-1-yl}-2-methyl-benzoyl)-pyrrolidin-2-carbonsäuremethylester | | |
| **5** | 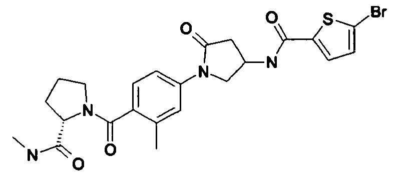 | (M+H)⁺ = 534.46 | 3.44 min |
| | (2S)-1-(4-{4-[(5-Brom-thiophen-2-carbonyl)-amino]-2-oxo-pyrrolidin-1-yl}-2-methyl-benzoyl)-pyrrolidin-2-carbonsäuremethylamid | | |
| **6** | 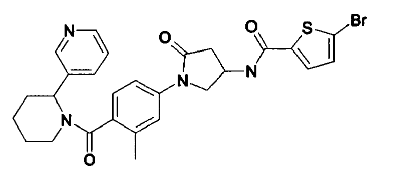 | (M+H)⁺ = 568.52 | 3.34 min |
| | 5-Brom-thiophen-2-carbonsäure-{1-[3-methyl-4-(3,4,5,6-tetrahydro-2H-[2,3']bipyridinyl-1-carbonyl)-phenyl]-5-oxo-pyrrolidin-3-yl}-amid | | |
| **7** | 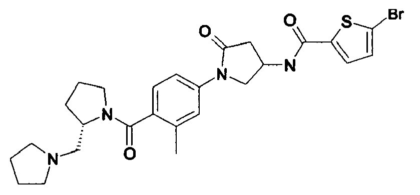 | (M+H)⁺ = 560.54 | 3.32 min |
| | 5-Brom-thiophen-2-carbonsäure-{1-[3-methyl-4-((2S)-2-pyrrolidin-1-ylmethyl-pyrrolidin-1-carbonyl)-phenyl]-5-oxo-pyrrolidin-3-yl}-amid | | |
| **8** | 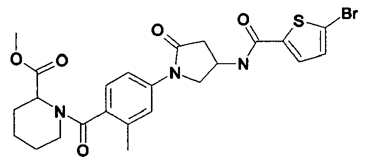 | (M+H)⁺ = 549.47 | 4.03 min |
| | 1-(4-{4-[(5-Brom-thiophen-2-carbonyl)-amino]-2-oxo-pyrrolidin-1-yl}-2-methyl-benzoyl)-piperidin-2-carbonsäuremethylester | | |
| **9** | 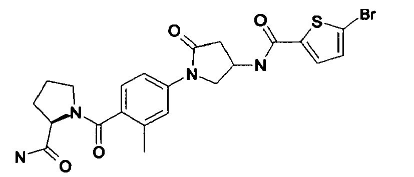 | (M⁺H)⁺ = 520.43 | 3.39 min |
| | (2*R*)-1-(4-{4-[(5-Brom-thiophen-2-carbonyl)-amino]-2-oxo-pyrrolidin-1-yl}-2-methyl-benzoyl)-pyrrolidin-2-carbonsäureamid | | |
| **10** | 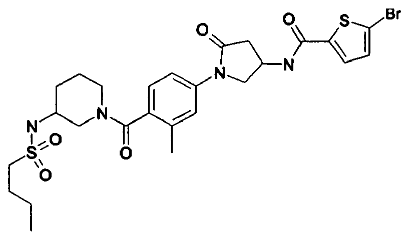 | (M+H)⁺ = 626.62 | 3.90 min |
| | 5-Brom-thiophen-2-carbonsäure-(1-{4-[3-(butan-1-sulfonylamino)-piperidin-1-carbonyl]-3-methyl-phenyl}-5-oxo-pyrrolidin-3-yl)-amid | | |
| **11** | 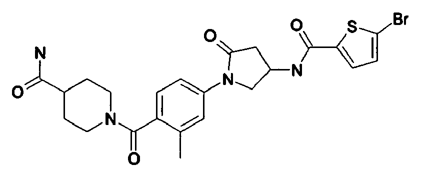 | (M⁺H)⁺ = 534.46 | 3.35 min |
| | 1-(4-{4-[(5-Brom-thiophen-2-carbonyl)-amino]-2-oxo-pyrrolidin-1-yl}-2-methyl-benzoyl)-piperidin-4-carbonsäureamid | | |
| **12** | 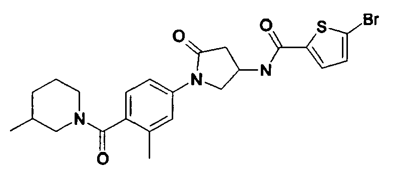 | (M+H)⁺ = 505.46 | 4.13 min |
| | 5-Brom-thiophen-2-carbonsäure-{1-[3-methyl-4-(3-methyl-piperidin-1-carbonyl)-phenyl]-5-oxo-pyrrolidin-3-yl}-amid | | |
| **13** | 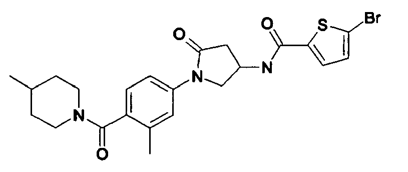 | (M⁺H)⁺ = 505.46 | 4.14 min |
| | 5-Brom-thiophen-2-carbonsäure-{1-[3-methyl-4-(4-methyl-piperidin-1-carbonyl)-phenyl]-5-oxo-pyrrolidin-3-yl}-amid | | |
| **14** | 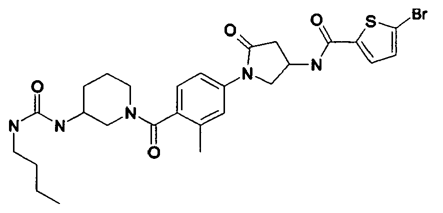 | (M+H)⁺ = 605.58 | 3.81 min |
| | 5-Brom-thiophen-2-carbonsäure-(1-{4-[3-(3-butyl-ureido)-piperidin-1-carbonyl]-3-methyl-phenyl}-5-oxo-pyrrolidin-3-yl)-amid | | |
| **15** | 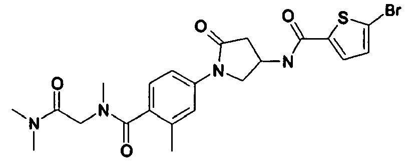 | (M⁺H)⁺ = 522.44 | 3.48 min |
| | 5-Brom-thiophen-2-carbonsäure-{1-[4-(dimethylcarbamoylmethyl-methyl-carbamoyl)-3-methyl-phenyl]-5-oxo-pyrrolidin-3-yl}-amid | | |
| **16** | 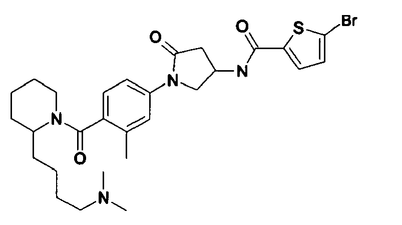 | (M⁺H)⁺ = 590.61 | 3.42 min |
| | 5-Brom-thiophen-2-carbonsäure-(1-{4-[2-(4-dimethylamino-butyl)-piperidin-1-carbonyl]-3-methyl-pheny}-5-oxo-pyrrolidin-3-yl)-amid | | |
| **17** | 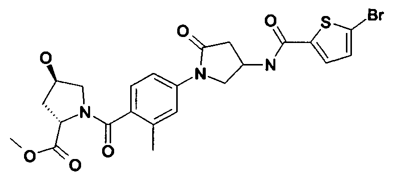 | (M⁺H)⁺ = 551.44 | 3.50 min |
| | (2*S*,4*R*)-1-(4-{4-[(5-Brom-thiophen-2-carbonyl)-amino]-2-oxo-pyrrolidin-1-yl}-2-methyl-benzoyl)-4-hydroxy-pyrrolidin-2-carbonsäuremethylester | | |
| **18** | 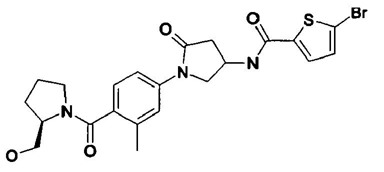 | (M⁺H)⁺ = 507.43 | 3.55 min |
| | 5-Brom-thiophen-2-carbonsäure-{1-[4-(R-2-hydroxymethyl-pyrrolidin-1-carbonyl)-3-methyl-phenyl]-5-oxo-pyrrolidin-3-yl}-amid | | |
| **19** | 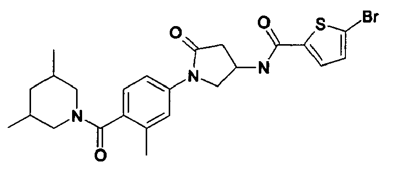 | (M⁺H)⁺ = 519.48 | 4.31 min |
| | 5-Brom-thiophen-2-carbonsäure-{1-[4-(3,5-dimethyl-piperidin-1-carbonyl)-3-methyl-phenyl]-5-oxo-pyrrolidin-3-yl}-amid | | |
| **20** | 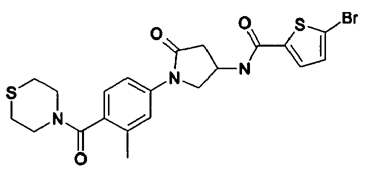 | (M+H)⁺ = 509.47 | 3.89 min |
| | 5-Brom-thiophen-2-carbonsäure-{1-[3-methyl-4-(thiomorpholin-4-carbonyl)-phenyl]-5-oxo-pyrrolidin-3-yl}-amid | | |
| **21** | 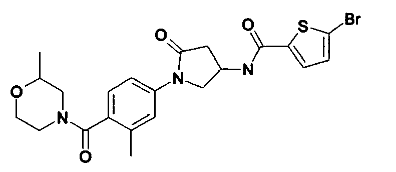 | (M⁺H)⁺ = 507.43 | 3.73 min |
| | 5-Brom-thiophen-2-carbonsäure-{1-[3-methyl-4-(2-methyl-morpholin-4-carbonyl)-phenyl]-5-oxo-pyrrolidin-3-yl}-amid | | |
| **22** | 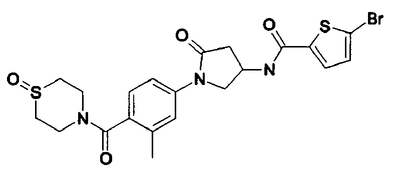 | (M+H)⁺ = 525.47 | 3.34 min |
| | 5-Brom-thiophen-2-carbonsäure-{1-[3-methyl-4-(1-oxo-1λ⁴-thiomorpholin-4-carbonyl)-phenyl]-5-oxo-pyrrolidin-3-yl}-amid | | |
| **23** | 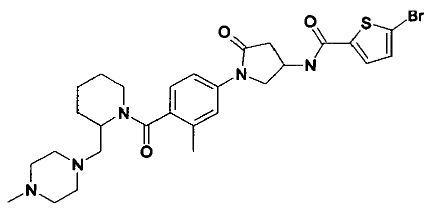 | (M⁺H)⁺ = 603.60 | 3.26 min |
| | 5-Brom-thiophen-2-carbonsäure-(1-{3-methyl-4-[2-(4-methyl-piperazin-1-ylmethyl)-piperidin-1-carbonyl]-phenyl}-5-oxo-pyrrolidin-3-yl)-amid | | |
| **24** | 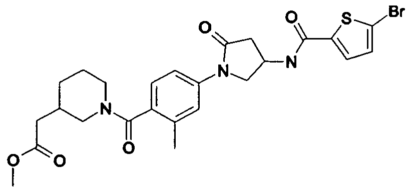 | (M⁺H)⁺ = 563.49 | 3.91 min |
| | 1-(4-{4-[(5-Brom-thiophen-2-carbonyl)-amino]-2-oxo-pyrrolidin-1-yl}-2-methyl-benzoyl)-piperidin-3-yl]essigsäuremethylester | | |
| **25** | 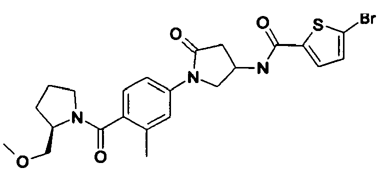 | (M+H)⁺ = 521.46 | 3.85 min |
| | 5-Brom-thiophen-2-carbonsäure-{1-[4-((2*R*)-2-methoxymethyl-pyrrolidin-1-carbonyl)-3-methyl-phenyl]-5-oxo-pyrrolidin-3-yl}-amid | | |
| **26** | 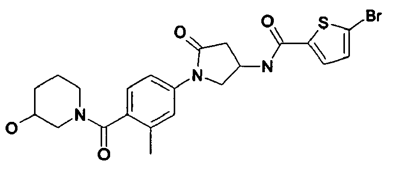 | (M⁺H)⁺ = 507.43 | 3.50 min |
| | 5-Brom-thiophen-2-carbonsäure-{1-[4-(3-hydroxy-piperidin-1-carbonyl)-3-methyl-phenyl]-5-oxo-pyrrolidin-3-yl}-amid | | |
| **27** | 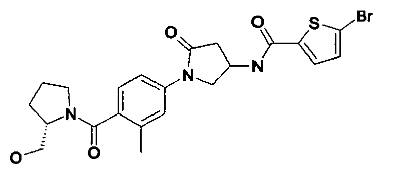 | (M+H)⁺ = 507.43 | 3.55 min |
| | 5-Brom-thiophen-2-carbonsäure-{1-[4-((2S)-2-hydroxymethyl-pyrrolidin-1-carbonyl)-3-methyl-phenyl]-5-oxo-pyrrolidin-3-yl}-amid | | |
| **28** | 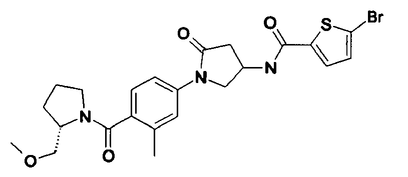 | (M+H)⁺ = 521.46 | 3.86 min |
| | 5-Brom-thiophen-2-carbonsäure-{1-[4-((2S)-2-methoxymethyl-pyrrolidin-1-carbonyl)-3-methyl-phenyl]-5-oxo-pyrrolidin-3-yl}-amid | | |
| **29** | 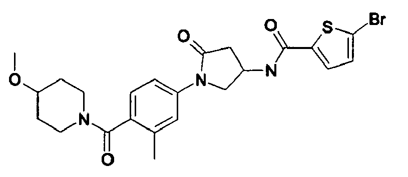 | (M+H)⁺ = 521.46 | 3.74 min |
| | 5-Brom-thiophen-2-carbonsäure-{1-[4-(4-methoxy-piperidin-1-carbonyl)-3-methyl-phenyl]-5-oxo-pyrrolidin-3-yl}-amid | | |
| **30** | 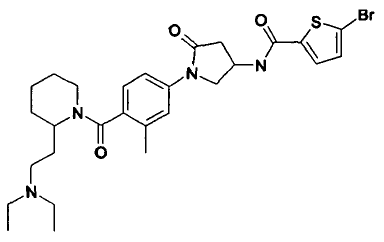 | (M+H)⁺ = 590.61 | 3.40 min |
| | 5-Brom-thiophen-2-carbonsäure-(1-{4-[2-(2-diethylamino-ethyl)-piperidin-1-carbonyl]-3-methyl-phenyl}-5-oxo-pyrrolidin-3-yl)-amid | | |
| **31** | 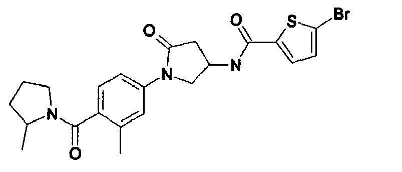 | (M+H)⁺ = 491.43 | 3.90 min |
| | 5-Brom-thiophen-2-carbonsäure-{1-[3-methyl-4-(2-methyl-pyrrolidin-1-carbonyl)-phenyl]-5-oxo-pyrrolidin-3-yl}-amid | | |
| **32** | 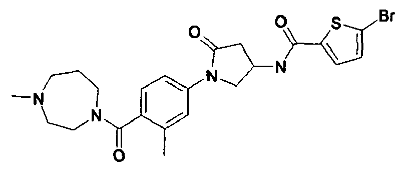 | (M⁺H)⁺ = 520.47 | 3.14 min |
| | 5-Brom-thiophen-2-carbonsäure-{1-[3-methyl-4-(4-methyl-[1,4]diazepan-1-carbonyl)-phenyl]-5-oxo-pyrrolidin-3-yl}-amid | | |
| **33** | 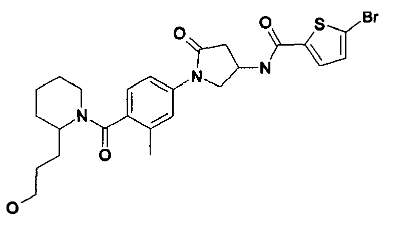 | (M+H)⁺ = 549.51 | 3.74 min |
| | 5-Brom-thiophen-2-carbonsäure-(1-{4-[2-(3-hydroxy-propyl)-piperidin-1-carbonyl]-3-methyl-phenyl}-5-oxo-pyrrolidin-3-yl)-amid | | |
| **34** | 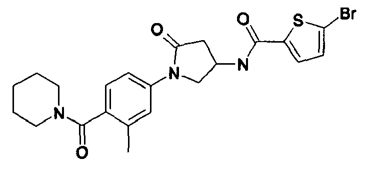 | (M⁺H)⁺ = 491.43 | 3.95 min |
| | 5-Brom-thiophen-2-carbonsäure-{1-[3-methyl-4-(piperidin-1-carbonyl)-phenyl]-5-oxo-pyrrolidin-3-yl}-amid | | |
| **35** | 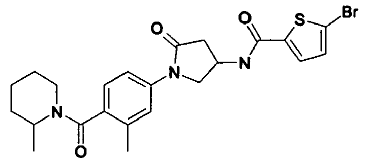 | (M⁺H)⁺ = 505.46 | 4.07 min |
| | 5-Brom-thiophen-2-carbonsäure-{1-[3-methyl-4-(2-methyl-piperidin-1-carbonyl)-phenyl]-5-oxo-pyrrolidin-3-yl}-amid | | |
| **36** | 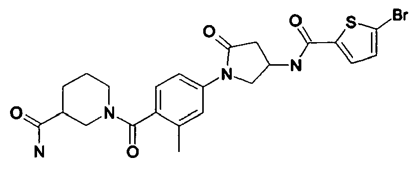 | (M+H)⁺ = 534.46 | 3.42 min |
| | 1-(4-{4-[(5-Brom-thiophen-2-carbonyl)-amino]-2-oxo-pyrrolidin-1-yl}-2-methyl-benzoyl)-piperidin-3-carbonsäureamid | | |
| **37** | 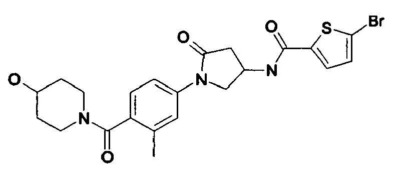 | (M⁺H)⁺ = 507.43 | 3.42 min |
| | 5-Brom-thiophen-2-carbonsäure-{1-[4-(4-hydroxy-piperidin-1-carbonyl)-3-methyl-phenyl]-5-oxo-pyrrolidin-3-yl}-amid | | |
| **38** | 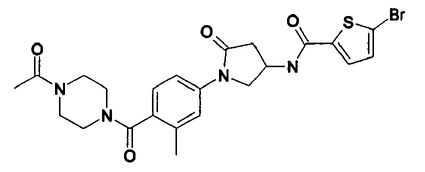 | (M⁺H)⁺ = 534.46 | 3.44 min |
| | 5-Brom-thiophen-2-carbonsäure-{1-[4-(4-acetyl-piperazin-1-carbonyl)-3-methyl-phenyl]-5-oxo-pyrrolidin-3-yl}-amid | | |
| **39** | 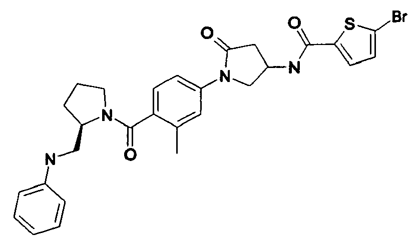 | (M⁺H)⁺ = 582.54 | 3.79 min |
| | 5-Brom-thiophen-2-carbonsäure-{1-[3-methyl-4-((2*R*)-2-phenylaminomethyl-pyrrolidin-1-carbonyl)-phenyl]-5-oxo-pyrrolidin-3-yl}-amid | | |
| **40** | 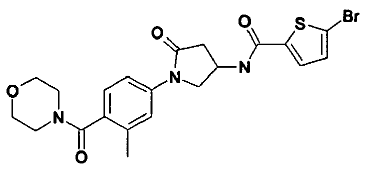 | (M⁺H)⁺ = 493.40 | 3.59 min |
| | 5-Brom-thiophen-2-carbonsäure-{1-[3-methyl-4-(morpholin-4-carbonyl)-phenyl]-5-oxo-pyrrolidin-3-yl}-amid | | |
| **41** | 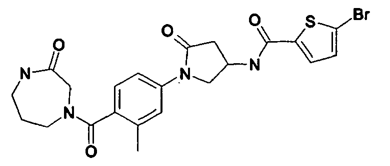 | (M⁺H)⁺ = 520.43 | 3.35 min |
| | 5-Brom-thiophen-2-carbonsäure-{1-[3-methyl-4-(3-oxo-[1,4]diazepan-1-carbonyl)-phenyl]-5-oxo-pyrrolidin-3-yl}-amid | | |
| **42** | 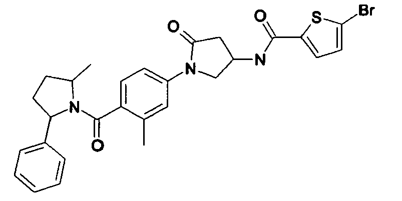 | (M⁺H)⁺ = 567.53 | 4.38 min |
| | 5-Brom-thiophen-2-carbonsäure-{1-[3-methyl-4-(2-methyl-5-phenyl-pyrrolidin-1-carbonyl)-phenyl]-5-oxo-pyrrolidin-3-yl}-amid | | |
| **43** | 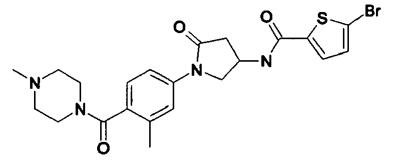 | (M⁺H)⁺ = 506.45 | 3.14 min |
| | 5-Brom-thiophen-2-carbonsäure-{1-[3-methyl-4-(4-methyl-piperazin-1-carbonyl)-phenyl]-5-oxo-pyrrolidin-3-yl}-amid | | |
| **44** | 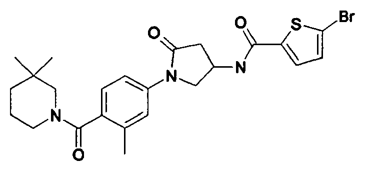 | (M⁺H)⁺ = 519.48 | 4.23 min |
| | 5-Brom-thiophen-2-carbonsäure-{1-[4-(3,3-dimethyl-piperidin-1-carbonyl)-3-methyl-phenyl]-5-oxo-pyrrolidin-3-yl}-amid | | |
| **45** | 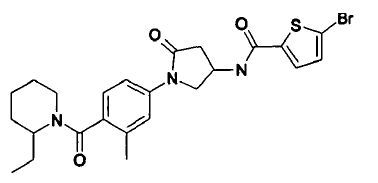 | (M+H)⁺ = 519.48 | 4.22 min |
| | 5-Brom-thiophen-2-carbonsäure-{1-[4-(2-ethyl-piperidin-1-carbonyl)-3-methyl-phenyl]-5-oxo-pyrrolidin-3-yl}-amid | | |
| **46** | 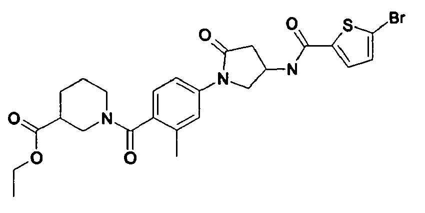 | (M⁺H)⁺ = 563.49 | 4.03 min |
| | 1-(4-{4-[(5-Brom-thiophen-2-carbonyl)-amino]-2-oxo-pyrrolidin-1-yl}-2-methyl-benzoyl)-piperidin-3-carbonsäureethylester | | |
| **47** | 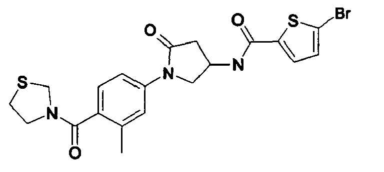 | (M+H)⁺ = 495.44 | 3.85 min |
| | 5-Brom-thiophen-2-carbonsäure-{1-[3-methyl-4-(thiazolidin-3-carbonyl)-phenyl]-5-oxo-pyrrolidin-3-yl}-amid | | |
| **48** | 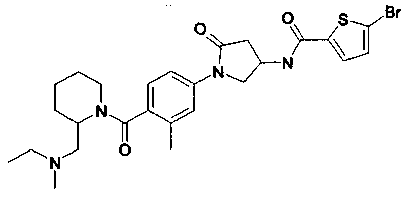 | (M+H)⁺ = 562.55 | 3.34 min |
| | 5-Brom-thiophen-2-carbonsäure-[1-(4-{2-[(ethyl-methyl-amino)-methyl]-piperidin-1-carbonyl}-3-methyl-phenyl)-5-oxo-pyrrolidin-3-yl]-amid | | |
| **49** | 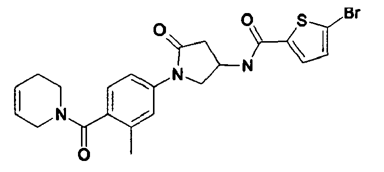 | (M⁺H)⁺ = 489.41 | 3.91 min |
| | 5-Brom-thiophen-2-carbonsäure-{1-[4-(3,6-dihydro-2H-pyridin-1-carbonyl)-3-methyl-phenyl]-5-oxo-pyrrolidin-3-yl}-amid | | |
| **50** | 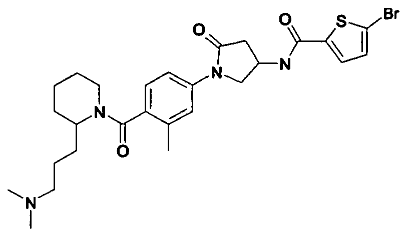 | (M⁺H)⁺ = 576.58 | 3.36 min |
| | 5-Brom-thiophen-2-carbonsäure-(1-{4-[2-(3-dimethylamino-propyl)-piperidin-1-carbonyl]-3-methyl-phenyl}-5-oxo-pyrrolidin-3-yl)-amid | | |
| **51** | 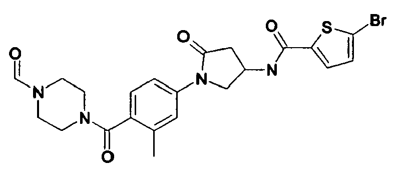 | (M⁺H)⁺ = 520.43 | 3.43 min |
| | 5-Brom-thiophen-2-carbonsäure-{1-[4-(4-formyl-piperazin-1-carbonyl)-3-methyl-phenyl]-5-oxo-pyrrolidin-3-yl}-amid | | |
| **52** | 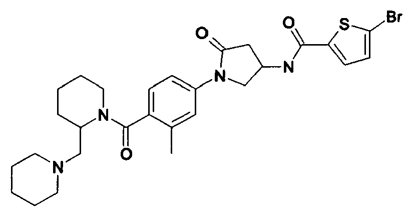 | (M⁺H)⁺ = 588.59 | 3.39 min |
| | 5-Brom-thiophen-2-carbonsäure-{1-[3-methyl-4-(2-piperidin-1-ylmethyl-piperidin-1-carbonyl)-phenyl]-5-oxo-pyrrolidin-3-yl}-amid | | |
| **53** | 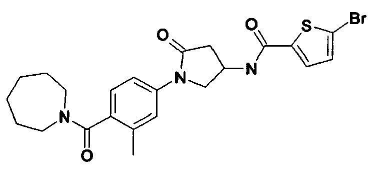 | (M⁺H)⁺ = 505.46 | 4.06 min |
| | 5-Brom-thiophen-2-carbonsäure-{1-[4-(azepan-1-carbonyl)-3-methyl-phenyl]-5-oxo-pyrrolidin-3-yl}-amid | | |
| **54** | 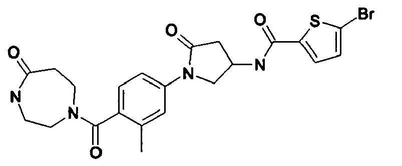 | (M+H)⁺ = 520.43 | 3.34 min |
| | 5-Brom-thiophen-2-carbonsäure-{1-[3-methyl-4-(5-oxo-[1,4]diazepan-1-carbonyl)-phenyl]-5-oxo-pyrrolidin-3-yl}-amid | | |
| **55** | 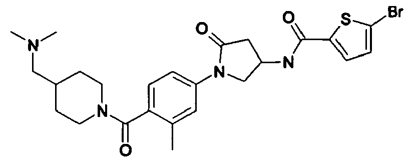 | (M⁺H)⁺ = 548.53 | 3.16 min |
| | 5-Brom-thiophen-2-carbonsäure-{1-[4-(4-dimethylamino-piperidin-1-carbonyl)-3-methyl-phenyl]-5-oxo-pyrrolidin-3-yl}-amid | | |
| **56** | 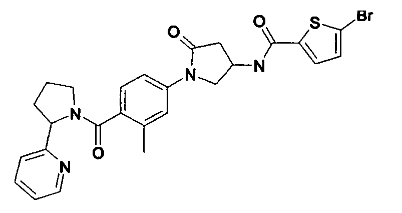 | (M⁺H)⁺ = 554.49 | 3.30 min |
| | 5-Brom-thiophen-2-carbonsäure-{1-[3-methyl-.4-(2-pyridin-2-yl-pyrrolidin-1-carbonyl)-phenyl]-5-oxo-pyrrolidin-3-yl}-amid | | |
| **57** | 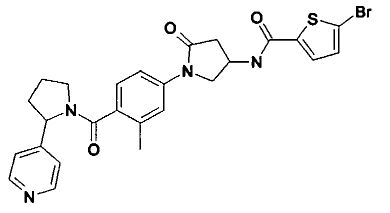 | (M⁺H)⁺ = 554.49 | 3.24 min |
| | 5-Brom-thiophen-2-carbonsäure-{1-[3-methyl-4-(2-pyridin-4-yl-pyrrolidin-1-carbonyl)-phenyl]-5-oxo-pyrrolidin-3-yl}-amid | | |
| **58** | 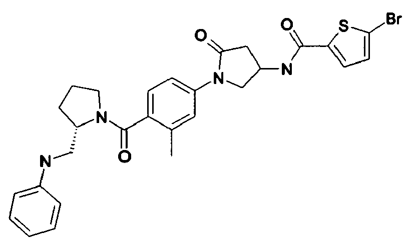 | (M⁺H)⁺ = 582.54 | 3.79 min |
| | 5-Brom-thiophen-2-carbonsäure-{1-[3-methyl-4-((2S)-2-phenylaminomethyl-pyrrolidin-1-carbonyl)-phenyl]-5-oxo-pyrrolidin-3-yl}-amid | | |
| **59** | 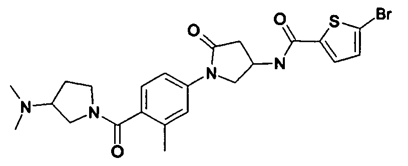 | (M⁺H)⁺ = 520.47 | 3.12 min |
| | 5-Brom-thiophen-2-carbonsäure-{1-[4-(3-dimethylamino-pyrrolidin-1-carbonyl)-3-methyl-phenyl]-5-oxo-pyrrolidin-3-yl}-amid | | |
| **60** | 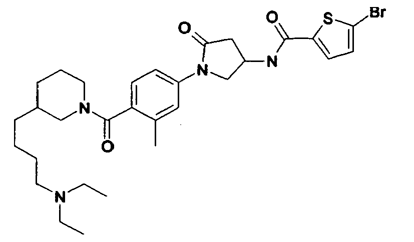 | (M⁺H)⁺ = 618.66 | 3.47 min |
| | 5-Brom-thiophen-2-carbonsäure-(1-{4-[3-(4-diethylamino-butyl)-piperidin-1-carbonyl]-3-methyl-phenyl}-5-oxo-pyrrolidin-3-yl)-amid | | |
| **61** | 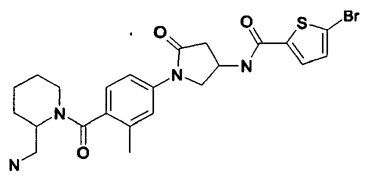 | (M⁺H)⁺ = 520.47 | 3.27 min |
| | 5-Brom-thiophen-2-carbonsäure-{1-[4-(2-aminomethyl-piperidin-1-carbonyl)-3-methyl-phenyl]-5-oxo-pyrrolidin-3-yl}-amid | | |
| **62** | 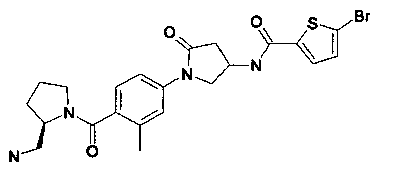 | (M⁺H)⁺ = 506.45 | 3.24 min |
| | 5-Brom-thiophen-2-carbonsäure-{1-[4-((2*R*)-2-aminomethyl-pyrrolidin-1-carbonyl)-3-methyl-phenyl]-5-oxo-pyrrolidin-3-yl}-amid | | |
| **63** | 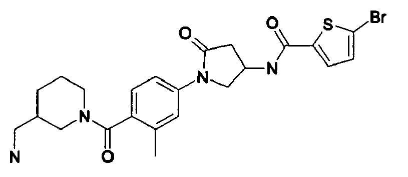 | (M⁺H)⁺ = 520.47 | 3.20 min |
| | 5-Brom-thiophen-2-carbonsäure-{1-[4-(3-aminomethyl-piperidin-1-carbonyl)-3-methyl-phenyl]-5-oxo-pyrrolidin-3-yl}-amid | | |
| **64** | 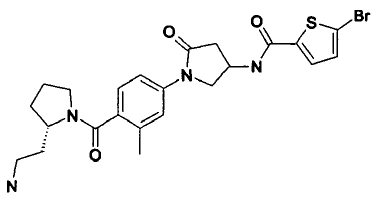 | (M⁺H)⁺ = 520.47 | 3.27 min |
| | 5-Brom-thiophen-2-carbonsäure-(1-{4-[(2S)-2-(2-amino-ethyl)-pyrrolidin-1-carbonyl]-3-methyl-phenyl}-5-oxo-pyrrolidin-3-yl)-amid | | |
| **65** | 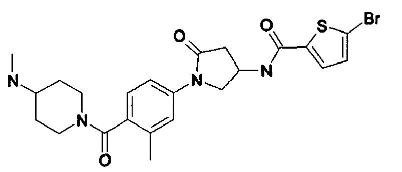 | (M⁺H)⁺ = 520.47 | 3.15 min |
| | 5-Brom-thiophen-2-carbonsäure-{1-[3-methyl-4-(4-methylamino-piperidin-1-carbonyl)-phenyl]-5-oxo-pyrrolidin-3-yl}-amid | | |
| **66** | 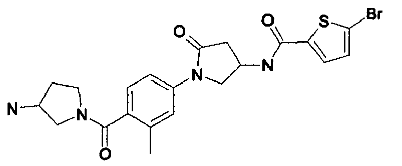 | (M⁺H)⁺ = 492.42 | 3.11 min |
| | 5-Brom-thiophen-2-carbonsäure-{1-[4-(3-amino-pyrrolidin-1-carbonyl)-3-methyl-phenyl]-5-oxo-pyrrolidin-3-yl}-amid | | |
| **67** | 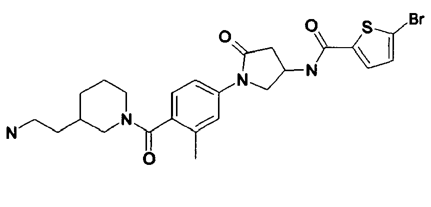 | (M⁺H)⁺ = 534.50 | 3.24 min |
| | 5-Brom-thiophen-2-carbonsäure-(1-{4-[3-(2-amino-ethyl)-piperidin-1-carbonyl]-3-methyl-phenyl}-5-oxo-pyrrolidin-3-yl)-amid | | |
| **68** | 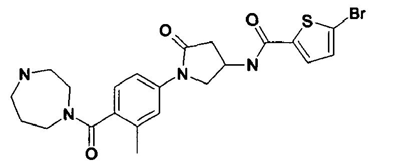 | (M⁺H)⁺ = 506.45 | 3.15 min |
| | 5-Brom-thiophen-2-carbonsäure-{1-[4-([1,4]diazepan-1-carbonyl)-3-methyl-phenyl]-5-oxo-pyrrolidin-3-yl}-amid | | |
| **69** | 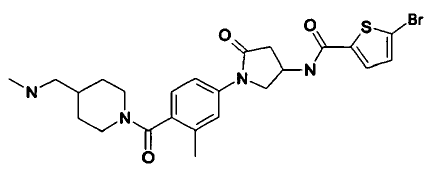 | (M⁺H)⁺ = 534.50 | 3.16 min |
| | 5-Brom-thiophen-2-carbonsäure-{1-[3-methyl-4-(4-methylaminomethyl-piperidin-1-carbonyl)-phenyl]-5-oxo-pyrrolidin-3-yl}-amid | | |
| **70** | 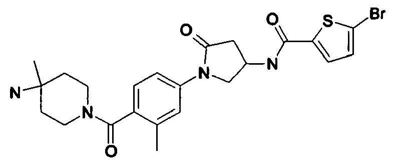 | (M⁺H)⁺ = 520.47 | 3.15 min |
| | 5-Brom-thiophen-2-carbonsäure-{1-[4-(4-amino-4-methyl-piperidin-1-carbonyl)-3-methyl-phenyl]-5-oxo-pyrrolidin-3-yl}amid | | |
| **71** | 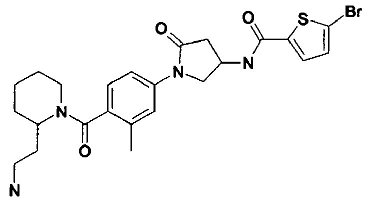 | (M+H)⁺ = 534.50 | 3.32 min |
| | 5-Brom-thiophen-2-carbonsäure-(1-{4-[2-(2-amino-ethyl)-piperidin-1-carbonyl]-3-methyl-phenyl}-5-oxo-pyrrolidin-3-yl)-amid | | |
| **72** | 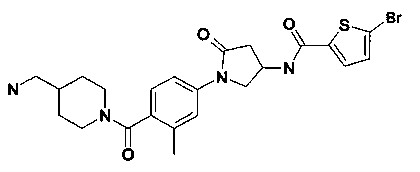 | (M⁺H)⁺ = 520.47 | 3.15 min |
| | 5-Brom-thiophen-2-carbonsäure-{1-[4-(4-aminomethyl-piperidin-1-carbonyl)-3-methyl-phenyl]-5-oxo-pyrrolidin-3-yl}amid | | |
| **73** | 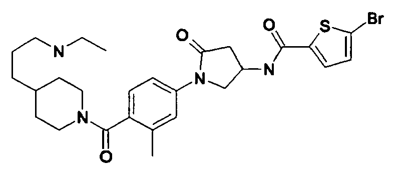 | (M⁺H)⁺ = 576.58 | 4.37 min |
| | 5-Brom-thiophen-2-carbonsäure-(1-{4-[4-(3-ethylamino-propyl)-piperidin-1-carbonyl]-3-methyl-phenyl}-5-oxo-pyrrolidin-3-yl)-amid | | |
| **74** | 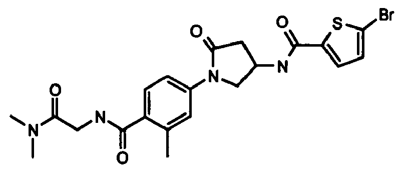 | (M+H)⁺ = 508.42 | 3.47 min |
| | 5-Brom-thiophen-2-carbonsäure-{1-[4-(dimethylcarbamoylmethyl-carbamoyl)-3-methyl-phenyl]-5-oxo-pyrrolidin-3-yl}-amid | | |
| **75** | 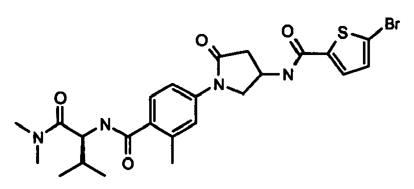 | (M+H)⁺ = 522.44 | 3.55 min |
| | 5-Brom-thiophen-2-carbonsäure-{1-[4-(1-dimethylcarbamoyl-2-methyl-propylcarbamoyl)-3-methyl-phenyl]-5-oxo-pyrrolidin-3-yl}-amid | | |
| **76** | 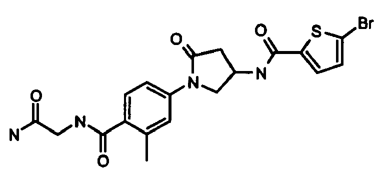 | (M+H)⁺ = 480.36 | 3.32 min |
| | 5-Brom-thiophen-2-carbonsäure-{1-[4-(carbamoylmethyl-carbamoyl)-3-methyl-phenyl]-5-oxo-pyn-olidin-3-yl}-amid | | |
| **77** | 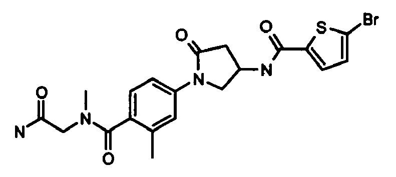 | (M+H)⁺ = 494.39 | 3.34 min |
| | 5-Brom-thiophen-2-carbonsäure-{1-[4-(carbamoylmethyl-methyl-carbamoyl)-3-methyl-phenyl]-5-oxo-pyrrolidin-3-yl}-amid | | |
| **78** | 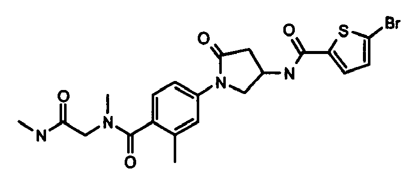 | (M+H)⁺ = 508.42 | 3.40 min |
| | 5-Brom-thiophen-2-carbonsäure-{1-[3-methyl-4-(methyl-methylcarbamoylmethyl-carbamoyl)-phenyl]-5-oxo-pyrrolidin-3-yl}-amid | | |
| **79** | 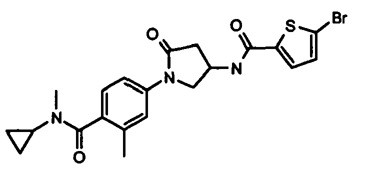 | (M+H)⁺ = 477.40 | 3.82 min |
| | 5-Brom-thiophen-2-carbonsäure-{1-[4-(cyclopropyl-methyl-carbamoyl)-3-methyl-phenyl]-5-oxo-pyrrolidin-3-yl}-amid | | |
| **80** | 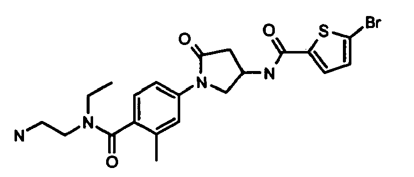 | (M+H)⁺ = 494.43 | 3.22 min |
| | 5-Brom-thiophen-2-carbonsäure-(1-{4-[(2-amino-ethyl)-ethyl-carbamoyl]-3-methyl-phenyl)-5-oxo-pyrrolidin-3-yl)-amid | | |
| **81** | 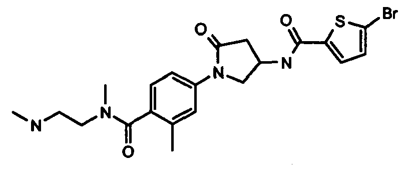 | (M+H)⁺ = 494.43 | 3.18 min |
| | 5-Brom-thiophen-2-carbonsäure-(1-{3-methyl-4-[methyl-(2-methylamino-ethyl)-carbamoyl]-phenyl}-5-oxo-pyrrolidin-3-yl)-amid | | |
| **82** | 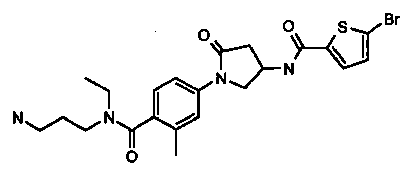 | (M+H)⁺ = 508.46 | 3.24 min |
| | 5-Brom-thiophen-2-carbonsäure-(1-{4-[(3-amino-propyl)-ethyl-carbamoyl]-3-methyl-phenyl}-5-oxo-pyrrolidin-3-yl)-amid | | |
| **83** | 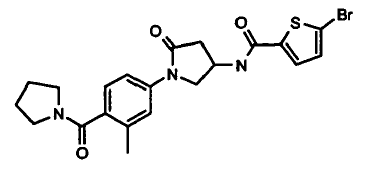 | (M+H)⁺ = 476/478 (Bromisotope) | 0.17 (Kieselgel, Dichlormethan/I sopropanol 95:5) |
| | 5-Brom-thiophen-2-carbonsäure{1-[3-methyl-4-(pyrrolidin-1-carbonyl)-phenyl]-5-oxo-pyrrolidin-3-yl}-amid | | |

### Beispiel 84

### 5-Brom-thiophen-2-carbonsäure-[1-(2'-dimethylaminomethyl-biphenyl-4-yl)-5-oxo-pyrrolidin-3-yl]-amid

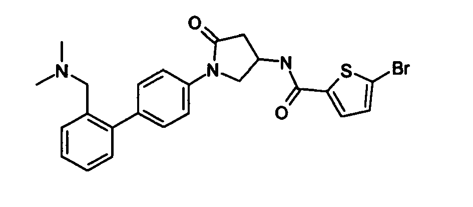

### (a) 1-(4-Brom-phenyl)-5-oxo-pyrrolidin-3-carbonsäure

Hergestellt analog Beispiel 1 b aus 4-Bromanilin und Itaconsäure.
Ausbeute: 98 %
Rₜ-Wert: 4.07 min
C₁₁H₁₀BrNO₃ (284.11)
Massenspektrum: (M+H)⁺ = 284/286 (Bromisotope)

### (b) [1-(4-Brom-phenyl)-5-oxo-pyrrolidin-3-yl]-carbaminsäure-tert-butylester

Hergestellt analog Beispiel 1c aus 1-(4-Brom-phenyl)-5-oxo-pyrrolidin-3-carbonsäure, tert.-Butanol und DPPA.
Ausbeute: 60%
Rₜ-Wert: 4.90 min
C₁₅H₁₉BrN₂O₃ (355.23)
Massenspektrum: (M+H)⁺ = 355/357 (Bromisotope)

### (c) [1,(3'-Dimethylaminomethyl-biphenyl-4-yl)-5-oxo-pyrrolidin-3-yl]-carbaminsäure-tert-butylester

525 mg (1.48 mmol) [1-(4-Brom-phenyl)-5-oxo-pyn-olidin-3-yl]-carbaminsäure-tert-butylester werden in 30 ml Toluol und 5 ml Wasser gelöst und mit 318 mg (1.77 mmol) 2-(*N*,*N*-Dimethylaminomethyl)phenylboronsäure, 48 mg (0.15 mmol) Tetrabutylammoniumbromid und 314 mg (2.96 mmol) Kaliumcarbonat versetzt, zum Schluß werden 173 mg (0.15 mmol)
Tetrakis(triphenylphosphin)Palladium(0) zugegeben. Die Reaktionslösung wird für 90 Minuten bei 400 Watt in der Mikrowelle auf Rückfluß erhitzt.

Anschließend gibt man nochmals 330 mg (1.84 mmol) 2-(*N*,*N-*Dimethylaminomethyl)phenylboronsäure zu und erwärmt für eine Stunde bei 400 Watt in der Mikrowelle. Die Reaktionslösung wird i.Vak. bis zur Trockene eingeengt. Der Rückstand wird mittels Reversed Phase Chromatographie gereinigt.
Ausbeute: 130 mg (21%)
Rₜ-Wert: 4.17 min
C₂₄H₃₁N₃O₃ (409.52)
Massenspektrum: (M+H)⁺ = 410

### (d) 4-Amino-1-(2'-dimethylaminomethyl-biphenyl-4-yl)-pyrrolidin-2-on-dihydrochlorid

130 mg (0.32 mmol) [1,(3'-Dimethylaminomethyl-biphenyl-4-yl)-5-oxo-pyrrolidin-3-yl]-carbaminsäure-tert-butylester werden in 5 ml Chlorwasserstoff in Dioxan (4 M) gelöst, für zwei Stunden bei Raumtemperatur gerührt und anschließend bis zur Trockene am Rotationsverdampfer eingeengt.
Ausbeute: quantitativ
Rₜ-Wert: 3.08 min
C₁₉H₂₃N₃O x 2 HCl (382.33)
C₁₉H₂₃N₃O (309.41)
Massenspektrum: (M+H)⁺ = 310

### (e) 5-Brom-thiophen-2-carbonsäure[1-(2'-dimethylaminomethyl-biphenyl-4-yl)-5-oxo-pyrrolidin-3-yl]amid

Hergestellt aus 4-Amino-1-(2'-dimethylaminomethyl-biphenyl-4-yl)-pyrrolidin-2-on-dihydrochlorid, TBTU, NMM und 5-Brom-thiophen-2-carbonsäure analog Beispiel 1g. Die Reinigung erfolgt mittels Reversed Phase Chromatographie.
Ausbeute: 34 %
Rₜ-Wert :4.27 min
C₂₄H₂₄BrN₃O₄S x C₂HF₃O₂ (612.46)
C₂₄H₂₄BrN₃O₄S (498.45)
Massenspektrum: (M+H)⁺ = 498/500 (Bromisotope)

Analog wurden folgende Verbindungen hergestellt:

| **Nr.** | **Strukturformel** | **Ausbeute** | **Massenpeak(s)** | **R_{f}-Wert bzw. Rₜ** |
|---|---|---|---|---|
| | **Name** | | | |
| **85** | 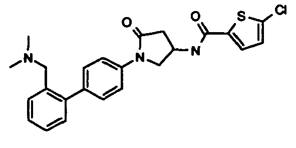 | Σ: 7.3% | (M+H)⁺ = 454/456 (Chlorisotope) | 4.24 min |
| | 5-Chlor-thiophen-2-carbonsäure[1-(2'-dimethylaminomethyl-biphenyl-4-yl)-5-oxo-pyrrolidin-3-yl]amid | | | |
| **86** | 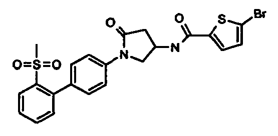 | Σ: 9.5% | (M+H)⁺ = 519/521 (Bromisotope) | 4.81 min |
| | 5-Brom-thiophen-2-carbonsäure-[1-(2'-methansulfonyl-biphenyl-4-yl)-5-oxo-pyrrolidin-3-yl]-amid | | | |
| **87** | 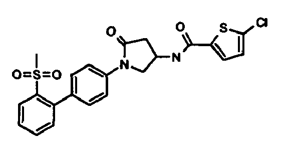 | Σ: 9.7% | (M+H)⁺ = 475/477 (Chlorisotope) | 4.73 min |
| | 5-Chlor-thiophen-2-carbonsäure-[1-(2'-methansulfonyl-biphenyl-4-yl)-5-oxo-pyrrolidin-3-yl]-amid | | | |
| **88** | 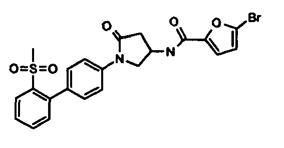 | Σ: 10.1% | (M+H)⁺ = 503/505 (Bromisotope) | 4.43 min |
| | 5-Brom-furan-2-carbonsäure-[1-(2'-methansulfonyl-biphenyl-4-yl)-5-oxo-pyrrolidin-3-yl]-amid | | | |
| **89** | 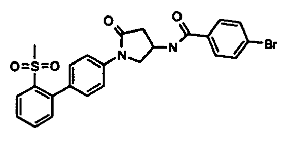 | Σ: 7.4% | (M+H)⁺ = 513/515 (Bromisotope) | 4.75 min |
| | 4-Brom-*N-*[1-(2'-methansulfonyl-biphenyl-4-yl)-5-oxo-pyrrolidin-3-yl]-benzamid | | | |
| **90** | 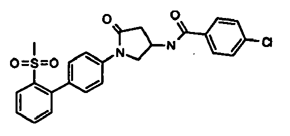 | Σ: 4.9% | (M+H)⁺ = 569/571 (Chlorisotope) | 4.65 min |
| | 4-Chlor-*N-*[1-(2'-methansulfonyl-biphenyl-4-yl)-5-oxo-pyrrolidin-3-yl]-benzamid | | | |
| **91** | 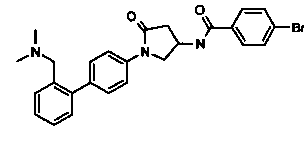 | Σ: 4.6% | (M+H)⁺ = 492/494 (Bromisotope) | 4.25 min |
| | 4-Brom-*N-*[1-(2'-dimethylaminomethyl-biphenyl-4-yl)-5-oxo-pyrrolidin-3-yl]-benzamid | | | |
| **92** | 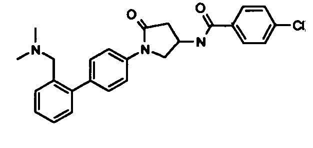 | Σ: 5.7% | (M+H)⁺ = 448/450 (Chlorisotope) | 4.17 min |
| | 4-Chlor-*N-*[1-(2'-dimethylaminomethyl-biphenyl-4-yl)-5-oxo-pyrrolidin-3-yl]-benzamid | | | |

### Beispiel 93

### (R)-5-Brom-thiophen-2-carbonsäure-{1-[3-methyl-4-(pyrrolidin-1-sulfonyl)-phenyl]-5-oxo-pyrrolidin-3-yl}-amid

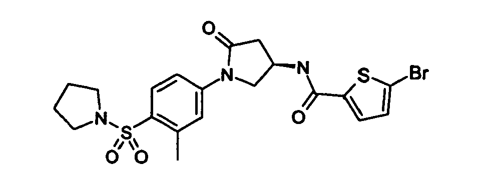

### (a) 4-Acetylamino-2-methyl-benzolsulfonylchlorid

*N-*m-Tolylacetamid (1,0 g; 6,7 mmol) werden portionsweise in 2,2 ml Chlorsulfonsäure eingetragen. Diese Mischung wird für 2 Stunden auf 60°C erwärmt und anschließend auf Eiswasser gegossen. Man extrahiert dreimal mit Ethylacetat, trocknet die vereinten organischen Phasen mit Natriumsulfat und destilliert das Lösungsmittel am Rotationsverdampfer ab. Es bleibt ein gelbes Öl zurück.
Ausbeute: 1.47 g (89%)
R_{f}-Wert: 0,62 (Kieselgel, Dichlormethan/Ethanol 9:1)
C₉H₁₀NO₃S (247.70)
Massenspektrum: (M+H)⁺ = 247/249 (Chlorisotope)

### (b) N-[3-Methyl-4-(pyrrolidin-1-sulfonyl)-phenyl]-acetamid

Man suspendiert 1,45 g 4-Acetylamino-2-methyl-benzolsulfonylchlorid (5,85 mmol) bei 0°C in 19 ml Natronlauge (1 M) und tropft anschließend 0,51 ml Pyrrolidin (6,47 mmol), gelöst in 9 ml Aceton innerhalb einer halben Stunde zu. Die Lösung wird über Nacht auf Raumtemperatur erwärmt und dann mit Salzsäure (2 M) angesäuert. Man extrahiert die Suspension zweimal mit Ethylacetat. Die vereinigten organischen Phasen werden über Natriumsulfat getrocknet und eingeengt. Es bleibt ein braunes Öl zurück.
Ausbeute: 1.25 g (76 %)
R_{f}-Wert: 0,56 (Kieselgel, Dichlormethan/Ethanol 9:1)
C₁₃H₁₈N₂O₃S (282.36)
Massenspektrum: (M+H)⁺ = 283

### (c) 3-Methyl-4-(pyrrolidin-1-sulfonyl)-phenylamin

Man löst 0,6 g *N-*[3-Methyl-4-(pyrrolidin-1-sulfonyl)-phenyl]-acetamid (2,13 mmol) in 5 ml Ethanol und gibt anschließend 10 ml Salzsäure (6 N) bei Raumtemperatur zu. Die Mischung wird über Nacht bei Raumtemperatur gerührt und dann dreimal mit Dichlormethan extrahiert. Die vereinten organischen Phasen werden nacheinander mit 5%iger Natriumhydrogencarbonat-Lösung und mit Wasser gewaschen. Die wässrige Phase wird mit Dichlormethan reextrahiert und mit der schon vorhandenen Dichlormethan-Phase vereinigt, über Natriumsulfat getrocknet und eingeengt. Man erhält ein gelbes Öl, das langsam auskristallisiert.
Ausbeute: 430 mg (84 %)
R_{f}-Wert: 0,80 (Kieselgel, Dichlormethan/Ethanol 9:1)
C₁₁H₁₆N₂O₂S (240.32)
Massenspektrum: (M+H)⁺ = 241

### (d) (R)-(2-Hydroxy-1-{[3-methyl-4-(pyrrolidin-1-sulfonyl)-phenylcarbamoyl]-methyl}-ethyl)-carbaminsäurebenzylester

Man löst 500 mg 3-Methyl-4-(pyrrolidin-1-sulfonyl)-phenylamin (2,08 mmol) in 40 ml Dichlormethan und tropft bei 0°C langsam 1,04 ml Trimethylaluminium in Toluol (2 M, 2,08 mmol) zu. Nach 15 Minuten gibt man eine Lösung von 489 mg (*R*)-(5-Oxo-tetrahydrofuran-3-yl)-carbaminsäurebenzylester in 20 ml Dichlormethan zu und rührt drei Tage bei Raumtemperatur. Anschließend wird die Mischung zur Trockene eingeengt und mit 100 ml Salzsäure (0,5 N) aufgenommen und dreimal mit Diethylether extrahiert. Die vereinigten organischen Phasen werden über Natriumsulfat getrocknet und eingeengt. Der Rückstand wird mittels Reversed-Phase-HPLC gereinigt.
Ausbeute: 210 mg (21 %)
Rₜ-Wert: 4.45 min
C₂₃H₂₉N₃O₆S (475.56)
Massenspektrum: (M+H)⁺ = 476

### (e) (R)-{1-[3-Methyl-4-(pyrrolidin-1-sulfonyl)-phenyl]-5-oxo-pyrrolidin-3-yl}-carbaminsäurebenzylester

Man löst 210 mg (*R*)-2-Hydroxy-1-{[3-methyl-4-(pyrrolidin-1-sulfonyl)-phenylcarbamoyl]-methyl}-ethyl)-carbaminsäurebenzylester (442 µmol) in 5 ml THF und tropft eine Lösung aus 203 mg Di-tert.-butylazodicarboxylat (883 µmol) und 220 µl Tributylphosphin (883 µmol) in 5 ml THF zu. Die Mischung wird über Nacht bei Raumtemperatur gerührt und anschließend zur Trockene eingeengt, mit TFA angesäuert und mittels Reversed-Phase-HPLC gereinigt.
Ausbeute: 48 mg (24 %)
Rₜ-Wert: 4.88 min
C₂₃H₂₇N₃O₅S (457.54)
Massenspektrum: (M+H)⁺ = 458

### (f) (R)-4-Amino-1-[3-methyl-4-(pyrrolidin-1-sulfonyl)-phenyl]-pyrrolidin-2-on

45 mg (98 µmol) (*R*)-{1-[3-Methyl-4-(pyrrolidin-1-sulfonyl)-phenyl]-5-oxo-pyrrolidin-3-yl}-carbaminsäurebenzylester werden in 10 ml Methanol gelöst, mit 25 mg Palladium auf Kohle versetzt und in einer Parr-Apparatur bei 3 bar Wasserstoffdruck bei Raumtemperatur 8 Stunden lang hydriert.

Die Mischung wird vom Katalysator abfiltriert und am Rotationsverdampfer zur Trockene eingeengt.
Ausbeute: quantitativ
Rₜ-Wert: 3.50 min
C₁₅H₂₁N₃O₃S (323.41)

### (g) (R)-5-Brom-thiophen-2-carbonsäure-{1-[3-methyl-4-(pyrrolidin-1-sulfonyl)-phenyl]-5-oxo-pyrrolidin-3-yl}-amid

Hergestellt analog Beispiel 1e aus 5-Brom-thiophen-2-carbonsäure und (*R*)-4-Amino-1-[3-methyl-4-(pyrrolidin-1-sulfonyl)-phenyl]-pyrrolidin-2-on mit TBTU und NMM in DMF und anschließender Reinigung durch Reversed-Phase-Chromatographie.
Ausbeute: 40%
Rₜ-Wert: 4.96 min
C₂₀H₂₂BrN₃O₄S₂ (512.44)
Massenspektrum: (M+H)⁺ = 512/514 (Bromisotope)

Analog wurde folgende Verbindung hergestellt:

| **Nr.** | **Strukturformel** | **Ausbeute** | **Massenpeak(s)** | **R_{f}-Wert bzw. Rₜ** |
|---|---|---|---|---|
| | **Name** | | | |
| **94** | 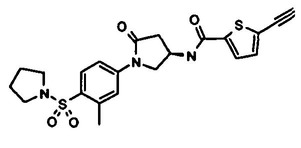 | Σ: 1.2% | (M+H)⁺ = 458 | 4.75 min |
| | (*R*)-5-Ethinyl-thiophen-2-carbonsäure-{1-[3-methyl-4-(3-oxo-morpholin-4-yl)-phenyl]-5-oxo-pyrrolidin-3-yl}-amid | | | |
| **95** | 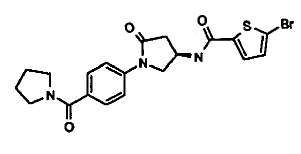 | Σ: 2.7% | (M+H)⁺ = 462/464 (Bromisotope) | 2.75 min |
| | (*R*)-5-Brom-thiophen-2-carbonsäure-{5-oxo-1-[4-(pyrrolidin-1-carbonyl)-phenyl]-pyrrolidin-3-yl}-amid | | | |
| **96** | 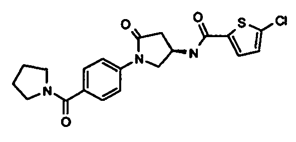 | Σ: 3.4% | (M+H)⁺ = 418/420 (Chlorisotope) | 2.72 min |
| | (*R*)-5-Chlor-thiophen-2-carbonsäure-{5-oxo-1-[4-(pyrrolidin-1-carbonyl)-phenyl]-pyrrolidin-3-yl}-amid | | | |

### Beispiel 97

### 5-Brom-thiophen-2-carbonsäure-{(3R)-1-[3-methyl-4-((2S)-2-pyrrolidin-1-ylmethyl-pyrrolidin-1-carbonyl)-phenyl]-5-oxo-pyrrolidin-3-yl}-amid (als Trifluoracetat-Salz)

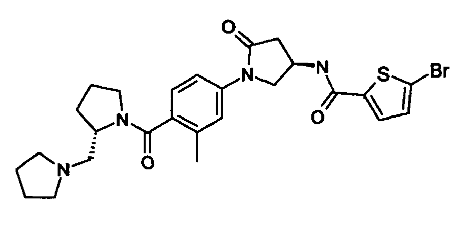

### (a) 4-Benzylamino-2-methyl-benzoesäure-tert.-butylester

4-Brom-2-methyl-benzoesäure-tert.-butylester (12 g; 43.4 mmol), Cäsiumcarbonat (21.2 g, 65 mmol), Palladium(II)-acetat (1 g, 4.45 mmol) und BINAP (2.37 g, 4.40 mmol) werden unter Stickstoff-Athmosphäre in 150 ml Toluol suspendiert und 10 Minuten bei Raumtemperatur gerührt. Dann wird Benzylamin (5.7 ml, 52.1 mmol) zugetropft und die Mischung für zwei Tage auf 100°C erwärmt. Anschließend wird die Mischung abgekühlt, vom Angelösten filtriert und zur Trockene eingedampft. Der ölige Rückstand wird chromatographisch (Silicagel, Petrolether/Ethylacetat 98:2) gereinigt. Man erhält weisse Kristalle.
Ausbeute: 10.15 g (79%)
R_{f}-Wert: 0,62 (Kieselgel, Petrolether/Ethylacetat 1:1)
C₁₉H₂₃NO₂ (297.39)
Massenspektrum: (M+H)⁺ = 298

### (b) 4-Amino-2-methyl-benzoesäure-tert.-butylester

Die Herstellung erfolgt analog Beispiel 93 f aus 4-Benzylamino-benzoesäure-*tert*.-butylester durch katalytische Hydrierung in Ethanol.
Ausbeute: 82 %
R_{f}-Wert: 0,72 (Kieselgel, Dichlormethan/Methanol 50:1)
C₁₂H₁₇NO₂ (207.27)
Massenspektrum: (M+H-C(CH₃)₃)⁺ = 152

### (c) (R)-4-(3-Benzyloxycarbonylamino-4-hydroxy-butyrylamino)-2-methyl-benzoesäure-tert.-butylester

Die Herstellung erfolgt analog Beispiel 93 d aus 4-Amino-benzoesäure-*tert*.-butylester und (*R*)-(5-Oxo-tetrahydrofuran-3-yl)-carbaminsäurebenzylester durch Reaktion mit Trimethylaluminium in Dichlormethan.
Ausbeute: 28 %
Rₜ-Wert: 3.15 min
C₂₄H₃₀N₂O₆ (442.51)
Massenspektrum: (M+H)⁺ = 443

### (d) (R)-4-(4-Benzyloxycarbonylamino-2-oxo-pyrrolidin-1-yl)-2-methyl-benzoesäure-tert.-butylester

Die Herstellung erfolgt analog Beispiel 93 e aus (*R*)-4-(3-Benzyloxycarbonylamino-4-hydroxy-butyrylamino)-2-methyl-benzoesäure-*tert*.-butylester.
Ausbeute: 80 %
Rₜ-Wert: 3.45 min
C₂₄H₂₈N₂O₅ (424.49)
Massenspektrum: (M+H)⁺ = 425

### (e) (R)-4-(4-Amino-2-oxo-pyrrolidin-1-yl)-2-methyl-benzoesäure-tert-butylester

Die Herstellung erfolgt analog Beispiel 93 f aus (*R*)-4-(4-Benzyloxycarbonylamino-2-oxo-pyrrolidin-1-yl)-2-methyl-benzoesäure-*tert*.-butylester durch katalytische Hydrierung.
Ausbeute: 74 %
Rₜ-Wert: 2.44 min
C₁₆H₂₂N₂O₃ (290.36)
Massenspektrum: (M+H)⁺ = 291

### (f) (R)-4-[(5-Brom-thiophen-2-carbonyl)-amino]-2-oxo-pyrrolidin-1-yl)-2-methyl-benzoesäure-tert.-butylester

Die Herstellung erfolgt analog Beispiel 93 g aus (*R*)-4-(4-Amino-2-oxo-pyrrolidin-1-yl)-2-methyl-benzoesäure-*tert*.-butylester und 5-Brom-thiophen-2-carbonsäure.
Ausbeute: 81 %
Rₜ-Wert: 3.51 min
C₂₁H₂₃BrN₂O₄S (479.39)
Massenspektrum: (M-H)- = 477/479 (Brom-Isotope)

### (g) (R)-4-[(5-Brom-thiophen-2-carbonyl)-amino]-2-oxo-pyrrolidin-1-yl)-2-methyl-benzoesäure

(*R*)-4-[(5-Brom-thiophen-2-carbonyl)-amino]-2-oxo-pyrrolidin-1-yl)-2-methyl-benzoesäure-*tert*.-butylester (96 mg; 200 µmol) weren in 1 ml Dichlormethan gelöst und mit 0,5 ml Trifluoressigsäure versetzt. Man rührt 1,5 Stunden bei Raumtemperatur und engt dann zur Trockene ein. Man erhält gelbliche Kristalle.
Ausbeute: 86 mg (quantitativ)
Rₜ-Wert: 4.39 min
C₁₇H₁₅BrN₂O₄S (423.28)

### (h) 5-Brom-thiophen-2-carbonsäure-{(3R)-1-[3-methyl-4-((2S)-2-pyrrolidin-1-ylmethyl-pyrrolidin-1-carbonyl)-phenyl]-5-oxo-pyrrolidin-3-yl}-amid (als Trifluoracetat-Salz)

Hergestellt analog Beispiel 1e aus (*R*)-4-[(5-Brom-thiophen-2-carbonyl)-amino]-2-oxo-pyrrolidin-1-yl)-2-methyl-benzoesäure und (S)-1-(2-Pyrrolidinylmethyl)-pyrrolidin mit TBTU und NMM in DMF und anschließender Reinigung durch Reversed-Phase-Chromatographie.
Ausbeute: 62 %
Rₜ-Wert: 4.23 min
C₂₆H₃₁BrN₄O₃S (559.53)
Massenspektrum: (M+H)⁺ = 559/561 (Bromisotope)

Analog wurden folgende Verbindungen hergestellt:

| **Nr.** | **Strukturformel** | **Ausbeute** | **Massenpeak(s)** | **R_{f}-wert bzw. Rₜ** |
|---|---|---|---|---|
| | **Name** | | | |
| **98** | 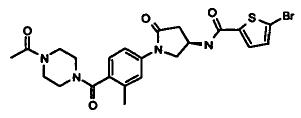 | Σ: 6.6% | (M+H)⁺ = 433/435 (Bromisotope) | 3.90 min |
| | (*R*)-5-Brom-thiophen-2-carbonsäure-{1-[4-(4-acetyl-piperazin-1-carbonyl)-3-methyl-phenyl]-5-oxo-pyrrolidin-3-yl}-amid | | | |
| **99** | 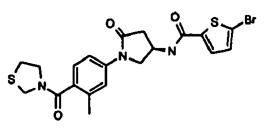 | Σ: 3.3% | (M+H)⁺ = 494/496 (Bromisotope) | 4.49 min |
| | (*R*)-5-Brom-thiophen-2-carbonsäure-{1-[3-methyl-4-(thiazolidin-3-carbonyl)-phenyl]-5-oxo-pyrrolidin-3-yl}-amid | | | |

## Patentansprüche

1. Substituierte Pyrrolidinone der allgemeinen Formel 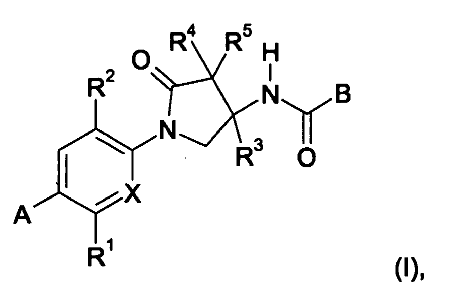 **dadurch gekennzeichnet, dass**
A eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluoratome, eine oder zwei C₁₋₃-Alkyl-, C₂₋₃-Alkenyl-, C₂₋₃-Alkinyl-, Hydroxy-C₁₋₃-alkyl-, C₁₋₃-Alkyloxy-C₁₋₃-alkyl-, Phenyl-C₁₋₃-alkyl-, 1,1- Diphenyl-C₁₋₃-alkyl-, Heteroaryl-C₁₋₃-alkyl-, Amino-C₁₋₃-alkyl-, C₃₋₆-Cycloalkylamino-C₁₋₃-alkyl-, Phenylamino-C₁₋₃-alkyl-, C₁₋₅-Alkylamino-C₁₋₃-alkyl-, Di-(C₁₋₅-alkyl)-amino-C₁₋₅-alkyl-, *N-* (C₃₋₆-Cycloalkyl)-C₁₋₃-alkylamino-C₁₋₃-alkyl-, eine 4- bis 7-gliedrige Cycloalkylenimino-C₁₋₃-alkyl-, *N-*(C₁₋₃-Alkylcarbonyl)-C₁₋₃-alkylamino- C₁₋₃-alkyl-, Carboxy-C₁₋₃-alkyl-, C₁₋₃-Alkyloxycarbonyl-C₁₋₃-alkyl-, Aminocarbonyl-C₁₋₃-alkyl-, C₁₋₃-Alkylaminocarbonyl-C₁₋₃-alkyl-, Di- (C₁₋₃-alkyl)-aminocarbonyl-C₁₋₃-alkyl-, eine 4- bis 7-gliedrige Cycloalkyleniminocarbonyl-C₁₋₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino-C₁₋₃-alkyl-, Aminocarbonylamino-C₁₋₃-alkyl-, C₁₋₅-Alkylaminocarbonylamino-C₁₋₃-alkyl-, Di-(C₁₋₃-alkyl)- aminocarbonylamino-C₁₋₃-alkyl-, C₁₋₅-Alkylsulfonylamino, C₁₋₅- alkylaminocarbonylamino, Carboxy-, C₁₋₃-Alkyloxycarbonyl-, Benzyloxycarbonyl-, C₁₋₃-Alkylcarbonyl-, Aminocarbonyl-, C₁₋₃-Alkylaminocarbonyl-, Di-(C₁₋₃-alkyl)-aminocarbonyl-, *N-*(C₃₋₇-Cycloalkyl)-C₁₋₅-alkylaminocarbonyl-, *N-*(Phenyl-C₁₋₃- alkyl)-C₁₋₅-alkylaminocarbonyl-, eine 4- bis 7-gliedrige Cycloalkyleniminocarbonyl-, Aminocarbonyl-C₁₋₃-alkylaminocarbonyl- , Hydroxy-, C₁₋₃-Alkyloxy-, Allyloxy-, Propargyloxy-, Benzyloxy-, Amino-, C₁₋₃-Alkylamino-, Di-(C₁₋₃-alkyl)-amino-, eine 4- bis 7- gliedrige Cycloalkylenimino-, Trifluormethylcarbonylamino-, *N-*(C₁₋₃-Alkyl)-piperazin-4-yl-C₁₋₃-alkyl-, eine Phenyl- oder eine 5- bis 6-gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom, eine Sulfinyl- oder Sulfonylgruppe ersetzt sein kann oder
eine Methylengruppe in 4-Position einer 6- oder 7-gliedrigen Cycloalkyleniminogruppe durch ein Sauerstoff- oder Schwefelatom, eine Carbonyl-, Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7- gliedrigen Cycloalkyleniminoreste, bei denen eine Methylengruppe durch ein Sauerstoff- oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Hydroxy-C₁₋₃-alkyl, C₁-₅-alkoxy-C₁₋₃-alkyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₃₋₆-Cycloalkylamino-C₁₋₃-alkyl-, Phenyl-, Phenyl-C₁-₃-alkyl-, Heteroaryl-, Heteroaryl-C₁₋₃-alkyl-, Aminocarbonyl-, C₁₋₃-Alkylaminocarbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl-, C₂₋₃-Alkenyl-, C₂₋₃-Alkinyl-, oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Benzyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₁₋₅-Alkyloxycarbonylamino-C₁₋₃-alkyl-, C₃₋₆-Cycloalkylamino-C₁₋₃-alkyl-, Aminocarbonyl-, C₁₋₃-Alkylamino-carbonyl-, *N-*(C₃₋₇-Cycloalkyl)-C₁₋₃-alkylaminocarbonyl-, *N-*(Phenyl-C₁₋₃-alkyl)-C₁₋₅-alkylaminocarbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 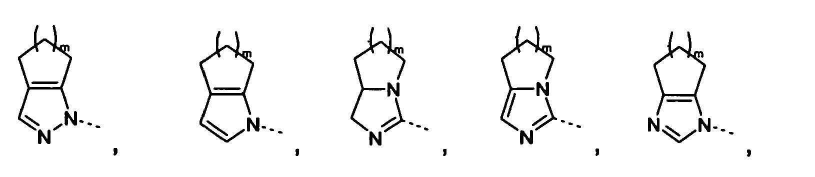 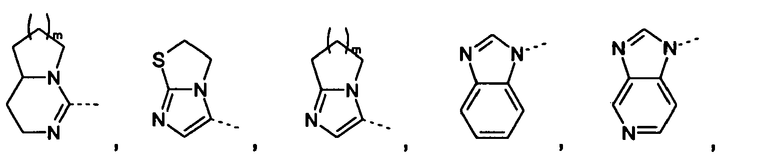 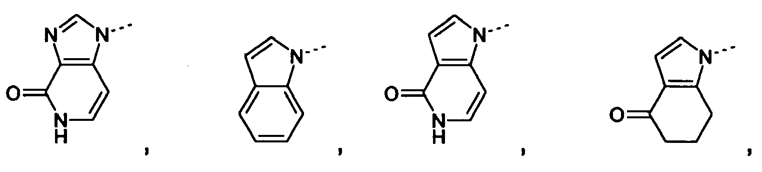 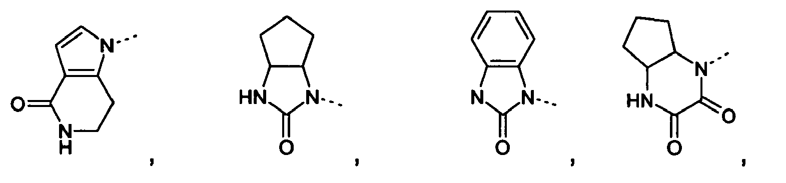 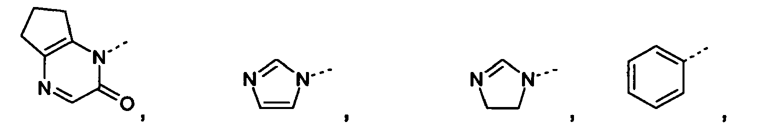 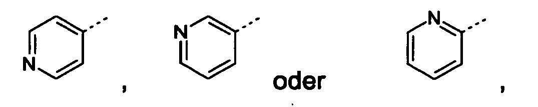 die jeweils an einem Kohlenstoffatom durch eine C₁₋₃-Alkyl-, C₁₋₃-Alkyloxy-, C₁₋₃-Alkyloxycarlbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, C₁₋₃-alkylsulfonyl-C₁₋₃-alkyl, C₁₋₃-alkylsulfonylamino-C₁₋₃-alkyl, C₁₋₃-alkylsulfonylamino, C₁₋₃-alkylcarbonylamino-C₁₋₃-alkyl-, C₁₋₃-alkylcarbonylamino-, Amino-C₁₋₃-alkyl-, C₁₋₃-Alkylamino-C₁₋₃-alkyl-, Di-(C₁₋₃-alkylyamino-C₁₋₃-alkyl-, Hydroxy-C₁₋₃-alkyl-, C₁₋₅-alkoxy-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
R¹ ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
R² ein Wasserstoff- oder Halogenatom oder eine C₁₋₃-Alkylgruppe bedeutet,
X ein Stickstoffatom oder eine CH-Gruppe bedeutet,
R³ ein Wasserstoffatom oder eine C₁₋₃-Alkylgruppe bedeutet,
R⁴ und R⁵ jeweils unabhängig voneinander
ein Wasserstoffatom, eine Hydroxygruppe, eine OR⁹-Gruppe, eine C₂₋₆-Alkenyl- oder C₂₋₆-Alkinylgruppe,
eine geradkettige oder verzweigte C₁₋₆-Alkylgruppe,
wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁-₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfonyl-, Carboxy-, C₁₋₅-Alkyloxycarbonyl-, Aminocarbonyl-,
C₁₋₅-Alkylaminocarbonyl-, Di-(C₁₋₅-alkyl)-aminocarbonyl-, C₄₋₆- Cycloalkyleniminocarbonyl-, Aminosulfonyl-, C₁₋₅-Al- kylaminosulfonyl-, Di-(C₁₋₅-alkyl)-aminosulfonyl-, C₄₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, *N-*(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
eine Phenyl-, oder Heteroarylgruppe die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, oder eine C₁₋₅-Alkyloxycarbonyl- C₁₋₅-alkyloxygruppe substituiert sein kann,
eine 3- bis 7-gliedrige Cycloalkyl-, Cycloalkylenimino-, Cycloalkyl- C₁₋₅-alkyl- oder Cycloalkylenimino-C₁₋₃-alkylgruppe, in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder-N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkylenimino-, Cycloalkyl-C₁₋₅-alkyl- oder Cycloalkylenimino-C₁₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkylenimino-, Cycloalkyl-C₁₋₅-alkyl- oder Cycloalkylenimino-C₁₋₃-alkylgruppe an einer oder zwei -CH₂- Gruppen durch jeweils eine oder zwei C₁₋₃-Alkylgruppen substituiert sein kann,
bedeutet,
mit der Maßgabe, dass R⁴und R⁵ nicht gleichzeitig als Hydroxy- oder OR⁹-Gruppen definiert sein können, oder
R⁴ und R⁵ zusammen mit dem Kohlenstoffatom, an dem sie gebunden sind, eine C₃₋₈-Cycloalkyl- oder C₃₋₈-Cycloalkenylgruppe bilden,
wobei eine der Methylengruppen einer C₄₋₈-Cycloalkylgruppe durch ein Sauerstoff- oder Schwefelatom oder eine -N(R⁷)-, oder eine Carbonyl-, Sulfinyl- oder Sulfonylgruppe ersetzt sein kann, und/oder
zwei direkt benachbarte Methylengruppen einer C₄₋₈-Cycloalkylgruppe zusammen durch eine -C(O)N(R⁸)- oder - S(O)₂N(R⁸)-Gruppe ersetzt sein können, und/oder
drei direkt benachbarte Methylengruppen einer C₆₋₈- Cycloalkylgruppe zusammen durch eine -OC(O)N(R⁸)-, - N(R⁸)C(O)N(R⁸)- oder -N(R⁸)S(O)₂N(R⁸)-Gruppe ersetzt sein können,
wobei 1 bis 3 Kohlenstoffatome einer C₃₋₈-Cycloalkylgruppe gegebenenfalls unabhängig voneinander durch jeweils ein oder zwei gleiche oder unterschiedliche Halogenatome oder C₁₋₅-Alkyl-, Nitril-, Hydroxy-, C₁₋₅-Alkyloxy-, C₁₋₅-Alkylcarbonyloxy-, Carboxy- C₁₋₅-alkyl, C₁₋₅-Alkyloxycarbonyl-C₁₋₅-alkyl, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, *N-*(C₁₋₅-Alkylsulfonyl)-C₁₋₅-alkylamino- oder C₃₋₆-Cycloalkylcarbonylaminogruppen substituiert sein können,
wobei 1 bis 2 Kohlenstoffatome einer C₃₋₈-Cycloalkenylgruppe gegebenenfalls unabhängig voneinander durch jeweils eine C₁₋₅- Alkyl-, Nitril-, Carboxy-C₁₋₅-alkyl, C₁₋₅-Alkyloxycarbonyl-C₁₋₅-alkyl, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonylgruppen substituiert sein können,
und 1 bis 2 Kohlenstoffatome einer C₄₋₈-Cycloalkenylgruppe, die nicht durch eine Doppelbindung an ein anderes Kohlenstoffatom gebunden sind, gegebenenfalls unabhängig voneinander durch ein Fluoratom oder eine Hydroxy-, C₁₋₅-Alkyloxy-, C₁₋₅-Alkylcarbonyloxy-, C₁₋₅-Alkylsulfanyl-, C₁₋₅-Alkylsulfonyl-, Amino-, C₁₋₅-Alkylamino-, Di-(C₁₋₅-alkyl)-amino-, C₁₋₅-Alkylcarlbonylamino-, C₁₋₅-Alkylsulfonylamino-, *N-*(C₁₋₅-Alkylsulfonyl)-C₁₋₅-alkylamino- oder C₃₋₆-Cycloalkylcarbonylaminogruppen substituiert sein können,
mit der Maßgabe, dass eine solche, zusammen aus R⁴ und R⁵ gebildete C₃₋₈-Cycloalkyl- oder C₃₋₈-Cycloalkenylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder
in der ein an die cyclische Gruppe gebundener Substituent, der sich dadurch auszeichnet, daß ein Heteroatom aus der Gruppe Sauerstoff, Stickstoff, Schwefel und Halogenatom direkt an die cyclische Gruppe gebunden ist, von einem anderen Heteroatom aus der Gruppe Sauerstoff, Stickstoff und Schwefel, mit Ausnahme der Sulfongruppe, durch genau eine, gegebenenfalls substituierte, Methylengruppe getrennt ist, und/oder
in der zwei Sauerstoffatome direkt miteinander verbunden sind,
ausgeschlossen ist,
R⁷ jeweils unabhängig voneinander ein Wasserstoffatom, eine Hydroxy-, eine Formyl-, eine C₁₋₅-Alkyl-, C₁₋₅-Alkylcarbonyl-, C₁₋₅-Alkyloxycarbonyl- oder C₁₋₅-Alkylsulfonylgruppe bedeutet,
R⁸ jeweils unabhängig voneinander ein Wasserstoffatom oder eine C₁₋₅-Alkylgruppe bedeutet,
R⁹ eine geradkettige oder verzweigte C₁₋₆-Alkylgruppe,
wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, C₁₋₅-alkyloxycarbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, *N-*(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
mit der Maßgabe, dass der Ersatz von Wasserstoffatomen des ersten Kohlenstoffatoms der geradkettigen oder verzweigten C₁₋₆-Alkylgruppe durch Substituenten aus der Gruppe Sauerstoff, Schwefel oder Stickstoff ausgeschlossen ist,
eine Phenyl-, Heteroaryl-, Phenyl-C₁-₅-alkyl- oder Heteroaryl- C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können,
eine 3- bis 7-gliedrige Cycloalkyl-, Cycloalkyl-C₁₋₅-alkyl- oder Cycloalkylenimino-C₂₋₃-alkylgruppe,
in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkyl-C₁₋₅-alkyl- oder Cycloalkylenimino-C₂₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkyl-C₁₋₅-alkyl- oder Cycloalkylenimino-C₂₋₃-alkylgruppe an einer oder zwei -CH₂-Gruppen durch jeweils eine oder zwei C₁₋₃- Alkylgruppen substituiert sein kann,
bedeutet,
B einen Rest der allgemeinen Formel 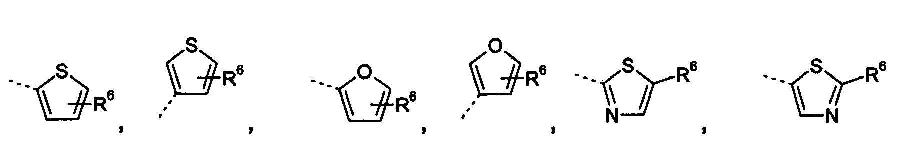 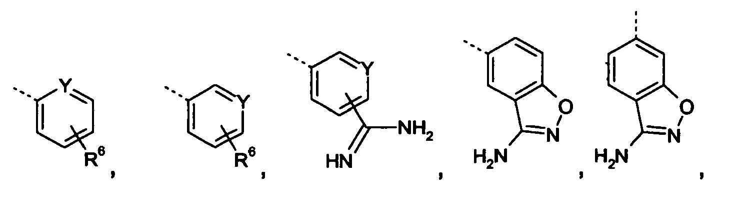 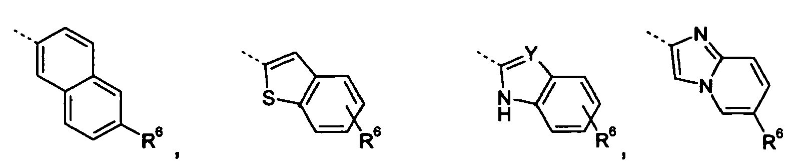 oder 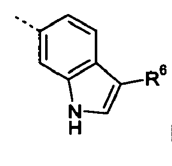 bedeutet,
Y ein Stickstoffatom oder eine CH-Gruppe bedeutet,
R⁶ ein Wasserstoff-, ein Halogenatom, eine Nitrilgruppe, eine C₂₋₃-Alkenyl-, C₂₋₃-Alkinyl-, eine C₁₋₃-Alkylgruppe, oder eine C₁₋₃-Alkoxygruppe bedeutet, wobei die Wasserstoffatome der C₁₋₃-Alkyl- oder C₁₋₃-Alkoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome und die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine C₃₋₆-Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält, und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

2. Substituierte Pyrrolidinone der allgemeinen Formel I nach Anspruch 1, **dadurch gekennzeichnet, dass**
A eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, C₂₋₃-Alkenyl-, C₂₋₃-Alkinyl-, Hydroxy- C₁₋₃-alkyl-, C₁₋₃-Alkyloxy-C₁₋₃-alkyl-, Phenyl-C₁₋₃-alkyl-, 1,1-Diphenyl- C₁₋₃-alkyl-, Heteroaryl-C₁₋₃-alkyl-, Amino-C₁₋₃-alkyl-, C₃₋₆-Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, *N-*(C₃₋₆-Cycloalkyl)-C₁₋₃-alkylamino-C₁₋₃-alkyl-, eine 4- bis 7-gliedrige Cycloalkylenimino-C₁₋₃-alkyl-, *N-*(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine 4- bis 7-gliedrige Cycloalkyleniminocarbonyl-C₁₋₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino- C₁₋₃-alkyl-, Aminocarbonylamino-C₁₋₃-alkyl-, C₁₋₅-Alkylaminocarbonylamino- C₁₋₃-alkyl-, Di-(C₁₋₃-alkyl)-aminocarbonylamino-C₁₋₃-alkyl-, C₁₋₅- Alkylsulfonylamino, C₁₋₅-alkylaminocarbonylamino, Carboxy-, C₁₋₃-Alkyloxycarbonyl-, Benzyloxycarbonyl-, C₁₋₃-Alkylcarbonyl-, Aminocarbonyl-, C₁₋₃-Alkylaminocarbonyl-, Di-(C₁₋₃-alkyl)-aminocarbonyl-, *N-*(C₃₋₇-Cycloalkyl)-C₁₋₅-alkylaminocarbonyl-, *N-*(Phenyl-C₁₋₃-alkyl)-C₁₋₅- alkylaminocarbonyl-, eine 4- bis 7-gliedrige Cycloalkyleniminocarbonyl-, Aminocarbonyl-C₁₋₃-alkylaminocarbonyl-, Hydroxy-, C₁₋₃-Alkyloxy-, Allyloxy-, Propargyloxy-, Benzyloxy-, Amino-, C₁₋₃-Alkylamino-, Di-(C₁₋₃-alkylamino-, eine 4- bis 7-gliedrige Cycloalkylenimino-, Trifluormethylcarbonylamino-, *N-*(C₁₋₃-Alkyl)-piperazin-4-yl-C₁₋₃-alkyl-, eine Phenyl- oder eine 5- bis 6- gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom, eine Sulfinyl- oder Sulfonylgruppe ersetzt sein kann oder
eine Methylengruppe in 4-Position einer 6- oder 7-gliedrigen Cycloalkyleniminogruppe durch ein Sauerstoff- oder Schwefelatom, eine Carbonyl-, Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Hydroxy-C₁₋₃-alkyl, C₁₋₅-alkoxy-C₁₋₃-alkyl, Amino-C₁₋₃-alkyl-, C₁₋₃-Alkylamino-C₁₋₃-alkyl-, Di-(C₁₋₃-alkyl)-amino-C₁.₃-alkyl-, eine 4- bis 7-gliedrige Cycloalkylenimino-C₁₋₃-alkyl-, C₃₋₆-Cycloalkylamino-C₁₋₃-alkyl-, Phenyl-, Phenyl-C₁₋₃-alkyl-, Heteroaryl-, Heteroaryl-C₁₋₃-alkyl-, Aminocarbonyl-, C₁₋₃-Alkylaminocarlbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl-, C₂₋₃-Alkenyl-, C₂₋₃-Alkinyl-, oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Benzyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₁₋₅-Alkyloxycarbonylamino-C₁₋₃-alkyl-, C₃₋₆-Cycloalkylamino-C₁₋₃-alkyl-, Aminocarbonyl-, C₁₋₃-Alkylamino-carbonyl-, *N-*(C₃₋₇-Cycloalkyl)-C₁₋₃-alkylaminocarbonyl-, *N-*(Phenyl-C₁₋₃-alkyl)-C₁₋₅-alkylaminocarbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 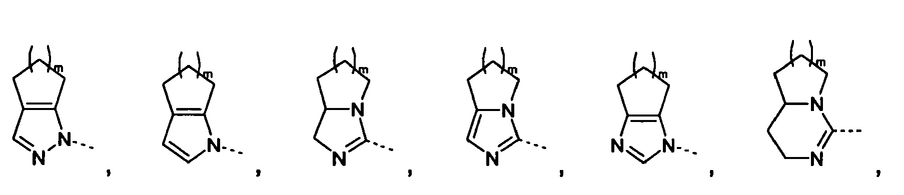 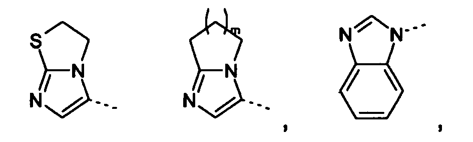 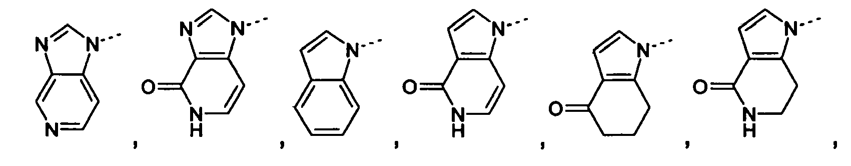 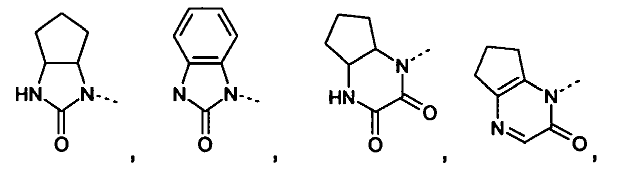 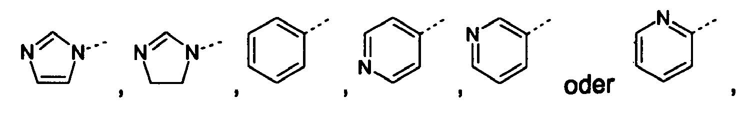 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, C₁₋₃-alkylsulfonyl-C₁₋₃-alkyl, C₁₋₃-alkylsulfonylamino-C₁₋₃-alkyl, C₁₋₃-alkylsulfonylamino, C₁₋₃-alkylcarbonylamino-C₁₋₃-alkyl-, C₁₋₃-alkylcarbonylamino-, Amino-C₁₋₃-alkyl-, C₁₋₃-Alkylamino-C₁₋₃-alkyl-, Di-(C₁₋₃-alkyl)-amino-C₁₋₃-alkyl-, Hydroxy-C₁₋₃-alkyl-, C₁₋₅-alkoxy-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
R¹ ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
R² ein Wasserstoff- oder Halogenatom oder eine C₁₋₃-Alkylgruppe bedeutet,
X ein Stickstoffatom oder eine CH-Gruppe bedeutet,
R³ ein Wasserstoffatom oder eine C₁₋₃-Alkylgruppe bedeutet,
R⁴ und R⁵ jeweils unabhängig voneinander
ein Wasserstoffatom, eine Hydroxygruppe, eine OR⁹-Gruppe, eine C₂₋₆-Alkenyl- oder C₂₋₆-Alkinylgruppe,
eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, Aminosulfonyl-, C₁₋₅-Al- kylaminosulfonyl-, Di-(C₁₋₅-alkyl)-aminosulfonyl-, C₄₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, *N-*(C₁₋₅-Alkylsulfonyl)-C₁₋₅-alkylamino- oder C₃₋₆-Cycloalkylcarbonylaminogruppe substituiert sein kann, wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
eine Phenyl-, oder Heteroarylgruppe die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, oder eine C₁₋₅-Alkyloxycarbonyl- C₁₋₅-alkyloxygruppe substituiert sein kann,
eine 3- bis 7-gliedrige Cycloalkyl-, Cycloalkylenimino-, Cycloalkyl- C₁₋₅-alkyl- oder Cycloalkylenimino-C₁₋₃-alkylgruppe, in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkylenimino-, Cycloalkyl-C₁₋₅-alkyl- oder Cycloalkylenimino-C₁₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist, wobei eine wie oben definierte 3- bis 7-gliedrige Cycloalkyl-, Cycloalkylenimino-, Cycloalkyl-C₁₋₅-alkyl- oder Cycloalkylenimino-C₁₋₃-alkylgruppe an einer oder zwei -CH₂- Gruppen durch jeweils eine oder zwei C₁₋₃-Alkylgruppen substituiert sein kann,
bedeutet,
mit der Maßgabe, dass R⁴ und R⁵ nicht gleichzeitig als Hydroxy- oder OR⁹-Gruppen definiert sein können, oder
R⁴ und R⁵ zusammen mit dem Kohlenstoffatom, an dem sie gebunden sind, eine C₃₋₈-Cycloalkyl- oder C₃₋₈-Cycloalkenylgruppe bilden,
wobei eine der Methylengruppen einer C₄₋₈-Cycloalkylgruppe durch ein Sauerstoff- oder Schwefelatom oder eine -N(R⁷)-, oder eine Carbonyl-, Sulfinyl- oder Sulfonylgruppe ersetzt sein kann, und/oder
zwei direkt benachbarte Methylengruppen einer C₄₋₈-Cycloalkylgruppe zusammen durch eine -C(O)N(R⁸)- oder - S(O)₂N(R⁸)-Gruppe ersetzt sein können, und/oder drei direkt benachbarte Methylengruppen einer C₆₋₈- Cycloalkylgruppe zusammen durch eine -OC(O)N(R⁸)-, - N(R⁸)C(O)N(R⁸)- oder -N(R⁸)S(O)₂N(R⁸)-Gruppe ersetzt sein können,
wobei 1 bis 3 Kohlenstoffatome einer C₃₋₈-Cycloalkylgruppe gegebenenfalls unabhängig voneinander durch jeweils ein oder zwei gleiche oder unterschiedliche Halogenatome oder C₁₋₅-Alkyl-, Nitril-, Hydroxy-, C₁₋₅-Alkyloxy-, C₁₋₅-Alkylcarbonyloxy-, Carboxy- C₁₋₅-alkyl, C₁₋₅-Alkyloxycarbonyl-C₁₋₅-alkyl, C₁₋₅-Alkylsulfanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₃₋₆-Cycloalkyleniminocarbonyl-, Aminosulfonyl-, C₁₋₅-Alkylaminosulfonyl-, Di-(C₁₋₅-alkyly)-aminosulfonyl-, C₃₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, *N-*(C₁₋₅-Alkylsulfonyl)-C₁₋₅-alkylamino- oder C₃₋₆-Cycloalkylcarbonylaminogruppen substituiert sein können,
wobei 1 bis 2 Kohlenstoffatome einer C₃₋₈-Cycloalkenylgruppe gegebenenfalls unabhängig voneinander durch jeweils eine C₁₋₅- Alkyl-, Nitril-, Carboxy-C₁₋₅-alkyl, C₁₋₅-Alkyloxycarbonyl-C₁₋₅-alkyl, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonylgruppen substituiert sein können,
und 1 bis 2 Kohlenstoffatome einer C₄₋₈-Cycloalkenylgruppe, die nicht durch eine Doppelbindung an ein anderes Kohlenstoffatom gebunden sind, gegebenenfalls unabhängig voneinander durch ein Fluoratom oder eine Hydroxy-, C₁₋₅-Alkyloxy-, C₁₋₅-Alkylcarbonyloxy-, C₁₋₅-Alkylsulfanyl-, C₁₋₅-Alkylsulfonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N-*(C₁₋₅-Alkylsulfonyl)-C₁₋₅-alkylamino- oder C₃₋₆-Cycloalkylcarbonylaminogruppen substituiert sein können, mit der Maßgabe, dass eine solche, zusammen aus R⁴ und R⁵ gebildete C₃₋₈-Cycloalkyl- oder C₃₋₈-Cycloalkenylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder
in der ein an die cyclische Gruppe gebundener Substituent, der sich dadurch auszeichnet, daß ein Heteroatom aus der Gruppe Sauerstoff, Stickstoff, Schwefel und Halogenatom direkt an die cyclische Gruppe gebunden ist, von einem anderen Heteroatom aus der Gruppe Sauerstoff, Stickstoff und Schwefel, mit Ausnahme der Sulfongruppe, durch genau eine, gegebenenfalls substituierte, Methylengruppe getrennt ist, und/oder in der zwei Sauerstoffatome direkt miteinander verbunden sind,
ausgeschlossen ist,
R⁷ jeweils unabhängig voneinander ein Wasserstoffatom, eine Hydroxy-, eine Formyl-, eine C₁₋₅-Alkyl-, C₁₋₅-Alkylcarbonyl-, C₁₋₅-Alkyloxycarbonyl- oder C₁₋₅-Alkylsulfonylgruppe bedeutet,
R⁸ jeweils unabhängig voneinander ein Wasserstoffatom oder eine C₁₋₅-Alkylgruppe bedeutet,
R⁹ eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, C₁₋₅-alkyloxycarbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, *N-*(C₁₋₅-Alkylsulfonyl)-C₁₋₅-alkylamino- oder C₃₋₆-Cycloalkylcarbonylaminogruppe substituiert sein kann, wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann, mit der Maßgabe, dass der Ersatz von Wasserstoffatomen des ersten Kohlenstoffatoms der geradkettigen oder verzweigten C₁₋₆-Alkylgruppe durch Substituenten aus der Gruppe Sauerstoff, Schwefel oder Stickstoff ausgeschlossen ist,
eine Phenyl-, Heteroaryl-, Phenyl-C₁₋₅-alkyl- oder Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können,
eine 3- bis 7-gliedrige Cycloalkyl-, Cycloalkyl-C₁₋₅-alkyl- oder Cycloalkylenimino-C₂₋₃-alkylgruppe,
in der bei 4- bis 7-gliedrigen Cyclen im cyclischen Teil eine Methylengruppe gegebenenfalls durch eine -N(R⁷)-Gruppe, ein Sauerstoff oder Schwefelatom oder eine -S(O)- oder-S(O)₂-Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7-gliedrige Cycloalkyl-, Cycloalkyl-C₁₋₅-alkyl- oder Cycloalkylenimino-C₂₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkyl-C₁₋₅-alkyl- oder Cycloalkylenimino-C₂₋₃-alkylgruppe an einer oder zwei -CH₂-Gruppen durch jeweils eine oder zwei C₁₋₃-Alkylgruppen substituiert sein kann,
bedeutet,
B einen Rest der allgemeinen Formel 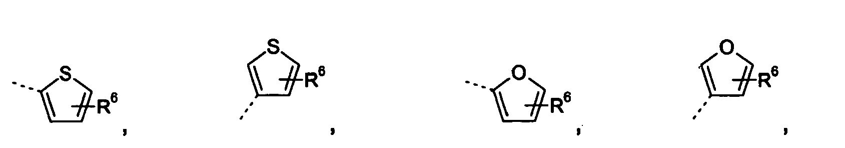 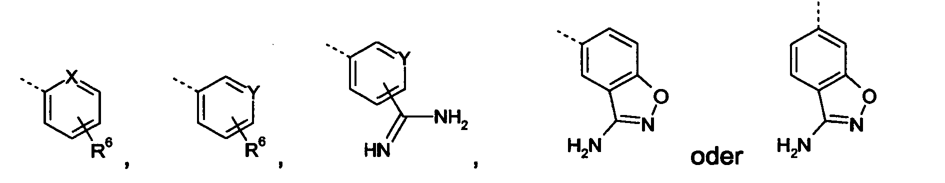 bedeutet,
Y ein Stickstoffatom oder eine CH-Gruppe bedeutet,
R⁶ ein Wasserstoff- , ein Halogenatom, eine Nitrilgruppe, eine C₂₋₃-Alkenyl-, C₂₋₃-Alkinyl-, eine C₁₋₃-Alkylgruppe, oder eine C₁₋₃-Alkoxygruppe bedeutet, wobei die Wasserstoffatome der C₁₋₃-Alkyl- oder C₁₋₃-Alkoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-AlkylaminO-C₂₋₃-alkyl-, Di-(C₁₋₃-alkyl)-amino-C₂-₃-alkyl-, eine C₃₋₆-Cycloalkylenimino-C₁₋₃-alkyl- oder Phenyl-C₁₋₃-alkylgruppe substituierte Iminogruppe oder ein Sauerstoff oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

3. Substituierte Pyrrolidinone der allgemeinen Formel I nach Anspruch 1, **dadurch gekennzeichnet, dass**
A eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, C₂₋₃-Alkenyl-, C₂₋₃-Alkinyl-, Hydroxy- C₁₋₃-alkyl-, C₁₋₃-Alkyloxy-C₁₋₃-alkyl-, Phenyl-C₁₋₃-alkyl-, 1,1-Diphenyl- C₁₋₃-alkyl-, Heteroaryl-C₁₋₃-alkyl-, Amino-C₁₋₃-alkyl-, C₃₋₆-Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, *N-*(C₃₋₆-Cycloalkyl)-C₁₋₃-alkylamino-C₁₋₃-alkyl-, eine 4- bis 7-gliedrige Cycloalkylenimino-C₁₋₃-alkyl-, *N-*(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine 4- bis 7-gliedrige Cycloalkyleniminocarbonyl-C₁-₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino- C₁₋₃-alkyl-, Aminocarbonylamino-C₁₋₃-alkyl-, C₁₋₅-Alkylaminocarbonylamino- C₁₋₃-alkyl-, Di-(C₁₋₃-alkyl)-aminocarbonylamino-C₁₋₃-alkyl-, C₁₋₅- Alkylsulfonylamino, C₁₋₅-alkylaminocarbonylamino, Carboxy-, C₁₋₃-Alkyloxycarbonyl-, Benzyloxycarbonyl-, C₁₋₃-Alkylcarbonyl-, Aminocarbonyl-, C₁₋₃-Alkylaminocarbonyl-, Di-(C₁₋₃-alkyl)-aminocarbonyl-, *N-*(C₃₋₇-Cycloalkyl)-C₁₋₅-alkylaminocarbonyl-, *N-*(Phenyl-C₁₋₃-alkyl)-C₁₋₅- alkylaminocarbonyl-, eine 4- bis 7-gliedrige Cycloalkyleniminocarbonyl-, Aminocarbonyl-C₁₋₃-alkylaminocarbonyl-, Hydroxy-, C₁₋₃-Alkyloxy-, Allyloxy-, Propargyloxy-, Benzyloxy-, Amino-, C₁₋₃-Alkylamino-, Di-(C₁₋₃-alkyl)-amino-, eine 4- bis 7-gliedrige Cycloalkylenimino-, Trifluormethylcarbonylamino-, *N-*(C₁₋₃-Alkyl)-piperazin-4-yl-C₁₋₃-alkyl-, eine Phenyl- oder eine 5- bis 6- gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder
eine Methylengruppe in 4-Position einer 6- oder 7-gliedrigen Cycloalkyleniminogruppe durch ein Sauerstoff- oder Schwefelatom, eine Carbonyl-, Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Hydroxy-C₁₋₃-alkyl, C₁₋₅-alkoxy-C₁₋₃-alkyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₃₋₆-Cycloalkylamino-C₁₋₃-alkyl-, Phenyl-, Phenyl-C₁₋₃-alkyl-, Heteroaryl-, Heteroaryl-C₁₋₃-alkyl-, Aminocarbonyl-, C₁₋₃-Alkylaminocarbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl-, C₂₋₃-Alkenyl-, C₂₋₃-Alkinyl- oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Benzyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₁₋₅-Alkyloxycarbonylamino-C₁₋₃-alkyl-, C₃₋₆-Cycloalkylamino-C₁₋₃-alkyl-, Aminocarbonyl-, C₁₋₃-Alkylamino-carbonyl-, *N-*(C₃₋₇-Cycloalkyl)-C₁₋₃-alkylaminocarbonyl-, *N-*(Phenyl-C₁₋₃-alkyl)-C₁₋₅-alkylaminocarbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 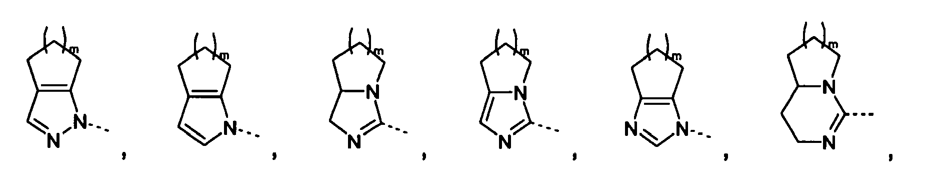 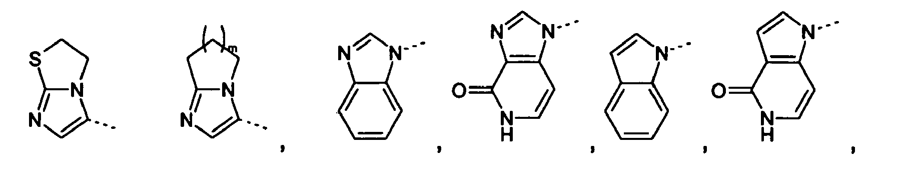 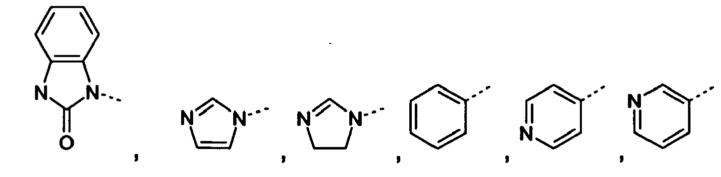 oder 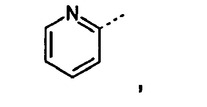 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, C₁₋₃-alkylsulfonyl-C₁₋₃-alkyl, C₁₋₃-alkylsulfonylamino-C₁₋₃-alkyl, C₁₋₃-alkylsulfonylamino, C₁₋₃-alkylcarbonylamino-C₁₋₃-alkyl-, C₁₋₃-alkylcarbonylamino-, Amino-C₁₋₃-alkyl-, C₁₋₃-Alkylamino-C₁₋₃-alkyl-, Di-(C₁₋₃-alkyly)-amino-C₁₋₃-alkyl-, Hydroxy-C₁₋₃-alkyl-, C₁₋₅-alkoxy-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-aminocarbonylgruppe substituiert sein kann und wobei
m die Zahl 1 oder 2 bedeutet,
R¹ ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
R² ein Wasserstoff- oder Halogenatom oder eine Methylgruppe bedeutet,
X ein Stickstoffatom oder eine CH-Gruppe bedeutet,
R³ ein Wasserstoffatom oder eine C₁₋₃-Alkylgruppe bedeutet,
R⁴ und R⁵ jeweils unabhängig voneinander
ein Wasserstoffatom, eine Hydroxygruppe, eine OR⁹-Gruppe, eine C₂₋₆-Alkenyl- oder C₂₋₆-Alkinylgruppe,
eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅-Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsufanyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, Aminosulfonyl-, C₁₋₅-Al- kylaminosulfonyl-, Di-(C₁₋₅-alkyl)-aminosulfonyl-, C₄₋₆-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, *N-*(C₁₋₅-Alkylsulfonyl)-C₁₋₅-alkylamino- oder C₄₋₆-Cycloalkylcarbonylaminogruppe substituiert sein kann,
wobei in den 6- bis 7-gliedrigen Cyclen der C₃₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
eine Phenyl-, Heteroaryl-, Phenyl-C₁₋₅-alkyl- oder Heteroaryl- C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können, eine 3- bis 7-gliedrige Cycloalkyl-, Cycloalkylenimino-, Cycloalkyl- C₁₋₅-alkyl- oder Cycloalkylenimino-C₁₋₃-alkylgruppe,
in der bei 4- bis 7-gliedrigen Cyclen im cyclischen Teil eine Methylengruppe gegebenenfalls durch eine -N(R⁷)-Gruppe, ein Sauerstoff oder Schwefelatom oder eine -S(O)- oder -S(O)₂- Gruppe ersetzt sein kann, oder
in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkylenimino-, Cycloalkyl-C₁₋₅-alkyl- oder Cycloalkylenimino-C₁₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkylenimino-, Cycloalkyl-C₁₋₅-alkyl- oder Cycloalkylenimino-C₁₋₃-alkylgruppe an einer oder zwei -CH₂- Gruppen durch jeweils eine oder zwei C₁₋₃-Alkylgruppen substituiert sein kann,
bedeutet,
mit der Maßgabe, dass R⁴ und R⁵ nicht gleichzeitig als Hydroxy- oder OR⁹-Gruppen definiert sein können,
R⁷ jeweils unabhängig voneinander ein Wasserstoffatom, eine Hydroxy-, eine Formyl-, eine C₁₋₃-Alkyl-, C₁₋₃-Alkylcarbonyl-, C₁₋₃-Alkyloxycarbonyl- oder C₁₋₃-Alkylsulfonylgruppe bedeutet,
R⁸ jeweils unabhängig voneinander ein Wasserstoffatom oder eine C₁₋₃-Alkylgruppe bedeutet,
R⁹ eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und die gegebenenfalls durch eine C₃₋₅- Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl- C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, C₁₋₅-alkyloxycarlbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, *N-*(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann, mit der Maßgabe, dass der Ersatz von Wasserstoffatomen des ersten Kohlenstoffatoms der geradkettigen oder verzweigten C₁₋₆-Alkylgruppe durch Substituenten aus der Gruppe Sauerstoff, Schwefel oder Stickstoff ausgeschlossen ist,
eine Phenyl-, oder Heteroarylgruppe die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, oder eine C₁₋₅-Alkyloxycarbonyl- C₁₋₅-alkyloxygruppe substituiert sein kann;
eine 3- bis 7-gliedrige Cycloalkyl-, Cycloalkyl-C₁₋₅-alkyl- oder Cycloalkylenimino-C₂₋₃-alkylgruppe,
in der bei 4- bis 7-gliedrigen Cyclen im cyclischen Teil eine Methylengruppe gegebenenfalls durch eine -N(R⁷)-Gruppe, ein Sauerstoff- oder Schwefelatom oder eine -S(O)- oder -S(O)₂- Gruppe ersetzt sein kann, oder in der bei 4- bis 7-gliedrigen Cyclen im cyclischen Teil zwei benachbarte Methylengruppen zusammen gegebenenfalls durch eine -C(O)N(R⁸)- oder -S(O)₂N(R⁸)-Gruppe ersetzt sein kann, oder
in der bei 6- bis 7-gliedrigen Cyclen im cyclischen Teil drei benachbarte Methylengruppen zusammen gegebenenfalls durch eine substituierte -OC(O)N(R⁸)- oder -N(R⁸)C(O)N(R⁸)- oder -N(R⁸)S(O)₂N(R⁸)-Gruppe ersetzt sein kann,
mit der Maßgabe, dass eine wie oben definierte 3- bis 7- gliedrige Cycloalkyl-, Cycloalkyl-C₁₋₅-alkyl- oder Cycloalkylenimino-C₂₋₃-alkylgruppe, in der zwei Heteroatome aus der Gruppe Sauerstoff und Stickstoff durch genau eine gegebenenfalls substituierte -CH₂-Gruppe voneinander getrennt sind, ausgeschlossen ist,
wobei eine wie oben definierte 3- bis 7-gliedrige Cycloalkyl-, Cycloalkyl-C₁₋₅-alkyl- oder Cycloalkylenimino-C₂₋₃-alkylgruppe an einer oder zwei -CH₂-Gruppen durch jeweils eine oder zwei C₁₋₃- Alkylgruppen substituiert sein kann,
bedeutet,
B einen Rest der allgemeinen Formel 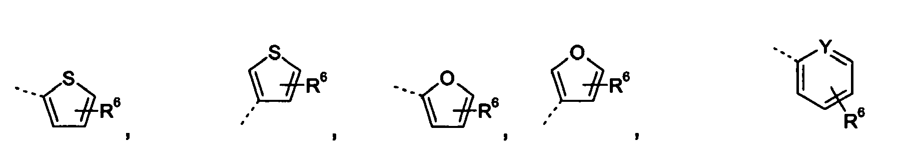 oder 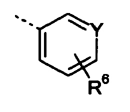 bedeutet,
Y ein Stickstoffatom oder eine CH-Gruppe bedeutet,
R⁶ ein Wasserstoff-, ein Halogenatom, eine Ethinyl-, eine Methylgruppe, eine Methoxygruppe bedeutet, wobei die Wasserstoffatome der Methoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome und die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine C₃₋₆-Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

4. Substituierte Pyrrolidinone der allgemeinen Formel I nach Anspruch 1, **dadurch gekennzeichnet, dass**
A eine 4- bis 7-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, Hydroxy-C₁₋₃-alkyl-, C₁₋₃-Alkyloxy- C₁₋₃-alkyl-, Heteroaryl-C₁₋₃-alkyl-, Amino-C₁₋₃-alkyl-, C₃₋₆-Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, *N-*(C₃₋₆-Cycloalkyl)-C₁₋₃-alkylamino-C₁₋₃-alkyl-, eine 4- bis 7-gliedrige Cycloalkylenimino-C₁₋₃-alkyl-, *N-*(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine 4- bis 7-gliedrige Cycloalkyleniminocarbonyl-C₁₋₃-alkyl-, C₁₋₅-Alkyloxycarbonylamino- C₁₋₃-alkyl-, C₁₋₃-Alkylcarbonylamino-C₁₋₃-alkyl-, C₁₋₅-Alkylsulfonylamino- C₁₋₃-alkyl-, Aminocarbonylamino-C₁₋₃-alkyl-, C₁₋₅-Alkylaminocarbonylamino- C₁₋₃-alkyl-, Di-(C₁₋₃-alkylyaminocarbonylamino-C₁₋₃-alkyl-, C₁₋₅- Alkylsulfonylamino, C₁₋₅-alkylaminocarbonylamino, Carboxy-, C₁₋₃-Alkyloxycarbonyl-, Benzyloxycarbonyl-, C₁₋₃-Alkylcarbonyl-, Aminocarbonyl-, C₁₋₃-Alkylaminocarbonyl-, Di-(C₁₋₃-alkyl)-aminocarbonyl-, *N-*(C₃₋₇-Cycloalkyl)-C₁₋₅-alkylaminocarbonyl-, *N-*(Phenyl-C₁₋₃-alkyl)-C₁₋₅- alkylaminocarbonyl-, eine 4- bis 7-gliedrige Cycloalkyleniminocarbonyl-, Aminocarbonyl-C₁₋₃-alkylaminocarbonyl-, Hydroxy-, C₁₋₃-Alkyloxy-, Allyloxy-, Propargyloxy-, Benzyloxy-, Amino-, C₁₋₃-Alkylamino-, Di-(C₁₋₃-alkyl)-amino-, eine 4- bis 7-gliedrige Cycloalkylenimino-, *N-*(C₁₋₃-Alkyl)-piperazin-4-yl- C₁₋₃-alkyl-, eine Phenyl- oder eine 5- bis 6-gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder
eine Methylengruppe in 4-Position einer 6- oder 7-gliedrigen Cycloalkyleniminogruppe durch ein Sauerstoff- oder Schwefelatom, eine Sulfinyl- oder Sulfonylgruppe oder durch eine gegebenenfalls durch eine C₁₋₃-Alkyl-, Hydroxy-, Formyl- oder C₁₋₃-Alkylcarbonylgruppe substituierte - NH-Gruppe ersetzt sein kann, wobei zusätzlich eine Methylengruppe benachbart zur voranstehend genannten gegebenenfalls substituierten -NH-Gruppe durch eine Carbonyl-, Sulfinyl- oder Sulfonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom, eine Sulfinyl- oder Sulfonylgruppe ersetzt ist, zwei Heteroatome durch mindestens zwei Kohlenstoffatome voneinander getrennt sind,
eine gegebenenfalls durch eine oder zwei C₁₋₃-Alkyl-, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, C₃₋₆-Cycloalkylamino-C₁₋₃-alkyl-, Heteroaryl-, Heteroaryl-C₁₋₃-alkyl-, Aminocarbonyl-, C₁₋₃-Alkylaminocarbonyl-, Di-(C₁₋₃-alkyl)-aminocarbonyl- oder 4- bis 7-gliedrige Cycloalkyleniminocarbonylgruppen substituierte 5- bis 7-gliedrige Cycloalkenyleniminocarbonyl- oder Cycloalkenyleniminosulfonylgruppe, wobei die Doppelbindung nicht an ein Stickstoffatom gebunden ist und mit einer 5- oder 6-gliedrigen Heteroarylgruppe kondensiert sein kann,
eine gegebenenfalls durch eine oder zwei C₁₋₅-Alkyl- oder C₃₋₆-Cycloalkylgruppen substituierte Aminocarbonyl- oder Aminosulfonylgruppe,
wobei die Substituenten gleich oder verschieden sein können und
jeweils eine der C₁₋₅-Alkylgruppen durch eine oder zwei Hydroxy-C₁₋₃-alkyl-, C₁₋₃-Alkoxy-C₁₋₃-alkyl-, Amino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, Aminocarbonyl-, C₁₋₃-Alkylamino-carbonyl-, Di-(C₁₋₃-alkyl)-aminocarbonyl- oder eine 4- bis 7-gliedrige Cycloalkyleniminocarbonylgruppe substituiert sein kann,
oder eine Gruppe der Formel 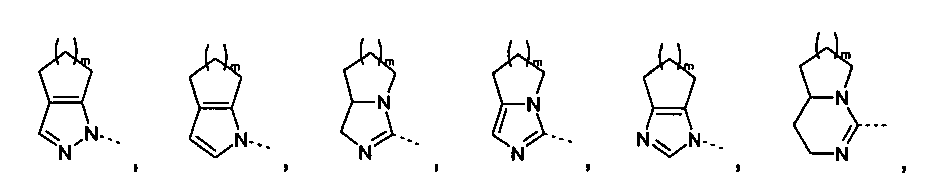 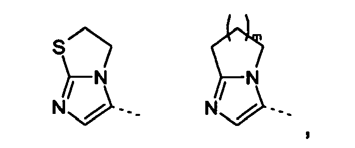 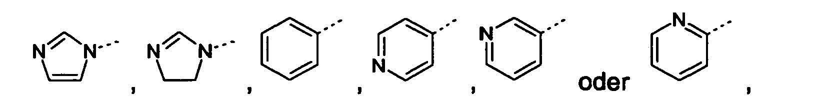 die jeweils an einem Kohlenstoffatom durch eine C₁₋₃-Alkyl-, C₁₋₃-Alkyloxy-, C₁₋₃-Alkyloxycarbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-amino-carbonylgruppe substituiert sein kann und wobei
m die Zahl 1 oder 2 bedeutet,
R¹ ein Wasserstoff- oder Halogenatom, eine C₁₋₃-Alkyl- oder C₁₋₃-Alkoxygruppe, wobei die Wasserstoffatome der C₁₋₃-Alkyl- oder C₁₋₃-Alkoxygruppe gegebenenfalls ganz oder teilweise durch Fluoratome ersetzt sein können, eine C₂₋₃-Alkenyl-, C₂₋₃-Alkinyl-, Nitril-, Nitro- oder Aminogruppe bedeutet,
R² ein Wasserstoff- oder Fluoratom oder eine Methylgruppe bedeutet,
X ein Stickstoffatom oder eine CH-Gruppe bedeutet,
R³ ein Wasserstoffatom oder eine C₁₋₃-Alkylgruppe bedeutet,
R⁴ ein Wasserstoffatom, eine geradkettige oder verzweigte C₁₋₄-Alkylgruppe, wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine C1-₃-Alkyloxygruppe, wobei die Wasserstoffatome der C₁₋₃-Alkyloxygruppe gegebenenfalls ganz oder teilweise durch Fluoratome ersetzt sein können, substituiert sein kann, bedeutet
R⁵ ein Wasserstoffatom, eine Hydroxygruppe, eine OR⁹-Gruppe, eine C₂₋₄-Alkenyl- oder C₂₋₄-Alkinylgruppe,
eine geradkettige oder verzweigte C₁₋₄-Alkylgruppe, wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine C₃₋₅- Cycloalkylgruppe, eine Nitril-, Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl- C₁₋₅-alkyloxy-, Mercapto-, C₁₋₅-Alkylsulfanyl-, C₁₋₅-Alkylsulfonyl-, Carboxy- , C₁₋₅-Alkyloxycarbonyl-, Aminocarbonyl-, C₁₋₅-Alkylaminocarbonyl-, Di- (C₁₋₅-alkyl)-aminocarbonyl-, C₄₋₆-Cycloalkyleniminocarbonyl-, Aminosulfonyl-, C₁₋₅-Alkylaminosulfonyl-, Di-(C₁₋₅-alkyly)-aminosulfonyl-, C₄₋₆-Cycloalkyleniminosulfonyl-, Amino-, C₁₋₅-Alkylamino-, Di-(C₁₋₅-alkyl)- amino-, C₁₋₅-Alkylcarbonylamino-, C₁₋₅-Alkylsulfonylamino-, *N-*(C₁₋₅-Alkylsulfonyl)-C₁₋₅-alkylamino- oder C₃₋₆-Cycloalkylcarbonyl- aminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff -oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten -NR⁷-Gruppe durch eine Carbonylgruppe ersetzt sein kann,
eine Phenyl-, Heteroaryl-, Phenyl-C₁₋₅-alkyl- oder Heteroaryl- C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein können,
bedeutet,
R⁷ jeweils unabhängig voneinander ein Wasserstoffatom, eine C₁₋₃-Alkyl-, C₁₋₃-Alkylcarbonyl-, C₁₋₃-Alkyloxycarbonyl- oder C₁₋₃-Alkylsulfonylgruppe bedeutet,
R⁹ eine geradkettige oder verzweigte C₁₋₄-Alkylgruppe, wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine C₃₋₅-Cycloalkylgruppe, eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycamonyl-C₁₋₅-alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₆- Cycloalkyleniminocarbonyl-, C₁₋₅-alkyloxycarbonylamino-, Amino-, C₁₋₅-Alkylamino-, Di-(C₁₋₅-alkyl)-amino-, C₁₋₅-Alkylcarbonylamino-, C₁₋₅-Alkylsulfonylamino-, *N*-(C₁₋₅-Alkylsulfonyl)-C₁₋₅-alkylamino- oder C₃₋₆-Cycloalkylcarbonylaminogruppe substituiert sein kann,
wobei in den 6- bis 7-gliedrigen Cyclen der C₄₋₆- Cycloalkyleniminocarbonylgruppe im cyclischen Teil eine Methylengruppe in 4-Position einer 6- oder 7-gliedrigen Cycloalkyleniminogruppe durch ein Sauerstoff- oder Schwefelatom, durch eine Carbonyl-, Sulfinyl-, Sulfonyl- oder - NR⁷-Gruppe ersetzt sein kann und zusätzlich eine Methylengruppe benachbart zu einer vorstehend erwähnten - NR⁷-Gruppe durch eine Carbonylgruppe ersetzt sein kann,
mit der Maßgabe, dass der Ersatz von Wasserstoffatomen des ersten Kohlenstoffatoms der geradkettigen oder verzweigten C₁₋₄-Alkylgruppe durch Substituenten aus der Gruppe Sauerstoff, Schwefel oder Stickstoff ausgeschlossen ist,
eine Phenyl-, oder Heteroarylgruppe
die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann,
eine Phenyl-C₁₋₅-alkyl- oder Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Halogenatomen, C₁₋₅-Alkyl-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppen substituiert sein kann, und die gegebenenfalls im C₁₋₅-alkyl-Teil durch eine Hydroxy-, eine C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, oder eine C₁₋₅-Alkyloxycarbonyl- C₁₋₅-alkyloxygruppe substituiert sein kann;
bedeutet,
B einen Rest der allgemeinen Formel 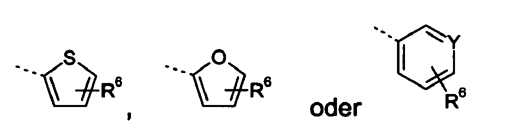 bedeutet,
Y ein Stickstoffatom oder eine CH-Gruppe bedeutet,
R⁶ ein Wasserstoff- , ein Halogenatom, eine Ethinyl-, eine Methylgruppe, eine Methoxygruppe bedeutet, wobei die Wasserstoffatome der Methoxygruppe gegebenenfalls ganz oder teilweise durch Fluoratome ersetzt sein können,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine C₃₋₆-Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

5. Substituierte Pyrrolidinone der allgemeinen Formel I nach Anspruch 1, **dadurch gekennzeichnet, dass**
A eine 5- bis 6-gliedrige Cycloalkyleniminocarbonyl- oder Cycloalkyleniminosulfonylgruppe, wobei
der Cycloalkyleniminoteil im Kohlenstoffgerüst durch ein oder zwei Fluor- atome, eine oder zwei C₁₋₃-Alkyl-, Hydroxy-C₁₋₃-alkyl-, C₁₋₃-Alkyloxy- C₁₋₃-alkyl-, Heteroaryl-C₁₋₃-alkyl-, Amino-C₁₋₃-alkyl-, C₃₋₆-Cycloalkylamino- C₁₋₃-alkyl-, Phenylamino-C₁₋₃-alkyl-, C₁₋₅-Alkylamino-C₁₋₃-alkyl-, Di- (C₁₋₅-alkyl)-amino-C₁₋₅-alkyl-, *N-*(C₃₋₆-Cycloalkyl)-C₁₋₃-alkylamino-C₁₋₃-alkyl-, eine 4- bis 7-gliedrige Cycloalkylenimino-C₁₋₃-alkyl-, *N-*(C₁₋₃-Alkylcarbonyl)- C₁₋₃-alkylamino-C₁₋₃-alkyl-, Carboxy-C₁₋₃-alkyl-, C₁₋₃-Alkyloxycarbonyl- C₁₋₃-alkyl-, Aminocarbonyl-C₁₋₃-alkyl-, C₁₋₃-Alkylaminocarbonyl-C₁₋₃-alkyl-, Di-(C₁₋₃-alkyl)-aminocarbonyl-C₁₋₃-alkyl-, eine C₁₋₃-Alkylcarbonylamino- C₁₋₃-alkyl-, C₁₋₅-Alkylaminocarbonylamino-C₁₋₃-alkyl-, Di-(C₁₋₃-alkyl)- aminocarbonylamino-C₁₋₃-alkyl-, C₁₋₅-Alkylsulfonylamino, C₁₋₅- alkylaminocarbonylamino, Carboxy-, C₁₋₃-Alkyloxycarbonyl-, Aminocarbonyl-, C₁₋₃-Alkylaminocarbonyl-, Di-(C₁₋₃-alkyl)-aminocarbonyl-, *N-*(C₃₋₇-Cycloalkyl)-C₁₋₅-alkylaminocarbonyl-, *N-*(Phenyl-C₁₋₃-alkyl)-C₁₋₅- alkylaminocarbonyl-, Hydroxy-, C₁₋₃-Alkyloxy, Amino-, C₁₋₃-Alkylamino-, Di- (C₁₋₃-alkyl)-amino-, *N-*(C₁₋₃-Alkyl)-piperazin-4-yl-C₁₋₃-alkyl-, eine Phenyl- oder eine 6-gliedrige Heteroarylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder eine Methylengruppe in 4-Position einer 6- bis 7-gliedrigen Cycloalkyleniminogruppe durch ein Sauerstoff oder Schwefelatom oder durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom ersetzt ist, zwei Heteroatome durch mindestens zwei Kohlenstoffatome voneinander getrennt sind,
oder A eine Gruppe der Formel 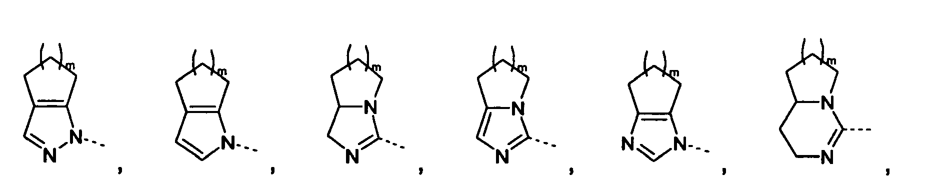 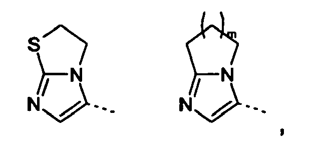 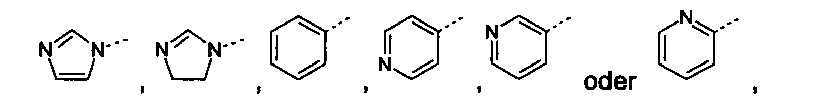 die jeweils an einem Kohlenstoffatom durch eine C₁₋₃-Alkyl-, C₁₋₃-Alkyloxy-, C₁₋₃-Alkyloxycarlbonyl-, C₁₋₅-Alkyloxycarbonylamino-C₁₋₃-alkyl-, C₁₋₃-alkylsulfonyl-, C₁₋₃-alkyl-sulfonyl-C₁₋₃-alkyl-, Aminosulfonyl, Amino-C₁₋₃-alkyl-, C₁₋₃-Alkylamino-C₁₋₃-alkyl-, Di-(C₁₋₃-alkyl)-amino-C₁₋₃-alkyl-, eine 4- bis 7-gliedrige Cycloalkylenimino-C₁₋₃-alkyl-, Morpholin-4-yl-C₁₋₃-alkyl-, Piperazinyl-C₁₋₃-alkyl-, *N-*(C₁₋₃-Alkyl)-piperazin-4-yl-C₁₋₃-alkyl-, Aminocarbonyl-, C₁₋₃-Alkylaminocarbonyl- oder Di-(C₁₋₃-alkyl)-amino-carbonylgruppe substituiert sein kann und wobei
m die Zahl 1 oder 2 bedeutet,
R¹ ein Wasserstoff- , Fluor-, Chlor- oder Bromatom, eine Methyl- oder Methoxygruppe, wobei die Wasserstoffatome der Methyl- oder Methoxygruppe gegebenenfalls ganz oder teilweise durch Fluoratome ersetzt sein können, bedeutet,
R² ein Wasserstoff- oder Fluoratom bedeutet,
X ein Stickstoffatom oder eine CH-Gruppe bedeutet,
R³ ein Wasserstoffatom oder eine C₁₋₃-Alkylgruppe bedeutet,
R⁴ ein Wasserstoffatom bedeutet,
R⁵ ein Wasserstoffatom, eine Hydroxygruppe, eine OR⁹-Gruppe, eine C₂₋₄-Alkenyl- oder C₂₋₄-Alkinylgruppe,
eine geradkettige oder verzweigte C₁₋₄-Alkylgruppe, wobei die Wasserstoffatome gegebenenfalls ganz oder teilweise durch Fluoratome ersetzt sein können, und die gegebenenfalls durch eine Hydroxy-, eine C₁₋₃-Alkyloxygruppe, wobei die Wasserstoffatome der C₁₋₃-Alkyloxygruppe ganz oder teilweise durch Fluoratome ersetzt sein können, eine Benzyloxy-, C₁₋₃-Alkylcarbonyloxy-, C₁₋₃-Alkyloxycarbonyl-, Aminocarbonyl-, C₁₋₃-Alkylaminocarbonyl-, Di-(C₁₋₃-alkyl)-aminocarbonyl- C₄₋₆-Cycloalkyleniminocarbonyl-, Amino-, C₁₋₃-Alkylamino-, Di- (C₁₋₃-alkyl)-amino-, C₁₋₃-Alkylcarbonylamino-, oder C₁₋₃-Alkyl- sulfonylaminogruppe substituiert sein kann,
eine Phenyl-, oder C-verknüpfte Heteroarylgruppe wobei die Heteroarylgruppe ausgewählt ist aus der Gruppe bestehend aus Pyrrolyl, Oxazolyl, Imidazolyl, Furanyl, Thiophenyl, Thiazolyl, 1,2,3- Triazolyl, 1,2,4-Triazolyl, Tetrazolyl, Pyridinyl, Pyrimidinyl und Pyrazinyl, und die im Heteroarylteil gegebenenfalls ein- bis zweifach durch gleiche oder unterschiedliche Substituenten ausgewählt aus der Gruppe bestehend aus Halogenatomen, C₁₋₃-Alkyl-, Hydroxy-, C₁₋₃-Alkyloxy-, Mono-, Di- und Trifluormethoxygruppen substituiert sein kann,
eine Phenyl-C₁₋₃-alkyl-, Heteroaryl-C₁₋₃-alkyl-gruppe, wobei die Heteroarylgruppe ausgewählt ist aus der Gruppe bestehend aus Pyrrolyl, Oxazolyl, Imidazolyl, Furanyl, Thiophenyl, Thiazolyl, 1,2,3- Triazolyl, 1,2,4-Triazolyl, Tetrazolyl, Pyridinyl, Pyrimidinyl und Pyrazinyl, und die im Heteroarylteil gegebenenfalls ein- bis zweifach durch gleiche oder unterschiedliche Substituenten ausgewählt aus der Gruppe bestehend aus Halogenatomen, C₁₋₃-Alkyl-, Hydroxy-, C₁₋₃-Alkyloxy-, Mono-, Di- und Trifluormethoxygruppen substituiert sein kann, und die gegebenenfalls im C₁₋₃-alkyl-Teil durch eine Hydroxy-, eine C₁₋₃-Alkyloxygruppe, wobei die Wasserstoffatome der C₁₋₃-Alkyloxygruppe gegebenenfalls ganz oder teilweise durch Fluoratome ersetzt sein können, eine C₁₋₅-Alkylcarbonyloxy-, oder eine C₁₋₅-Alkyloxycarbonyloxygruppe substituiert sein kann;
bedeutet,
R⁹ eine geradkettige oder verzweigte C₁₋₄-Alkylgruppe, wobei die Wasserstoffatome der geradkettigen oder verzweigten C₁₋₄-Alkylgruppe gegebenenfalls ganz oder teilweise durch Fluoratome ersetzt sein können, und die gegebenenfalls durch eine Hydroxy-, eine C₁₋₃-Alkyloxygruppe, wobei die Wasserstoffatome der C₁₋₃-Alkyloxygruppe gegebenenfalls ganz oder teilweise durch Fluoratome ersetzt sein können, eine Benzyloxy-, C₁₋₃-Alkyloxycarbonyl-, Aminocarbonyl-, C₁₋₃-Alkylaminocarbonyl-, Di-(C₁₋₃-alkyl)-aminocarbonyl-, C₄₋₆-Cycloalkyleniminocarbonyl-, Amino-, C₁₋₃-Alkylamino-, Di-(C₁₋₃-alkyl)- amino-, C₁₋₃-Alkylcarbonylamino-, C₁₋₃-Alkylsulfonylaminogruppe substituiert sein kann,
mit der Maßgabe, dass der Ersatz von Wasserstoffatomen des ersten Kohlenstoffatoms der geradkettigen oder verzweigten C₁₋₄-Alkylgruppe durch Substituenten aus der Gruppe Sauerstoff, Schwefel oder Stickstoff ausgeschlossen ist,
eine Phenyl-, Phenyl-C₁₋₂-alkyl-, Heteroaryl-C₁₋₂-alkyl- oder C-verknüpfte Heteroarylgruppe
wobei die Heteroarylgruppe ausgewählt ist aus der Gruppe bestehend aus Pyrrolyl, Oxazolyl, Imidazolyl, Furanyl, Thiophenyl, Thiazolyl, 1,2,3-Triazolyl, 1,2,4-Triazolyl, Tetrazolyl, Pyridinyl, Pyrimidinyl und Pyrazinyl, und die im Heteroarylteil gegebenenfalls ein- bis zweifach durch gleiche oder unterschiedliche Substituenten ausgewählt aus der Gruppe bestehend aus Halogenatomen, C₁₋₃-Alkyl-, Hydroxy-, C₁₋₃-Alkyloxy-, Mono-, Di- und Trifluormethoxygruppen substituiert sein kann,
bedeutet,
B einen Rest der allgemeinen Formel 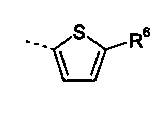 bedeutet,
R⁶ ein Wasserstoff-, ein Chlor- oder Bromatom, eine Ethinyl-, eine Methyl- oder eine Methoxygruppe bedeutet,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

6. Substituierte Pyrrolidinone der allgemeinen Formel I nach Anspruch 1, **dadurch gekennzeichnet, dass**
A eine 5- bis 6-gliedrige Cycloalkyleniminocarbonyl- oder Cycloalkyleniminosulfonylgruppe, wobei
der Cycloalkyleniminoteil im Kohlenstoffgerüst durch eine oder zwei C₁₋₃-Alkyl-, Hydroxy-C₁₋₃-alkyl-, C₁₋₃-Alkyloxy-C₁₋₃-alkyl-, Pyridinyl-C₁₋₃-alkyl-, Amino-C₁₋₃-alkyl-, C₃₋₆-Cycloalkylamino-C₁₋₃-alkyl-, Phenylamino-C₁₋₃-alkyl-, C₁₋₅-Alkylamino-C₁₋₃-alkyl-, Di-(C₁₋₅-alkyl)-amino-C₁₋₅-alkyl-, N-Pyrrolidinyl- C₁₋₃-alkyl-, C₁₋₃-Alkyloxycarbonyl-C₁₋₃-alkyl-, C₁₋₃-Alkyloxycarbonyl-, Aminocarbonyl-, C₁₋₃-Alkylaminocarbonyl-, Di-(C₁₋₃-alkyl)-aminocarbonyl-, Hydroxy-, C₁₋₃-Alkyloxy, C₁₋₃-Alkylamino-, Di-(C₁₋₃-alkyl)-amino-, *N*-(C₁₋₃-Alkyl)-piperazin-4-yl-C₁₋₃-alkyl-, eine Phenyl- oder eine Pyridinylgruppe substituiert sein kann mit der Maßgabe, dass bei der Substitution einer der Iminogruppe benachbarten Methylengruppe zwei Heteroatome durch mindestens zwei Kohlenstoffatome voneinander getrennt sind, und/oder
eine Methylengruppe in 3-Position einer 5-gliedrigen Cycloalkyleniminogruppe durch ein Schwefelatom ersetzt sein kann oder eine Methylengruppe in 4-Position einer 6- bis 7-gliedrigen Cycloalkyleniminogruppe durch ein Sauerstoff- oder Schwefelatom, durch eine gegebenenfalls durch eine C₁₋₃-Alkyl-, Hydroxy-, Formyl- oder C₁₋₃-Alkylcarbonylgruppe substituierte -NH-Gruppe ersetzt sein kann, wobei zusätzlich eine Methylengruppe benachbart zur voranstehend genannten gegebenenfalls substituierten -NH-Gruppe durch eine Carbonylgruppe ersetzt sein kann, mit der Maßgabe, dass
bei der Substitution der voranstehend genannten 6- bis 7-gliedrigen Cycloalkyleniminoreste, bei denen eine Methylengruppe durch ein Sauerstoff- oder Schwefelatom ersetzt ist, zwei Heteroatome durch mindestens zwei Kohlenstoffatome voneinander getrennt sind,
oder A eine Gruppe der Formel 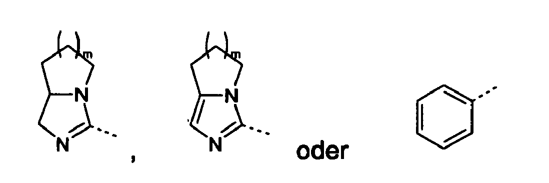 die jeweils an einem Kohlenstoffatom durch eine C₁₋₃-Alkyl-, Methylsulfonylmethyl-, Aminosulfonyl, Amino-C₁₋₃-alkyl-, C₁₋₃-Alkylamino-C₁₋₃-alkyl- oder eine Di-(C₁₋₃-alkyl)-amino-C₁₋₃-alkyl-gruppe substituiert sein kann und wobei
m die Zahl 1 oder 2 bedeutet,
R¹ ein Wasserstoff-, Fluor-, Chlor- oder Bromatom, eine Methyl-, Trifluormethyl- oder Methoxygruppe bedeutet,
R² ein Wasserstoffatom bedeutet,
X ein Stickstoffatom oder eine CH-Gruppe bedeutet,
R³ ein Wasserstoffatom oder eine Methylgruppe bedeutet,
R⁴ ein Wasserstoffatom bedeutet,
R⁵ ein Wasserstoffatom, eine Hydroxygruppe, eine OR⁹-Gruppe, eine Allyl- oder Methallylgruppe,
eine Methylgruppe, die durch eine C₁₋₃-alkyl-, Hydroxy-, OR⁹-Gruppe-, Aminocarbonyl-, Pyridin-4-yl, Pyridin-3-yl, Pyridin-2-yl, Pyrazin-2-yl, Pyrazin-3-yl- oder Phenylgruppe substituiert sein kann,
eine Phenylgruppe
bedeutet,
R⁹ eine geradkettige oder verzweigte C₁₋₄-Alkylgruppe, die gegebenenfalls durch eine Hydroxy-, eine C₁₋₃-Alkoxygruppe, eine Benzyloxy- oder eine Di-(C₁₋₃-alkyl)-aminogruppe substituiert sein kann, mit der Maßgabe, dass der Ersatz von Wasserstoffatomen des ersten Kohlenstoffatoms der geradkettigen oder verzweigten C₁₋₄-Alkylgruppe durch Substituenten aus der Gruppe Sauerstoff oder Stickstoff ausgeschlossen ist,
bedeutet,
B einen Rest der allgemeinen Formel 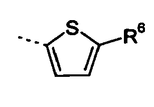 bedeutet,
R⁶ ein Chlor- oder Bromatom oder eine Ethinylgruppe bedeutet,
wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
wobei die in den voranstehend erwähnten Definitionen enthaltenen Alkyl-, Alkenyl-, Alkinyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

7. Substituierte Pyrrolidinone der allgemeinen Formel I nach einem der Ansprüche 1 bis 6, **dadurch gekennzeichnet, dass** der Rest B die Gruppe 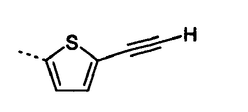 bedeutet,
deren Tautomere, deren Enantiomere, deren Diastereomere, deren Gemische und deren Salze.

8. Substituierte Pyrrolidinone der allgemeinen Formel I nach einem der Ansprüche 1 bis 6, **dadurch gekennzeichnet, dass**
der Rest B die Gruppe 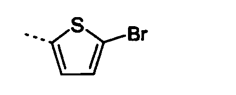 bedeutet,
deren Tautomere, deren Enantiomere, deren Diastereomere, deren Gemische und deren Salze.

9. Substituierte Pyrrolidinone der allgemeinen Formel I nach einem der Ansprüche 1 bis 8, **dadurch gekennzeichnet, dass**
der Rest A die Gruppe 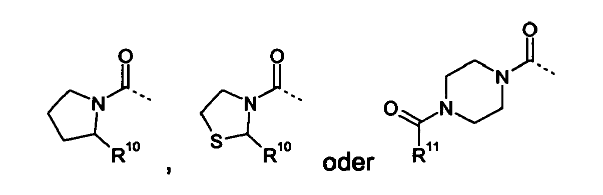 bedeutet, wobei
R¹⁰ das Wasserstoffatom, eine Methyl-, Aminomethyl-, C₁₋₃-Alkylamino-C₁₋₂-alkyl-, Di-(C₁₋₃-alkyl)-amino-C₁₋₂-alkyl-, Pyrrolidin-1-yl-methyl- oder 2-(Pyrrolidin-1-yl)-ethylgruppe,
R¹¹ das Wasserstoffatom oder eine Methylgruppe bedeutet,
deren Tautomere, deren Enantiomere, deren Diastereomere, deren Gemische und deren Salze.

10. Physiologisch verträgliche Salze der Verbindungen gemäß einem der Ansprüche 1 bis 9.

11. Arzneimittel, enthaltend eine Verbindung nach einem der Ansprüche 1 bis 9 öder ein physiologisch verträgliches Salz gemäß Anspruch 10 neben gegebenenfalls einem oder mehreren inerten Trägerstoffen und/oder Verdünnungsmitteln.

12. Verwendung einer Verbindung nach einem der Ansprüche 1 bis 9, oder eines physiologisch verträglichen Salzes gemäß Anspruch 10 zur Herstellung eines Arzneimittels zur Vorbeugung und Behandlung venöser und arterieller thrombotischer Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulcerativen Colitis, der Prophylaxe und Behandlung der Koronarthrombose, der Prophylaxe des Schlaganfalls und der Verhinderung der Occlusion von Shunts, zur antithrombotischen Unterstützung bei einer thrombolytischen Behandlung, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, zur Prophylaxe und Behandlung der rheumatoiden Arthritis, zur Verhütung oder Verhinderung von Fibrin-abhängigen Gewebsadhäsionen und/oder Narbengewebebildung sowie zur Förderung von Wundheilungsprozessen.

13. Verfahren zur Herstellung eines Arzneimittels gemäß Anspruch 11, **dadurch gekennzeichnet, dass** auf nichtchemischem Wege eine Verbindung nach einem der Ansprüche 1 bis 9 oder ein physiologisch verträgliches Salz gemäß Anspruch 10 in einen oder mehrere inerte Trägerstoffe und/oder Verdünnungsmittel eingearbeitet wird.

## Claims

1. Substituted Pyrrolidinones of general formula 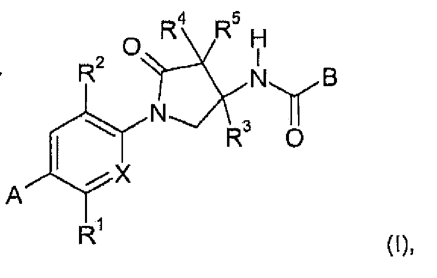 **characterised in that**
A denotes a 4- to 7-membered cycloalkyleneiminocarbonyl or cycloalkyleneiminosulphonyl group, while
the cycloalkyleneimino moiety in the carbon skeleton may be substituted by one or two fluorine atoms, one or two C₁₋₃-alkyl, C₂₋₃-alkenyl, C₂₋₃-alkynyl, hydroxy-C₁₋₃-alkyl, C₁₋₃-alkyloxy-C₁₋₃-alkyl, phenyl-C₁₋₃-alkyl, 1,1-diphenyl-C₁₋₃-alkyl, heteroaryl-C₁₋₃-alkyl, amino-C₁₋₃-alkyl, C₃₋₆- cycloalkylamino-C₁₋₃-alkyl, phenylamino-C₁₋₃-alkyl, C₁₋₅-alkylamino- C₁₋₃-alkyl, di-(C₁₋₅-alkyl)-amino-C₁₋₅-alkyl, *N*-(C₃₋₆-cycloalkyl)- C₁₋₃-alkylamino-C₁₋₃-alkyl, a 4- to 7-membered cycloalkyleneimino- C₁₋₃-alkyl, *N*-(C₁₋₃-alkylcarbonyl)-C₁₋₃-alkylamino-C₁₋₃-alkyl, carboxy- C₁₋₃-alkyl, C₁₋₃-alkyloxycarbonyl-C₁₋₃-alkyl, aminocarbonyl-C₁₋₃-alkyl, C₁₋₃-alkylaminocarbonyl-C₁₋₃-alkyl, di-(C₁₋₃-alkyl)-aminocarbonyl- C₁₋₃-alkyl, a 4- to 7-membered cycloalkyleneiminocarbonyl-C₁₋₃-alkyl, C₁₋₅-alkyloxycarbonylamino-C₁₋₃-alkyl, C₁₋₃-alkylcarbonylamino-C₁₋₃-alkyl, C₁₋₃-alkylsulphonylamino-C₁₋₃-alkyl, aminocarbonylamino-C₁₋₃-alkyl, C₁₋₅-alkylaminocarbonylamino-C₁₋₃-alkyl, di-(C₁₋₃-alkyl)- aminocarbonylamino-C₁₋₃-alkyl, C₁₋₅-alkylsulphonylamino, C₁₋₅- alkylaminocarbonylamino, carboxy, C₁₋₃-alkyloxycarbonyl, benzyloxycarbonyl, C₁₋₃-alkylcarbonyl, aminocarbonyl, C₁₋₃-alkylaminocarbonyl, di-(C₁₋₃-alkyl)-aminocarbonyl, *N*-(C₃₋₇- cycloalkyl)-C₁₋₆-alkylaminocarbonyl, *N*-(phenyl-C₁₋₃-alkyl)-C₁₋₅-alkylamino- carbonyl, a 4- to 7-membered cycloalkyleneiminocarbonyl, aminocarbonyl-C₁₋₃-alkylaminocarbonyl, hydroxy, C₁₋₃-alkyloxy, allyloxy, propargyloxy, benzyloxy, amino, C₁₋₃-alkylamino, di-(C₁₋₃-alkyl)-amino, a 4- to 7-membered cycloalkyleneimino, trifluoromethylcarbonylamino, *N*-(C₁₋₃-alkyl)-piperazin-4-yl-C₁₋₃-alkyl, a phenyl or a 5- to 6-membered heteroaryl group, with the proviso that in the substitution of a methylene group adjacent to the imino group two heteroatoms are separated from one another by at least two carbon atoms, and/or
a methylene group in the 3-position of a 5-membered cycloalkyleneimino group may be replaced by a sulphur atom, a sulphinyl or sulphonyl group or
a methylene group in the 4-position of a 6- or 7-membered cycloalkyleneimino group may be replaced by an oxygen or sulphur atom, a carbonyl, sulphinyl or sulphonyl group or by an -NH group optionally substituted by a C₁₋₃-alkyl, hydroxy, formyl or C₁₋₃-alkylcarbonyl group, while additionally a methylene group adjacent to the previously mentioned optionally substituted -NH group may be replaced by a carbonyl, sulphinyl or sulphonyl group, with the proviso that
during the substitution of the previously mentioned 6- to 7-membered cycloalkyleneimino groups, wherein a methylene group is replaced by an oxygen or sulphur atom, a sulphinyl or sulphonyl group, two heteroatoms are separated from one another by at least two carbon atoms,
a 5- to 7-membered cycloalkenyleneiminocarbonyl or cycloalkenyleneiminosulphonyl group optionally substituted by one or two C₁₋₃-alkyl, hydroxy-C₁₋₃-alkyl, C₁₋₅-alkoxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, C₃₋₆-cycloalkylamino-C₁₋₃-alkyl, phenyl, phenyl-C₁₋₃-alkyl, heteroaryl), heteroaryl-C₁₋₃-alkyl, aminocarbonyl, C₁₋₃-alkylaminocarbonyl, di-(C₁₋₃-alkyl)-aminocarbonyl or 4- to 7-membered cycloalkyleneiminocarbonyl groups, wherein the double bond is not bound to a nitrogen atom and may be fused to a 5- or 6-membered heteroaryl group,
an aminocarbonyl or aminosulphonyl group optionally substituted by one or two C₁₋₅-alkyl, C₂₋₃-alkenyl, C₂₋₃-alkynyl or C₃₋₆-cycloalkyl groups,
while the substituents may be identical or different and
in each case one of the C₁₋₅-alkyl groups may be substituted by one or two hydroxy-C₁₋₃-alkyl, C₁₋₃-alkoxy-C₁₋₃-alkyl, benzyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, C₁₋₅-alkyloxycarbonylamino-C₁₋₃-alkyl, C₃₋₆-cycloalkylamino-C₁₋₃-alkyl, aminocarbonyl, C₁₋₃-alkylamino-carbonyl, *N*-(C₃₋₇-cycloalkyl)-C₁₋₃-alkylaminocarbonyl, *N*-(phenyl-C₁₋₃-alkyl)-C₁₋₅-alkylaminocarbonyl, di-(C₁₋₃-alkyl)-aminocarbonyl or a 4- to 7-membered cycloalkyleneiminocarbonyl group,
or a group of formula 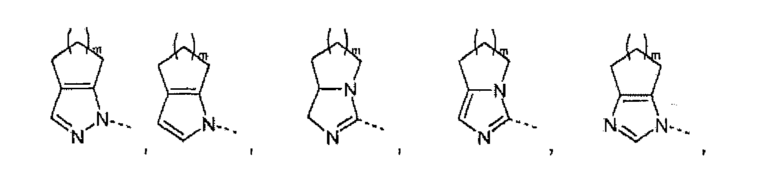 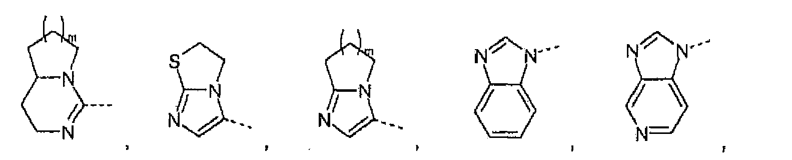 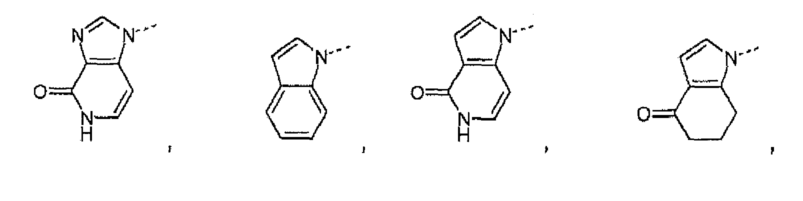 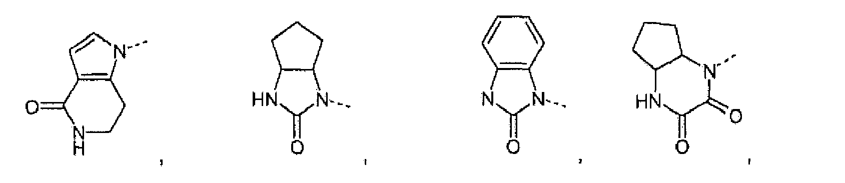 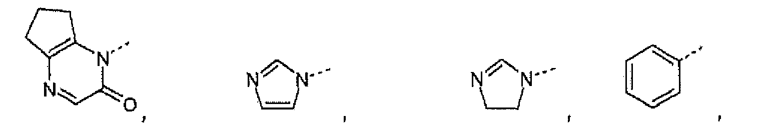 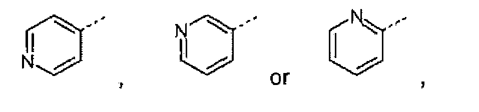 which may be substituted in each case at a carbon atom by a C₁₋₃-alkyl, C₁₋₃-alkyloxy, C₁₋₃-alkyloxycarbonyl, C₁₋₅-alkyloxycarbonylamino-C₁₋₃-alkyl, C₁₋₃-alkyl-sulphonyl, C₁₋₃-alkyl-sulphonyl-C₁₋₃-alkyl, aminosulphonyl, C₁₋₃-alkylsulphonyl-C₁₋₃-alkyl, C₁₋₃-alkylsulphonylamino-C₁₋₃-alkyl, C₁₋₃-alkylsulphonylamino, C₁₋₃-alkylcarbonylamino-C₁₋₃-alkyl, C₁₋₃-alkylcarbonylamino, amino-C₁₋₃-alkyl, C₁₋₃-alkylamino-C₁₋₃-alkyl, di-(C₁₋₃-alkyl)-amino-C₁₋₃-alkyl, hydroxy-C₁₋₃-alkyl, C₁₋₅-alkoxy-C₁₋₃-alkyl, a 4- to 7-membered cycloalkyleneimino-C₁₋₃-alkyl, morpholin-4-yl-C₁₋₃-alkyl, piperazinyl-C₁₋₃-alkyl, *N*-(C₁₋₃-alkyl)-piperazin-4-yl-C₁₋₃-alkyl, aminocarbonyl, C₁₋₃-alkylaminocarbonyl or di-(C₁₋₃-alkyl)-aminocarbonyl group and wherein
m denotes the number 1 or 2,
R¹ denotes a hydrogen or halogen atom, a C₁₋₃-alkyl or C₁₋₃-alkoxy group, while the hydrogen atoms of the C₁₋₃-alkyl or C₁₋₃-alkoxy group may optionally be wholly or partly replaced by fluorine atoms, a C₂₋₃-alkenyl, C₂₋₃-alkynyl, nitrile, nitro or amino group,
R² denotes a hydrogen or halogen atom or a C₁₋₃-alkyl group,
X denotes a nitrogen atom or a CH- group,
R³ denotes a hydrogen atom or a C₁₋₃-alkyl group,
R⁴ and R⁵ each independently of one another denote
a hydrogen atom, a hydroxy group, an OR⁹ group, a C₂₋₆-alkenyl or C₂₋₆-alkynyl group,
a straight-chain or branched C₁₋₆-alkyl group, while the hydrogen atoms of the straight-chain or branched C₁₋₆-alkyl group may optionally be wholly or partly replaced by fluorine atoms, and which may optionally be substituted by a C₃₋₅- cycloalkyl group, a nitrile, hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy- C₁₋₅-alkyloxy, C₁₋₅-alkyloxycarbonyl-C₁₋₅-alkyloxy, mercapto, C₁₋₅-alkylsulphanyl, C₁₋₅-alkylsulphonyl, carboxy, C₁₋₅-alkyloxycarbonyl, aminocarbonyl, C₁₋₅-alkylaminocarbonyl, di- (C₁₋₅-alkyl)-aminocarbonyl, C₄₋₆-cycloalkyleneiminocarbonyl, aminosulphonyl, C₁₋₆-alkylaminosulphonyl, di-(C₁₋₅-alkyl)- aminosulphonyl, C₄₋₆-cycloalkyleneiminosulphonyl, amino, C₁₋₅-alkylamino, di-(C₁₋₅-alkyl)-amino, C₁₋₅-alkylcarbonylamino, C₁₋₅-alkylsulphonylamino, *N*-(C₁₋₅-alkylsulphonyl)-C₁₋₅-alkylamino or C₃₋₆-cycloalkylcarbonylamino group, while in the 6- to 7-membered cyclic groups of the C₄₋₆- cycloalkyleneiminocarbonyl group in the cyclic moiety a methylene group in the 4-position of a 6- or 7-membered cycloalkyleneimino group may be replaced by an oxygen or sulphur atom, by a carbonyl, sulphinyl, sulphonyl or -NR⁷ group and additionally a methylene group adjacent to an above- mentioned -NR⁷ group may be replaced by a carbonyl group,
a phenyl or heteroaryl group which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₆-alkyloxy, mono-, di- or trifluoromethoxy, carboxy- and C₁₋₅-alkyloxycarbonyl groups,
a phenyl-C₁₋₅-alkyl or heteroaryl-C₁₋₅-alkyl group, which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups, and which may optionally be substituted in the C₁₋₅-alkyl moiety by a hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy, or a C₁₋₅-alkyloxycarbonyl-C₁₋₅-alkyloxy group,
a 3- to 7-membered cycloalkyl, cycloalkyleneimino, cycloalkyl- C₁₋₅-alkyl or cycloalkyleneimino-C₁₋₃-alkyl group, wherein in 4- to 7-membered cyclic groups in the cyclic moiety a methylene group may optionally be replaced by an -N(R⁷) group, an oxygen or sulphur atom or a -S(O) or -S(O)₂ group, or
wherein in 4- to 7-membered cyclic groups in the cyclic moiety two adjacent methylene groups together may optionally be replaced by a -C(O)N(R⁸) or -S(O)₂N(R⁸) group, or
wherein in 6- to 7-membered cyclic groups in the cyclic moiety three adjacent methylene groups together may optionally be replaced by a substituted-OC(O)N(R⁸) or -N(R⁸)C(O)N(R⁸) or -N(R⁸)S(O)₂N(R⁸) group,
with the proviso that a 3- to 7-membered cycloalkyl, cycloalkyleneimino, cycloalkyl-C₁₋₅-alkyl or cycloalkyleneimino- C₁₋₃-alkyl group as hereinbefore defined wherein two heteroatoms selected from oxygen and nitrogen are separated from one another by precisely one optionally substituted -CH₂ group, is excluded,
while a 3- to 7-membered cycloalkyl, cycloalkyleneimino, cycloalkyl- C₁₋₅-alkyl or cycloalkyleneimino-C₁₋₃-alkyl group as hereinbefore defined may be substituted at one or two -CH₂ groups by one or two C₁₋₃-alkyl groups in each case, with the proviso that R⁴ and R⁵ may not simultaneously be defined as hydroxy or OR⁹ groups, or
R⁴ and R⁵ together with the carbon atom to which they are bound form a C₃₋₈- cycloalkyl or C₃₋₈-cycloalkenyl group,
while one of the methylene groups of a C₄₋₈-cycloalkyl group may be replaced by an oxygen or sulphur atom or a -N(R⁷), or a carbonyl, sulphinyl or sulphonyl group, and/or
two directly adjacent methylene groups of a C₄₋₈-cycloalkyl group may together be replaced by a -C(O)N(R⁸) or -S(O)₂N(R⁸) group, and/or
three directly adjacent methylene groups of a C₈₋₈-cycloalkyl group may together be replaced by a -OC(O)N(R⁸), -N(R⁸)C(O)N(R⁸) or -N(R⁸)S(O)₂N(R⁸) group,
while 1 to 3 carbon atoms of a Cₐ₋₈-cycloalkyl group may optionally be substituted independently of one another by in each case one or two identical or different halogen atoms or C₁₋₅-alkyl, nitrile, hydroxy, C₁₋₅-alkyloxy, C₁₋₅-alkylcarbonyloxy, carboxy-C₁₋₅-alkyl, C₁₋₅- alkyloxycarbonyl-C₁₋₅-alkyl, C₁₋₅-alkylsulphanyl, C₁₋₅-alkylsulphonyl, carboxy, C₁₋₅-alkyloxycarbonyl, aminocarbonyl, C₁₋₅-alkylaminocarbonyl, di-(C₁₋₅-alkyl)-aminocarbonyl, C₃₋₆- cycloalkyleneiminocarbonyl, aminosulphonyl, C₁₋₅-alkylaminosulphonyl, di-(C₁₋₅-alkyl)-aminosulphonyl, C₃₋₆- cycloalkyleneiminosulphonyl, amino, C₁₋₅-alkylamino, di-(C₁₋₅-alkyl)- amino, C₁₋₅-alkylcarbonylamino, C₁₋₅-alkylsulphonylamino, *N*-(C₁₋₅-alkylsulphonyl)-C₁₋₅-alkylamino or C₃₋₆-cycloalkylcarbonyl- amino groups,
while 1 to 2 carbon atoms of a C₃₋₈-cycloalkenyl group may optionally be substituted independently of one another by in each case a C₁₋₅- alkyl, nitrile, carboxy-C₁₋₅-alkyl, C₁₋₅-alkyloxycarbonyl-C₁₋₅-alkyl, carboxy, C₁₋₅-alkyloxycarbonyl, aminocarbonyl, C₁₋₅-alkylaminocarbonyl, di-(C₁₋₅-alkyl)-aminocarbonyl, C₃₋₆- cycloalkyleneiminocarbonyl, aminosulphonyl, C₁₋₅-alkylaminosulphonyl, di-(C₁₋₅-alkyl)-aminosulphonyl, C₃₋₆- cycloalkyleneiminosulphonyl groups,
and 1 to 2 carbon atoms of a C₄₋₈-cycloalkenyl group which are not linked to another carbon atom by a double bond, may optionally be substituted independently of one another by a fluorine atom or a hydroxy, C₁₋₅-alkyloxy, C₁₋₅-alkylcarbonyloxy, C₁₋₆-alkylsulphanyl, C₁₋₅-alkylsulphonyl, amino, C₁₋₅-alkylamino di-(C₁₋₅-alkyl)-amino, C₁₋₅-alkylcarbonylamino, C₁₋₅-alkylsulphonylamino, *N*-(C₁₋₅-alkylsulphonyl)-C₁₋₅-alkylamino or C₃₋₆-cycloalkylcarbonyl- amino groups,
with the proviso that a C₃₋₈-cycloalkyl or C₃₋₈-cycloalkenyl group of this kind formed from R⁴ and R⁵ together,
wherein two heteroatoms in the cyclic group comprising oxygen and nitrogen are separated from one another by precisely one optionally substituted -CH₂ group, and/or
wherein one or both methylene groups of the cyclic group which are directly connected to the carbon atom to which the groups R⁴ and R⁵ are bound, are replaced by a heteroatom selected from among oxygen, nitrogen and sulphur, and/or
wherein a substituent bound to the cyclic group, which is **characterised in that** a heteroatom selected from among oxygen, nitrogen, sulphur and halogen atom is bound directly to the cyclic group, is separated from another heteroatom selected from among oxygen, nitrogen and sulphur, with the exception of the sulphone group, by precisely one optionally substituted methylene group, and/or
wherein two oxygen atoms are directly joined together,
is excluded,
R⁷ in each case independently of one another denotes a hydrogen atom, a hydroxy, a formyl, a C₁₋₅-alkyl, C₁₋₆-alkylcarbonyl, C₁₋₅-alkyloxycarbonyl or C₁₋₅-alkylsulphonyl group,
R⁸ in each case independently of one another denotes a hydrogen atom or a C₁₋₅-alkyl group,
R⁹ denotes a straight-chain or branched d₁₋₆-alkyl group,
while the hydrogen atoms of the straight-chain or branched C₁₋₆-alkyl group may optionally be wholly or partly replaced by fluorine atoms, and which may optionally be substituted by a C₃₋₅-cycloalkyl group, a hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₆-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, carboxy, C₁₋₅-alkyloxycarbonyl, aminocarbonyl, C₁₋₅-alkylaminocarbonyl, di-(C₁₋₆-alkyl)-aminocarbonyl, C₄₋₆-cycloalkyleneiminocarbonyl, C₁₋₅-alkyloxycarbonylamino, amino, C₁₋₅-alkylamino, di-(C₁₋₅-alkyl)-amino, C₁₋₅-alkylcarbonylamino, C₁₋₅-alkylsulphonylamino, *N*-(C₁₋₅-alkylsulphonyl)-C₁₋₅-alkylamino or C₃₋₆-cycloalkylcarbonylamino group,
while in the 6- to 7-membered cyclic groups of the C₄₋₆- cycloalkyleneiminocarbonyl group in the cyclic moiety |a| methylene group in the 4-position of a 6- or 7-membered cycloalkyleneimino group may be replaced by an oxygen or sulphur atom, by a carbonyl, sulphinyl, sulphonyl or -NR⁷ group and additionally a methylene group adjacent to an above- mentioned -NR⁷ group may be replaced by a carbonyl group,
with the proviso that replacement of hydrogen atoms of the first carbon atom of the straight-chain or branched C₁₋₆-alkyl group by substltuents from the group comprising oxygen, sulphur or nitrogen is excluded,
a phenyl, heteroaryl, phenyl-C₁₋₆-alkyl or heteroaryl-C₁₋₅-alkyl group, which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups,
a 3- to 7-membered cycloalkyl, cycloalkyl-C₁₋₅-alkyl or cycloalkyleneimino- C₂₋₃-alkyl group,
wherein in 4- to 7-membered cyclic groups in the cyclic moiety a methylene group may optionally be replaced by a -N(R⁷) group, an oxygen or sulphur atom or a -S(O) or -S(O)₂ group, or
wherein in 4- to 7-membered cyclic groups in the cyclic moiety two adjacent methylene groups together may optionally be replaced by a -C(O)N(R⁸) or -S(O)₂N(R⁸) group, or
wherein in 6- to 7-membered cyclic groups in the cyclic moiety three adjacent methylene groups together may optionally be replaced by a substituted -OC(O)N(R⁸) or -N(R⁸)C(O)N(R⁸) or -N(R⁸)S(O)₂N(R⁸) group,
with the proviso that a 3- to 7-membered cycloalkyl, cycloalkyl- C₁₋₅-alkyl or cycloalkyleneimino-C₂₋₃-alkyl group as hereinbefore defined wherein two heteroatoms selected from oxygen and nitrogen are separated from one another by precisely one optionally substituted -CH₂ group, is excluded,
while a 3- to 7-membered cycloalkyl, cycloalkyl-C₁₋₅-alkyl or cycloalkyloneimino-C₂₋₃-alkyl group as hereinbefore defined may be substituted at one or two -CH₂ groups by one or two C₁₋₃-alkyl groups in each case,
B denotes a group of general formula 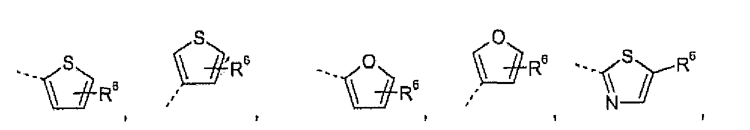 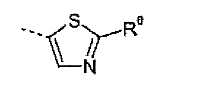 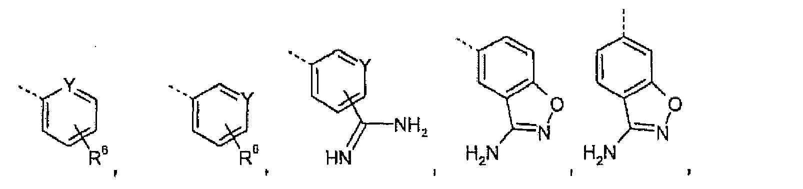 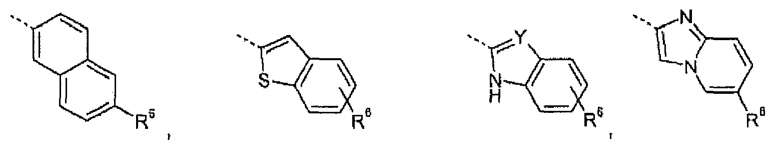 or 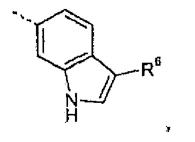
Y denotes a nitrogen atom or a CH- group,
R⁶ denotes a hydrogen, a halogen atom, a nitrile group, a C₂₋₃-alkenyl, C₂₋₃-alkynyl, a C₁₋₃-alkyl group, or a C₁₋₃-alkoxy group, while the hydrogen atoms of the C₁₋₃-alkyl or C₁₋₃-alkoxy group may optionally be wholly or partly replaced by fluorine atoms,
while, unless otherwise stated, by the "heteroaryl group" mentioned hereinbefore in the definitions is meant a monocyclic 5- or 6-membered heteroaryl group, while
the 6-membered heteroaryl group contains one, two or three nitrogen atoms and
the 5-membered heteroaryl group contains an imino group optionally substituted by a C₁₋₃-alkyl, phenyl or phenyl-C₁₋₃-alkyl group, or an oxygen or sulphur atom or
an imino group optionally substituted by a C₁₋₃-alkyl, phenyl, amino-C₂₋₃-alkyl, C₁₋₃-alkylamino-C₂₋₃-alkyl, di-(C₁₋₃-alkyl)-amino-C₂₋₃-alkyl, a C₃₋₆-cycloalkyleneimino-C₁₋₃-alkyl or phenyl-C₁₋₃-alkyl group or an oxygen or sulphur atom and additionally a nitrogen atom or
an imino group optionally substituted by a C₁₋₃-alkyl or phenyl-C₁₋₃-alkyl group and two or three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms
and the bond is effected via a nitrogen atom or a carbon atom of the heterocyclic moiety or a fused-on phenyl ring,
while, unless otherwise stated, by the term "halogen atom" mentioned hereinbefore in the definitions is meant an atom selected from among fluorine, chlorine, bromine and iodine,
while the alkyl, alkenyl, alkynyl and alkoxy groups contained in the afore-mentioned definitions which have more than two carbon atoms may unless otherwise stated be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated to the contrary, may be wholly or partly replaced by fluorine atoms,
the tautomers, the enantiomers, the diastereomers, the mixtures thereof and the salts thereof.

2. Substituted pyrrolidinones of general formula I according to claim 1, **characterised in that**
A denotes a 4- to 7-membered cycloalkyleneiminocarbonyl or cycloalkyleneiminosulphonyl group, while
the cycloalkyleneimino moiety in the carbon skeleton may be substituted by one or two fluorine atoms, one or two C₁₋₃-alkyl, C₂₋₃-alkenyl, C₂₋₃-alkynyl, hydroxy C₁₋₃-alkyl, C₁₋₃-alkyloxy-C₁₋₃-alkyl, phenyl-C₁₋₃-alkyl, 1,1-diphenyl-C₁₋₃-alkyl, heteroaryl-C₁₋₃-alkyl, amino-C₁₋₃-alkyl, C₃₋₆-cycloalkylamino-C₁₋₃-alkyl, phenylamino-C₁₋₃-alkyl, C₁₋₅-alkylamino-C₁₋₃-alkyl, di-(C₁₋₅-alkyl)-amino-C₁₋₅-alkyl, *N*-(C₃₋₆-cycloalkyl)-C₁₋₃-alkylamino-C₁₋₃-alkyl, a 4- to 7-membered cycloalkyleneimino-C₁₋₃-alkyl, *N*-(C₁₋₃-alkylcarbonyl)-C₁₋₃-alkylamino-C₁₋₃-alkyl, carboxy-C₁₋₃-alkyl, C₁₋₃-alkyloxycarbonyl-C₁₋₃-alkyl, aminocarbonyl-C₁₋₃-alkyl, C₁₋₃-alkylaminocarbonyl-C₁₋₃-alkyl, di-(C₁₋₃-alkyl)-aminocarbonyl-C₁₋₃-alkyl, a 4- to 7-membered cycloalkyleneiminocarbonyl-C₁₋₃-alkyl, C₁₋₅-alkyloxycarbonylamino-C₁₋₃-alkyl, C₁₋₃-alkylcarbonylamino-C₁₋₃-alkyl, C₁₋₅-alkylsulphonylamino-C₁₋₃-alkyl, aminocarbonylamino-C₁₋₃-alkyl, C₁₋₅-alkylaminocarbonylamino-C₁₋₃-alkyl, di-(C₁₋₃-alkyl)-aminocarbonylamino- C₁₋₃-alkyl, C₁₋₅-alkylsulphonylamino, C₁₋₅-alkylaminocarbonylamino, carboxy, C₁₋₃-alkyloxycarbonyl, benzyloxycarbonyl, C₁₋₃-alkylcarbonyl, aminocarbonyl, C₁₋₃-alkylaminocarbonyl, di-(C₁₋₃-alkyl)-aminocarbonyl, *N*-(C₃₋₇-cycloalkyl)-C₁₋₅- alkylaminocarbonyl, *N*-(phenyl-C₁₋₃-alkyl)-C₁₋₅-alkylaminocarbonyl, a 4- to 7- membered cycloalkyleneiminocarbonyl, aminocarbonyl-C₁₋₃-alkylaminocarbonyl, hydroxy, C₁₋₃-alkyloxy, allyloxy, propargyloxy, benzyloxy, amino, C₁₋₃-alkylamino, di-(C₁₋₃-alkyl)-amino, a 4- to 7-membered cycloalkyleneimino, trifluoromethylcarbonylamino, *N*-(C₁₋₃-alkyl)-piperazin-4-yl-C₁₋₃-alkyl, a phenyl or a 5- to 6-membered heteroaryl group, with the proviso that in the substitution of a methylene group adjacent to the imino group two heteroatoms are separated from one another by at least two carbon atoms, and/or
a methylene group in the 3-position of a 5-membered cycloalkyleneimino group may be replaced by a sulphur atom, a sulphinyl or sulphonyl group or
a methylene group in the 4-position of a 6- or 7-membered cycloalkyleneimino group may be replaced by an oxygen or sulphur atom, a carbonyl, sulphinyl or sulphonyl group or by an -NH group optionally substituted by a C₁₋₃-alkyl, hydroxy, formyl or C₁₋₃-alkylcarbonyl group, while additionally a methylene group adjacent to the previously mentioned optionally substituted -NH group may be replaced by a carbonyl, sulphinyl or sulphonyl group, with the proviso that
in the substitution of the previously mentioned 6- to 7-membered cyclo- alkyleneimino groups, wherein a methylene group is replaced by an oxygen or sulphur atom, a sulphinyl or sulphonyl group, two heteroatoms are separated from one another by at least two carbon atoms,
a 5- to 7-membered cycloalkenyleneiminocarbonyl or cycloalkenyleneiminosulphonyl group optionally substituted by one or two C₁₋₃-alkyl, hydroxy-C₁₋₃-alkyl, C₁₋₅-alkoxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, C₃₋₆-cycloalkylamino-C₁₋₃-alkyl, phenyl, phenyl-C₁₋₃-alkyl, heteroaryl, heteroaryl-C₁₋₃-alkyl, aminocarbonyl, C₁₋₃-alkylaminocarbonyl, di-(C₁₋₃-alkyl)-aminocarbonyl or 4- to 7-membered cycloalkyleneiminocarbonyl groups, while the double bond is not bound to a nitrogen atom and may be fused to a 5- or 6-membered heteroaryl group,
an aminocarbonyl or aminosulphonyl group optionally substituted by one or two C₁₋₆-alkyl, C₂₋₃-alkenyl, C₂₋₃-alkynyl, or C₃₋₈-cycloalkyl groups,
while the substituents may be identical or different and
in each case one of the C₁₋₅-alkyl groups may be substituted by one or two hydroxy-C₁₋₃-alkyl, C₁₋₃-alkoxy-C₁₋₃-alkyl, benzyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, C₁₋₅-alkyloxycarbonylamino-C₁₋₃-alkyl, C₃₋₆-cycloalkylamino-C₁₋₃-alkyl, aminocarbonyl, C₁₋₃-alkylamino-carbonyl, *N*-(C₃₋₇-cycloalkyl)-C₁₋₃-alkylaminocarbonyl, *N*-(phenyl-C₁₋₃-alkyl)-C₁₋₅-alkylaminocarbonyl, di-(C₁₋₃-alkyl)-aminocarbonyl or a 4- to 7-membered cycloalkyleneiminocarbonyl group,
or a group of formula 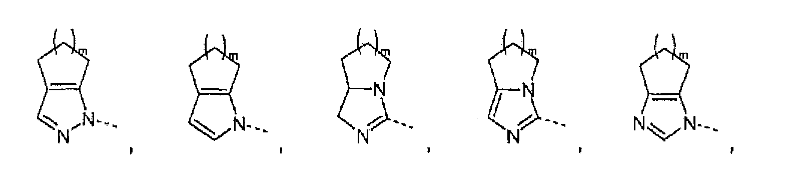 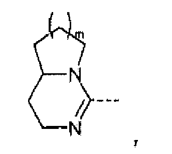 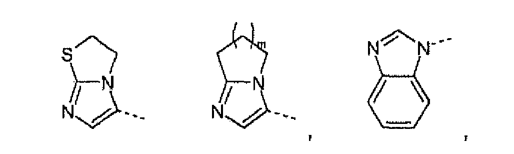 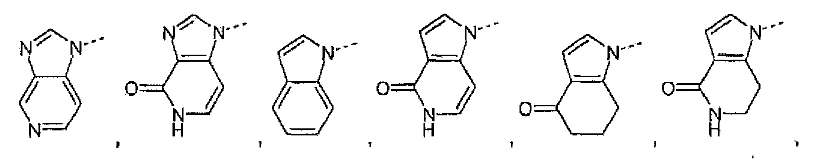 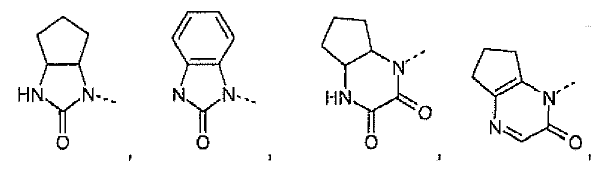 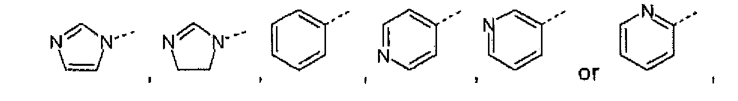 which may be substituted in each case at a carbon atom by a C₁₋₃-alkyl, C₁₋₃-alkyloxy, C₁₋₃-alkyloxycarbonyl, C₁₋₅-alkyloxycarbonylamino-C₁₋₃-alkyl, C₁₋₃-alkyl-sulphonyl, C₁₋₃-alkyl-sulphonyl-C₁₋₃-alkyl, aminosulphonyl, C₁₋₃-alkylsulphonyl-C₁₋₃-alkyl, C₁₋₃-alkylsulphonylamino-C₁₋₃-alkyl, C₁₋₃-alkylsulphonylamino, C₁₋₃-alkylcarbonylamino-C₁₋₃-alkyl, C₁₋₃-alkylcarbonylamino, amino-C₁₋₃-alkyl, C₁₋₃-alkylamino-C₁₋₃-alkyl, di-(C₁₋₃-alkyl)-amino-C₁₋₃-alkyl, hydroxy-C₁₋₃-alkyl, C₁-₅-alkoxy-C₁-₃-alkyl, a 4- to 7-membered cycloalkyleneimino-C₁₋₃-alkyl, morpholin-4-yl-C₁₋₃-alkyl, piperazinyl-C₁₋₃-alkyl, *N*-(C₁₋₃-alkyl)-piperazin-4-yl-C₁₋₃-alkyl, aminocarbonyl, C₁₋₃-alkylaminocarbonyl or di-(C₁₋₃-alkyl)-aminocarbonyl group and wherein
m denotes the number 1 or 2,
R¹ denotes a hydrogen or halogen atom, a C₁₋₃-alkyl or C₁₋₃-alkoxy group, while the hydrogen atoms of the C₁₋₃-alkyl or C₁₋₃-alkoxy group may optionally be wholly or partly replaced by fluorine atoms, a C₂₋₃-alkenyl, C₂₋₃-alkynyl, nitrile, nitro or amino group,
R² denotes a hydrogen or halogen atom or a C₁₋₃-alkyl group,
X denotes a nitrogen atom or a CH- group,
R³ denotes a hydrogen atom or a C₁₋₃-alkyl group,
R⁴ and R⁵ each independently of one another denote
a hydrogen atom, a hydroxy group, an OR⁹ group, a C₂₋₆-alkenyl or C₂₋₆-alkynyl group,
a straight-chain or branched C₁₋₅-alkyl group, while the hydrogen atoms of the straight-chain or branched C₁₋₆-alkyl group may optionally be wholly or partly replaced by fluorine atoms, and which may optionally be substituted by a C₃₋₅- cycloalkyl group, a nitrile, hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy- C₁₋₅-alkyloxy, C₁₋₅-alkyloxycarbonyl-C₁₋₅-alkyloxy, mercapto,
C₁₋₅-alkylsulphanyl, C₁₋₅-alkylsulphonyl, carboxy, C₁₋₅-alkyloxycarbonyl, aminocarbonyl, C₁₋₅-alkylaminocarbonyl, di- (C₁₋₅-alkyl)-aminocarbonyl, C₄₋₆-cycloalkyleneiminocarbonyl, aminosulphonyl, C₁₋₅-alkylaminosulphonyl, di-(C₁₋₅calkyl)- aminosulphonyl, C₄₋₆-cycloalkyleneiminosulphonyl, amino, C₁₋₅-alkylamino, di-(C₁₋₅-alkyl)-amino, C₁₋₅alkylcarbonylamino, C₁₋₅-alkylsulphonylamino, *N*-(C₁₋₅-alkylsulphonyl)-C₁₋₅-alkylamino or C₃₋₆-cycloalkyloarbonylamino group,
while in the 6- to 7-membered cyclic groups of the C₄₋₆- cycloalkyleneiminocarbonyl group in the cyclic moiety a methylene group in the 4-position of a 6- or 7-membered cycloalkyleneimino group may be replaced by an oxygen or sulphur atom, by a carbonyl, sulphinyl, sulphonyl or -NR⁷ group and additionally a methylene group adjacent to an above- mentioned -NR⁷ group may be replaced by a carbonyl group,
a phenyl or heteroaryl group
which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups,
a phenyl-C₁₋₅-alkyl or heteroaryl-C₁₋₅-alkyl group,
which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups, and which may optionally be substituted in the C₁₋₅-alkyl-molety by a hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy, or a C₁₋₅-alkyloxycarbonyl-C₁₋₅-alkyloxy group, a 3- to 7-membered cycloalkyl, cycloalkyleneimino, cycloalkyl- C₁₋₅-alkyl or cycloalkyleneimino-C₁₋₃-alkyl group,
wherein in 4- to 7-membered cyclic groups in the cyclic moiety a methylene group may optionally be replaced by an -N(R⁷) group, an oxygen or sulphur atom or a -S(O) or -S(O)₂ group, or
wherein in 4- to 7-membered cyclic groups in the cyclic moiety two adjacent methylene groups together may optionally be replaced by a -C(O)N(R⁸) or -S(O)₂N(R⁸) group, or
wherein in 6- to 7-membered cyclic groups in the cyclic moiety three adjacent methylene groups together may optionally be replaced by a substituted -OC(O)N(R⁸) or -N(R⁸)C(O)N(R⁸) or -N(R⁸)S(O)₂N(R⁸) group,
with the proviso that a 3- to 7-membered cycloalkyl, cycloalkyleneimino, cycloalkyl-C₁₋₅-alkyl or cycloalkyleneimino- C₁₋₃-alkyl group as hereinbefore defined wherein two heteroatoms selected from oxygen and nitrogen are separated from one another by precisely one optionally substituted -CH₂ group, is excluded,
while a 3- to 7-membered cycloalkyl, cycloalkyleneimino, cycloalkyl- C₁₋₅-alkyl or cycloalkyleneimino-C₁₋₃-alkyl group as hereinbefore defined may be substituted at one or two -CH₂ groups by one or two C₁₋₃-alkyl groups in each case,
with the proviso that R⁴ and R⁵ may not simultaneously be defined as hydroxy or OR⁹ groups, or
R⁴ and R⁵ together with the carbon atom to which they are bound form a C₃₋₈- cycloalkyl, or C₃₋₈-cycloalkenyl group, while one of the methylene groups of a C₄₋₈-cycloalkyl group may be replaced by an oxygen or sulphur atom or an -N(R⁷), or a carbonyl, sulphinyl or sulphonyl group, and/or
two directly adjacent methylene groups of a C₄₋₈-cycloalkyl group may together be replaced by a -C(O)N(R⁸) or -S(O)₂N(R⁸) group, and/or
three directly adjacent methylene groups of a C₆₋₈-cycloalkyl group may together be replaced by a -OC(O)N(R⁸), -N(R⁸)C(C)N(R⁸) or -N(R⁸)S(O)₂N(R⁸) group,
while 1 to 3 carbon atoms of a C₃₋₈-cycloalkyl group may optionally be substituted Independently of one another by in each case one or two identical or different halogen atoms or C₁₋₅-alkyl, nitrile, hydroxy, C₁₋₅-alkyloxy, C₁₋₅-alkylcarbonyloxy, carboxy-C₁₋₅-alkyl, C₁₋₅- alkyloxycarbonyl-C₁₋₅-alkyl, C₁₋₅-alkylsulphanyl, C₁₋₅-alkylsulphonyl, carboxy, C₁₋₅-alkyloxycarbonyl aminocarbonyl, C₁₋5-alkylaminocarbonyl, di-(C₁₋₅-alkyl)-aminocarbonyl, C₃₋₈- cycloalkyleneiminocarbonyl, aminosulphonyl, C₁₋₅-alkylaminosulphonyl, di-(C₁₋₅-alkyl)-aminosulphonyl, C₃₋₆- cycloalkyleneiminosulphonyl, amino, C₁₋₅-alkylamino, di-(C₁₋₅-alkyl)- amino, C₁₋₅-alkylcarbonylamino, C₁₋₅-alkylsulphonylamino, *N-*(C₁₋₅-alkylsulphonyl)-C₁₋₅-alkylamino or C₃₋₈-cycloalkylcarbonyl- amino groups,
while 1 to 2 carbon atoms of a C₃₋₈-cycloalkenyl group may optionally be substituted independently of one another by in each case a C₁₋₅- alkyl, nitrile, carboxy-C₁₋₅-alkyl, C₁₋₆-alkyloxycarbonyl-C₁₋₅-alkyl, carboxy, C₁₋₅-alkyloxycerbonyl, aminocarbonyl, C₁₋₅-alkylaminocarbonyl, di-(C₁₋₅-alkyl)-aminocarbonyl, C₃₋₆- cycloalkyleneiminocarbonyl, aminosulphonyl, C₁₋₅-alkylaminosulphonyl, di-(C₁₋₅-alkyl)-aminosulphonyl, C₃₋₆- cycloalkyleneiminosulphonyl groups,
and 1 to 2 carbon atoms of a C₄₋₈-cycloalkenyl group which are not linked to another carbon atom by a double bond may optionally be substituted independently of one another by a fluorine atom or a hydroxy, C₁₋₅-alkyloxy, C₁₋₅-alkylcarbonyloxy, C₁₋₅-alkylsulphanyl, C₁₋₅-alkylsulphonyl, amino, C₁₋₅-alkylamino, di-(C₁₋₅-alkyl)-amino, C₁₋₅-alkylcarbonylamino, C₁₋₅-alkylsulphonylamino, *N-*(C₁₋₅-alkylsulphonyl)-C₁₋₅-alkylamino or C₃₋₈-cycloalkylcarbonyl- amino groups,
with the proviso that a C₃₋₈-cycloalkyl or C₃₋₈-cycloalkenyl group of this kind formed from R⁴ and R⁶ together,
wherein two heteroatoms in the cyclic group comprising oxygen and nitrogen are separated from one another by precisely one optionally substituted -CH₂ group, and/or
wherein one or both methylene groups of the cyclic group which are directly connected to the carbon atom to which the groups R⁴ and R⁵ are bound, are replaced by a heteroatom selected from among oxygen, nitrogen and sulphur, and/or
wherein a substituent bound to the cyclic group, which is **characterised in that** a heteroatom selected from among oxygen, nitrogen, sulphur and halogen atom is bound directly to the cyclic group, is separated from another heteroatom selected from among oxygen, nitrogen and sulphur, with the exception of the sulphone group, by precisely one optionally substituted methylene group, and/or
wherein two oxygen atoms are directly joined together,
is excluded,
R⁷ each independently of one another denote a hydrogen atom, a hydroxy, a formyl, a C₁₋₅-alkyl, C₁₋₅-alkylcarbonyl, C₁₋₅-alkyloxycarbonyl or C₁₋₅-alkylsulphonyl group,
R⁸ each independently of one another denote a hydrogen atom or a C₁₋₅-alkyl group,
R⁹ denotes a straight-chain or branched C₁₋₆-alkyl group, while the hydrogen atoms of the straight-chain or branched C₁₋₆-alkyl group may optionally be wholly or partly replaced by fluorine atoms, and which may optionally be substituted by a C₃₋₅-cycloalkyl group, a hydroxy, a C₁₋₆-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy, C₁₋₅alkyloxycarbonyl-C₃₋₅-alkyloxy, carboxy, C₁₋₅-alkyloxycarbonyl, aminocarbonyl, C₁₋₅-alkylaminocarbonyl, di-(C₁₋₅-alkyl)-aminocarbonyl, C₄₋₆-cycloalkyleneiminocarbonyl, C₁₋₅-alkyloxycarbonylamino, amino, C₁₋₅alkylamino, di-(C₁₋₅-alkyl)-amino, C₁₋₅-alkylcarbonylamino, C₁₋₅-alkylsulphonylamino, *N-*(C₁₋₅-alkylsulphonyl)-C₁₋₅-alkylamino or C₃₋₆-cycloalkylcarbonylamino group,
while in the 6- to 7-membered cyclic groups of the C₄₋₆- cycloalkyleneiminocarbonyl group in the cyclic moiety a methylene group in the 4-position of a 6- or 7-membered cycloalkyleneimino group may be replaced by an oxygen or sulphur atom, by a carbonyl, sulphinyl, sulphonyl or -NR⁷ group and additionally a methylene group adjacent to an above- mentioned -NR⁷ group may be replaced by a carbonyl group,
with the proviso that the replacement of hydrogen atoms of the first carbon atom of the straight-chain or branched C₁₋₆-alkyl group by substituents from the group comprising oxygen, sulphur or nitrogen is excluded,
a phenyl, heteroaryl, phenyl-C₁₋₅-alkyl or heteroaryl-C₁₋₅-alkyl group, which may optionally be mono- to trisubstituted in the phenyl or heteroaryl moiety by identical or different substituents selected from among halogen atoms, C₁₋₅-alkyl, di-(C₁₋₆-alkyl)-amino, hydroxy, C₁₋₅-alkyloxy, mono-, di- or trifluoromethoxy, carboxy- and C₁₋₅-alkyloxycarbonyl groups,
a 3- to 7-membered cycloalkyl, cycloalkyl-C₁₋₅-alkyl or cycloalkyleneimino- C₂₋₃-alkyl group,
wherein in 4- to 7-membered cyclic groups in the cyclic moiety a methylene group may optionally be replaced by a -N(R⁷) group, an oxygen or sulphur atom or a -S(O) or -S(O)₂ group, or
wherein in 4- to 7-membered cyclic groups in the cyclic moiety two adjacent methylene groups together may optionally be replaced by a -C(O)N(R⁸) or -S(O)₂N(R⁸) group, or
wherein in 6- to 7-membered cyclic groups in the cyclic moiety three adjacent methylene groups together may optionally be replaced by a substituted -OC(O)N(R⁸) or-N(R⁸)C(O)N(R⁸) or -N(R⁸)S(O)₂N(R⁸) group,
with the proviso that a 3- to 7-membered cycloalkyl, cycloalkyl- C₁₋₅-alkyl or cycloalkyleneimino-C₂₋₃-alkyl group as hereinbefore defined wherein two heteroatoms selected from oxygen and nitrogen are separated from one another by precisely one optionally substituted -CH₂ group, is excluded,
while a 3- to 7-membered cycloalkyl, cycloalkyl-C₁₋₅-alkyl or cycloalkyleneimino-C₂₋₃-alkyl group as hereinbefore defined may be substituted at one or two -CH₂ groups by one or two C₁₋₃-alkyl groups in each case,
B denotes a group of general formula 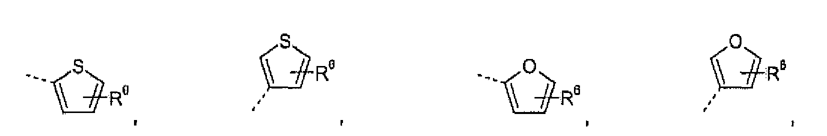 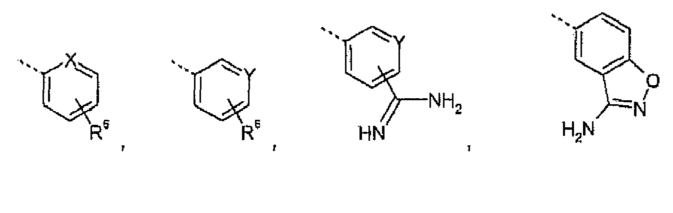 or 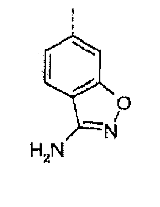
Y denotes a nitrogen atom or a CH- group,
R⁶ denotes a hydrogen, a halogen atom, a nitrile group, a C₂₋₃-alkenyl, C₂₋₃-alkynyl, a C₁₋₃-alkyl group, or a C₁₋₃-alkoxy group, while the hydrogen atoms of the C₁₋₃-alkyl or C₁₋₃-alkoxy group may optionally be wholly or partly replaced by fluorine atoms,
while, unless otherwise stated, by the "heteroaryl group" mentioned hereinbefore in the definitions is meant a monocyclic 5- or 6-membered heteroaryl group, while
the 6-membered heteroaryl group contains one, two or three nitrogen atoms and
the 5-membered heteroaryl group contains an imino group optionally substituted by a C₁₋₃-alkyl, phenyl or phenyl-C₁₋₃-alkyl group, or an oxygen or sulphur atom or
an imino group optionally substituted by a C₁₋₃-alkyl, phenyl, amino-C₂₋₃-alkyl, C₁₋₃-alkylamino-C₂₋₃-alkyl, di-(C₁₋₃-alkyl)-amino-C₂₋₃-alkyl, a C₃₋₆-cycloalkyleneimino-C₁₋₃-alkyl or phenyl-C₁₋₃-alkyl group or an oxygen or sulphur atom and additionally a nitrogen atom or
an imino group optionally substituted by a C₁₋₃-alkyl or phenyl-C₁₋₃-alkyl group and two or three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms
and the bond is effected via a nitrogen atom or a carbon atom of the heterocyclic moiety or a fused-on phenyl ring,
while, unless otherwise stated, by the term "halogen atom" mentioned hereinbefore in the definitions is meant an atom selected from among fluorine, chlorine, bromine and iodine,
while the alkyl, alkenyl, alkynyl and alkoxy groups contained in the afore-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated to the contrary, may be wholly or partly replaced by fluorine atoms,
the tautomers, the enantiomers, the diastereomers, the mixtures thereof and the salts thereof.

3. Substituted pyrrolidinones of general formula I according to claim 1, **characterised in that**
A denotes a 4- to 7-membered cycloalkyleneiminocarbonyl or cycloalkyleneiminosulphonyl group, while
the cycloalkyleneimino moiety in the carbon skeleton may be substituted by one or two fluorine atoms, one or two C₁₋₃-alkyl, C₂₋₃-alkenyl, C₂₋₃-alkynyl, hydroxy- C₁₋₃-alkyl, C₁₋₃-alkyloxy-C₁₋₃-alkyl, phenyl-C₁₋₃-alkyl, 1,1-diphenyl-C₁₋₃-alkyl, heteroaryl-C₁₋₃-alkyl, amino-C₁₋₃-alkyl, C₃₋₆-cycloalkylamino-C₁₋₃-alkyl, phenylamino-C₁₋₃-alkyl, C₁₋₅-alkylamino-C₁₋₃-alkyl, di-(C₁₋₅-alkyl)-amino-C₁₋₅-alkyl, *N-*(C₃₋₈-cycloalkyl)-C₁₋₃-alkylamino-C₁₋₃-alkyl, a 4- to 7-membered cycloalkyleneimino-C₁₋₃-alkyl, *N-*(C₁₋₃-alkylcarbonyl)-C₁₋₃-alkylamino-C₁₋₃-alkyl, carboxy-C₁₋₃-alkyl, C₁₋₃-alkyloxycarbonyl-C₁₋₃-alkyl, aminocarbonyl-C₁₋₃-alkyl, C₁₋₃-alkylaminocarbonyl-C₁₋₃-alkyl, di-(C₁₋₃-alkyl)-aminocarbonyl-C₁₋₃-alkyl, a 4- to 7-membered cycloalkyleneiminocarbonyl-C₁₋₃-alkyl, C₁₋₅alkyloxycarbonylamino-C₁₋₃-alkyl, C₁₋₃-alkylcarbonylamino-C₁₋₃-alkyl, C₁₋₃-alkylsulphonylamino-C₁₋₃-alkyl, aminocarbonylamino-C₁₋₃-alkyl, C₁₋₃-alkylaminocarbonylamino-C₁₋₃-alkyl, di-(C₁₋₃-alkyl)-aminocarbonylamino- C₁₋₃-alkyl, C₁₋₅-alkylsulphonylamino, C₁₋₅-alkylaminocarbonylamino, carboxy, C₁₋₃-alkyloxycarbonyl, benzyloxycarbonyl, C₁₋₃-alkylcarbonyl, aminocarbonyl, C₁₋₃-alkylaminocarbonyl, di-(C₁₋₃-alkyl)-aminocarbonyl, *N-*(C₃₋₇-cycloalkyl)-C₁₋₅- alkylaminocarbonyl, *N-*(phenyl-C₁₋₃-alkyl)-C₁₋₅-alkylaminocarbonyl, a 4- to 7- membered cycloalkyleneiminocarbonyl, aminocarbonyl-C₁₋₃-alkylaminocarbonyl, hydroxy, C₁₋₃-alkyloxy, allyloxy, propargyloxy, benzyloxy, amino, C₁₋₃-alkylamino, di-(C₁₋₃-alkyl)-amino, a 4- to 7-membered cycloalkyleneimino, trlfluoromethylcarbonylamino, *N-*(C₁₋₃-alkyl)-piperazin-4-yl-C₁₋₃-alkyl, a phenyl or a 5- to 6-membered heteroaryl group, with the proviso that in the substitution of a methylene group adjacent to the imino group two heteroatoms are separated from one another by at least two carbon atoms, and/or
a methylene group in the 3-position of a 5-membered cycloalkyleneimino group may be replaced by a sulphur atom or
a methylene group in the 4-position of a 6- or 7-membered cycloalkyleneimino group may be replaced by an oxygen or sulphur atom, a carbonyl, sulphinyl or sulphonyl group or by an -NH group optionally substituted by a C₁₋₃-alkyl, hydroxy, formyl or C₁₋₃-alkylcarbonyl group, while additionally a methylene group adjacent to the previously mentioned optionally substituted -NH group may be replaced by a carbonyl, sulphinyl or sulphonyl group, with the proviso that
in the substitution of the previously mentioned 6- to 7-membered cyclo- alkyleneimino groups, wherein a methylene group is replaced by an oxygen or sulphur atom, a sulphinyl or sulphonyl group, two heteroatoms are separated from one another by at least two carbon atoms,
a 5- to 7-membered cycloalkenyleneiminocarbonyl or cycloalkenyleneiminosulphonyl group optionally substituted by one or two C₁₋₃-alkyl, hydroxy-C₁₋₃-alkyl, C₁₋₅-alkoxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, C₃₋₈-cycloalkylamino-C₁₋₃-alkyl, phenyl, phenyl-C₁₋₃-alkyl, heteroaryl, heteroaryl-C₁₋₃-alkyl, aminocarbonyl, C₁₋₃-alkylaminocarbonyl, di-(C₁₋₃-alkyl)-aminocarbonyl or 4- to 7-membered cycloalkylenelminocarbonyl groups, while the double bond is not bound to a nitrogen atom and may be fused to a 5- or 6-membered heteroaryl group,
an aminocarbonyl or aminosulphonyl group optionally substituted by one or two C₁₋₅-alkyl, C₂₋₃-alkenyl, C₂₋₃-alkynyl, or C₃₋₆-cycloalkyl groups,
while the substituents may be identical or different and
in each case one of the C₁₋₅-alkyl groups may be substituted by one or two hydroxy-C₁₋₃-alkyl, C₁₋₃-alkoxy-C₁₋₃-alkyl, benzyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, C₁₋₅-alkyloxycarbonylamino-C₁₋₃-alkyl, C₃₋₆-cycloalkylamlno-C₁₋₃-alkyl, aminocarbonyl, C₁₋₃-alkylamino-carbonyl, *N-*(C₃₋₇-cycloalkyl)-C₁₋₃-alkylaminocarbonyl, *N-*(phenyl-C₁₋₃-alkyl)-C₁₋₅-alkylaminocarbonyl, di-(C₁₋₃-alkyl)-aminocarbonyl or a 4- to 7-membered cycloalkyleneiminocarbonyl group,
or a group of formula 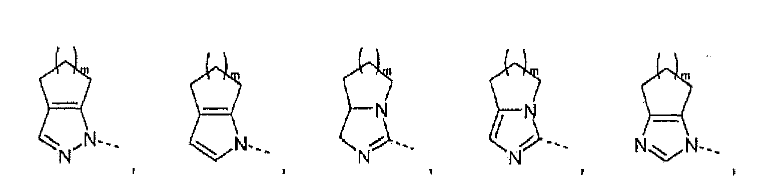 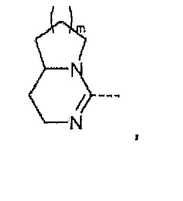 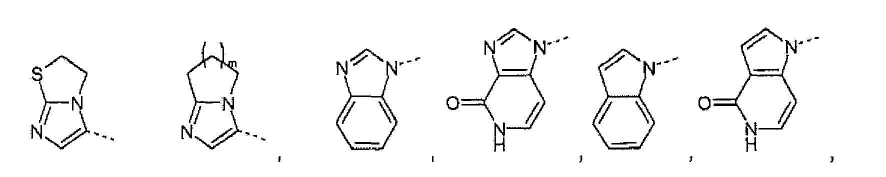 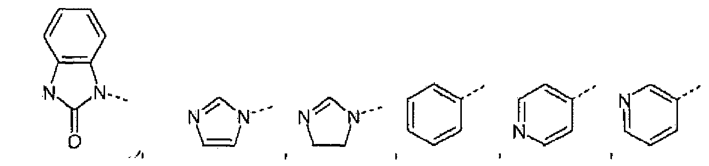 or 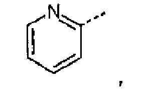 which may be substituted in each case at a carbon atom by a C₁₋₃-alkyl, C₁₋₃-alkyloxy, C₁₋₃-alkyloxycarbonyl, C₁₋₅-alkyloxycarbonylamino-C₁₋₃-alkyl, C₁₋₃-alkyl-sulphonyl, C₁₋₃-alkyl-sulphonyl-C₁₋₃-alkyl, aminosulphonyl, C₁₋₃-alkylsulphonyl-C₁₋₃-alkyl, C₁₋₃-alkylsulphonylamino-C₁₋₃-alkyl, C₁₋₃-alkylsulphonylamino, C₁₋₃-alkylcarbonylamino-C₁₋₃-alkyl, C₁₋₃-alkylcarbonylamino, amino-C₁₋₃-alkyl, C₁₋₃-alkylamino-C₁₋₃-alkyl, di-(C₁₋₃-alkyl)-amino-C₁₋₃-alkyl, hydroxy-C₁₋₃-alkyl, C₁₋₅-alkoxy-C₁₋₃-alkyl, a 4- to 7-membered cycloalkyleneimino-C₁₋₃-alkyl, morpholin-4-yl-C₁₋₃-alkyl, piperazinyl-C₁₋₃-alkyl, *N-*(C₁₋₃-alkyl)-piperazin-4-yl-C₁₋₃-alkyl, aminocarbonyl, C₁₋₃-alkylaminocarbonyl or di-(C₁₋₃-alkyl)-aminocarbonyl group and wherein
m denotes the number 1 or 2,
R¹ denotes a hydrogen or halogen atom, a C₁₋₃-alkyl or C₁₋₃-alkoxy group, while the hydrogen atoms of the C₁₋₃-alkyl or C₁₋₃-alkoxy group may optionally be wholly or partly replaced by fluorine atoms, a C₂₋₃-alkenyl, C₂₋₃-alkynyl, nitrile, nitro or amino group,
R² denotes a hydrogen or halogen atom or a methyl group,
X denotes a nitrogen atom or a CH- group,
R³ denotes a hydrogen atom or a C₁₋₃-alkyl group,
R⁴ and R⁵ each independently of one another denote
a hydrogen atom, a hydroxy group, an OR⁹ group, a C₂₋₆-alkenyl or C₂₋₆-alkynyl group,
a straight-chain or branched C₁₋₆-alkyl group, while the hydrogen atoms of the straight-chain or branched C₁₋₆-alkyl group may optionally be wholly or partly replaced by fluorine atoms, and which may optionally be substituted by a C₃₋₅- cycloalkyl group, a nitrile, hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy- C₁₋₅-alkyloxy, C₁₋₅-alkyloxycarbonyl-C₁₋₅-alkyloxy, mercapto, C₁₋₅-alkylsulphanyl, C₁₋₅-alkylsulphonyl, carboxy, C₁₋₅-alkyloxycarbonyl, aminocarbonyl, C₁₋₅-alkylaminocarbonyl, di- (C₁₋₅-alkyl)-aminocarbonyl, C₄₋₆-cycloalkyleneiminocarbonyl, aminosulphonyl, C₁₋₅-alkylaminosulphonyl, di-(C₁₋₅-alkyl)- aminosulphonyl, C₄₋₆-cycloalkyleneiminosulphonyl, amino, C₁₋₅-alkylamino, di-(C₁₋₅-alkyl)-amino, C₁₋₅-alkylcarbonylamino, C₁₋₅-alkylsulphonylamino, *N-*(C₁₋₅-alkylsulphonyl)-C₁₋₅-alkylamino or C₃₋₆-cycloalkylcarbonylamino group,
while in the 6- to 7-membered cyclic groups of the C₃₋₆- cycloalkyleneiminocarbonyl group in the cyclic moiety a methylene group in the 4-position of a 6- or 7-membered cycloalkyleneimino group may be replaced by an oxygen or sulphur atom or by a carbonyl, sulphinyl, sulphonyl or -NR⁷ group and additionally a methylene group adjacent to an above- mentioned -NR⁷ group may be replaced by a carbonyl group,
a phenyl, heteroaryl, phenyl-C₁₋₅-alkyl or heteroaryl-C₁₋₅-alkyl group, which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups,
a 3- to 7-membered cycloalkyl, cycloalkyleneimino, cycloalkyl- C₁₋₅-alkyl or cycloalkyleneimino-C₁₋₃-alkyl group,
wherein in 4- to 7-membered cyclic groups in the cyclic moiety a methylene group may optionally be replaced by a -N(R⁷) group, an oxygen or sulphur atom or a -S(O) or -S(O)₂ group, or
wherein in 4- to 7-membered cyclic groups in the cyclic moiety two adjacent methylene groups together may optionally be replaced by a -C(O)N(R⁸) or-S(O)₂N(R⁸) group, or
wherein In 6- to 7-membered cyclic groups in the cyclic moiety three adjacent methylene groups together may optionally be replaced by a substituted -OC(O)N(R⁸) or -N(R⁸)C(O)N(R⁸) or -N(R⁸)S(O)₂N(R⁸) group,
with the proviso that a 3- to 7-membered cycloalkyl, cycloalkyleneimino, cycloalkyl-C₁₋₅-alkyl or cycloalkyleneimino- C₁₋₃-alkyl group as hereinbefore defined wherein two heteroatoms selected from oxygen and nitrogen are separated from one another by precisely one optionally substituted -CH₂ group, is excluded,
while a 3- to 7-membered cycloalkyl, cycloalkyleneimino, cycloalkyl- C₁₋₅-alkyl or cycloalkyleneimino-C₁₋₃-alkyl group as hereinbefore defined may be substituted at one or two -CH₂ groups by one or two C₁₋₃-alkyl groups in each case,
with the proviso that R⁴ and R⁵ may not simultaneously be defined as hydroxy or OR⁹ groups,
R⁷ each independently of one another denote a hydrogen atom, a hydroxy, a formyl, a C₁₋₃-alkyl, C₁₋₃-alkylcarbonyl, C₁₋₃-alkyloxycarbonyl or C₁₋₃-alkylsulphonyl group,
R⁸ each independently of one another denote a hydrogen atom or a C₁₋₃-alkyl group
R⁸ denotes a straight-chain or branched C₁₋₆-alkyl group, while the hydrogen atoms of the straight-chain or branched C₁₋₆-alkyl group may optionally be wholly or partly replaced by fluorine atoms, and which may optionally be substituted by a C₃₋₅-cycloalkyl group, a hydroxy, a C₁₋₅-alkyloxy group, while the hydrogen atoms of the C₁₋₆-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy- C₁₋₅-alkyloxy,C₁₋₅-alkyloxycarbonyl-C₁₋₅-alkyloxy, carboxy, C₁₋₅-alkyloxycarbonyl, aminocarbonyl, C₁₋₅-alkylaminocarbonyl, di-(C₁₋₅-alkyl)- aminocarbonyl, C₄₋₈-cycloalkyleneiminocarbonyl, C₁₋₅alkyloxycarbonylamino, amino, C₁₋₅-alkylamino, di-(C₁₋₅-alkyl)-amino, C₁₋₅-alkylcarbonylamino, C₁₋₅-alkylsulphonylamino, *N-*(C₁₋₅-alkylsulphonyl)-C₁₋₅-alkylamino or C₃₋₆- cycloalkylcarbonylamino group,
while in the 6- to 7-membered cyclic groups of the C₄₋₆- cycloalkyleneiminocarbonyl group in the cyclic moiety a methylene group in the 4-position of a 6- or 7-membered cycloalkyleneimino group may be replaced by an oxygen or sulphur atom, by a carbonyl, sulphinyl, sulphonyl or -NR⁷ group and additionally a methylene group adjacent to an above-mentioned -NR⁷ group may be replaced by a carbonyl group, with the proviso that the replacement of hydrogen atoms of the first carbon atom of the straight-chain or branched C₁₋₆-alkyl group by substituents from the group comprising oxygen, sulphur or nitrogen is excluded,
a phenyl or heteroaryl group
which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups,
a phenyl-C₁₋₆-alkyl or heteroaryl-C₁₋₅-alkyl group, which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups, and which may optionally be substituted in the C₁₋₅-alkyl-moiety by a hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy, or a C₁₋₅-alkyloxycarbonyl-C₁₋₅-alkyloxy group;
a 3- to 7-membered cycloalkyl, cycloalkyl-C₁₋₅-alkyl or cycloalkyleneimino- C₂₋₃-alkyl group,
wherein in 4- to 7-membered cyclic groups in the cyclic moiety a methylene group may optionally be replaced by an -N(R⁷) group, an oxygen or sulphur atom or a -S(O) or -S(O)₂ group, or
wherein in 4- to 7-membered cyclic groups in the cyclic moiety two adjacent methylene groups together may optionally be replaced by a -C(O)N(R⁸) or -S(O)₂N(R⁸) group, or
wherein in 6- to 7-membered cyclic groups in the cyclic moiety three adjacent methylene groups together may optionally be replaced by a substituted -OC(O)N(R⁸) or -N(R⁸)C(O)N(R⁸) or -N(R⁸)S(O)₂N(R⁸) group,
with the proviso that a 3- to 7-membered cycloalkyl, cycloalkyl- C₁₋₆-alkyl or cycloalkyleneimino-C₂₋₃-alkyl group as hereinbefore defined wherein two heteroatoms selected from oxygen and nitrogen are separated from one another by precisely one optionally substituted -CH₂ group, is excluded,
while a 3- to 7-membered cycloalkyl, cycloalkyl-C₁₋₅-alkyl or cycloalkyleneimino-C₂₋₃-alkyl group as hereinbefore defined may be substituted at one or two -CH₂ groups by one or two C₁₋₃-alkyl groups in each case,
B denotes a group of general formula 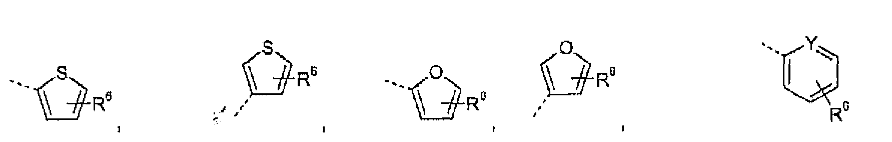 or 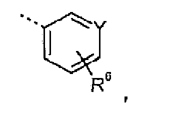
Y denotes a nitrogen atom or a CH- group,
R⁶ denotes a hydrogen, a halogen atom, an ethynyl, a methyl group, a methoxy group, while the hydrogen atoms of the methoxy group may optionally be wholly or partly replaced by fluorine atoms,
while, unless otherwise stated, by the "heteroaryl group" mentioned hereinbefore in the definitions is meant a monocyclic 5- or 6-membered heteroaryl group, while
the 6-membered heteroaryl group contains one, two or three nitrogen atoms and
the 5-membered heteroaryl group contains an imino group optionally substituted by a C₁₋₃-alkyl, phenyl or phenyl-C₁₋₃-alkyl group, or an oxygen or sulphur atom or
an imino group optionally substituted by a C₁₋₃-alkyl, phenyl, amino-C₂₋₃-alkyl, C₁₋₃-alkylamino-C₂₋₃-alkyl, di-(C₁₋₃-alkyl)-amino-C₂₋₃-alkyl, a C₃₋₆-cycloalkyleneimino-C₁₋₃-alkyl or phenyl-C₁₋₃-alkyl group or an oxygen or sulphur atom and additionally a nitrogen atom or
an imino group optionally substituted by a C₁₋₃-alkyl or phenyl-C₁₋₃-alkyl group and two or three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms
and the bond is effected via a nitrogen atom or a carbon atom of the heterocyclic moiety or a fused-on phenyl ring,
while, unless otherwise stated, by the term "halogen atom" mentioned hereinbefore in the definitions is meant an atom selected from among fluorine, chlorine, bromine and iodine,
while the alkyl, alkenyl, alkynyl and alkoxy groups contained in the afore-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated to the contrary, may be wholly or partly replaced by fluorine atoms,
the tautomers, the enantiomers, the diastereomers, the mixtures thereof and the salts thereof.

4. Substituted pyrrolidinones of general formula I according to claim 1, **characterised in that**
A denotes a 4- to 7-membered cycloalkyleneiminocarbonyl or cycloalkyleneiminosulphonyl group, while
the cycloalkyleneimino moiety in the carbon skeleton may be substituted by one or two fluorine atoms, one or two C₁₋₃-alkyl, hydroxy-C₁₋₃-alkyl, C₁₋₃-alkyloxy- C₁₋₃-alkyl, heteroaryl-C₁₋₃-alkyl, amino-C₁₋₃-alkyl, C₃₋₆-cycloalkylamino-C₁₋₃-alkyl, phenylamino-C₁₋₃-alkyl, C₁₋₅-alkylamino-C₁₋₃-alkyl, di-(C₁₋₅-alkyl)-amino-C₁₋₅-alkyl, *N-*(C₃₋₆-cycloalkyl)-C₁₋₃-alkylamino-C₁₋₃-alkyl, a 4- to 7-membered cycloalkyleneimino-C₁₋₃-alkyl, *N-*(C₁₋₃-alkylcarbonyl)-C₁₋₃-alkylamino-C₁₋₃-alkyl, carboxy-C₁₋₃-alkyl, C₁₋₃-alkyloxycarbonyl-C₁₋₃-alkyl, aminocarbonyl-C₁₋₃-alkyl, C₁₋₃-alkylaminocarbonyl-C₁₋₃-alkyl, di-(C₁₋₃-alkyl)-aminocarbonyl-C₁₋₃-alkyl, a 4- to 7-membered cycloalkyleneiminocarbonyl-C₁₋₃-alkyl, C₁₋₅-alkyloxycarbonylamino-C₁₋₃-alkyl, C₁₋₃-alkylcarbonylamino-C₁₋₃-alkyl, C₁₋₅-alkylsulphonylamino-C₁₋₃-alkyl, aminocarbonylamino-C₁₋₃-alkyl, C₁₋₅-alkylaminocarbonylamino-C₁₋₃-alkyl, di-(C₁₋₃-alkyl)-aminocarbonylamino- C₁₋₃-alkyl, C₁₋₅-alkylsulphonylamino, C₁₋₅-alkylaminocarbonylamino, carboxy, C₁₋₃-alkyloxycarbonyl, benzyloxycarbonyl, C₁₋₃-alkylcarbonyl, aminocarbonyl, C₁₋₃-alkylaminocarbonyl, di-(C₁₋₃-alkyl)-aminocarbonyl, *N-*(C₃₋₇-cycloalkyl)-C₁₋₅- alkylaminocarbonyl, *N-*(phenyl-C₁₋₃-alkyl)-C₁₋₅-alkylaminocarbonyl, a 4- to 7- membered cycloalkyleneiminocarbonyl, aminocarbonyl-C₁₋₃-alkylaminocarbonyl, hydroxy, C₁₋₃-alkyloxy, allyloxy, propargyloxy, benzyloxy, amino, C₁₋₃-alkylamino, di-(C₁₋₃-alkyl)-amino, a 4- to 7-membered cycloalkyleneimino, *N-*(C₁₋₃-alkyl)- piperazin-4-yl-C₁₋₃-alkyl, a phenyl or a 5- to 6-membered heteroaryl group, with the proviso that in the substitution of a methylene group adjacent to the imino group two heteroatoms are separated from one another by at least two carbon atoms, and/or
a methylene group in the 3-position of a 5-membered cycloalkyleneimino group may be replaced by a sulphur atom or
a methylene group in the 4-position of a 6- or 7-membered cycloalkyleneimino group may be replaced by an oxygen or sulphur atom, a sulphinyl or sulphonyl group or by an -NH group optionally substituted by a C₁₋₃-alkyl, hydroxy, formyl or C₁₋₃-alkylcarbonyl group, while additionally a methylene group adjacent to the previously mentioned optionally substituted -NH group may be replaced by a carbonyl, sulphinyl or sulphonyl group, with the proviso that
In the substitution of the previously mentioned 6- to 7-membered cyclo- alkyleneimino groups, wherein a methylene group is replaced by an oxygen or sulphur atom, a sulphinyl or sulphonyl group, two heteroatoms are separated from one another by at least two carbon atoms,
a 5- to 7-membered cycloalkenyleneiminocarbonyl or cycloalkenyleneiminosulphonyl group optionally substituted by one or two C₁₋₃-alkyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, C₃₋₆-cycloalkylamino-C₁₋₃-alkyl, heteroaryl, heteroaryl-C₁₋₃-alkyl, aminocarbonyl, C₁₋₃-alkylaminocarbonyl, di-(C₁₋₃-alkyl)-aminocarbonyl or 4- to 7-membered cycloalkyleneiminocarbonyl groups, while the double bond is not bound to a nitrogen atom and may be fused to a 5- or 6-membered heteroaryl group,
an aminocarbonyl or aminosulphonyl group optionally substituted by one or two C₁₋₅-alkyl, or C₃₋₆-cycloalkyl groups,
while the substituents may be identical or different and
in each case one of the C₁₋₅-alkyl groups may be substituted by one or two hydroxy-C₁₋₃-alkyl, C₁₋₃-alkoxy-C₁₋₃-alkyl, amino-C₁₋₃-alkyl, di-(C₁₋₃-alkyl)-amino-C₁₋₃-alkyl, a 4- to 7-membered cycloalkyleneimino-C₁₋₃-alkyl, aminocarbonyl, C₁₋₃-alkylamino-carbonyl, di-(C₁₋₃-alkyl)-aminocarbonyl or a 4- to 7-membered cycloalkyleneiminocarbonyl group,
or a group of formula 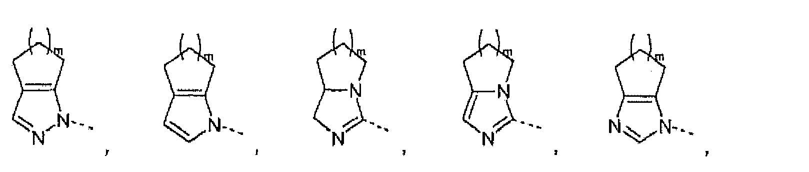 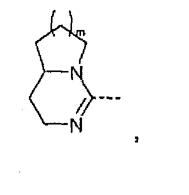 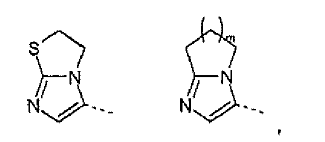 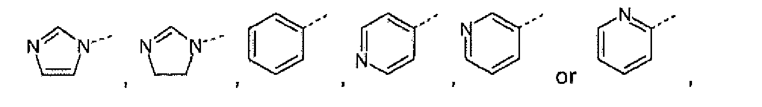 which may be substituted in each case at a carbon atom by a C₁₋₃-alkyl, C₁₋₃-alkyloxy, C₁₋₃-alkyloxycarbonyl, C₁₋₅-alkyloxycarbonylamino-C₁₋₃-alkyl, C₁₋₃-alkyl-sulphonyl, C₁₋₃-alkyl-sulphonyl-C₁₋₃-alkyl, aminosulphonyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, morpholin-4-yl-C₁₋₃-alkyl, piperazinyl-C₁₋₃-alkyl, *N-*(C₁₋₃-alkyl)-piperazin-4-yl-C₁₋₃-alkyl, aminocarbonyl, C₁₋₃-alkylaminocarbonyl or di-(C₁₋₃-alkyl)-aminocarbonyl group and wherein
m denotes the number 1 or 2,
R¹ denotes a hydrogen or halogen atom, a C₁₋₃-alkyl or C₁₋₃-alkoxy group, while the hydrogen atoms of the C₁₋₃-alkyl or C₁₋₃-alkoxy group may optionally be wholly or partly replaced by fluorine atoms, a C₂₋₃-alkenyl, C₂₋₃-alkynyl, nitrile, nitro or amino group,
R² denotes a hydrogen or fluorine atom or a methyl group,
X denotes a nitrogen atom or a CH- group,
R³ denotes a hydrogen atom or a C₁₋₃-alkyl group,
R⁴ denotes a hydrogen atom,
a straight-chain or branched C₁₋₄-alkyl group, while the hydrogen atoms of the straight-chain or branched C₁₋₄-alkyl group may optionally be wholly or partly replaced by fluorine atoms, and which may optionally be substituted by a C₁₋₃-alkyloxy group, while the hydrogen atoms of the C₁₋₃-alkyloxy group may optionally be wholly or partly replaced by fluorine atoms,
R⁵ denotes a hydrogen atom, a hydroxy group, an OR⁹ group, a C₂₋₄-alkenyl or C₂₋₄-alkynyl group,
a straight-chain or branched C₁₋₄-alkyl group, while the hydrogen atoms of the straight-chain or branched C₁₋₄-alkyl group may optionally be wholly or partly replaced by fluorine atoms, and which may optionally be substituted by a C₃₋₅-cycloalkyl group, a nitrile, hydroxy, a C₁₋₆-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy C₁₋₅-alkyloxycarbonyl-C₁₋₅-alkyloxy, mercapto, C₁₋₅-alkylsulphanyl, C₁₋₅-alkylsulphonyl, carboxy, C₁₋₅-alkyloxycarbonyl, aminocarbonyl, C₁₋₅-alkylaminocarbonyl, di-(C₁₋₅-alkyl)-aminocarbonyl, C₄₋₆- cycloalkyleneiminocarbonyl, aminosulphonyl, C₁₋₅-alkylaminosulphonyl, di- (C₁₋₅-alkyl)-aminosulphonyl, C₄₋₆-cycloalkyleneiminosulphonyl amino, C₁₋₅-alkylamino, di-(C₁₋₅-alkyl)-amino, C₁₋₅-alkylcarbonylamino, C₁₋₅-alkyl- sulphonylamino, *N-*(C₁₋₅-alkylsulphonyl)-C₁₋₅-alkylamino or C₃₋₅- cycloalkylcarbonylamino group,
while in the 6- to 7-membered cyclic groups of the C₄₋₆- cycloalkyleneiminocarbonyl group in the cyclic moiety a methylene group in the 4-position of a 6- or 7-membered cycloalkyleneimino group may be replaced by an oxygen or sulphur atom, by a carbonyl, sulphinyl, sulphonyl or -NR⁷ group and additionally a methylene group adjacent to an above-mentioned -NR⁷ group may be replaced by a carbonyl group
a phenyl, heteroaryl, phenyl-C₁₋₅-alkyl or heteroaryl-C₁₋₅-alkyl group,
which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups,
R⁷ each independently of one another denote a hydrogen atom, a C₁₋₃-alkyl, C₁₋₃-alkylcarbonyl, C₁₋₃-alkyloxycarbonyl or C₁₋₃-alkylsulphonyl group,
R⁹ denotes a straight-chain or branched C₁₋₄-alkyl group,
while the hydrogen atoms of the straight-chain or branched C₁₋₄-alkyl group may optionally be wholly or partly replaced by fluorine atoms, and which may optionally be substituted by a C₃₋₅-cycloalkyl group, a hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₆-alkyloxy, C₁₋₅-alkyloxycarbonyl-C₁₋₅-alkyloxy, carboxy, C₁₋₅-alkyloxycarbonyl, aminocarbonyl, C₁₋₅-alkylaminocarbonyl, di-(C₁₋₅-alkyl)-aminocarbonyl, C₄₋₆-cycloalkyleneiminocarbonyl, C₁₋₅-alkyloxycarbonylamino, amino, C₁₋₅-alkylamino, di-(C₁₋₅-alkyl)-amino, C₁₋₅-alkylcarbonylamino, C₁₋₅-alkylsulphonylamino, *N-*(C₁₋₅-alkylsulphonyl)-C₁₋₅-alkylamino or C₃₋₆-cycloalkylcarbonylamino group,
while in the 6- to 7-membered cyclic groups of the C₄₋₆- cycloalkyleneiminocarbonyl group in the cyclic moiety a methylene group in the 4-position of a 6- or 7-membered cycloalkyleneimino group may be replaced by an oxygen or sulphur atom, by a carbonyl, sulphinyl, sulphonyl or -NR⁷ group and additionally a methylene group adjacent to an above- mentioned -NR⁷ group may be replaced by a carbonyl group
with the proviso that the replacement of hydrogen atoms of the first carbon atom of the straight-chain or branched C₁₋₄-alkyl group by substituents from the group comprising oxygen, sulphur or nitrogen is excluded,
a phenyl or heteroaryl group
which may optionally be mono- to trisubstituted in the phenyl or heteroaryl moiety by identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups,
a phenyl-C₁₋₅-alkyl or heteroaryl-C₁₋₅-alkyl group,
which may optionally be mono- to trisubstituted in the phenyl or heteroaryl moiety by Identical or different substituents selected from among halogen atoms, C₁₋₅-alkyl, di-(C₁₋₅-alkyl)-amino, hydroxy, C₁₋₅-alkyloxy, mono-, di- or trifluoromethoxy, carboxy- and C₁₋₅-alkyloxycarbonyl groups, and which may optionally be substituted in the C₁₋₅-alkyl-moiety by a hydroxy, a C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy, or a C₁₋₅-alkyloxycarbonyl-C₁₋₅-alkyloxy group;
B denotes a group of general formula 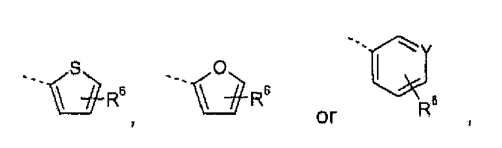
Y denotes a nitrogen atom or a CH- group,
R⁶ denotes a hydrogen, a halogen atom, an ethynyl, a methyl group, a methoxy group, while the hydrogen atoms of the methoxy group may optionally be wholly or partly replaced by fluorine atoms,
while, unless otherwise stated, by the "heteroaryl group" mentioned hereinbefore in the definitions is meant a monocyclic 5- or 6-membered heteroaryl group, while
the 6-membered heteroaryl group contains one, two or three nitrogen atoms and
the 5-membered heteroaryl group contains an imino group optionally substituted by a C₁₋₃-alkyl, phenyl or phenyl-C₁₋₃-alkyl group, an oxygen or sulphur atom or
an imino group optionally substituted by a C₁₋₃-alkyl, phenyl, amino-C₂₋₃-alkyl, C₁₋₃-alkylamino-C₂₋₃-alkyl, di-(C₁₋₃-alkyl)-amino-C₂₋₃-alkyl, a C₃₋₆-cycloalkyleneimino-C₁₋₃-alkyl or phenyl-C₁₋₃-alkyl group or an oxygen or sulphur atom and additionally a nitrogen atom or
an imino group optionally substituted by a C₁₋₃-alkyl or phenyl-C₁₋₃-alkyl group and two or three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms
and the bond is effected via a nitrogen atom or a carbon atom of the heterocyclic moiety or a fused-on phenyl ring,
while, unless otherwise stated, by the term "halogen atom" mentioned hereinbefore In the definitions is meant an atom selected from among fluorine, chlorine, bromine and iodine,
while the alkyl, alkenyl, alkynyl and alkoxy groups contained in the afore-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated to the contrary, may be wholly or partly replaced by fluorine atoms,
the tautomers, the enantiomers, the diastereomers, the mixtures thereof and the salts thereof.

5. Substituted pyrrolidinones of general formula I according to claim 1, **characterised in that**
A denotes a 5- to 6-membered cycloalkyleneiminocarbonyl or cycloalkyleneiminosulphonyl group, while
the cycloalkyleneimino moiety in the carbon skeleton may be substituted by one or two fluorine atoms, one or two C₁₋₃-alkyl, hydroxy-C₁₋₃-alkyl, C₁₋₃-alkyloxy- C₁₋₃-alkyl, heteroaryl-C₁₋₃-alkyl, amino-C₁₋₃-alkyl, C₃₋₆-cycloalkylamino-C₁₋₃-alkyl, phenylamino-C₁₋₃-alkyl, C₁₋₅-alkylamino-C₁₋₃-alkyl, di-(C₁₋₅-alkyl)-amino-C₁₋₅-alkyl, *N*-(C₃₋₆-cycloalkyl)-C₁₋₃-alkylamino-C₁₋₃-alkyl, a 4- to 7-membered cycloalkyleneimlno-C₁₋₃-alkyl, *N*-(C₁₋₃-alkylcarbonyl)-C₁₋₃-alkylamino-C₁₋₃-alkyl, carboxy-C₁₋₃-alkyl, C₁₋₃-alkyloxycarbonyl-C₁₋₃-alkyl, aminocarbonyl-C₁₋₃-alkyl, C₁₋₃-alkylaminocarbonyl-C₁₋₃-alkyl, di-(C₁₋₃-alkyl)-aminocarbonyl-C₁₋₃-alkyl, a C₁₋₃-alkylcarbonylamino-Cᵢ-₃-alkyl, C₁₋₅-alkylaminocarbonylamino-C₁₋₃-alkyl, di- (C₁₋₃-alkyl)-aminocarbonylamino-C₁₋₃-alkyl, C₁₋₅-alkylsulphonylamino, C₁₋₅- alkylaminocarbonylamino, carboxy, C₁₋₃-alkyloxycarbonyl aminocarbonyl, C₁₋₃-alkylaminocarbonyl, di-(C₁₋₃-alkyl)-aminocarbonyl, *N*-(C₃₋₇-cycloalkyl)-C₁₋₅- alkylaminocarbonyl, *N*-(phenyl-C₁₋₃-alkyl)-C₁₋₅-alkylaminocarbonyl, hydroxy, C₁₋₃-alkyloxy, amino, C₁₋₃-alkylamino, di-(C₁₋₃-alkyl)-amino, *N*-(C₁₋₃-alkyl)- piperazin-4-yl-C₁₋₃-alkyl, a phenyl or a 6-membered heteroaryl group, with the proviso that in the substitution of a methylene group adjacent to the imino group two heteroatoms are separated from one another by at least two carbon atoms, and/or
a methylene group in the 3-position of a 5-membered cycloalkyleneimino group may be replaced by a sulphur atom or
a methylene group in the 4-position of a 6- to 7-membered cycloalkyleneimino group may be replaced by an oxygen or sulphur atom or by an -NH group optionally substituted by a C₁₋₃-alkyl, hydroxy, formyl or C₁₋₃-alkylcarbonyl group, while additionally a methylene group adjacent to the previously mentioned optionally substituted -NH group may be replaced by a carbonyl group, with the proviso that
in the substitution of the previously mentioned 6- to 7-membered cyclo- alkyleneimino groups, wherein a methylene group is replaced by an oxygen or sulphur atom, two heteroatoms are separated from one another by at least two carbon atoms,
or A denotes a group of formula 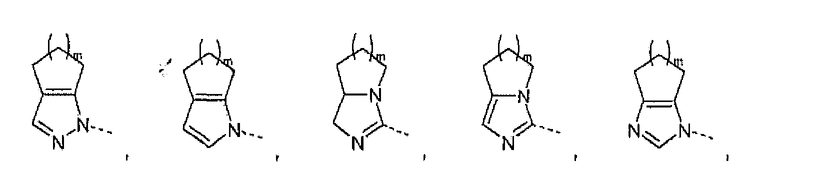 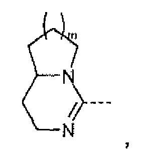 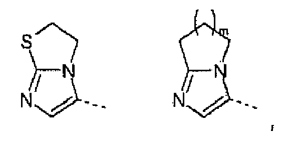 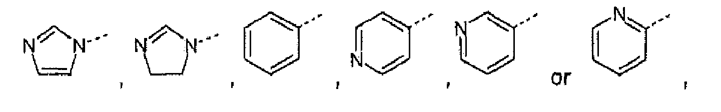 which may be substituted in each case at a carbon atom by a C₁₋₃-alkyl, C₁₋₃-alkyloxy, C₁₋₃-alkyloxycarbonyl, C₁₋₅-alkyloxycarbonylamino-C₁₋₃-alkyl, C₁₋₃-alkyl-sulphonyl, C₁₋₃-alkyl-sulphonyl-C₁₋₃-alkyl, aminosulphonyl, amino-C₁₋₃-alkyl, C₁₋₃-alkylamino-C₁₋₃-alkyl, di-(C₁₋₃-alkyl)-amino-C₁₋₃-alkyl, a 4- to 7-membered cycloalkyleneimino-C₁₋₃-alkyl, morpholin-4-yl-C₁₋₃-alkyl, piperazinyl-C₁₋₃-alkyl, *N*-(C₁₋₃-alkyl)-piperazin-4-yl-C₁₋₃-alkyl, aminocarbonyl, C₁₋₃-alkylaminocarbonyl or di-(C₁₋₃-alkyl)-aminocarbonyl group and wherein
m denotes the number 1 or 2,
R¹ denotes a hydrogen, fluorine, chlorine or bromine atom, a methyl or methoxy group, while the hydrogen atoms of the methyl or methoxy group may optionally be wholly or partly replaced by fluorine atoms,
R² denotes a hydrogen or fluorine atom,
X denotes a nitrogen atom or a CH- group,
R³ denotes a hydrogen atom or a C₁₋₃-alkyl group,
R⁴ denotes a hydrogen atom,
R⁶ denotes a hydrogen atom, a hydroxy group, an OR⁹ group, a C₂₋₄-alkenyl or C₂₋₄-alkynyl group,
a straight-chain or branched C₁₋₄-alkyl group, while the hydrogen atoms may optionally be wholly or partly replaced by fluorine atoms, and which may optionally be substituted by a hydroxy, a C₁₋₃-alkyloxy group, while the hydrogen atoms of the C₁₋₃-alkyloxy group may be wholly or partly replaced by fluorine atoms, a benzyloxy, C₁₋₃-alkylcarbonyloxy, C₁₋₃-alkyloxycarbonyl, aminocarbonyl, C₁₋₃-alkylaminocarbonyl , di-(C₁₋₃-alkyl)-aminocarbonyl, C₄₋₆- cycloalkyleneiminocarbonyl, amino, C₁₋₃-alkylamino, di-(C₁₋₃-alkyl)-amino, C₁₋₃-alkylcarbonylamino, or C₁₋₃-alkylsulphonylamino group,
a phenyl or C-linked heteroaryl group while the heteroaryl group is selected from among pyrrolyl, oxazolyl, imidazolyl, furanyl, thlophenyl, thiazolyl, 1,2,3-triazvlyl, 1,2,4-triazolyl, tetrazolyl, pyridinyl, pyrimidinyl and pyrazinyl, and which may optionally be mono- to disubstituted in the heteroaryl moiety by Identical or different substituents selected from among halogen atoms, C₁₋₃-alkyl, hydroxy, C₁₋₃-alkyloxy, mono-, di- and trifluoromethoxy groups,
a phenyl-C₁₋₃-alkyl, heteroaryl-C₁₋₃-alkyl- group, while the heteroaryl group is selected from among pyrrolyl, oxazolyl, imidazolyl, furanyl, thiophenyl, thiazofyl, 1,2,3-triazolyl, 1,2,4-triazolyl, tetrazolyl, pyridinyl, pyrimidinyl and pyrazinyl, and which may optionally be mono- to disubstituted in the heteroaryl moiety by identical or different substituents selected from among halogen atoms, C₁₋₃-alkyl, hydroxy, C₁₋₃-alkyloxy, mono-, di- and trifluoromethoxy groups, and which may optionally be substituted in the C₁₋₃-alkyl moiety by a hydroxy, a C₁₋₃-alkyloxy group, while the hydrogen atoms of the C₁₋₃-alkyloxy group may optionally be wholly or partly replaced by fluorine atoms, a C₁₋₅-alkylcarbonyloxy, or a C₁₋₅-alkyloxycarbonyloxy group;
R⁹ denotes a straight-chain or branched C₁₋₄-alkyl group, while the hydrogen atoms of the straight-chain or branched C₁₋₄-alkyl group may optionally be wholly or partly replaced by fluorine atoms, and which may optionally be substituted by a hydroxy, a C₁₋₃-alkyloxy group, while the hydrogen atoms of the C₁₋₃-alkyloxy group may optionally be wholly or partly replaced by fluorine atoms, a benzyloxy, C₁₋₃-alkyloxycarbonyl, aminocarbonyl, C₁₋₃-alkylaminocarbonyl, di-(C₁₋₃-alkyl)-aminocarbonyl, C₄₋₆- cycloalkyleneiminocarbonyl, amino, C₁₋₃-alkylamino, di-(C₁₋₃-alkyl)-amino, C₁₋₃-alkylcarbonylamino, C₁₋₃-alkylsulphonylamino group,
with the proviso that the replacement of hydrogen atoms of the first carbon atom of the straight-chain or branched C₁₋₄-alkyl group by substituents from the group comprising oxygen, sulphur or nitrogen is excluded,
a phenyl, phenyl-C₁₋₂-alkyl, heteroaryl-C₁₋₂-alkyl or C-linked heteroaryl group
while the heteroaryl group is selected from among pyrrolyl, oxazolyl, imidazolyl, furanyl, thiophenyl, thiazolyl, 1,2,3-triazolyl, 1,2,4-triazolyl, tetrazolyl, pyridinyl, pyrimidinyl and pyrazinyl, and which may optionally be mono- to disubstituted in the heteroaryl moiety by identical or different substituents selected from among halogen atoms, C₁₋₃-alkyl, hydroxy, C₁₋₃-alkyloxy, mono-, di- and trifluoromethoxy groups,
B denotes a group of general formula 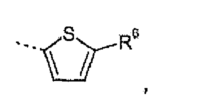
R⁶ denotes a hydrogen, a chlorine or bromine atom, an ethynyl, a methyl or a methoxy group,
while, unless otherwise stated, by the term "halogen atom" mentioned hereinbefore in the definitions is meant an atom selected from among fluorine, chlorine, bromine and iodine,
while the alkyl, alkenyl, alkynyl and alkoxy groups contained in the afore-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated to the contrary, may be wholly or partly replaced by fluorine atoms,
the tautomers, the enantiomers, the diastereomers, the mixtures thereof and the salts thereof.

6. Substituted pyrrolidinones of general formula I according to claim 1, **characterised in that**
A denotes a 5- to 6-membered cycloalkyleneiminocarbonyl or cycloalkyleneiminosulphonyl group, while
the cycloalkyleneimino moiety may be substituted in the carbon skeleton by one or two C₁₋₃-alkyl, hydroxy-C₁₋₃-alkyl, C₁₋₃-alkyloxy-C₁₋₃-alkyl, pyridinyl-C₁₋₃-alkyl, amino-C₁₋₃-alkyl, C₃₋₆-cycloalkylamino-C₁₋₃-alkyl, phenylamino-C₁₋₃-alkyl, C₁₋₅-alkylamino-C₁₋₃-alkyl, di-(C₁₋₅-alkyl)-amino-C₁₋₅-alkyl, N-pyrrolidinyl- C₁₋₃-alkyl, C₁₋₃-alkyloxycarbonyl-C₁₋₃-alkyl, C₁₋₃-alkyloxycarbonyl, aminocarbonyl, C₁₋₃-alkylaminocarbonyl, di-(C₁₋₃-alkyl)-aminocarbonyl, hydroxy, C₁₋₃-alkyloxy, C₁₋₃-alkylemino, di-(C₁₋₃-alkyl)-amino, *N*-(C₁₋₃-alkyl)-piperazin-4-yl-C₁₋₃-alkyl, a phenyl or a pyridinyl group, with the proviso that in the substitution of a methylene group adjacent to the imino group two heteroatoms are separated from one another by at least two carbon atoms, and/or
a methylene group in the 3-position of a 5-membered cycloalkyleneimino group may be replaced by a sulphur atom or
a methylene group in the 4-position of a 6- to 7-membered cycloalkyleneimino group may be replaced by an oxygen or sulphur atom, by an -NH group optionally substituted by a C₁₋₃-alkyl, hydroxy, formyl or C₁₋₃-alkylcarbonyl group, while additionally a methylene group adjacent to the previously mentioned optionally substituted -NH group may be replaced by a carbonyl, group, with the proviso that
in the substitution of the previously mentioned 6- to 7-membered cyclo- alkyleneimino groups wherein a methylene group is replaced by an oxygen or sulphur atom, two heteroatoms are separated from one another by at least two carbon atoms,
or A denotes a group of formula 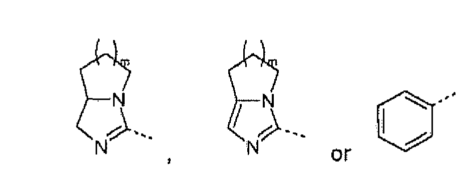 which may be substituted in each case at a carbon atom by a C₁₋₃-alkyl, methylsulphonylmethyl, aminosulphonyl, amino-C₁₋₃-alkyl, C₁₋₃-alkylamino-C₁₋₃-alkyl or a di-(C₁₋₃-alkyl)-amino-C₁₋₃-alkyl group and wherein
m denotes the number 1 or 2,
R¹ denotes a hydrogen, fluorine, chloride or bromine atom, a methyl, trifluoromethyl or methoxy group,
R² denotes a hydrogen atom,
X denotes a nitrogen atom or a CH- group,
R³ denotes a hydrogen atom or a methyl group,
R⁴ denotes a hydrogen atom,
R⁵ denotes a hydrogen atom, a hydroxy group, an OR⁹ group, an allyl or methallyl group,
a methyl group which may be substituted by a C₁₋₃-alkyl, hydroxy, OR⁹ group, aminocarbonyl, pyridin-4-yl, pyridin-3-yl, pyridin-2-yl, pyrazin-2-yl, pyrazin-3-yl or phenyl group,
or a phenyl group,
R⁹ denotes a straight-chain or branched C₁₋₄-alkyl group, which may optionally be substituted by a hydroxy, a C₁₋₃-alkoxy group, a benzyloxy or a di-(C₁₋₃-alkyl)-amino group, with the proviso that the replacement of hydrogen atoms of the first carbon atom of the straight-chain or branched C₁₋₄-alkyl group by substituents from the group oxygen or nitrogen is excluded,
B denotes a group of general formula 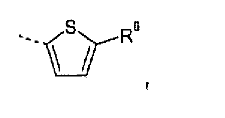
R⁶ denotes a chlorine or bromine atom or an ethynyl group,
while, unless otherwise stated, by the term "halogen atom" mentioned hereinbefore in the definitions is meant an atom selected from among fluorine, chlorine, bromine and iodine,
while the alkyl, alkenyl, alkynyl and alkoxy groups contained in the afore-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated to the contrary, may be wholly or partly replaced by fluorine atoms,
the tautomers, the enantiomers, the diastereomers, the mixtures thereof and the salts thereof,

7. Substituted pyrrolidinones of general formula I according to one of claims 1 to 6, **characterised in that** the group B denotes the group 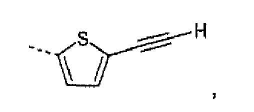 the tautomers, the enantiomers, the diastereomers, the mixtures thereof and the salts thereof.

8. Substituted pyrrolidinones of general formule according to one of claims 1 to 6, **characterised in that**
the group B denotes the group 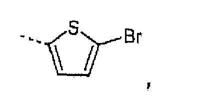 the tautomers, the enantiomers, the diastereomers, the mixtures thereof and the salts thereof.

9. Substituted Pyrrolidinones of general formula I according to one of claims 1 to 8, **characterised in that**
the group A denotes the group 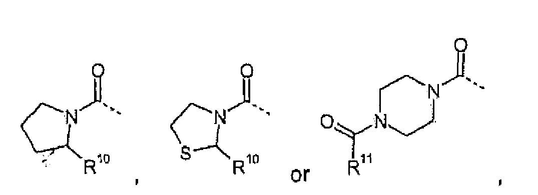 where
R¹⁰ denotes the hydrogen atom, a methyl, aminomethyl, C₁₋₃-alkylamino-C₁₋₂-alkyl, di-(C₁₋₃-alkyl)-amino-C₁₋₂-alkyl, pyrrolldin-1-yl-methyl or 2-(pyrrolidin-1-yl)-ethyl group,
R¹¹ denotes the hydrogen atom or a methyl group,
the tautomers, the enantiomers, the diastereomers, the mixtures thereof and the salts thereof.

10. Physiologically acceptable salts of the compounds according to one of claims 1 to 9.

11. Pharmaceutical compositions containing a compound according to one of claims 1 to 9 or a physiologically acceptable salt according to claim 10 optionally together with one or more inert carriers and/or diluents.

12. Use of a compound according to one of claims 1 to 9 or a physiologically acceptable salt according to claim 10 for preparing a pharmaceutical composition for the prevention and treatment of venous and arterial thrombotic diseases, and the prevention and treatment of pulmonary embolism, disseminated intravascular coagulation and severe sepsis, for the prevention and prophylaxis of DVT in patients with exacerbated COPD, for treating ulcerative colitis, for prophylaxis and treatment of coronary thrombosis, for preventing stroke and for preventing the occlusion of shunts, for antithrombotic support in thrombolytic treatment, for the prophylaxis and treatment of Ischaemic events in patients with all forms of coronary heart disease, for preventing metastasis and the growth of tumours and inflammatory processes, for prophylaxis and treatment of rheumatoid arthritis, for preventing or averting fibrin-dependent tissue adhesions and/or the formation of scar tissue and for promoting wound healing processes,

13. Process for preparing a pharmaceutical composition according to claim 11, **characterised in that** a compound according to one of claims 1 to 9 or a physiologically acceptable salt according to claim 10 is incorporated into one or more inert carriers and/or diluents by a non-chemical method.

## Revendications

1. Pyrrolidinones substituées de formule générale 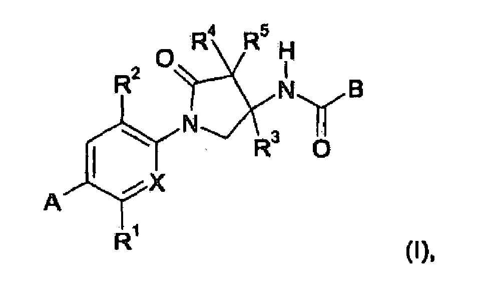 **caractérisées en ce que**
A représente un groupe cycloalkyléniminocarbonyle ou cycloalkyléniminosulfonyle à 4 à 7 chaînons, où
la partie cycloalkylénimino peut être substituée dans le squelette carboné par un ou deux atomes de fluor, un ou deux groupes C₁₋₃-alkyle, C₂₋₃-alcényle, C₂₋₃-alcynyle, hydroxy-C₁₋₃-alkyle, C₁₋₃-alkyloxy-C₁₋₃-alkyle, phényl-C₁₋₃-alkyle, 1,1-diphényl-C₁₋₃-alkyle, hétéroaryl-C₁₋₃-alkyle, amino-C₁₋₃-alkyle, C₃₋₆-cycloalkylamino-C₁₋₃-alkyle, phénylamino-C₁₋₃-alkyle, C₁₋₅-alkylamino-C₁₋₃-alkyle, di-(C₁₋₅-alkyl)-amino-C₁₋₅-alkyle, *N*-(C₃₋₆-cycloalkyl)-C₁₋₃-alkylamino-C₁₋₃-alkyle, un groupe cycloalkylénimino-C₁₋₃-alkyle à 4 à 7 chaînons, *N*-(C₁₋₃-alkylcarbonyl)-C₁₋₃-alkylamino-C₁₋₃-alkyle, carboxy-C₁₋₃-alkyle, C₁₋₃-alkyloxycarbonyl-C₁₋₃-alkyle, aminocarbonyl-C₁₋₃-alkyle, C₁₋₃-alkylaminocarbonyl-C₁₋₃-alkyle, di-(C₁₋₃-alkyl)-aminocarbonyl-C₁₋₃-alkyle, un groupe cycloalkyléniminocarbonyl-C₁₋₃-alkyle à 4 à 7 chaînons, C₁₋₅-alkyloxycarbonylamino-C₁₋₃-alkyle, C₁₋₃-alkylcarbonylamino-C₁₋₃-alkyle, C₁₋₅-alkylsulfonylamino-C₁₋₃-alkyle, aminocarbonylamino-C₁₋₃-alkyle, C₁₋₅-alkylaminocarbonylamino-C₁₋₃-alkyle, di-(C₁₋₃-alkyl)-aminocarbonylamino-C₁₋₃-alkyle, C₁₋₅-alkylsulfonylamino, C₁₋₅-alkylaminocarbonylamino, carboxy, C₁₋₃-alkyloxycarbonyle, benzyloxycarbonyle, C₁₋₃-alkylcarbonyle, aminocarbonyle, C₁₋₃-alkylaminocarbonyle, di-(C₁₋₃-alkyl)-aminocarbonyle, *N*-(C₃₋₇-cycloalkyl)-C₁₋₅-alkylaminocarbonyle, *N*-(phényl-C₁₋₃-alkyl)-C₁₋₅-alkylaminocarbonyle, un groupe cycloalkyléniminocarbonyle à 4 à 7 chaînons, aminocarbonyl-C₁₋₃-alkylaminocarbonyle, hydroxy, C₁₋₃-alkyloxy, allyloxy, propargyloxy, benzyloxy, amino, C₁₋₃-alkylamino, di-(C₁₋₃-alkyl)-amino, un groupe cycloalkylénimino à 4 à 7 chaînons, trifluorométhylcarbonylamino, *N*-(C₁₋₃-alkyl)-pipérazin-4-yl-C₁₋₃-alkyle, un groupe phényle ou un groupe hétéroaryle à 5 à 6 chaînons, avec la condition que, lors de la substitution d'un groupe méthylène voisin du groupe imino, deux hétéroatomes soient séparés l'un de l'autre par au moins deux atomes de carbone, et/ou
un groupe méthylène en position 3 d'un groupe cycloalkylénimino à 5 chaînons peut être remplacé par un atome de soufre, un groupe sulfinyle ou sulfonyle ou
un groupe méthylène en position 4 d'un groupe cycloalkylénimino à 6 ou 7 chaînons peut être remplacé par un atome d'oxygène ou de souffre, un groupe carbonyle, sulfinyle ou sulfonyle ou par un groupe -NH-éventuellement substitué par un groupe C₁₋₃-alkyle, hydroxy, formyle ou C₁₋₃-alkylcarbonyle, où en outre un groupe méthylène voisin du groupe -NH- éventuellement substitué cité précédemment peut être remplacé par un groupe carbonyle, sulfinyle ou sulfonyle, avec la condition que
lors de la substitution des groupements cycloalkylénimino à 6 à 7 chaînons cités précédemment, dans lesquels un groupe méthylène est remplacé par un atome d'oxygène ou de soufre, un groupe sulfinyle ou sulfonyle, deux hétéroatomes sont séparés l'un de l'autre par au moins deux atomes de carbone,
un groupe cycloalcényléniminocarbonyle ou cycloalcényléniminosulfonyle à 5 à 7 chaînons éventuellement substitué par un ou deux groupes C₁₋₃-alkyle, hydroxy-C₁₋₃-alkyle, C₁₋₅-alcoxy-C₁₋₃-alkyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, C₃₋₆-cycloalkylamino-C₁₋₃-alkyle, phényle, phényl-C₁₋₃-alkyle, hétéroaryle, hétéroaryl-C₁₋₃-alkyle, aminocarbonyle, C₁₋₃-alkylaminocarbonyle, di-(C₁₋₃-alkyl)-aminocarbonyle ou cycloalkyléniminocarbonyle à 4 à 7 chaînons, où la double liaison n'est pas liée à un atome d'azote et peut être condensée avec un groupe hétéroaryle à 5 ou 6 chaînons,
un groupe aminocarbonyle ou aminosulfonyle éventuellement substitué par un ou deux groupes C₁₋₅-alkyle, C₂₋₃-alcényle, C₂₋₃-alcynyle ou C₃₋₆-cycloalkyle,
où les substituants peuvent être identiques ou différents et
dans chaque cas l'un des groupes C₁₋₅-alkyle peut être substitué par un ou deux groupes hydroxy-C₁₋₃-alkyle, C₁₋₃-alcoxy-C₁₋₃-alkyle, benzyloxy-C₁₋₃-alkyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, C₁₋₅-alkyloxycarbonylamino-C₁₋₃-alkyle, C₃₋₆-cycloalkylamino-C₁₋₃-alkyle, aminocarbonyle, C₁₋₃-alkylaminocarbonyle, *N*-(C₃₋₇-cycloalkyl)-C₁₋₃-alkylaminocarbonyle, *N*-(phényl-C₁₋₃-alkyl)-C₁₋₅-alkylaminocarbonyle, di-(C₁₋₃-alkyl)-aminocarbonyle ou un groupe cycloalkyléniminocarbonyle à 4 à 7 chaînons,
ou un groupe de formule 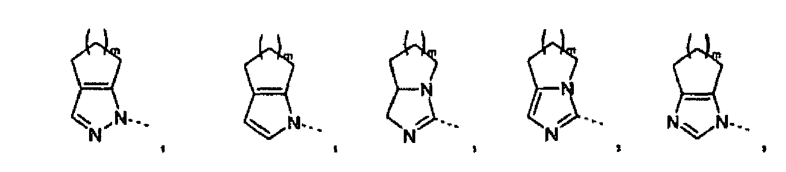 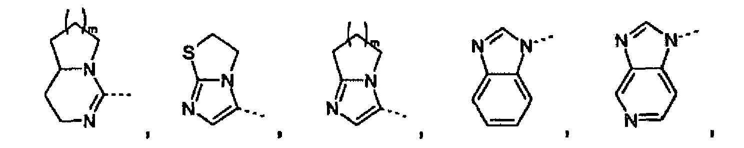 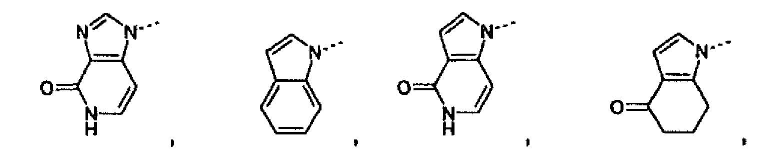 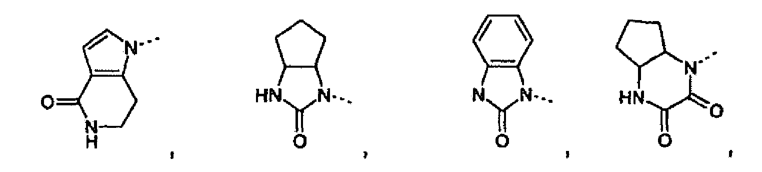 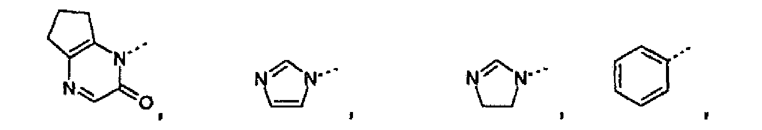 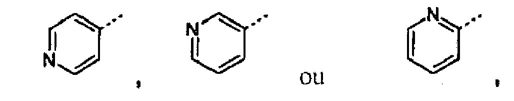 qui peut être substitué dans chaque cas sur un atome de carbone par un groupe C₁₋₃-alkyle, C₁₋₃-alkyloxy, C₁₋₃-alkyloxycarbonyle, C₁₋₅-alkyloxycarbonylamino-C₁₋₃-alkyle, C₁₋₃-alkylsulfonyle, C₁₋₃-alkylsulfonyl-C₁₋₃-alkyle, aminosulfonyle, C₁₋₃-alkylsulfonyl-C₁₋₃-alkyle, C₁₋₃-alkylsulfonylamino-C₁₋₃-alkyle, C₁₋₃-alkylsulfonylamino, C₁₋₃-alkylcarbonylamino-C₁₋₃-alkyle, C₁₋₃-alkylcarbonylamino, amino-C₁₋₃-alkyle, C₁₋₃-alkylamino-C₁₋₃-alkyle, di-(C₁₋₃-alkyl)-amino-C₁₋₃-alkyle, hydroxy-C₁₋₃-alkyle, C₁₋₅-alcoxy-C₁₋₃-alkyle, un groupe cycloalkylénimino-C₁₋₃-alkyle à 4 à 7 chaînons, morpholin-4-yl-C₁₋₃-alkyle, pipérazinyl-C₁₋₃-alkyle, *N*-(C₁₋₃-alkyl)-pipérazin-4-yl-C₁₋₃-alkyle, aminocarbonyle, C₁₋₃-alkylamino-carbonyl ou di-(C₁₋₃-alkyl)-aminocarbonyle, et où
m représente le nombre 1 ou 2,
R¹ représente un atome d'hydrogène ou d'halogène, un groupe C₁₋₃-alkyle ou C₁₋₃-alcoxy, où les atomes d'hydrogène du groupe C₁₋₃-alkyle ou C₁₋₃-alcoxy peuvent éventuellement être remplacés totalement ou en partie par des atomes de fluor, un groupe C₂₋₃-alcényle, C₂₋₃-alcynyle, nitrile, nitro ou amino,
R² représente un atome d'hydrogène ou d'halogène ou un groupe C₁₋₃-alkyle,
X représente un atome d'azote ou un groupe CH,
R³ représente un atome d'hydrogène ou un groupe C₁₋₃-alkyle,
R⁴ et R⁵ représentent dans chaque cas indépendamment l'un de l'autre
un atome d'hydrogène, un groupe hydroxy, un groupe OR⁹, un groupe C₂₋₆-alcényle ou C₂₋₆-alcynyle,
un groupe C₁₋₆-alkyle linéaire ou ramifié,
où les atomes d'hydrogène du groupe C₁₋₆-alkyle linéaire ou ramifié peuvent éventuellement être remplacés totalement ou en partie par des atomes de fluor, et qui peut éventuellement être substitué par un groupe C₃₋₅-cycloalkyle, un groupe nitrile, hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁-₅-alkyloxy, mercapto, C₁₋₅-alkylsulfanyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₄₋₆-cycloalkyléniminocarbonyle, aminosulfonyle, C₁₋₅-alkylaminosulfonyle, di-(C₁₋₅-alkyl)-aminosulfonyle, C₄₋₆-cycloalkyléniminosulfonyle, amino, C₁₋₅-alkylamino, di-(C₁₋₅-alkyl)-amino, C₁₋₅-alkylcarbonylamina, C₁₋₅-alkylsulfonylamino, *N*-(C₁₋₅-alkylsulfonyl)-C₁₋₅-alkylamino ou C₃₋₆-cycloalkylcarbonylamino,
où, dans les cycles à 6 à 7 chaînons du groupe C₄₋₆-cycloalkyléniminocarbonyle, dans la partie cyclique un groupe méthylène en position 4 d'un groupe cycloalkylénimino à 6 ou 7 chaînons peut être remplacé par un atome d'oxygène ou de soufre, par un groupe carbonyle, sulfinyle, sulfonyle ou -NR⁷- et en outre un groupe méthylène voisin d'un groupe -NR⁷- cité précédemment peut être remplacé par un groupe carbonyle,
un groupe phényle ou hétéroaryle
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle,
un groupe phényl-C₁₋₅-alkyle ou hétéroaryl-C₁₋₅-alkyle,
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle, et qui peut éventuellement être substitué dans la partie C₁₋₅-alkyle par un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy ou un groupe C₁₋₅-alkyloxycarbonyl-C₁₋₅-alkyloxy,
un groupe cycloalkyle, cycloalkylénimino, cycloalkyl-C₁₋₅-alkyle ou cycloalkylénimino-C₁₋₃-alkyle à 3 à 7 chaînons,
dans lequel, dans le cas de cycles à 4 à 7 chaînons, dans la partie cyclique un groupe méthylène peut éventuellement être remplacé par un groupe -N(R⁷)-, un atome d'oxygène ou de soufre ou un groupe -S(O)- ou -S(O)₂-, ou
dans lequel, dans le cas de cycles à 4 à 7 chaînons, dans la partie cyclique deux groupes méthylène voisins peuvent éventuellement être remplacés ensemble par un groupe -C(O)N(R⁸)- ou -S(O)₂N(R⁸)-, ou
dans lequel, dans le cas de cycles à 6 à 7 chaînons, dans la partie cyclique trois groupes méthylène voisins peuvent éventuellement être remplacés ensemble par un groupe -OC(O)N(R⁹)- ou -N(R⁸)C(O)N(R⁸)- ou -N(R⁸)S(O)₂N(R⁸)- substitué,
avec la condition qu'un groupe cycloalkyle, cycloalkylénimino, cycloalkyl-C₁₋₅-alkyle ou cycloalkylénimino-C₁₋₃-alkyle à 3 à 7 chaînons défini comme ci-dessus, dans lequel deux hétéroatomes du groupe de l'oxygène et de l'azote sont séparés l'un de l'autre par exactement un groupe -CH₂-éventuellement substitué est exclus,
où un groupe cycloalkyle, cycloalkylénimino, cycloalkyl-C₁₋₅-alkyle ou cycloalkylénimino-C₁₋₃-alkyle à 3 à 7 chaînons défini comme ci-dessus peut être substitué sur un ou deux groupes -CH₂- par un ou deux groupes C₁₋₃-alkyle à chaque fois,
avec la condition que R⁴ et R⁵ ne puissent pas être définis en même temps comme des groupes hydroxy ou OR⁹, ou
R⁴ et R⁵ forment avec l'atome de carbone auquel ils sont liés un groupe C₃₋₈-cycloalkyle ou C₃₋₈-cycloalcényle,
où l'un des groupes méthylène d'un groupe C₄₋₈-cycloalkyle peut être remplacé par un atome d'oxygène ou de soufre ou un groupe -N(R⁷)- ou un groupe carbonyle, sulfinyle ou sulfonyle, et/ou
deux groupes méthylène d'un groupe C₄₋₈-cycloalkyle qui sont directement voisins peuvent être remplacés ensemble par un groupe -C(O)N(R⁸)- ou -S(O)₂N(R⁸)-, et/ou
trois groupes méthylène d'un groupe C₆₋₈-cycloalkyle qui sont directement voisins peuvent être remplacés ensemble par un groupe -OC(O)N(R⁸)-, -N(R⁸)C(O)N(R⁸)- ou -N(R⁸)S(O)₂N(R⁸)-,
où 1 à 3 atomes de carbone d'un groupe C₃₋₈-cycloalkyle peuvent éventuellement être substitués indépendamment les uns des autres dans chaque cas par un ou deux atomes d'halogène identiques ou différents ou des groupes C₁₋₅-alkyle, nitrile, hydroxy, C₁₋₅-alkyloxy, C₁₋₅-alkylcarbonyloxy, carboxy-C₁₋₅-alkyle, C₁₋₅-alkyloxycarbonyl-C₁₋₅-alkyle, C₁₋₅-alkylsulfanyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₃₋₆-cyclo-alkyléniminocarbonyle, aminosulfonyle, C₁₋₅-alkylaminosulfonyle, di-(C₁₋₅-alkyl)-aminosulfonyle, C₃₋₆-cycloalkyléniminosulfonyle, amino, C₁₋₅-alkyl-amino, di-(C₁₋₅-alkyl)-amino, C₁₋₅-alkylcarbonylamino, C₁₋₅-alkylsulfonyl-amino, *N*-(C₁₋₅-alkylsulfonyl)-C₁₋₅-alkylamino ou C₃₋₆-cycloalkylcarbonyl-amino,
où 1 à 2 atomes de carbone d'un groupe C₃₋₈-cycloalcényle peuvent éventuellement être substitués indépendamment l'un de l'autre dans chaque cas par un groupe C₁₋₅-alkyle, nitrile, carboxy-C₁₋₅-alkyle, C₁₋₅-alkyloxycarbonyl-C₁₋₅-alkyle, carboxy, C₁₋₅-alkyloxycarbonyle, aminocarbonyle, C₁₋₅-alkylaminocarbonyle, di-(C₁₋₅-alkyl)-aminocarbonyle, C₃₋₆-cycloalkyléniminocarbonyle, aminosulfonyle, C₁₋₅-alkylaminosulfonyle, di-(C₁₋₅-alkyl)-aminosulfonyle, C₃₋₆-cycloalkyléniminosulfonyle,
et 1 à 2 atomes de carbone d'un groupe C₄₋₈-cycloalcényle, qui ne sont pas liés par une double liaison à un autre atome de carbone, peuvent éventuellement être substitués indépendamment l'un de l'autre par un atome de fluor ou un groupe hydroxy, C₁₋₅-alkyloxy, C₁₋₅-alkylcarbonyloxy, C₁₋₅-alkylsulfanyle, C₁₋₅-alkylsulfonyle, amino, C₁₋₅-alkylamino, di-(C₁₋₅-alkyl)-amino, C₁₋₅-alkylcarbonylamino, C₁₋₅-alkylsulfanylamino, *N*-(C₁₋₅-alkylsulfonyl)-C₁₋₅-alkylamino ou C₃₋₆-cycloalkylcarbonylamino,
avec la condition qu'un tel groupe C₃₋₈-cycloalkyle ou C₃₋₈-cycloalcényle formé à partir de R⁴ et R⁵,
dans lequel deux hétéroatomes du groupe de l'oxygène et de l'azote sont séparés l'un de l'autre dans le cycle par exactement un groupe -CH₂-éventuellement substitué, et/ou
dans lequel l'un ou les deux groupes méthylène du cycle, qui sont liés directement à l'atome de carbone auquel sont liés les groupements R⁴ et R⁵, sont remplacés par un hétéroatome du groupe de l'oxygène, de l'azote et du soufre, et/ou
dans lequel un substituant lié au groupe cyclique qui se **caractérise en ce qu'**un hétéroatome du groupe de l'oxygène, de l'azote, du soufre et un atome d'halogène est lié directement au groupe cyclique, est séparé par exactement un groupe méthylène éventuellement substitué d'un autre hétéroatome du groupe de l'oxygène, de l'azote et du soufre, à l'exception du groupe sulfone, et/ou
dans lequel deux atomes d'oxygène sont liés directement entre eux, est exclus,
R⁷ représente indépendamment des autres un atome d'hydrogène, un groupe hydroxy, un groupe formyle, un groupe C₁₋₅-alkyle, C₁₋₅-alkyl carbonyle, C₁₋₅-alkyloxycarbonyle ou C₁₋₅-alkylsulfonyle,
R⁸ représente indépendamment des autres un atome d'hydrogène ou un groupe C₁₋₅-alkyle,
R⁹ représente un groupe C₁₋₆-alkyle linéaire ou ramifié,
où les atomes d'hydrogène du groupe C₁₋₆-alkyle linéaire ou ramifié peuvent éventuellement être remplacés totalement ou en partie par des atomes de fluor, et qui peut éventuellement être substitué par un groupe C₃₋₅-cycloalkyle, un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, carboxy, C₁₋₅-alkyloxycarbonyle, amino-carbonyle, C₁₋₅-alkylaminocarbonyte, di-(C₁₋₅-alkyl)-aminocarbonyle, C₄₋₆-cycloalkyléniminocarbonyle, C₁₋₅-alkyloxycarbonylamino, amino, C₁₋₃-alkylamino, di-(C₁₋₅-alkyl)-amino, C₁₋₅-alkylcarbonylamino, C₁₋₅-alkyl-sulfonylamino, *N*-(C₁₋₅-alkylsulfonyl)-C₁₋₅-alkylamino ou C₃₋₆-cycloalkyl-carbonylamino,
où dans les cycles à 6 à 7 chaînons du groupe C₄₋₆-cycloalkyléniminocarbonyle, dans la partie cyclique un groupe méthylène en position 4 d'un groupe cycloalkylénimino à 6 à 7 chaînons peut être remplacé par un atome d'oxygène ou de soufre, par un groupe carbonyle, sulfinyle, sulfonyle ou -NR⁷- et en outre un groupe méthylène voisin d'un groupe -NR⁷- cité précédemment peut être remplacé par un groupe carbonyle,
avec la condition que le remplacement d'atomes d'hydrogène du premier atome de carbone du groupe C₁₋₆-alkyle linéaire ou ramifié par des substituants du groupe de l'oxygène, du soufre ou de l'azote est exclus,
un groupe phényle, hétéroaryle, phényl-C₁₋₅-alkyle ou hétéroaryl-C₁₋₅-alkyle, qui peuvent éventuellement être substitués une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle,
un groupe cycloalkyle, cycloalkyl-C₁₋₅-alkyle ou cycloalkylénimino-C₂₋₃-alkyle à 3 à 7 chaînons,
dans lequel, dans le cas de cycles à 4 à 7 chaînons, dans la partie cyclique un groupe méthylène peut éventuellement être remplacé par un groupe -N(R⁷)-, un atome d'oxygène ou de soufre ou un groupe -S(O)- ou -S(O)₂-, ou
dans lequel, dans le cas de cycles à 4 à 7 chaînons, dans la partie cyclique deux groupes méthylène voisins peuvent éventuellement être remplacés ensemble par un groupe -C(O)N(R⁸)- ou -S(O)₂N(R⁸)-, ou
dans lequel, dans le cas de cycles à 6 à 7 chaînons, dans la partie cyclique trois groupes méthylène voisins peuvent éventuellement être remplacés ensemble par un groupe -OC(O)N(R⁸)- ou -N(R⁸)C(Q)N(R⁸)- ou -N(R⁸)S(O)₂N(R⁸)- substitué,
avec la condition qu'un groupe cycloalkyle, cycloalkyl-C₁₋₅-alkyle ou cycloalkylénimino-C₂₋₃-alkyle à 3 à 7 chaînons défini comme ci-dessus, dans lequel deux hétéroatomes du groupe de l'oxygène et de l'azote sont séparés l'un de l'autre par exactement un groupe -CH₂- éventuellement substitué, est exclus,
où un groupe cycloalkyle, cycloalkyl-C₁₋₅-alkyle ou cycloalkylénimino-C₂₋₃-alkyle à 3 à 7 chaînons défini comme ci-dessus peut être substitué sur un ou deux groupes -CH₂- dans chaque cas par un ou deux groupes C₁₋₃-alkyle,
B représente un groupement de formule générale 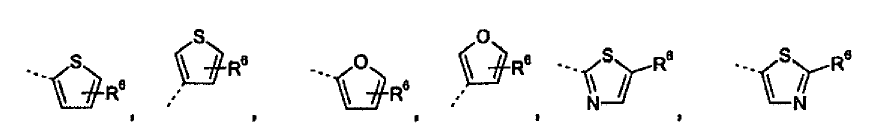 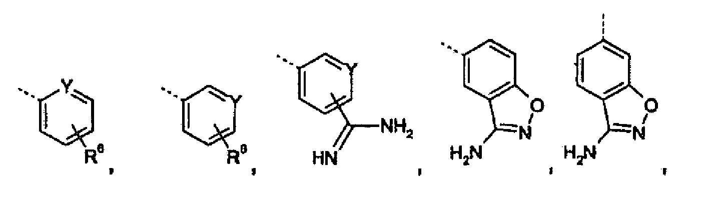 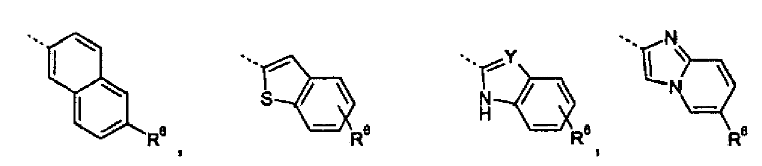 ou 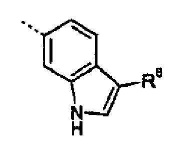 Y représente un atome d'azote ou un groupe CH,
R⁶ représente un atome d'hydrogène, un atome d'halogène, un groupe nitrile, un groupe C₂₋₃-alcényle, C₂₋₃-alcynyle, C₁₋₃-alkyle ou un groupe C₁₋₃-alcoxy, où les atomes d'hydrogène du groupe C₁₋₃-alkyle ou C₁₋₃-alcoxy peuvent éventuellement être remplacés totalement ou en partie par des atomes de fluor, où, sauf indication contraire, par l'expression « groupe hétéroaryle » citée précédemment dans les définitions, on doit entendre un groupe hétéroaryle à 5 ou 6 chaînons monocycliques, où
le groupe hétéroaryle à 6 chaînons contient un, deux ou trois atomes d'azote et
le groupe hétéroaryle à 5 chaînons contient un groupe imino éventuellement substitué par un groupe C₁₋₃-alkyle, phényle ou phényl-C₁₋₃-alkyle, un atome d'oxygène ou de soufre ou
un groupe imino éventuellement substitué par un groupe C₁₋₃-alkyle, phényle, amino-C₂₋₃-alkyle, C₁₋₃-alkylamino-C₂₋₃-alkyle, di-(C₁₋₃-alkyl)-amino-C₂₋₃-alkyle, un groupe C₃₋₆-cycloalkylénimino-C₁₋₃-alkyle ou phényl-C₁₋₃-alkyle ou un atome d'oxygène ou de soufre et en outre un atome d'azote ou
un groupe imino éventuellement substitué par un groupe C₁₋₃-alkyle ou phényl-C₁₋₃-alkyle et deux ou trois atomes d'azote,
et en outre un cycle phényle éventuellement substitué par un atome de fluor, de chlore ou de brome, un groupe C₁₋₃-alkyle, hydroxy, C₁₋₃-alkyloxy, amino, C₁₋₃-alkylamino, di-(C₁₋₃-alkyl)-amino ou C₃₋₆-cyclo-alkylénimino peut être condensé aux groupes hétéroaryle monocycliques cités précédemment par le biais de deux atomes de carbone voisins,
et la liaison se fait par le biais d'un atome d'azote ou par le biais d'un atome de carbone de la partie hétérocyclique ou d'un cycle phényle condensé,
où, sauf indication contraire, par l'expression « atome d'halogènes citée précédemment dans les définitions, on doit comprendre un atome du groupe du fluor, du chlore, du brome et de l'iode,
où les groupes alkyle, alcényle, alcynyle et alcoxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
et où les atomes d'hydrogène des groupes méthyle ou éthyle contenus dans les définitions citées précédemment, sauf indication contraire, peuvent être remplacés totalement ou en partie par des atomes de fluor,
leurs tautomères, leurs énantiomères, leurs diastéréoisomères, leurs mélanges et leurs sels.

2. Pyrrolidinones substituées de formule générale 1 selon la revendication 1, **caractérisées en ce que**
A représente un groupe cycloalkyléniminocarbonyle ou cycloalkyléniminosulfonyle à 4 à 7 chaînons, où
la partie cycloalkylénimino peut être substituée dans le squelette carboné par un ou deux atomes de fluor, un ou deux groupes C₁₋₃-alkyle, C₂₋₃-alcényle, C₂₋₃-alcynyle, hydroxy-C₁₋₃-alkyle, C₁₋₃-alkyloxy-C₁₋₃-alkyle, phényl-C₁₋₃-alkyle, 1,1-diphényl-C₁₋₃-alkyle, hétéroaryl-C₁₋₃-alkyle, amino-C₁₋₃-alkyle, C₃₋₆-cycloalkylamino-C₁₋₃-alkyle, phénylamino-C₁₋₃-alkyle, C₁₋₅-alkylamino-C₁₋₃-alkyle, di-(C₁₋₅-alkyl)-amino-C₁₋₅-alkyle, *N*-(C₃₋₆-cycloalkyl)-C₁₋₃-alkylamino-C₁₋₃-alkyle, un groupe cycloalkylénimino-C₁₋₃-alkyle à 4 à 7 chaînons, *N*-(C₁₋₃-alkylcarbonyl)-C₁₋₃-alkylamino-C₁₋₃-alkyle, carboxy-C₁₋₃-alkyle, C₁₋₃-alkyloxycarbonyl-C₁₋₃-alkyle, aminocarbonyl-C₁₋₃-alkyle, C₁₋₃-alkylaminocarbonyl-C₁₋₃-alkyle, di-(C₁₋₃-alkyl)-aminocarbonyl-C₁₋₃-alkyle, un groupe cycloalkyléniminocarbonyl-C₁₋₃-alkyle à 4 à 7 chaînons, C₁₋₅-alkyloxycarbonylamino-C₁₋₃-alkyle, C₁₋₃-alkylcarbonylamino-C₁₋₃-alkyle, C₁₋₅-alkylsulfonylamino-C₁₋₃-alkyle, aminocarbonylamino-C₁₋₃-alkyle, C₁₋₅-alkylaminocarbonylamino-C₁₋₃-alkyle, di-(C₁₋₃-alkyl)-aminocarbonylamino-C₁₋₃-alkyle, C₁₋₅-alkylsulfonylamino, C₁₋₅-alkylaminocarbonylamino, carboxy, C₁₋₃-alkyloxycarbonyle, benzyloxycarbonyle, C₁₋₃-alkylcarbonyle, aminocarbonyle, C₁₋₃-alkylaminocarbonyle, di-(C₁₋₃-alkyl)-aminocarbonyle, *N*-(C₃₋₇-cycloalkyl)-C₁₋₅-alkylaminocarbonyle, *N*-(phényl-C₁₋₃-alkyl)-C₁₋₅-alkylaminocarbonyle, un groupe cycloalkyléniminocarbonyle à 4 à 7 chaînons, aminocarbonyl-C₁₋₃-alkylaminocarbonyle, hydroxy, C₁₋₃-alkyloxy, allyloxy, propargyloxy, benzyloxy, amino, C₁₋₃-alkylamino, di-(C₁₋₃-alkyl)-amino, un groupe cycloalkylénimino à 4 à 7 chaînons, trifluorométhylcarbonylamino, *N*-(C₁₋₃-alkyl)-pipérazin-4-yl-C₁₋₃-alkyle, un groupe phényle ou un groupe hétéroaryle à 5 à 6 chaînons, avec la condition que, lors de la substitution d'un groupe méthylène voisin du groupe imino, deux hétéroatomes soient séparés l'un de l'autre par au moins deux atomes de carbone, et/ou
un groupe méthylène en position 3 d'un groupe cycloalkylénimino à 5 chaînons peut être remplacé par un atome de soufre, un groupe sulfinyle ou sulfonyle ou
un groupe méthylène en position 4 d'un groupe cycloalkylénimino à 6 ou 7 chaînons peut être remplacé par un atome d'oxygène ou de soufre, un groupe carbonyle, sulfinyle ou sulfonyle ou par un groupe -NH-éventuellement substitué par un groupe C₁₋₃-alkyle, hydroxy, formyle ou C₁₋₃-alkylcarbonyle, où en outre un groupe méthylène voisin du groupe -NH- éventuellement substitué cité précédemment peut être remplacé par un groupe carbonyle, sulfinyle ou sulfonyle, avec la condition que
lors de la substitution des groupements cycloalkylénimino à 6 à 7 chaînons cités précédemment, dans lesquels un groupe méthylène est remplacé par un atome d'oxygène ou de soufre, un groupe sulfinyle ou sulfonyle, deux hétéroatomes sont séparés l'un de l'autre par au moins deux atomes de carbone,
un groupe cycloalcényléniminocarbonyle ou cycloalcényléniminosulfonyle à 5 à 7 chaînons éventuellement substitué par un ou deux groupes C₁₋₃-alkyle, hydroxy-C₁₋₃-alkyle, C₁₋₅-alcoxy-C₁₋₃-alkyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, C₃₋₆-cycloalkylamino-C₁₋₃-alkyle, phényle, phényl-C₁₋₃-alkyle, hétéroaryle, hétéroaryl-C₁₋₃-alkyle, aminocarbonyle, C₁₋₃-alkylaminocarbonyle, di-(C₁₋₃-alkyl)-aminocarbonyle ou cycloalkyléniminocarbonyle à 4 à 7 chaînons, où la double liaison n'est pas liée à un atome d'azote et peut être condensée avec un groupe hétéroaryle à 5 ou 6 chaînons,
un groupe aminocarbonyle ou aminosulfonyle éventuellement substitué par un ou deux groupes C₁₋₅-alkyle, C₂₋₃-alcényle, C₂₋₃-alcynyle ou C₃₋₆-cycloalkyle,
où les substituants peuvent être identiques ou différents et
dans chaque cas l'un des groupes C₁₋₅-alkyle peut être substitué par un ou deux groupes hydroxy-C₁₋₃-alkyle, C₁₋₃-alcoxy-C₁₋₃-alkyle, benzyloxy-C₁₋₃-alkyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, C₁₋₅-alkyloxycarbonylamino-C₁₋₃-alkyle, C₃₋₆-cycloalkylamino-C₁₋₃-alkyle, aminocarbonyle, C₁₋₃-alkylaminocarbonyle, *N*-(C₃₋₇-cycloalkyl)-C₁₋₃-alkylaminocarbonyle, *N*-(phényl-C₁₋₃-alkyl)-C₁₋₅-alkylaminocarbonyle, di-(C₁₋₃-alkyl)-aminocarbonyle ou un groupe cycloalkyléniminocarbonyle à 4 à 7 chaînons,
ou un groupe de formule 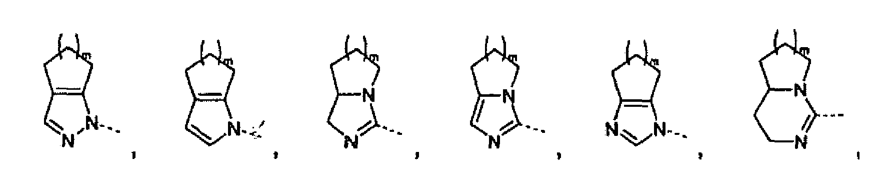 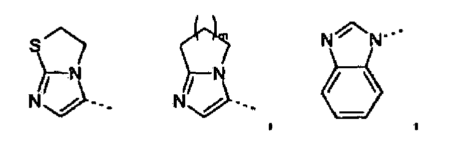 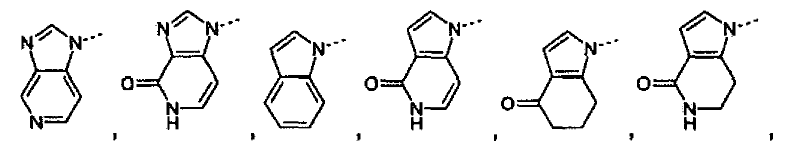 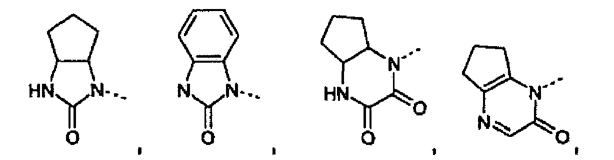 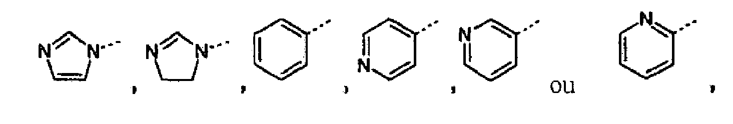 qui peut être substitué dans chaque cas sur un atome de carbone par un groupe C₁₋₃-alkyle, C₁₋₃-alkyloxy, C₁₋₃-alkyloxycarbanyle, C₁₋₅-alkyloxycarbonylamino-C₁₋₃-alkyle, C₁₋₃-alkylsulfanyle, C₁₋₃-alkylsulfonyl-C₁₋₃-alkyle, aminosulfonyle, C₁₋₃-alkylsulfonyl-C₁₋₃-alkyle, C₁₋₃-alkylsulfonylamino-C₁₋₃-alkyle, C₁₋₃-alkylsulfonylamino, C₁₋₃-alkylcarbonylamino-C₁₋₃-alkyle, C₁₋₃-alkylcarbonylamino, amino-C₁₋₃-alkyle, C₁₋₃-alkylamino-C₁₋₃-alkyle, di-(C₁₋₃-alkyl)-amino-C₁₋₃-alkyle, hydroxy-C₁₋₃-alkyle, C₁₋₅-alcoxy-C₁₋₃-alkyle, un groupe cycloalkylénimino-C₁₋₃-alkyle à 4 à 7 chaînons, morpholin-4-yl-C₁₋₃-alkyle, pipérazinyl-C₁₋₃-alkyle, *N*-(C₁₋₃-alkyl)-pipérazin-4-yl-C₁₋₃-alkyle, aminocarbonyle, C₁₋₃-alkylamino-carbonyle ou di-(C₁₋₃-alkyl)-aminocarbonyle, et où
m représente le nombre 1 ou 2,
R¹ représente un atome d'hydrogène ou d'halogène, un groupe C₁₋₃-alkyle ou C₁₋₃-alcoxy, où les atomes d'hydrogène du groupe C₁₋₃-alkyle ou C₁₋₃-alcoxy peuvent éventuellement être remplacés totalement ou en partie par des atomes de fluor, un groupe C₂₋₃-alcényle, C₂₋₃-alcynyle, nitrile, nitro ou amino,
R² représente un atome d'hydrogène ou d'halogène ou un groupe C₁₋₃-alkyle,
X représente un atome d'azote ou un groupe CH,
R³ représente un atome d'hydrogène ou un groupe C₁₋₃-alkyle,
R⁴ et R⁵ représentent dans chaque cas indépendamment l'un de l'autre
un atome d'hydrogène, un groupe hydroxy, un groupe OR⁹, un groupe C₂₋₆-alcényle ou C₂₋₆-alcynyle,
un groupe C₁₋₆-alkyle linéaire ou ramifié,
où les atomes d'hydrogène du groupe C₁₋₆-alkyle linéaire ou ramifié peuvent éventuellement être remplacés totalement ou en partie par des atomes de fluor, et qui peut éventuellement être substitué par un groupe C₃₋₅-cycloalkyle, un groupe nitrile, hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, mercapto, C₁₋₅-alkylsulfanyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₄₋₆-cycloalkyléniminocarbonyle, aminosulfonyle, C₁₋₅-alkylaminosulfonyle, di-(C₁₋₅-alkyl)-aminosulfonyle, C₄₋₆-cycloalkyléniminosulfonyle, amino, C₁₋₅-alkylamino, di-(C₁₋₅-alkyl)-amino, C₁₋₅-alkylcarbonylarnino, C₁₋₅-alkylsulfonylamino, *N*-(C₁₋₅-alkylsulfonyl)-C₁₋₅-alkylamino ou C₃₋₆-cycloalkylcarbonylamino,
où, dans les cycles à 6 à 7 chaînons du groupe C₄₋₆-cycloalkyléniminocarbonyle, dans la partie cyclique un groupe méthylène en position 4 d'un groupe cycloalkylénimino à 6 ou 7 chaînons peut être remplacé par un atome d'oxygène ou de soufre, par un groupe carbonyle, sulfinyle, sulfonyle ou -NR⁷- et en outre un groupe méthylène voisin d'un groupe -NR⁷- cité précédemment peut être remplacé par un groupe carbonyle,
un groupe phényle ou hétéroaryle
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle,
un groupe phényl-C₁₋₅-alkyle ou hétéroaryl-C₁₋₅-alkyle,
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle, et qui peut éventuellement être substitué dans la partie C₁₋₅-alkyle par un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy ou un groupe C₁₋₅-alkyloxycarbonyl-C₁₋₅-alkyloxy,
un groupe cycloalkyle, cycloalkylénimino, cycloalkyl-C₁₋₅-alkyle ou cycloalkylénimino-C₁₋₃-alkyle à 3 à 7 chaînons,
dans lequel, dans le cas de cycles à 4 à 7 chaînons, dans la partie cyclique un groupe méthylène peut éventuellement être remplacé par un groupe -N(R⁷)-, un atome d'oxygène ou de soufre ou un groupe -S(O)- ou -S(O)₂-, ou
dans lequel, dans le cas de cycles à 4 à 7 chaînons, dans la partie cyclique deux groupes méthylène voisins peuvent éventuellement être remplacés ensemble par un groupe -C(O)N(R⁸)- ou -S(O)₂N(R⁸)-, ou
dans lequel, dans le cas de cycles à 6 à 7 chaînons, dans la partie cyclique trois groupes méthylène voisins peuvent éventuellement être remplacés ensemble par un groupe -OC(O)N(R⁸)- ou -N(R⁸)C(O)N(R⁸)- ou -N(R⁸)S(O)₂N(R⁸)- substitué,
avec la condition qu'un groupe cycloalkyle, cycloalkylénimino, cycloalkyl-C₁₋₅-alkyle ou cycloalkylénimino-C₁₋₃-alkyle à 3 à 7 chaînons défini comme ci-dessus, dans lequel deux hétéroatomes du groupe de l'oxygène et de l'azote sont séparés l'un de l'autre par exactement un groupe -CH₂-éventuellement substitué est exclus,
où un groupe cycloalkyle, cycloalkylénimino, cycloalkyl-C₁₋₅-alkyle ou cycloalkylénimino-C₁₋₃-alkyle à 3 à 7 chaînons défini comme ci-dessus peut être substitué sur un ou deux groupes -CH₂- par un ou deux groupes C₁₋₃-alkyle à chaque fois,
avec la condition que R⁴ et R⁵ ne puissent pas être définis en même temps comme des groupes hydroxy ou OR⁹, ou
R⁴ et R⁵ forment avec l'atome de carbone auquel ils sont liés un groupe C₃₋₈-cycloalkyle ou C₃₋₈-cycloalcényle,
où l'un des groupes méthylène d'un groupe C₄₋₈-cycloalkyle peut être remplacé par un atome d'oxygène ou de soufre ou un groupe -N(R⁷)-, ou un groupe carbonyle, sulfinyle ou sulfonyle, et/ou
deux groupes méthylène d'un groupe C₄₋₈-cycloalkyle qui sont directement voisins peuvent être remplacés ensemble par un groupe -C(O)N(R⁸)- ou -S(O)₂N(R⁸)-, et/ou
trois groupes méthylène d'un groupe C₆₋₈-cycloalkyle qui sont directement voisins peuvent être remplacés ensemble par un groupe -OC(O)N(R⁸)-, -N(R⁸)C(O)N(R⁸)- ou -N(R⁸)S(O)₂N(R⁸)-,
où 1 à 3 atomes de carbone d'un groupe C₃₋₈-cycloalkyle peuvent éventuellement être substitués indépendamment les uns des autres dans chaque cas par un ou deux atomes d'halogène identiques ou différents ou des groupes C₁₋₅-alkyle, nitrile, hydroxy, C₁₋₅-alkyloxy, C₁₋₅-alkylcarbonyloxy, carboxy-C₁₋₅-alkyle, C₁₋₅-alkyloxycarbonyl-C₁₋₅-alkyle, C₁₋₅-alkylsulfanyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₃₋₆-cyclo-alkyléniminocarbonyle, aminosulfonyle, C₁₋₅-alkylaminosulfonyle, di-(C₁₋₅-alkyl)-aminosulfonyle, C₃₋₆-cycloalkyléniminosulfonyle, amino, C₁₋₅-alkylamino, di-(C₁₋₅-alkyl)-amino, C₁₋₅-alkylcarbonylamino, C₁₋₅-alkylsulfonylamino, *N*-(C₁₋₅-alkylsulfonyl)-C₁₋₅-alkylamino ou C₃₋₆-cycloalkylcarbonylamino,
où 1 à 2 atomes de carbone d'un groupe C₃₋₈-cycloalcényle peuvent éventuellement être substitués indépendamment l'un de l'autre dans chaque cas par un groupe C₁₋₅-alkyle, nitrile, carboxy-C₁₋₅-alkyle, C₁₋₅-alkyloxycarbonyl-C₁₋₅-alkyle, carboxy, C₁₋₅-alkyloxycarbonyle, aminocarbonyle, C₁₋₅-alkylaminocarbonyle, di-(C₁₋₅-alkyl)-aminocarbonyle, C₃₋₆-cycloalkyléniminocarbonyle, aminosulfonyle, C₁₋₅-alkylaminosulfonyle, di-(C₁₋₅-alkyl)-aminosulfonyle, C₃₋₆-cycloalkyléniminosulfonyle,
et 1 à 2 atomes de carbone d'un groupe C₄₋₈-cycloalcényle, qui ne sont pas liés par une double liaison à un autre atome de carbone, peuvent éventuellement être substitués indépendamment l'un de l'autre par un atome de fluor ou un groupe hydroxy, C₁₋₅-alkyloxy, C₁₋₅-alkylcarbonyloxy, C₁₋₅-alkylsulfanyle, C₁₋₅-alkylsulfonyle, amino, C₁₋₅-alkylamino, di-(C₁₋₅-alkyl)-amino, C₁₋₅-alkylcarbonylamino, C₁₋₅-alkylsulfonylamino, *N*-(C₁₋₅-alkylsulfonyl)-C₁₋₅-alkylamino ou C₃₋₆-cycloalkylcarbonylamino,
avec la condition qu'un tel groupe C₃₋₈-cycloalkyle ou C₃₋₈-cycloalcényle formé à partir de R⁴ et R⁵,
dans lequel deux hétéroatomes du groupe de l'oxygène et de l'azote sont séparés l'un de l'autre dans le cycle par exactement un groupe -CH₂-éventuellement substitué, et/ou
dans lequel l'un ou les deux groupes méthylène du cycle, qui sont liés directement à l'atome de carbone auquel sont liés les groupements R⁴ et R⁵, sont remplacés par un hétéroatome du groupe de l'oxygène, de l'azote et du soufre, et/ou
dans lequel un substituant lié au groupe cyclique qui se **caractérise en ce qu'**un hétéroatome du groupe de l'oxygène, de l'azote, du soufre et un atome d'halogène est lié directement au groupe cyclique, est séparé par exactement un groupe méthylène éventuellement substitué d'un autre hétéroatome du groupe de l'oxygène, de l'azote et du soufre, à l'exception du groupe sulfone, et/ou
dans lequel deux atomes d'oxygène sont liés directement entre eux, est exclus,
R⁷ représente indépendamment des autres un atome d'hydrogène, un groupe hydroxy, un groupe formyle, un groupe C₁₋₅-alkyle, C₁₋₅-alkylcarbonyle, C₁₋₅-alkyloxycarbonyle ou C₁₋₅-alkylsulfonyle,
R⁸ représente indépendamment des autres un atome d'hydrogène ou un groupe C₁₋₅-alkyle,
R⁹ représente un groupe C₁₋₆-alkyle linéaire ou ramifié,
où les atomes d'hydrogène du groupe C₁₋₆-alkyle linéaire ou ramifié peuvent éventuellement être remplacés totalement ou en partie par des atomes de fluor, et qui peut éventuellement être substitué par un groupe C₃₋₅-cycloalkyle, un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, carboxy, C₁₋₅-alkyloxycarbonyle, aminocarbonyle, C₁₋₅-alkylaminocarbonyle, di-(C₁₋₅-alkyl)-aminocarbonyle, C₄₋₆-cycloalkyléniminocarbonyle, C₁₋₅-alkyloxycarbonylamino, amino, C₁₋₅-alkylamino, di-(C₁₋₅-alkyl)-amino, C₁₋₅-alkylcarbonylamino, C₁₋₅-alkylsulfonylamino, *N*-(C₁₋₅-alkylsulfonyl)-C₁₋₅-alkylamino ou C₃₋₆-cycloalkylcarbonylamino,
où dans les cycles à 6 à 7 chaînons du groupe C₄₋₆-cycloalkyléniminocarbonyle, dans la partie cyclique un groupe méthylène en position 4 d'un groupe cycloalkylénimino à 6 à 7 chaînons peut être remplacé par un atome d'oxygène ou de soufre, par un groupe carbonyle, sulfinyle, sulfonyle ou -NR⁷- et en outre un groupe méthylène voisin d'un groupe -NR⁷- cité précédemment peut être remplacé par un groupe carbonyle,
avec la condition que le remplacement d'atomes d'hydrogène du premier atome de carbone du groupe C₁₋₆-alkyle linéaire ou ramifié par des substituants du groupe de l'oxygène, du soufre ou de l'azote est exclus,
un groupe phényle, hétéroaryle, phényl-C₁₋₅-alkyle ou hétéroaryl-C₁₋₅-alkyle, qui peuvent éventuellement être substitués une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle,
un groupe cycloalkyle, cycloalkyl-C₁₋₅-alkyle ou cycloalkylénimino-C₂₋₃-alkyle à 3 à 7 chaînons,
dans lequel, dans le cas de cycles à 4 à 7 chaînons, dans la partie cyclique un groupe méthylène peut éventuellement être remplacé par un groupe -N(R⁷)-, un atome d'oxygène ou de soufre ou un groupe -S(O)- ou -S(O)₂-, ou
dans lequel, dans le cas de cycles à 4 à 7 chaînons, dans la partie cyclique deux groupes méthylène voisins peuvent éventuellement être remplacés ensemble par un groupe -C(O)N(R⁸)- ou -S(O)₂N(R⁸)-, ou
dans lequel, dans le cas de cycles à 6 à 7 chaînons, dans la partie cyclique trois groupes méthylène voisins peuvent éventuellement être remplacés ensemble par un groupe -OC(C)N(R⁸)- ou -N(R⁸)C(O)N(R⁸)- ou -N(R⁸)S(O)₂N(R⁸)- substitué,
avec la condition qu'un groupe cycloalkyle, cycloalkyl-C₁₋₅-alkyle ou cycloalkylénimino-C₂₋₃-alkyle à 3 à 7 chaînons défini comme ci-dessus, dans lequel deux hétéroatomes du groupe de l'oxygène et de l'azote sont séparés l'un de l'autre par exactement un groupe -CH₂- éventuellement substitué, est exclus,
où un groupe cycloalkyle, cycloalkyl-C₁₋₅-alkyle ou cycloalkylénimino-C₂₋₃-alkyle à 3 à 7 chaînons défini comme ci-dessus peut être substitué sur un ou deux groupes -CH₂- dans chaque cas par un ou deux groupes C₁₋₃-alkyle,
B représente un groupement de formule générale 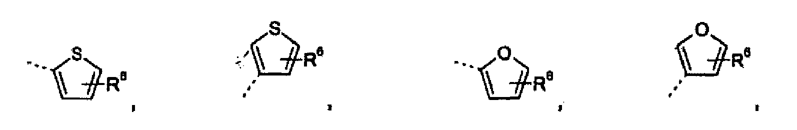 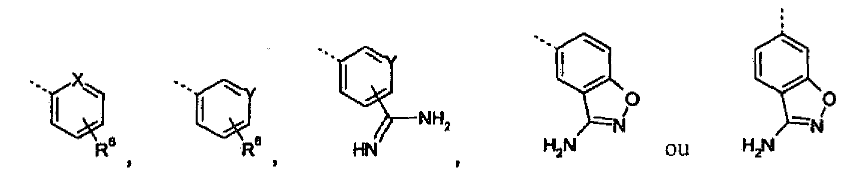 Y représente un atome d'azote ou un groupe CH,
R⁶ représente un atome d'hydrogène, un atome d'halogène, un groupe nitrile, un groupe C₂₋₃-alcényle, C₂₋₃-alcynyle, C₁₋₃-alkyle ou un groupe C₁₋₃-alcoxy, où les atomes d'hydrogène du groupe C₁₋₃-alkyle ou C₁₋₃-alcoxy peuvent éventuellement être remplacés totalement ou en partie par des atomes de fluor, où, sauf indication contraire, par l'expression « groupe hétéroaryle » citée précédemment dans les définitions, on doit entendre un groupe hétéroaryle à 5 ou 6 chaînons monocyclique, où
le groupe hétéroaryle à 6 chaînons contient un, deux ou trois atomes d'azote et
le groupe hétéroaryle à 5 chaînons contient un groupe imino éventuellement substitué par un groupe C₁₋₃-alkyle, phényle ou phényl-C₁₋₃-alkyle, un atome d'oxygène ou de soufre ou
un groupe imino éventuellement substitué par un groupe C₁₋₃-alkyle, phényle, amino-C₇₋₃-alkyle, C₁₋₃-alkylamino-C₂₋₃-alkyle, di-(C₁₋₃-alkyl)-amino-C₂₋₃-alkyle, un groupe C₃₋₆-cycloalkylénimino-C₁₋₃-alkyle ou phényl-C₁₋₃-alkyle ou un atome d'oxygène ou de soufre et en outre un atome d'azote ou
un groupe imino éventuellement substitué par un groupe C₁₋₃-alkyle ou phényl-C₁₋₃-alkyle et deux ou trois atomes d'azote,
et en outre un cycle phényle éventuellement substitué par un atome de fluor, de chlore ou de brome, un groupe C₁₋₃-alkyle, hydroxy, C₁₋₃-alkyloxy, amino, C₁₋₃-alkylamino, di-(C₁₋₃-alkyl)-amino ou C₃₋₆-cyclo-alkylénimino peut être condensé aux groupes hétéroaryle monocycliques cités précédemment par le biais de deux atomes de carbone voisins,
et la liaison se fait par le biais d'un atome d'azote ou par le biais d'un atome de carbone de la partie hétérocyclique ou d'un cycle phényle condensé,
où, sauf indication contraire, par l'expression « atome d'halogène » citée précédemment dans les définitions, on doit comprendre un atome du groupe du fluor, du chlore, du brome et de l'iode,
où les groupes alkyle, alcényle, alcynyle et alcoxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
et où les atomes d'hydrogène des groupes méthyle ou éthyle contenus dans les définitions citées précédemment, sauf indication contraire, peuvent être remplacés totalement ou en partie par des atomes de fluor,
leurs tautomères, leurs énantiomères, leurs diastéréoisomères, leurs mélanges et leurs sels.

3. Pyrrolidinones substituées de formule générale I selon la revendication 1, **caractérisées en ce que**
A représente un groupe cycloalkyléniminocarbonyle ou cycloalkyléniminosulfonyle à 4 à 7 chaînons, où
la partie cycloalkylénimino peut être substituée dans le squelette carboné par un ou deux atomes de fluor, un ou deux groupes C₁₋₃-alkyle, C₂₋₁-alcényle, C₂₋₃-alcynyle, hydroxy-C₁₋₃-alkyle, C₁₋₃-alkyloxy-C₁₋₃-alkyle, phényl-C₁₋₃-alkyle, 1,1-diphényl-C₁₋₃-alkyle, hétéroaryl-C₁₋₃-alkyle, amino-C₁₋₃-alkyle, C₃₋₆-cycloalkylamino-C₁₋₃-alkyle, phénylamino-C₁₋₃-alkyle, C₁₋₅-alkylamino-C₁₋₃-alkyle, di-(C₁₋₅-alkyl)-amino-C₁₋₅-alkyle, *N*-(C₃₋₆-cycloalkyl)-C₁₋₃-alkylamino-C₁₋₃-alkyle, un groupe cycloalkylénimino-C₁₋₃-alkyle à 4 à 7 chaînons, *N*-(C₁₋₃-alkylcarbonyl)-C₁₋₃-alkylamino-C₁₋₃-alkyle, carboxy-C₁₋₃-alkyle, C₁₋₃-alkyloxycarbonyl-C₁₋₃-alkyle, aminocarbonyl-C₁₋₃-alkyle, C₁₋₃-alkylaminocarbonyl-C₁₋₃-alkyle, di-(C₁₋₃-alkyl)-aminocarbonyl-C₁₋₃-alkyle, un groupe cycloalkyléniminocarbonyl-C₁₋₃-alkyle à 4 à 7 chaînons, C₁₋₅-alkyloxycarbonylamino-C₁₋₃-alkyle, C₁₋₃-alkylcarbonylamino-C₁₋₃-alkyle, C₁₋₅-alkylsulfonylamino-C₁₋₃-alkyle, aminocarbonylamino-C₁₋₃-alkyle, C₁₋₅-alkylaminocarbonylamino-C₁₋₃-alkyle, di-(C₁₋₃-alkyl)-aminocarbonylamino-C₁₋₃-alkyle, C₁₋₅-alkylsulfonylamino, C₁₋₅-alkylaminocarbonylamino, carboxy, C₁₋₃-alkyloxycarbonyle, benzyloxycarbonyle, C₁₋₃-alkylcarbonyle, aminocarbonyle, C₁₋₃-alkylaminocarbonyle, di-(C₁₋₃-alkyl)-aminocarbonyle, *N*-(C₃₋₇-cycloalkyl)-C₁₋₅-alkylaminocarbonyle, *N*-(phényl-C₁₋₃-alkyl)-C₁₋₅-alkylaminocarbonyle, un groupe cycloalkyléniminocarbonyle à 4 à 7 chaînons, aminocarbonyl-C₁₋₃-alkylaminocarbonyle, hydroxy, C₁₋₃-alkyloxy, allyloxy, propargyloxy, benzyloxy, amino, C₁₋₃-alkylamino, di-(C₁₋₃-alkyl)-amino, un groupe cycloalkylénimino à 4 à 7 chaînons, trifluorométhylcarbonylamino, *N*-(C₁₋₃-alkyl)-pipérazin-4-yl-C₁₋₃-alkyle, un groupe phényle ou un groupe hétéroaryle à 5 à 6 chaînons, avec la condition que, lors de la substitution d'un groupe méthylène voisin du groupe imino, deux hétéroatomes soient séparés l'un de l'autre par au moins deux atomes de carbone, et/ou
un groupe méthylène en position 3 d'un groupe cycloalkylénimino à 5 chaînons peut être remplacé par un atome de soufre ou
un groupe méthylène en position 4 d'un groupe cycloalkylénimino à 6 ou 7 chaînons peut être remplacé par un atome d'oxygène ou de soufre, un groupe carbonyle, sulfinyle ou sulfonyle ou par un groupe -NH- éventuellement substitué par un groupe C₁₋₃-alkyle, hydroxy, formyle ou C₁₋₃-alkylcarbonyle, où en outre un groupe méthylène voisin du groupe -NH- éventuellement substitué cité précédemment peut être remplacé par un groupe carbonyle, sulfinyle ou sulfonyle, avec la condition que
lors de la substitution des groupements cycloalkylénimino à 6 à 7 chaînons cités précédemment, dans lesquels un groupe méthylène est remplacé par un atome d'oxygène ou de soufre, un groupe sulfinyle ou sulfonyle, deux hétéroatomes sont séparés l'un de l'autre par au moins deux atomes de carbone,
un groupe cycloalcényléniminocarbonyle ou cycloalcényléniminosulfonyle à 5 à 7 chaînons éventuellement substitué par un ou deux groupes C₁₋₃-alkyle, hydroxy-C₁₋₃-alkyle, C₁₋₅-alcoxy-C₁₋₃-alkyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, C₃₋₆-cycloalkylamino-C₁₋₃-alkyle, phényle, phényl-C₁₋₃-alkyle, hétéroaryle, hétéroaryl-C₁₋₃-alkyle, aminocarbonyle, C₁₋₃-alkylaminocarbonyle, di-(C₁₋₃-alkyl)-aminocarbanyle ou cycloalkyléniminocarbonyle à 4 à 7 chaînons, où la double liaison n'est
pas liée à un atome d'azote et peut être condensée avec un groupe hétéroaryle à 5 ou 6 chaînons,
un groupe aminocarbonyle ou aminosulfonyle éventuellement substitué par un ou deux groupes C₁₋₅-alkyle, C₂₋₃-alcényle, C₂₋₃-alcynyle ou C₃₋₆-cycloalkyle,
où les substituants peuvent être identiques ou différents et
dans chaque cas l'un des groupes C₁₋₅-alkyle peut être substitué par un ou deux groupes hydroxy-C₁₋₃-alkyle, C₁₋₃-alcoxy-C₁₋₃-alkyle, benzyloxy-C₁₋₃-alkyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, C₁₋₅-alkyloxycarbonylamino-C₁₋₃-alkyle, C₃₋₆-cycloalkylamino-C₁₋₃-alkyle, aminocarbonyle, C₁₋₃-alkylaminocarbonyle, *N*-(C₃₋₇-cycloalkyl)-C₁₋₃-alkylaminocarbonyle, *N*-(phényl-C₁₋₃-alkyl)-C₁₋₅-alkylaminocarbonyle, di-(C₁₋₃-alkyl)-aminocarbonyle ou un groupe cycloalkyléniminocarbonyle à 4 à 7 chaînons,
ou un groupe de formule 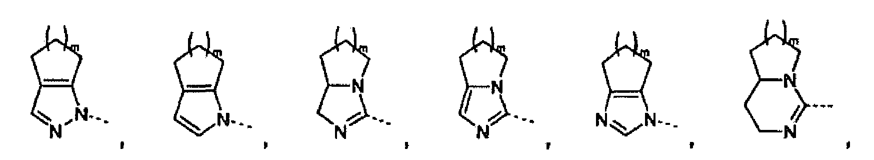 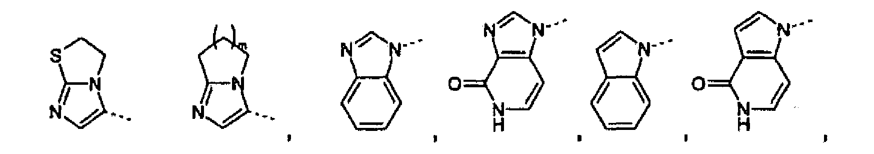 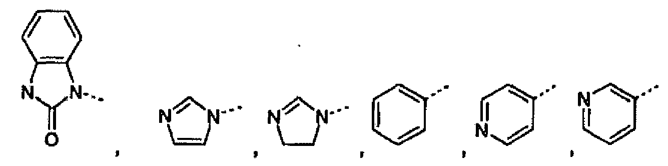 ou 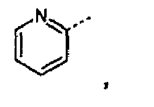 qui peut être substitué dans chaque cas sur un atome de carbone par un groupe C₁₋₃-alkyle, C₁₋₃-alkyloxy, C₁₋₃-alkyloxycarbonyle, C₁₋₅-alkyloxycarbonylamino-C₁₋₃-alkyle, C₁₋₃-alkylsulfonyle, C₁₋₃-alkylsulfonyl-C₁₋₃-alkyle, aminosulfonyle, C₁₋₃-alkylsulfonyl-C₁₋₃-alkyle, C₁₋₃-alkylsulfonylamino-C₁₋₃-alkyle, C₁₋₃-alkylsulfonylamino, C₁₋₃-alkylcarbonylamino-C₁₋₃-alkyle, C₁₋₃-alkylcarbonylamino, amino-C₁₋₃-alkyle, C₁₋₃-alkylamino-C₁₋₃-alkyle, di-(C₁₋₃-alkyl)-amino-C₁₋₃-alkyle, hydroxy-C₁₋₃-alkyle, C₁₋₅-alcoxy-C₁₋₃-alkyle, un groupe cycloalkylénimino-C₁₋₃-alkyle à 4 à 7 chaînons, morpholin-4-yl-C₁₋₃-alkyle, pipérazinyl-C₁₋₃-alkyle, *N*-(C₁₋₃-alkyl)-pipérazin-4-yl-C₁₋₃-alkyle, aminocarbonyle, C₁₋₃-alkylamino-carbonyle ou di-(C₁₋₃-alkyl)-aminocarbonyle, et où
m représente le nombre 1 ou 2,
R¹ représente un atome d'hydrogène ou d'halogène, un groupe C₁₋₃-alkyle ou C₁₋₃-alcoxy, où les atomes d'hydrogène du groupe C₁₋₃-alkyle ou C₁₋₃-alcoxy peuvent éventuellement être remplacés totalement ou en partie par des atomes de fluor, un groupe C₂₋₃-alcényle, C₂₋₃-alcynyle, nitrile, nitro ou amino,
R² représente un atome d'hydrogène ou d'halogène ou un groupe méthyle,
X représente un atome d'azote ou un groupe CH,
R³ représente un atome d'hydrogène ou un groupe C₁₋₃-alkyle,
R⁴ et R⁵ représentent dans chaque cas indépendamment l'un de l'autre
un atome d'hydrogène, un groupe hydroxy, un groupe OR⁹, un groupe C₂₋₆-alcényle ou C₂₋₆-alcynyle,
un groupe C₁₋₆-alkyle linéaire ou ramifié,
où les atomes d'hydrogène du groupe C₁₋₆-alkyle linéaire ou ramifié peuvent éventuellement être remplacés totalement ou en partie par des atomes de fluor, et qui peut éventuellement être substitué par un groupe C₃₋₅-cycloalkyle, un groupe nitrile, hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, mercapto, C₁₋₅-alkylsulfanyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₄₋₆-cycloalkyléniminocarbonyle, aminosulfonyle, C₁₋₅-alkylaminosulfonyle, di-(C ₁₋₅-alkyl)-aminosulfonyle, C₄₋₆-cycloalkyléniminosulfonyle, amino, C₁₋₅-alkylamino, di-(C₁₋₅-alkyl)-amino, C₁₋₅-alkylcarbonylamino, C₁₋₅-alkylsulfonylamino, *N*-(C₁₋₅-alkylsulfonyl)-C₁₋₅-alkylamino ou C₃₋₆-cycloalkylcarbonylamino,
où, dans les cycles à 6 à 7 chaînons du groupe C₃₋₆-cycloalkyléniminocarbonyle, dans la partie cyclique un groupe méthylène en position 4 d'un groupe cycloalkylénimino à 6 ou 7 chaînons peut être remplacé par un atome d'oxygène ou de soufre, par un groupe carbonyle, sulfinyle, sulfonyle ou -NR⁷- et en outre un groupe méthylène voisin d'un groupe -NR⁷- cité précédemment peut être remplacé par un groupe carbonyle,
un groupe phényle, hétéroaryle, phényl-C₁₋₅-alkyle ou hétéroaryl-C₁₋₅-alkyle,
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle,
un groupe cycloalkyle, cycloalkylénimino, cycloalkyl-C₁₋₅-alkyle ou cycloalkylénimino-C₁₋₃-alkyle à 3 à 7 chaînons,
dans lequel, dans le cas de cycles à 4 à 7 chaînons, dans la partie cyclique un groupe méthylène peut éventuellement être remplacé par un groupe -N(R⁷)-, un atome d'oxygène ou de soufre ou un groupe -S(O)- ou -S(O)₂-, ou
dans lequel, dans le cas de cycles à 4 à 7 chaînons, dans la partie cyclique deux groupes méthylène voisins peuvent éventuellement être remplacés ensemble par un groupe -C(O)N(R⁸)- ou -S(O)₂N(R⁸)-, ou
dans lequel, dans le cas de cycles à 6 à 7 chaînons, dans la partie cyclique trois groupes méthylène voisins peuvent éventuellement être remplacés ensemble par un groupe -OC(O)N(R⁸)- ou -N(R⁸)C(O)N(R⁸)- ou - N(R⁸)S(O)₂N(R⁸)- substitué,
avec la condition qu'un groupe cycloalkyle, cycloalkylénimino, cycloalkyl-C₁₋₅-alkyle ou cycloalkylénimino-C₁₋₃-alkyle à 3 à 7 chaînons défini comme ci-dessus, dans lequel deux hétéroatomes du groupe de l'oxygène et de l'azote sont séparés l'un de l'autre par exactement un groupe -CH₂-éventuellement substitué est exclus,
où un groupe cycloalkyle, cycloalkylénimino, cycloalkyl-C₁₋₅-alkyle ou cycloalkylénimino-C₁₋₃-alkyle à 3 à 7 chaînons défini comme ci-dessus peut être substitué sur un ou deux groupes -CH₂- par un ou deux groupes C₁₋₃-alkyle à chaque fois,
avec la condition que R⁴ et R⁵ ne puissent pas être définis en même temps comme des groupes hydroxy ou OR⁹,
R⁷ représente indépendamment des autres un atome d'hydrogène, un groupe hydroxy, un groupe formyle, un groupe C₁₋₅-alkyle, C₁₋₅-alkylcarbonyle, C₁₋₅-alkyloxycarbonyle ou C₁₋₅-alkylsulfonyle,
R⁸ représente indépendamment des autres un atome d'hydrogène ou un groupe C₁₋₅-alkyle,
R⁹ représente un groupe C₁₋₆-alkyle linéaire ou ramifié,
où les atomes d'hydrogène du groupe C₁₋₆-alkyle linéaire ou ramifié peuvent éventuellement être remplacés totalement ou en partie par des atomes de fluor, et qui peut éventuellement être substitué par un groupe C₃₋₅-cycloalkyl, un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, carboxy, C₁₋₅-alkyloxycarbonyle, aminocarbonyle, C₁₋₅-alkylaminocarbonyle, di-(C₁₋₅-alkyl)-aminocarbonyle, C₄₋₆-cycloalkyléniminocarbonyle, C₁₋₅-alkyloxycarbonylamino, amino, C₁₋₅-alkylamino, di-(C₁₋₅-alkyl)-amino, C₁₋₅-alkylcarbonylamino, C₁₋₅-alkylsulfonylarnino, *N*-(C₁₋₅-alkylsulfonyl)-C₁₋₅-alkylamino ou C₃₋₆-cycloalkylcarbonylamino,
où dans les cycles à 6 à 7 chaînons du groupe C₄₋₆-cycloalkyléniminocarbonyle, dans la partie cyclique un groupe méthylène en position 4 d'un groupe cycloalkylénimino à 6 à 7 chaînons peut être remplacé par un atome d'oxygène ou de soufre, par un groupe carbonyle, sulfinyle, sulfonyle ou -NR⁷- et en outre un groupe méthylène voisin d'un groupe -NR⁷- cité précédemment peut être remplacé par un groupe carbonyle,
avec la condition que le remplacement d'atomes d'hydrogène du premier atome de carbone du groupe C₁₋₆-alkyle linéaire ou ramifié par des substituants du groupe de l'oxygène, du soufre ou de l'azote est exclus,
un groupe phényle ou hétéroaryle,
qui peut éventuellement être substitué une à trois fois dans la partie phényle ou hétéroaryle par des substituant identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluoro-méthoxy, carboxy et C₁₋₅-alkyloxycarbonyle,
un groupe phényl-C₁₋₅-alkyle ou hétéroaryl-C₁₋₅-alkyle,
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle, et qui peut éventuellement être substitué dans la partie C₁₋₅-alkyle par un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy ou un groupe C₁₋₅-alkyloxycarbonyl-C₁₋₅-alkyloxy,
un groupe cycloalkyle, cycloalkyl-C₁₋₅-alkyle ou cycloalkylénimino-C₂₋₃-alkyle à 3 à 7 chaînons,
dans lequel, dans le cas de cycles à 4 à 7 chaînons, dans la partie cyclique un groupe méthylène peut éventuellement être remplacé par un groupe -N(R⁷)-, un atome d'oxygène ou de soufre ou un groupe -S(O)- ou -S(O)₂-, ou
dans lequel, dans le cas de cycles à 4 à 7 chaînons, dans la partie cyclique deux groupes méthylène voisins peuvent éventuellement être remplacés ensemble par un groupe -C(O)N(R⁸)- ou -S(O)₂N(R⁸)-, ou
dans lequel, dans le cas de cycles à 6 à 7 chaînons, dans la partie cyclique trois groupes méthylène voisins peuvent éventuellement être remplacés ensemble par un groupe -OC(O)N(R⁸)- ou -N(R⁸)C(O)N(R⁸)- ou -N(R⁸)S(O)₂N(R⁸)- substitué,
avec la condition qu'un groupe cycloalkyl, cycloalkyl-C₁₋₅-alkyle ou cycloalkylénimino-C₂₋₃-alkyle à 3 à 7 chaînons défini comme ci-dessus, dans lequel deux hétéroatomes du groupe de l'oxygène et de l'azote sont séparés l'un de l'autre par exactement un groupe -CH₂- éventuellement substitué, est exclus,
où un groupe cycloalkyle, cycloalkyl-C₁₋₅-alkyle ou cycloalkylénimino-C₂₋₃-alkyle à 3 à 7 chaînons défini comme ci-dessus peut être substitué sur un ou deux groupes -CH₂- dans chaque cas par un ou deux groupes C₁₋₃-alkyle,
B représente un groupement de formule générale 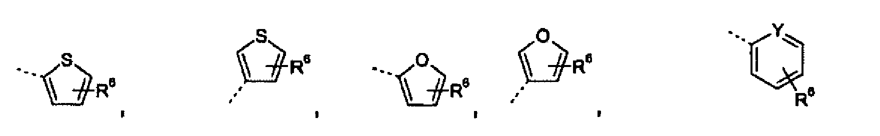 ou 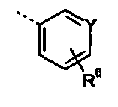 Y représente un atome d'azote ou un groupe CH,
R⁶ représente un atome d'hydrogène, un atome d'halogène, un groupe éthynyle, un groupe méthyle, un groupe méthoxy, où les atomes d'hydrogène du groupe méthoxy peuvent éventuellement être remplacés totalement ou en partie par des atomes de fluor,
où, sauf indication contraire, par l'expression « groupe hétéroaryle » citée précédemment dans les définitions, on doit entendre un groupe hétéroaryle à 5 ou 6 chaînons monocyclique, où
le groupe hétéroaryle à 6 chaînons contient un, deux ou trois atomes d'azote et
le groupe hétéroaryle à 5 chaînons contient un groupe imino éventuellement substitué par un groupe C₁₋₃-alkyle, phényle ou phényl-C₁₋₃-alkyle, un atome d'oxygène ou de soufre ou
un groupe imino éventuellement substitué par un groupe C₁₋₃-alkyle, phényle, amino-C₂₋₃-alkyle, C₁₋₃-alkylamino-C₁₋₃-alkyle, di-(C₁₋₃-alkyl)-amino-C₁₋₃-alkyle, un groupe C₃₋₆-cycloalkylénimino-C₁₋₃-alkyle ou phényl-C₁₋₃-alkyle ou un atome d'oxygène ou de soufre et en outre un atome d'azote ou
un groupe imino éventuellement substitué par un groupe C₁₋₃-alkyle ou phényl-C₁₋₃-alkyle et deux ou trois atomes d'azote,
et en outre un cycle phényle éventuellement substitué par un atome de fluor, de chlore ou de brome, un groupe C₁₋₃-alkyle, hydroxy, C₁₋₃-alkyloxy, amino, C₁₋₃-alkylamino, di-(C₁₋₃-alkyl)-amino ou C₃₋₆-cyclo-alkylénimino peut être condensé aux groupes hétéroaryle monocycliques cités précédemment par le biais de deux atomes de carbone voisins,
et la liaison se fait par le biais d'un atome d'azote ou par le biais d'un atome de carbone de la partie hétérocyclique ou d'un cycle phényle condensé,
où, sauf indication contraire, par l'expression « atome d'halogène » citée précédemment dans les définitions, on doit comprendre un atome du groupe du fluor, du chlore, du brome et de l'iode,
où les groupes alkyle, alcényle, alcynyle et alcoxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
et où les atomes d'hydrogène des groupes méthyle ou éthyle contenus dans les définitions citées précédemment, sauf indication contraire, peuvent être remplacés totalement ou en partie par des atomes de fluor,
leurs tautomères, leurs énantiomères, leurs diastéréoisomères, leurs mélanges et leurs sels.

4. Pyrrolidinones substituées de formule générale I selon la revendication 1, **caractérisées en ce que**
A représente un groupe cycloalkyléniminocarbonyle ou cycloalkyléniminosulfonyle à 4 à 7 chaînons, où
la partie cycloalkylénimino peut être substituée dans le squelette carboné par un ou deux atomes de fluor, un ou deux groupes C₁₋₃-alkyle, hydroxy-C₁₋₃-alkyle, C₁₋₃-alkyloxy-C₁₋₃-alkyle, hétéroaryl-C₁₋₃-alkyle, amino-C₁₋₃-alkyle, C₃₋₆-cycloalkylamino-C₁₋₃-alkyle, phénylamino-C₁₋₃-alkyle, C₁₋₅-alkylamino-C₁₋₃-alkyle, di-(C₁₋₅-alkyl)-amino-C₁₋₅-alkyle, *N*-(C₃₋₆-cycloalkyl)-C₁₋₃-alkylamino-C₁₋₃-alkyle, un groupe cycloalkylénimino-C₁₋₃-alkyle à 4 à 7 chaînons, *N*-(C₁₋₃-alkylcarbonyl)-C₁₋₃-alkylamino-C₁₋₃-alkyle, carboxy-C₁₋₃-alkyle, C₁₋₃-alkyloxycarbonyl-C₁₋₃-alkyle, aminocarbonyl-C₁₋₃-alkyle, C₁₋₃-alkylaminocarbonyl-C₁₋₃-alkyle, di-(C₁₋₃-alkyl)-aminocarbonyl-C₁₋₃-alkyle, un groupe cycloalkyléniminocarbonyl-C₁₋₃-alkyle à 4 à 7 chaînons, C₁₋₅-alkyloxycarbonylamino-C₁₋₃-alkyle, C₁₋₃-alkylcarbonylamino-C₁₋₃-alkyle, C₁₋₅-alkylsulfonylamino-C₁₋₃-alkyle, aminocarbonylamino-C₁₋₃-alkyle, C₁₋₅-alkylaminocarbonylamino-C₁₋₃-alkyle, di-(C₁₋₃-alkyl)-aminocarbonylamino-C₁₋₃-alkyle, C₁₋₅-alkylsulfonylamino, C₁₋₅-alkylaminocarbonylamino, carboxy, C₁₋₃-alkyloxycarbonyle, benzyloxycarbonyle, C₁₋₃-alkylcarbonyle, aminocarbonyle, C₁₋₃-alkylaminocarbonyle, di-(C₁₋₃-alkyl)-aminocarbonyle, *N*-(C₃₋₇-cycloalkyl)-C₁₋₅-alkylaminocarbonyle, *N*-(phényl-C₁₋₃-alkyl)-C₁₋₅-alkylaminocarbonyle, un groupe cycloalkyléniminocarbonyle à 4 à 7 chaînons, aminocarbonyl-C₁₋₃-alkylaminocarbonyle, hydroxy, C₁₋₃-alkyloxy, allyloxy, propargyloxy, benzyloxy, amino, C₁₋₃-alkylamino, di-(C₁₋₃-alkyl)-amino, un groupe cycloalkylénimino à 4 à 7 chaînons, *N*-(C₁₋₃-alkyl)-pipérazin-4-yl-C₁₋₃-alkyle, un groupe phényle ou un groupe hétéroaryle à 5 à 6 chaînons, avec la condition que, lors de la substitution d'un groupe méthylène voisin du groupe imino, deux hétéroatomes soient séparés l'un de l'autre par au moins deux atomes de carbone, et/ou
un groupe méthylène en position 3 d'un groupe cycloalkylénimino à 5 chaînons peut être remplacé par un atome de soufre ou
un groupe méthylène en position 4 d'un groupe cycloalkylénimino à 6 ou 7 chaînons peut être remplacé par un atome d'oxygène ou de soufre, un groupe sulfinyle ou sulfonyle ou par un groupe -NH- éventuellement substitué par un groupe C₁₋₃-alkyle, hydroxy, formyle ou C₁₋₃-alkylcarbonyle, où en outre un groupe méthylène voisin du groupe -NH- éventuellement substitué cité précédemment peut être remplacé par un groupe carbonyle, sulfonyle ou sulfonyle, avec la condition que
lors de la substitution des groupements cycloalkylénimino à 6 à 7 chaînons cités précédemment, dans lesquels un groupe méthylène est remplacé par un atome d'oxygène ou de soufre, un groupe sulfinyle ou sulfonyle, deux hétéroatomes sont séparés l'un de l'autre par au moins deux atomes de carbone,
un groupe cycloalcényléniminocarbonyle ou cycloalcényléniminosulfonyle à 5 à 7 chaînons éventuellement substitué par un ou deux groupes C₁₋₃-alkyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, C₃₋₆-cycloalkylamino-C₁₋₃-alkyle, hétéroaryle, hétéroaryl-C₁₋₃-alkyle, aminocarbonyle, C₁₋₃-alkylaminocarbonyle, di-(C₁₋₃-alkyl)-aminocarbonyle ou cycloalkyléniminocarbonyle à 4 à 7 chaînons, où la double liaison n'est pas liée à un atome d'azote et peut être condensée avec un groupe hétéroaryle à 5 ou 6 chaînons,
un groupe aminocarbonyle ou aminosulfonyle éventuellement substitué par un ou deux groupes C₁₋₅-alkyle ou C₃₋₆-cycloalkyle,
où les substituants peuvent être identiques ou différents et
dans chaque cas l'un des groupes C₁₋₅-alkyle peut être substitué par un ou deux groupes hydroxy-C₁₋₃-alkyle, C₁₋₃-almcoxy-C₁₋₃-alkyle, amino-C₁₋₃-alkyle, di-(C₁₋₃-alkyl)-amino-C₁₋₃-alkyle, un groupe cycloalkylénimino-C₁₋₃-alkyle à 4 à 7 chaînons, aminocarbonyle, C₁₋₃-alkylaminocarbonyle, di-(C₁₋₃-alkyl)-aminocarbonyle ou un groupe cycloalkyléniminocarbonyle à 4 à 7 chaînons,
ou un groupe de formule 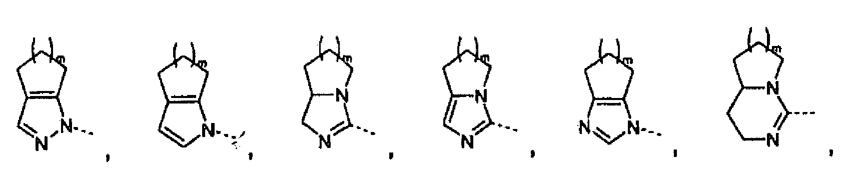 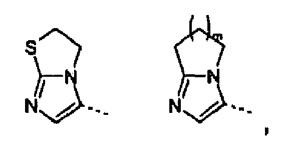 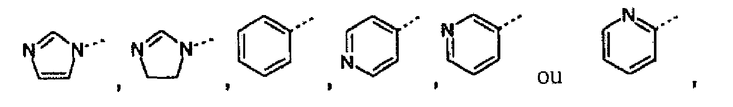 qui peut être substitué dans chaque cas sur un atome de carbone par un groupe C₁₋₃-alkyle, C₁₋₃-alkyloxy, C₁₋₃-alkyloxycarbonyle, C₁₋₅-alkyloxycarbonylamino-C₁₋₃-alkyle, C₁₋₃-alkylsulfonyle, C₁₋₃-alkylsulfonyl-C₁₋₃-alkyle, aminosulfonyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, morpholin-4-yl-C₁₋₃-alkyle, pipérazinyl-C₁₋₃-alkyle, *N*-(C₁₋₃-alkyl)-pipérazin-4-yl-C₁₋₃-alkyle, aminocarbonyle, C₁₋₃-alkylaminocarbonyle ou di-(C₁₋₃-alkyl)-aminocarbonyle, et où
m représente le nombre 1 ou 2,
R¹ représente un atome d'hydrogène ou d'halogène, un groupe C₁₋₃-alkyle ou C₁₋₃-alcoxy, où les atomes d'hydrogène du groupe C₁₋₃-alkyle ou C₁₋₃-alcoxy peuvent éventuellement être remplacés totalement ou en partie par des atomes de fluor, un groupe C₂₋₃-alcényle, C₁₋₃-alcynyle, nitrile, nitro ou amino,
R² représente un atome d'hydrogène ou d'halogène ou un groupe méthyle,
X représente un atome d'azote ou un groupe CH,
R³ représente un atome d'hydrogène ou un groupe C₁₋₃-alkyle,
R⁴ représente un atome d'hydrogène,
un groupe C₁₋₄-alkyle linéaire ou ramifié,
où les atomes d'hydrogène du groupe C₁₋₄-alkyle linéaire ou ramifié peuvent éventuellement être remplacés totalement ou en partie par des atomes de fluor, et qui peut éventuellement être substitué par un groupe C₁₋₃-alkyloxy, où les atomes d'hydrogène du groupe C₁₋₃-alkyloxy peuvent éventuellement être remplacés totalement ou en partie par des atomes de fluor,
R⁵ représente un atome d'hydrogène, un groupe hydroxy, un groupe OR⁹, un groupe C₂₋₄-alcényle ou C₂₋₄-alcynyle,
un groupe C₁₋₄-alkyle linéaire ou ramifié,
où les atomes d'hydrogène du groupe C₁₋₄-alkyle linéaire ou ramifié peuvent éventuellement être remplacés totalement ou en partie par des atomes de fluor, et qui peut éventuellement être substitué par un groupe C₃₋₅-cycloalkyle, un groupe nitrile, hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, mercapto, C₁₋₅-alkylsulfanyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₄₋₆-cycloalkyléniminocarbonyle, aminosulfonyle, C₁₋₅-alkylaminosulfonyle, di-(C₁₋₅-alkyl)-aminosulfonyle, C₄₋₆-cycloalkyléniminosulfonyle, amino, C₁₋₅-alkylamino, di-(C₁₋₅-alkyl)-amino, C₁₋₅-alkylcarbonylamino, C₁₋₅-alkylsulfonylamino, *N*-(C₁₋₅-alkylsulfonyl)-C₁₋₅-alkylamino ou C₃₋₆-cycloalkylcarbonylamino,
où, dans les cycles à 6 à 7 chaînons du groupe C₄₋₆-cycloalkyléniminocarbonyle, dans la partie cyclique un groupe méthylène en position 4 d'un groupe cycloalkylénimino à 6 ou 7 chaînons peut être remplacé par un atome d'oxygène ou de soufre, par un groupe carbonyle, sulfonyle, sulfonyle ou -NR⁷- et en outre un groupe méthylène voisin d'un groupe -NR⁷- cité précédemment peut être remplacé par un groupe carbonyle,
un groupe phényle, hétéroaryle, phényl-C₁₋₅-alkyle ou hétéroaryl-C₁₋₅-alkyle,
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle,
R⁷ représente indépendamment des autres un atome d'hydrogène, un groupe C₁₋₃-alkyle, C₁₋₃-alkylcarbonyle, C₁₋₃-alkyloxycarbonyle ou C₁₋₃-alkylsulfonyle,
R⁹ représente un groupe C₁₋₄-alkyle linéaire ou ramifié,
où les atomes d'hydrogène du groupe C₁₋₄-alkyle linéaire ou ramifié peuvent éventuellement être remplacés totalement ou en partie par des atomes de fluor, et qui peut éventuellement être substitué par un groupe C₃₋₅-cycloalkyle, un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, carboxy, C₁₋₅-alkyloxycarbonyle, aminocarbonyle, C₁₋₅-alkylaminocarbonyle, di-(C₁₋₅-alkyl)-aminocarbonyle, C₄₋₆-cycloalkyléniminocarbonyle, C₁₋₅-alkyloxycarbonylamino, amino, C₁₋₅-alkylamino, di-(C₁₋₅-alkyl)-amino, C₁₋₅-alkylcarbonylamino, C₁₋₅-alkylsulfonylamino, *N*-(C₁₋₅-alkylsulfonyl)-C₁₋₅-alkylamino ou C₃₋₆-cycloalkylcarbonylamino,
où dans les cycles à 6 à 7 chaînons du groupe C₄₋₆-cycloalkyléniminocarbonyle, dans la partie cyclique un groupe méthylène en position 4 d'un groupe cycloalkylénimino à 6 à 7 chaînons peut être remplacé par un atome d'oxygène ou de soufre, par un groupe carbonyle, sulfinyle, sulfonyle ou -NR⁷- et en outre un goupe méthylène voisin d'un groupe -NR⁷- cité précédemment peut être remplacé par un groupe carbonyle,
avec la condition que le remplacement d'atomes d'hydrogène du premier atome de carbone du groupe C₁₋₆-alkyle linéaire ou ramifié par des substituants du groupe de l'oxygène, du soufre ou de l'azote est exclus, un groupe phényle ou hétéroaryle,
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluoro-méthoxy, carboxy et C₁₋₅-alkyloxycarbonyle,
un groupe phényl-C₁₋₅-alkyle ou hétéroaryl-C₁₋₅-alkyle,
qui peut éventuellement être substitué une à trois fois dans la partie phényle ou hétéroaryle par des substituants identiques ou différents choisis dans le groupe consistant en les atomes d'halogène, les groupes C₁₋₅-alkyle, di-(C₁₋₅-alkyl)-amino, hydroxy, C₁₋₅-alkyloxy, mono-, di- ou trifluorométhoxy, carboxy et C₁₋₅-alkyloxycarbonyle, et qui peut éventuellement être substitué dans la partie C₁₋₅-alkyle par un groupe hydroxy, un groupe C₁₋₅-alkyloxy, où les atomes d'hydrogène du groupe C₁₋₅-alkyloxy peuvent éventuellement être remplacés totalement ou en partie par des atomes de fluor, un groupe allyloxy, propargyloxy, benzyloxy, C₁₋₅-alkylcarbonyloxy, C₁₋₅-alkyloxycarbonyloxy, carboxy-C₁₋₅-alkyloxy ou un groupe C₁₋₅-alkyloxycarbonyl-C₁₋₅-alkyloxy,
B représente un groupement de formule générale 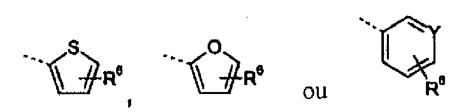 Y représente un atome d'azote ou un groupe CH,
R⁶ représente un atome d'hydrogène, un atome d'halogène, un groupe éthynyle, un groupe méthyle, un groupe méthoxy, où les atomes d'hydrogène du groupe méthoxy peuvent éventuellement être remplacés totalement ou en partie par des atomes de fluor,
où, sauf indication contraire, par l'expression « groupe hétéroaryle » citée précédemment dans les définitions, on doit entendre un groupe hétéroaryle à 5 ou 6 chaînons monocyclique, où
le groupe hétéroaryle à 6 chaînons contient un, deux ou trois atomes d'azote et
le groupe hétéroaryle à 5 chaînons contient un groupe imino éventuellement substitué par un groupe C₁₋₅-alkyle, phényle ou phényl-C₁₋₃-alkyle, un atome d'oxygène ou de soufre ou
un groupe imino éventuellement substitué par un groupe C₁₋₃-alkyle, phényle, amino-C₂₋₃-alkyle, C₁₋₃-alkylamino-C₂₋₃-alkyle, di-(C₁₋₃-alkyl)-amino-C₂₋₃-alkyle, un groupe C₃₋₆-cycloalkylénimino-C₁₋₃-alkyle ou phényl-C₁₋₃-alkyle ou un atome d'oxygène ou de soufre et en outre un atome d'azote ou
un groupe imino éventuellement substitué par un groupe C₁₋₃-alkyle ou phényl-C₁₋₃-alkyle et deux ou trois atomes d'azote,
et en outre un cycle phényle éventuellement substitué par un atome de fluor, de chlore ou de brome, un groupe C₁₋₃-alkyle, hydroxy, C₁₋₃-alkyloxy, amino, C₁₋₃-alkylamino, di-(C₁₋₃-alkyl)-amino ou C₃₋₆-cyclo-alkylénimino peut être condensé aux groupes hétéroaryle monocycliques cités précédemment par le biais de deux atomes de carbone voisins,
et la liaison se fait par le biais d'un atome d'azote ou par le biais d'un atome de carbone de la partie hétérocyclique ou d'un cycle phényle condensé,
où, sauf indication contraire, par l'expression « atome d'halogène » citée précédemment dans les définitions, on doit comprendre un atome du groupe du fluor, du chlore, du brome et de l'iode,
où les groupes alkyle, alcényle, alcynyle et alcoxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
et où les atomes d'hydrogène des groupes méthyle ou éthyle contenus dans les définitions citées précédemment, sauf indication contraire, peuvent être remplacés totalement ou en partie par des atomes de fluor,
leurs tautomères, leurs énantiomères, leurs diastéréoisomères, leurs mélanges et leurs sels.

5. Pyrrolidinones substituées de formule générale I selon la revendication 1, **caractérisées en ce que**
A représente un groupe cycloalkyléniminocarbonyle ou cycloalkyléniminosulfonyle à 5 à 6 chaînons, où
la partie cycloalkylénimino peut être substituée dans le squelette carboné par un ou deux atomes de fluor, un ou deux groupes C₁₋₃-alkyle, hydroxy-C₁₋₃-alkyle, C₁₋₃-alkyloxy-C₁₋₃-alkyle, hétéroaryl-C₁₋₃-alkyle, amino-C₁₋₃-alkyle, C₃₋₆-cycloalkylarnino-C₁₋₃-alkyle, phénylamino-C₁₋₃-alkyle, C₁₋₅-alkylamino-C₁₋₃-alkyle, di-(C₁₋₅-alkyl)-amino-C₁₋₅-alkyle, *N*-(C₃₋₆-cycloalkyl)-C₁₋₃-alkylamino-C₁₋₃-alkyle, un groupe cycloalkylénimino-C₁₋₃-alkyle à 4 à 7 chaînons, *N*-(C₁₋₃-alkylcarbonyl)-C₁₋₃-alkylamino-C₁₋₃-alkyle, carboxy-C₁₋₃-alkyle, C₁₋₃-alkyloxycarbonyl-C₁₋₃-alkyle, aminocarbonyl-C₁₋₃-alkyle, C₁₋₃-alkylaminacarbonyl-C₁₋₃-alkyle, di-(C₁₋₃-alkyl)-aminacarbonyl-C₁₋₃-alkyle, C₁₋₃-alkylcarbonylamino-C₁₋₃-alkyle, C₁₋₅-alkylaminocarbanylamino-C₁₋₃-alkyle, di-(C₁₋₃-alkyl)-aminocarbonylamino-C₁₋₃-alkyle, C₁₋₅-alkylsulfonylamino, C₁₋₅-alkylaminocarbonylamino, carboxy, C₁₋₃-alkyloxycarbonyle, aminocarbonyle, C₁₋₃-alkylaminocarbonyle, di-(C₁₋₃-alkyl)-aminocarbonyle, *N*-(C₃₋₇-cycloalkyl)-C₁₋₅-alkylaminocarbonyle, *N-*(phényl-C₁₋₃-alkyl)-C₁₋₅-alkylaminocarbonyle, hydroxy, C₁₋₃-alkyloxy, amino, C₁₋₃-alkylamino, di-(C₁₋₃-alkyl)-amino, *N*-(C₁₋₃-alkyl)-pipérazin-4-yl-C₁₋₃-alkyle, un groupe phényle ou un groupe hétéroaryle à 6 chaînons, avec
la condition que, lors de la substitution d'un groupe méthylène voisin du groupe imino, deux hétéroatomes soient séparés l'un de l'autre par au moins deux atomes de carbone, et/ou
un groupe méthylène en position 3 d'un groupe cycloalkylénimino à 5 chaînons peut être remplacé par un atome de soufre ou
un groupe méthylène en position 4 d'un groupe cycloalkylénimino à 6 à 7 chaînons peut être remplacé par un atome d'oxygène ou de soufre ou par un groupe -NH- éventuellement substitué par un groupe C₁₋₃-alkyle, hydroxy, formyle ou C₁₋₃-alkylcarbonyle, où en outre un groupe méthylène voisin du groupe -NH- éventuellement substitué cité précédemment peut être remplacé par un groupe carbonyle, avec la condition que
lors de la substitution des groupements cycloalkylénimino à 6 à 7 chaînons cités précédemment, dans lesquels un groupe méthylène est remplacé par un atome d'oxygène ou de soufre, deux hétéroatomes sont séparés l'un de l'autre par au moins deux atomes de carbone,
ou A représente un groupe de formule 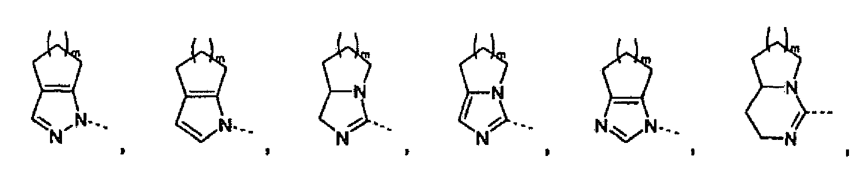 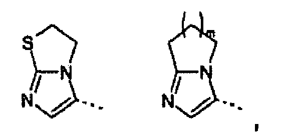 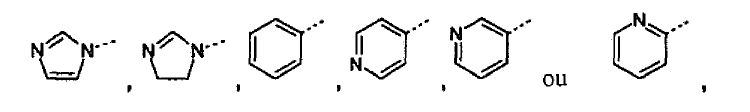 qui peut être substitué dans chaque cas sur un atome de carbone par un groupe C₁₋₃-alkyle, C₁₋₃-alkyloxy, C₁₋₃-alkyloxycarbonyle, C₁₋₅-alkyloxy-carbonylamino-C₁₋₃-alkyle, C₁₋₃-alkylsulfonyle, C₁₋₃-alkylsulfonyl-C₁₋₃-alkyle, aminosulfonyle, amino-C₁₋₃-alkyle, C₁₋₃-alkylamino-C₁₋₃-alkyle, di-(C₁₋₃-alkyl)-amino-C₁₋₃-alkyle, un groupe cycloalkylénimino-C₁₋₃-alkyle à 4 à 7 chaînons, morpholin-4-yl-C₁₋₃-alkyle, pipérazinyl-C₁₋₃-alkyle, *N*-(C₁₋₃-alkyl)-pipérazin-4-yl-C₁₋₃-alkyle, aminocarbonyle, C₁₋₃-alkylaminocarbonyle ou di-(C₁₋₃-alkyl)-aminocarbonyle, et où
m représente le nombre 1 ou 2,
R¹ représente un atome d'hydrogène, de fluor, de chlore ou de brome, un groupe méthyle ou méthoxy, où les atomes d'hydrogène du groupe méthyle ou méthoxy peuvent éventuellement être remplacés totalement ou en partie par des atomes de fluor,
R² représente un atome d'hydrogène ou de fluor,
X représente un atome d'azote ou un groupe CH,
R³ représente un atome d'hydrogène ou un groupe C₁₋₃-alkyle,
R⁴ représente un atome d'hydrogène,
R⁵ représente un atome d'hydrogène, un groupe hydroxy, un groupe OR⁹, un groupe C₂₋₄-alcényle ou C₂₋₄-alcynyle,
un groupe C₁₋₄-alkyle linéaire ou ramifié,
où les atomes d'hydrogène peuvent éventuellement être remplacés totalement ou en partie par des atomes de fluor, et qui peut éventuellement être substitué par un groupe hydroxy, un groupe C₁₋₃-alkyloxy, où les atomes d'hydrogène du groupe C₁₋₃-alkyloxy peuvent éventuellement être remplacés totalement ou en partie par des atomes de fluor, un groupe benzyloxy, C₁₋₃-alkylcarbonyloxy, C₁₋₃-alkyloxycarbonyle, aminocarbonyle, C₁₋₃-alkylaminocarbonyle, di-(C₁₋₃-alkyl)-aminocarbonyle, C₄₋₆-cycloalkyléniminocarbonyle, amino, C₁₋₃-alkylamino, di-(C₁₋₃-alkyl)-amino, C₁₋₃-alkylcarbonylamino ou C₁₋₃-alkylsulfonylamino,
un groupe phényle ou hétéroaryle lié par C
où le groupe hétéroaryle est choisi dans le groupe consistant en pyrrolyle, oxazolyle, imidazolyle, furanyle, thiophényle, thiazolyle, 1,2,3-triazolyle, 1,2,4-triazolyle, tétrazolyle, pyridinyle, pyrimidinyle et pyrazinyle, et qui peut éventuellement être substitué une à deux fois dans la partie hétéroaryle par des substituants identiques ou différents choisis dans le groupe consistant en les atomes d'halogène, les groupes C₁₋₃-alkyle, hydroxy, C₁₋₃-alkyloxy, mono-, di- ou trifluorométhoxy,
un groupe phényl-C₁₋₃-alkyle, hétéroaryl-C₁₋₃-alkyle,
où le groupe hétéroaryle est choisi dans le groupe consistant en pyrrolyle, oxazolyle, imidazolyle, furanyle, thiophényle, thiazolyle, 1,2,3-triazolyle, 1,2,4-triazolyle, tétrazolyle, pyridinyle, pyrimidinyle et pyrazinyle, et qui peut éventuellement être substitué une à deux fois dans la partie hétéroaryle par des substituants identiques ou différents choisis dans le groupe consistant en les atomes d'halogène, les groupes C₁₋₃-alkyle, hydroxy, C₁₋₃-alkyloxy, mono-, di- ou trifluorométhoxy, et qui peut éventuellement être substitué dans la partie C₁₋₃-alkyle par un groupe hydroxy, C₁₋₃-alkyloxy, où les atomes d'hydrogène peuvent éventuellement être remplacés totalement ou en partie par des atomes de fluor, un groupe C₁₋₅-alkylcarbonyloxy ou un groupe C₁₋₃-alkyloxycarbonyle ;
R⁹ représente un groupe C₁₋₄-alkyle linéaire ou ramifié,
où les atomes d'hydrogène du groupe C₁₋₄-alkyle linéaire ou ramifié peuvent éventuellement être remplacés totalement ou en partie par des atomes de fluor, et qui peut éventuellement être substitué par un groupe hydroxy, un groupe C₁₋₃-alkyloxy, où les atomes d'hydrogène du groupe C₁₋₃-alkyloxy peuvent éventuellement être remplacés totalement ou en partie par des atomes de fluor, un groupe benzyloxy, C₁₋₃-alkyloxycarbonyle, aminocarbonyle, C₁₋₃-alkylaminocarbonyle, di-(C₁₋₃-alkyl)-aminocarbonyle, C₄₋₆-cycloalkyléniminocarbonyle, amino, C₁₋₃-alkylamino, di-(C₁₋₃-alkyl)-amino, C₁₋₃-alkylcarbonylamino, C₁₋₃-alkylsulfonylamino,
avec la condition que le remplacement d'atomes d'hydrogène du premier atome de carbone du groupe C₁₋₆-alkyle linéaire ou ramifié par des substituants du groupe de l'oxygène, du soufre ou de l'azote est exclus,
un groupe phényle, phényl-C₁₋₂-alkyle, hétéroaryl-C₁₋₂-alkyle ou hétéroaryle lié par C
où le groupe hétéroaryle est choisi dans le groupe consistant en pyrrolyle, oxazolyle, imidazolyle, furanyle, thiophényle, thiazolyle, 1,2,3-triazolyle, 1,2,4-triazolyle, tétrazolyle, pyridinyle, pyrimidinyle et pyrazinyle, et qui peut éventuellement être substitué une à deux fois dans la partie hétéroaryle par des substituants identiques ou différents choisis dans le groupe consistant en les atomes d'halogène, les groupes C₁₋₃-alkyle, hydroxy, C₁₋₃-alkyloxy, mono-, di- ou trifluorométhoxy,
B représente un groupement de formule générale 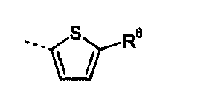 R⁶ représente un atome d'hydrogène, de chlore ou de brome, un groupe éthynyle, un groupe méthyle ou un groupe méthoxy,
où, sauf indication contraire, par l'expression « atome d'halogène » citée précédemment dans les définitions, on doit comprendre un atome du groupe du fluor, du chlore, du brome et de l'iode,
où les groupes alkyle, alcényle, alcynyle et alcoxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
et où les atomes d'hydrogène des groupes méthyle ou éthyle contenus dans les définitions citées précédemment, sauf indication contraire, peuvent être remplacés totalement ou en partie par des atomes de fluor,
leurs tautomères, leurs énantiomères, leurs diastéréoisomères, leurs mélanges et leurs sels.

6. Pyrrolidinones substituées de formule générale I selon la revendication 1, **caractérisées en ce que**
A représente un groupe cycloalkyléniminocarbonyle ou cycloalkyléniminosulfonyle à 5 à 6 chaînons, où
la partie cycloalkylénimino peut être substituée dans le squelette carboné par un ou deux groupes C₁₋₃-alkyle, hydroxy-C₁₋₃-alkyle, C₁₋₃-alkyloxy-C₁₋₃-alkyle, pyridinyl-C₁₋₃-alkyle, amino-C₁₋₃-alkyle, C₃₋₆-cycloalkylamino-C₁₋₃-alkyle, phénylamino-C₁₋₃-alkyle, C₁₋₅-alkylamino-C₁₋₃-alkyle, di-(C₁₋₅-alkyl)-amino-C₁₋₅-alkyle, N-pyrrolidinyl-C₁₋₃-alkyle, C₁₋₃-alkyloxycarbonyl-C₁₋₃-alkyle, C₁₋₃-alkyloxycarbonyle, aminocarbonyle, C₁₋₃-alkylaminocarbonyle, di-(C₁₋₃-alkyl)-aminocarbonyle, hydroxy, C₁₋₃-alkyloxy, C₁₋₃-alkylamino, di-(C₁₋₃-alkyl)-amino, *N*-(C₁₋₃-alkyl)-pipérazin-4-yl-C₁₋₃-alkyle, un groupe phényle ou un groupe pyridinyle avec la condition que, lors de la substitution d'un groupe méthylène voisin du groupe imino, deux hétéroatomes soient séparés l'un de l'autre par au moins deux atomes de carbone, et/ou
un groupe méthylène en position 3 d'un groupe cycloalkylénimino à 5 chaînons peut être remplacé par un atome de soufre ou
un groupe méthylène en position 4 d'un groupe cycloalkylénimino à 6 à 7 chaînons peut être remplacé par un atome d'oxygène ou de soufre ou par un groupe -NH- éventuellement substitué par un groupe C₁₋₃-alkyle, hydroxy, formyle ou C₁₋₃-alkylcarbonyle, où en outre un groupe méthylène voisin du groupe -NH- éventuellement substitué cité précédemment peut être remplacé par un groupe carbonyle, avec la condition que
lors de la substitution des groupements cycloalkylénimino à 6 à 7 chaînons cités précédemment, dans lesquels un groupe méthylène est remplacé par un atome d'oxygène ou de soufre, deux hétéroatomes sont séparés l'un de l'autre par au moins deux atomes de carbone,
ou A représente un groupe de formule 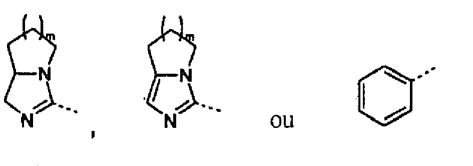 qui peut être substitué dans chaque cas sur un atome de carbone par un groupe C₁₋₃-alkyle, méthylsulfonylméthyle, aminosulfonyle, amino-C₁₋₃-alkyle, C₁₋₃-alkylamino-C₁₋₃-alkyle ou un groupe di-(C₁₋₃-alkyl)-amino-C₁₋₃-alkyle, et où
m représente le nombre 1 ou 2,
R¹ représente un atome d'hydrogène, de fluor, de chlore ou de brome, un groupe méthyle, trifluorométhyle ou méthoxy,
R² représente un atome d'hydrogène,
X représente un atome d'azote ou un groupe CH,
R³ représente un atome d'hydrogène ou un groupe méthyle,
R⁴ représente un atome d'hydrogène,
R⁵ représente un atome d'hydrogène, un groupe hydroxy, un groupe OR⁹, un groupe allyle ou méthallyle,
un groupe méthyle qui peut être substitué par un groupe C₁₋₃-alkyle, hydroxy, OR⁹, aminocarbonyle, pyridin-4-yle, pyridin-3-yle, pyridin-2-yle, pyrazin-2-yle, pyrazin-3-yle ou phényle,
un groupe phényle,
R⁹ représente un groupe C₁₋₄-alkyle linéaire ou ramifié,
qui peut éventuellement être substitué par un groupe hydroxy, un groupe C₁₋₃-alcoxy, un groupe benzyloxy ou un groupe di-(C₁₋₃-alkyl)-amino, avec la condition que le remplacement d'atomes d'hydrogène du premier atome de carbone du groupe C₁₋₄-alkyle linéaire ou ramifié par des substituants du groupe de l'oxygène ou de l'azote est exclus,
B représente un groupement de formule générale 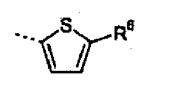 R⁶ représente un atome de chlore ou de brome, ou un groupe éthynyle, où, sauf indication contraire, par l'expression « atome d'halogène » citée précédemment dans les définitions, on doit comprendre un atome du groupe du fluor, du chlore, du brome et de l'iode,
où les groupes alkyle, alcényle, alcynyle et alcoxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
et où les atomes d'hydrogène des groupes méthyle ou éthyle contenus dans les définitions citées précédemment, sauf indication contraire, peuvent être remplacés totalement ou en partie par des atomes de fluor,
leurs tautomères, leurs énantiomères, leurs diastéréoisomères, leurs mélanges et leurs sels.

7. Pyrrolidinones substituées de formule générale I selon l'une des revendications 1 à 6 **caractérisées en ce que** le groupement B représente le groupe 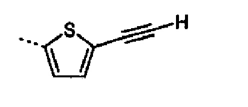 leurs tautomères, leurs énantiomères, leurs diastéréoisomères, leurs mélanges et leurs sels.

8. Pyrrolidinones substituées de formule générale I selon l'une des revendications 1 à 6 **caractérisées en ce que** le groupement B représente le groupe 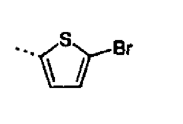 leurs tautomères, leurs énantiomères, leurs diastéréoisomères, leurs mélanges et leurs sels.

9. Pyrrolidinones substituées de formule générale 1 selon l'une des revendications 1 à 8 **caractérisées en ce que** le groupement A représente le groupe 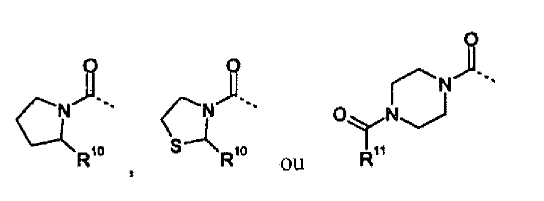 où
R¹⁰ représente l'atome d'hydrogène, un groupe méthyle, aminométhyle, C₁₋₃-alkylamino-C₁₋₂-alkyle, di-(C₁₋₃-alkyl)-amino-C₁₋₂-alkyle, pyrrolidin-1-yl-méthyle ou 2-(pyrrolidin-1-yl)-éthyle,
R¹¹ représente l'atome d'hydrogène ou un groupe méthyle,
leurs tautomères, leurs énantiomères, leurs diastéréoisomères, leurs mélanges et leurs sels.

10. Sels physiologiquement acceptables des composés selon l'une des revendications 1 à 9.

11. Médicament contenant un composé selon l'une des revendications 1 à 9 ou un sel physiologiquement acceptable selon la revendication 10 outre éventuellement un ou plusieurs vecteurs et/ou diluants inertes.

12. Utilisation d'un composé selon l'une des revendications 1 à 9 ou d'un sel physiologiquement acceptable selon la revendication 10 pour la production d'un médicament pour prévenir et traiter les maladies thrombotiques veineuse et artérielles, ainsi que pour prévenir et traiter l'embolie pulmonaire, la coagulation intravasculaire disséminée et la septicémie grave, la prévention et la prophylaxie de la DVT chez les patients ayant une exacerbation de la COPD, le traitement de la colite ulcéreuse, la prophylaxie et le traitement de la thrombose coronarienne, la prophylaxie de l'apoplexie cérébrale et la prévention de l'occlusion des pontages, pour le soutien antithrombotique lors d'un traitement thrombolytique, pour la prévention de la resténose à long terme après une PT(C)A, pour la prophylaxie et le traitement des accidents ischémiques chez les patients ayant toutes les formes de coronaropathie, pour la prévention de la formation de métastases et de la croissance des tumeurs et des processus inflammatoires, pour la prophylaxie et le traitement de la polyarthrite rhumatoïde, pour prévenir ou éviter les adhésions tissulaires dépendantes de la fibrine et/ou la formation de tissu cicatriciel ainsi que pour favoriser les processus de cicatrisation des plaies.

13. Procédé de production d'un médicament selon la revendication 11 **caractérisé en ce que**, par voie non chimique, un composé selon l'une des revendications 1 à 9 ou un sel physiologiquement acceptable selon la revendication 10 est incorporé dans un ou plusieurs vecteurs et/ou diluants inertes.
